(19) Europäisches Patentamt / European Patent Office / Office européen des brevets

(11) **EP 2 391 647 B1**

(12) **EUROPEAN PATENT SPECIFICATION**

(45) Date of publication and mention of the grant of the patent:
**26.07.2017 Bulletin 2017/30**

(21) Application number: **09789449.7**

(22) Date of filing: **02.02.2009**

(51) Int Cl.:
***C07K 14/705*** *(2006.01)* ***G01N 33/68*** *(2006.01)*

(86) International application number:
**PCT/US2009/032902**

(87) International publication number:
**WO 2010/087864 (05.08.2010 Gazette 2010/31)**

# (54) CELL LINES EXPRESSING NAV AND METHODS OF USING THEM

NAV EXPRIMIERENDE ZELLLINIEN UND VERFAHREN ZU DEREN ANWENDUNG

LIGNÉES CELLULAIRES EXPRIMANT LES NAV ET LEURS MÉTHODES D'UTILISATION

(84) Designated Contracting States:
**AT BE BG CH CY CZ DE DK EE ES FI FR GB GR HR HU IE IS IT LI LT LU LV MC MK MT NL NO PL PT RO SE SI SK TR**

(43) Date of publication of application:
**07.12.2011 Bulletin 2011/49**

(73) Proprietor: **Chromocell Corporation**
**North Brunswick, NJ 08902 (US)**

(72) Inventors:
- **SHEKDAR, Kambiz**
**New York**
**New York 10010 (US)**
- **DEDOVA, Olga**
**East Brunswick**
**New Jersey 08816 (US)**

(74) Representative: **Hally, Anna-Louise et al**
**FRKelly**
**27 Clyde Road**
**Dublin D04 F838 (IE)**

(56) References cited:
**WO-A-2009/094218** **WO-A2-2007/109324**
**US-A1- 2007 048 753**

- **MATHES CHRIS ET AL: "QPatch: The Missing Link Between HTS and Ion Channel Drug Discovery" COMBINATORIAL CHEMISTRY & HIGH THROUGHPUT SCREENING, vol. 12, no. 1, January 2009 (2009-01), pages 78-95, XP002544656 ISSN: 1386-2073**
- **ISOM LORI L ET AL: "Functional Co-expression of the beta-1 and Type IIA alpha Subunits of Sodium Channels in a Mammalian Cell Line" JOURNAL OF BIOLOGICAL CHEMISTRY, AMERICAN SOCIETY OF BIOLOCHEMICAL BIOLOGISTS, BIRMINGHAM, US, vol. 270, no. 7, 1 January 1995 (1995-01-01), pages 3306-3312, XP009119599 ISSN: 0021-9258**
- **COX JAMES J ET AL: "An SCN9A channelopathy causes congenital inability to experience pain" NATURE (LONDON),, vol. 444, no. 7121, 1 December 2006 (2006-12-01), pages 894-898, XP009119589**
- **ZHANG J-H ET AL: "A SIMPLE STATISTICAL PARAMETER FOR USE IN EVALUATION AND VALIDATION OF HIGH THROUGHPUT SCREENING ASSAYS" JOURNAL OF BIOMOLECULAR SCREENING, LARCHMONT, NY, US, vol. 4, no. 2, 1 April 1999 (1999-04-01), pages 67-73, XP001145867 ISSN: 1087-0571**
- **"Guidance for Assay Development & HTS", 1 January 2007 (2007-01-01), National Institutes of Health**
- **TRIVEDI SHEPHALI ET AL: "Cellular HTS assays for pharmacological characterization of Na(V)1.7 modulators", ASSAY AND DRUG DEVELOPMENT TECHNOLOGIES, vol. 6, no. 2, April 2008 (2008-04), pages 167-179, ISSN: 1540-658X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> CHROMOCELL CORPORATION

<120> CELL LINES EXPRESSING NAV AND METHODS OF USING THEM

<130> CHROMO/6 PCT 2 (002298-0013-WO2)

<140> PCT/US2009/032902
<141> 2009-02-02

<160> 35

<170> PatentIn version 3.5

<210> 1
<211> 25
<212> DNA
<213> Artificial Sequence

<220>
<221> source
<223> /note="Description of Artificial Sequence: Synthetic oligonucleotide"

<400> 1
gttcttaagg cacaggaact gggac 25

<210> 2
<211> 25
<212> DNA
<213> Artificial Sequence

<220>
<221> source
<223> /note="Description of Artificial Sequence: Synthetic oligonucleotide"

<400> 2
gaagttaacc ctgtcgttct gcgac 25

<210> 3
<211> 25
<212> DNA
<213> Artificial Sequence

<220>
<221> source
<223> /note="Description of Artificial Sequence: Synthetic oligonucleotide"

<400> 3
gttctatagg gtctgcttgt cgctc 25

<210> 4
<211> 34
<212> DNA
<213> Artificial Sequence

<220>
<221> source
<223> /note="Description of Artificial Sequence: Synthetic probe"

<400> 4
gccagtccca gttcctgtgc cttaagaacc tcgc 34

<210> 5
<211> 34
<212> DNA
<213> Artificial Sequence

<220>
<221> source
<223> /note="Description of Artificial Sequence: Synthetic probe"

<400> 5
gcgagtcgca gaacgacagg gttaacttcc tcgc 34

<210> 6
<211> 5997
<212> DNA
<213> Homo sapiens

<400> 6

```
atggagcaaa cagtgcttgt accaccagga cctgacagct tcaacttctt caccagagaa      60
tctcttgcgg ctattgaaag acgcattgca gaagaaaagg caaagaatcc caaaccagac     120
aaaaaagatg acgacgaaaa tggcccaaag ccaaatagtg acttggaagc tggaaagaac     180
cttccattta tttatggaga cattcctcca gagatggtgt cagagcccct ggaggacctg     240
gacccctact atatcaataa gaaaactttt atagtattga ataaagggaa ggccatcttc     300
cggttcagtg ccacctctgc cctgtacatt ttaactccct tcaatcctct taggaaaata     360
gctattaaga ttttggtaca ttcattattc agcatgctaa ttatgtgcac tattttgaca     420
aactgtgtgt ttatgacaat gagtaaccct cctgattgga caaagaatgt agaatacacc     480
ttcacaggaa tatatacttt tgaatcactt ataaaaatta ttgcaagggg attctgttta     540
gaagatttta ctttccttcg ggatccatgg aactggctcg atttcactgt cattacattt     600
gcgtacgtca cagagtttgt ggacctgggc aatgtctcgg cattgagaac attcagagtt     660
ctccgagcat tgaagacgat ttcagtcatt ccaggcctga aaaccattgt gggagccctg     720
atccagtctg tgaagaagct ctcagatgta atgatcctga ctgtgttctg tctgagcgta     780
tttgctctaa ttgggctgca gctgttcatg ggcaacctga ggaataaatg tatacaatgg     840
cctcccacca atgcttcctt ggaggaacat agtatagaaa agaatataac tgtgaattat     900
aatggtacac ttataaatga aactgtcttt gagtttgact ggaagtcata tattcaagat     960
tcaagatatc attatttcct ggagggtttt ttagatgcac tactatgtgg aaatagctct    1020
gatgcaggcc aatgtccaga gggatatatg tgtgtgaaag ctggtagaaa tcccaattat    1080
ggctacacaa gctttgatac cttcagttgg gctttttgt ccttgtttcg actaatgact    1140
caggacttct gggaaaatct ttatcaactg acattacgtg ctgctgggaa aacgtacatg    1200
```

atattttttg tattggtcat tttcttgggc tcattctacc taataaattt gatcctggct 1260
gtggtggcca tggcctacga ggaacagaat caggccacct tggaagaagc agaacagaaa 1320
gaggccgaat ttcagcagat gattgaacag cttaaaaagc aacaggaggc agctcagcag 1380
gcagcaacgg caactgcctc agaacattcc agagagccca gtgcagcagg caggctctca 1440
gacagctcat ctgaagcctc taagttgagt tccaagagtg ctaaggaaag aagaaatcgg 1500
aggaagaaaa gaaaacagaa agagcagtct ggtggggaag agaaagatga ggatgaattc 1560
caaaaatctg aatctgagga cagcatcagg aggaaaggtt ttcgcttctc cattgaaggg 1620
aaccgattga catatgaaaa gaggtactcc tccccacacc agtctttgtt gagcatccgt 1680
ggctccctat tttcaccaag gcgaaatagc agaacaagcc ttttcagctt tagagggcga 1740
gcaaaggatg tgggatctga gaacgacttc gcagatgatg agcacagcac ctttgaggat 1800
aacgagagcc gtagagattc cttgtttgtg ccccgacgac acggagagag acgcaacagc 1860
aacctgagtc agaccagtag gtcatcccgg atgctggcag tgtttccagc gaatgggaag 1920
atgcacagca ctgtggattg caatggtgtg gtttccttgg ttggtggacc ttcagttcct 1980
acatcgcctg ttggacagct tctgccagag ggaacaacca ctgaaactga aatgagaaag 2040
agaaggtcaa gttctttcca cgtttccatg gactttctag aagatccttc ccaaaggcaa 2100
cgagcaatga gtatagccag cattctaaca aatacagtag aagaacttga agaatccagg 2160
cagaaatgcc caccctgttg gtataaattt tccaacatat tcttaatctg ggactgttct 2220
ccatattggt taaaagtgaa acatgttgtc aacctggttg tgatggaccc atttgttgac 2280
ctggccatca ccatctgtat tgtcttaaat actcttttca tggccatgga gcactatcca 2340
atgacggacc atttcaataa tgtgcttaca gtaggaaact tggttttcac tgggatcttt 2400
acagcagaaa tgtttctgaa aattattgcc atggatcctt actattattt ccaagaaggc 2460
tggaatatct ttgacggttt tattgtgacg cttagcctgg tagaacttgg actcgccaat 2520
gtggaaggat tatctgttct ccgttcattt cgattgctgc gagttttcaa gttggcaaaa 2580
tcttggccaa cgttaaatat gctaataaag atcatcggca attccgtggg ggctctggga 2640
aatttaaccc tcgtcttggc catcatcgtc ttcatttttg ccgtggtcgg catgcagctc 2700
tttggtaaaa gctacaaaga ttgtgtctgc aagatcgcca gtgattgtca actcccacgc 2760
tggcacatga atgacttctt ccactccttc ctgattgtgt tccgcgtgct gtgtggggag 2820
tggatagaga ccatgtggga ctgtatggag gttgctggtc aagccatgtg ccttactgtc 2880
ttcatgatgg tcatggtgat tggaaaccta gtggtcctga atctctttct ggccttgctt 2940
ctgagctcat ttagtgcaga caaccttgca gccactgatg atgataatga aatgaataat 3000
ctccaaattg ctgtggatag gatgcacaaa ggagtagctt atgtgaaaag aaaaatatat 3060
gaatttattc aacagtcctt cattaggaaa caaaagattt tagatgaaat taaaccactt 3120

gatgatctaa acaacaagaa agacagttgt atgtccaatc atacagcaga aattgggaaa 3180
gatcttgact atcttaaaga tgtaaatgga actacaagtg gtataggaac tggcagcagt 3240
gttgaaaaat acattattga tgaaagtgat tacatgtcat tcataaacaa ccccagtctt 3300
actgtgactg taccaattgc tgtaggagaa tctgactttg aaaatttaaa cacggaagac 3360
tttagtagtg aatcggatct ggaagaaagc aaagagaaac tgaatgaaag cagtagctca 3420
tcagaaggta gcactgtgga catcggcgca cctgtagaag aacagcccgt agtggaacct 3480
gaagaaactc ttgaaccaga agcttgtttc actgaaggct gtgtacaaag attcaagtgt 3540
tgtcaaatca atgtggaaga aggcagagga aaacaatggt ggaacctgag aaggacgtgt 3600
ttccgaatag ttgaacataa ctggtttgag accttcattg ttttcatgat tctccttagt 3660
agtggtgctc tggcatttga agatatatat attgatcagc gaaagacgat taagacgatg 3720
ttggaatatg ctgacaaggt tttcacttac attttcattc tggaaatgct tctaaaatgg 3780
gtggcatatg gctatcaaac atatttcacc aatgcctggt gttggctgga cttcttaatt 3840
gttgatgttt cattggtcag tttaacagca aatgccttgg gttactcaga acttggagcc 3900
atcaaatctc tcaggacact aagagctctg agacctctaa gagccttatc tcgatttgaa 3960
gggatgaggg tggttgtgaa tgccctttta ggagcaattc catccatcat gaatgtgctt 4020
ctggtttgtc ttatattctg gctaattttc agcatcatgg gcgtaaattt gtttgctggc 4080
aaattctacc actgtattaa caccacaact ggtgacaggt ttgacatcga agacgtgaat 4140
aatcatactg attgcctaaa actaatagaa agaaatgaga ctgctcgatg gaaaaatgtg 4200
aaagtaaact ttgataatgt aggatttggg tatctctctt tgcttcaagt tgccacattc 4260
aaaggatgga tggatataat gtatgcagca gttgattcca gaaatgtgga actccagcct 4320
aagtatgaag aaagtctgta catgtatctt tactttgtta ttttcatcat ctttgggtcc 4380
ttcttcacct tgaacctgtt tattggtgtc atcatagata atttcaacca gcagaaaaag 4440
aagtttggag gtcaagacat ctttatgaca gaagaacaga agaaatacta taatgcaatg 4500
aaaaaattag gatcgaaaaa accgcaaaag cctatacctc gaccaggaaa caaatttcaa 4560
ggaatggtct ttgacttcgt aaccagacaa gtttttgaca taagcatcat gattctcatc 4620
tgtcttaaca tggtcacaat gatggtggaa acagatgacc agagtgaata tgtgactacc 4680
attttgtcac gcatcaatct ggtgttcatt gtgctattta ctggagagtg tgtactgaaa 4740
ctcatctctc tacgccatta ttattttacc attggatgga atatttttga ttttgtggtt 4800
gtcattctct ccattgtagg tatgtttctt gccgagctga tagaaaagta tttcgtgtcc 4860
cctaccctgt tccgagtgat ccgtcttgct aggattggcc gaatcctacg tctgatcaaa 4920
ggagcaaagg ggatccgcac gctgctcttt gctttgatga tgtcccttcc tgcgttgttt 4980
aacatcggcc tcctactctt cctagtcatg ttcatctacg ccatctttgg gatgtccaac 5040
tttgcctatg ttaagaggga agttgggatc gatgacatgt tcaactttga gacctttggc 5100

```
aacagcatga tctgcctatt ccaaattaca acctctgctg gctgggatgg attgctagca     5160
cccattctca acagtaagcc acccgactgt gaccctaata aagttaaccc tggaagctca     5220
gttaagggag actgtgggaa cccatctgtt ggaattttct tttttgtcag ttacatcatc     5280
atatccttcc tggttgtggt gaacatgtac atcgcggtca tcctggagaa cttcagtgtt     5340
gctactgaag aaagtgcaga gcctctgagt gaggatgact ttgagatgtt ctatgaggtt     5400
tgggagaagt ttgatcccga tgcaactcag ttcatggaat ttgaaaaatt atctcagttt     5460
gcagctgcgc ttgaaccgcc tctcaatctg ccacaaccaa acaaactcca gctcattgcc     5520
atggatttgc ccatggtgag tggtgaccgg atccactgtc ttgatatctt atttgctttt     5580
acaaagcggg ttctaggaga gagtggagag atggatgctc tacgaataca gatggaagag     5640
cgattcatgg cttccaatcc ttccaaggtc tcctatcagc caatcactac tactttaaaa     5700
cgaaaacaag aggaagtatc tgctgtcatt attcagcgtg cttacagacg ccacctttta     5760
aagcgaactg taaaacaagc ttcctttacg tacaataaaa acaaaatcaa aggtggggct     5820
aatcttctta taaaagaaga catgataatt gacagaataa atgaaaactc tattacagaa     5880
aaaactgatc tgaccatgtc cactgcagct tgtccacctt cctatgaccg ggtgacaaag     5940
ccaattgtgg aaaaacatga gcaagaaggc aaagatgaaa aagccaaagg gaaataa        5997
```

<210> 7
<211> 6018
<212> DNA
<213> Homo sapiens

<400> 7

```
atggcacagt cagtgctggt accgccagga cctgacagct tccgcttctt taccagggaa      60
tcccttgctg ctattgaaca acgcattgca gaagagaaag ctaagagacc caaacaggaa     120
cgcaaggatg aggatgatga aaatggccca aagccaaaca gtgacttgga agcaggaaaa     180
tctcttccat ttatttatgg agacattcct ccagagatgg tgtcagtgcc cctggaggat     240
ctggacccct actatatcaa taagaaaacg tttatagtat tgaataaagg gaaagcaatc     300
tctcgattca gtgccacccc tgccctttac attttaactc ccttcaaccc tattagaaaa     360
ttagctatta agattttggt acattcttta ttcaatatgc tcattatgtg cacgattctt     420
accaactgtg tatttatgac catgagtaac cctccagact ggacaaagaa tgtggagtat     480
acctttacag gaatttatac ttttgaatca cttattaaaa tacttgcaag ggcttttgt      540
ttagaagatt tcacattttt acgggatcca tggaattggt tggatttcac agtcattact     600
tttgcatatg tgacagagtt tgtggacctg ggcaatgtct cagcgttgag aacattcaga     660
gttctccgag cattgaaaac aatttcagtc attccaggcc tgaagaccat tgtgggggcc     720
ctgatccagt cagtgaagaa gctttctgat gtcatgatct tgactgtgtt ctgtctaagc     780
gtgtttgcgc taataggatt gcagttgttc atgggcaacc tacgaaataa atgtttgcaa     840
```

tggcctccag ataattcttc ctttgaaata aatatcactt ccttctttaa caattcattg 900
gatgggaatg gtactacttt caataggaca gtgagcatat ttaactggga tgaatatatt 960
gaggataaaa gtcactttta ttttttagag gggcaaaatg atgctctgct ttgtggcaac 1020
agctcagatg caggccagtg tcctgaagga tacatctgtg tgaaggctgg tagaaacccc 1080
aactatggct acacgagctt tgacaccttt agttgggcct ttttgtcctt atttcgtctc 1140
atgactcaag acttctggga aaacctttat caactgacac tacgtgctgc tgggaaaacg 1200
tacatgatat tttttgtgct ggtcattttc ttgggctcat tctatctaat aaatttgatc 1260
ttggctgtgg tggccatggc ctatgaggaa cagaatcagg ccacattgga agaggctgaa 1320
cagaaggaag ctgaatttca gcagatgctc gaacagttga aaaagcaaca agaagaagct 1380
caggcggcag ctgcagccgc atctgctgaa tcaagagact tcagtggtgc tggtgggata 1440
ggagtttttt cagagagttc ttcagtagca tctaagttga gctccaaaag tgaaaaagag 1500
ctgaaaaaca gaagaaagaa aaagaaacag aaagaacagt ctggagaaga agagaaaaat 1560
gacagagtcc gaaaatcgga atctgaagac agcataagaa gaaaaggttt ccgtttttcc 1620
ttggaaggaa gtaggctgac atatgaaaag agattttctt ctccacacca gtccttactg 1680
agcatccgtg gctccctttt ctctccaaga cgcaacagta gggcgagcct tttcagcttc 1740
agaggtcgag caaaggacat tggctctgag aatgactttg ctgatgatga gcacagcacc 1800
tttgaggaca atgacagccg aagagactct ctgttcgtgc cgcacagaca tggagaacgg 1860
cgccacagca atgtcagcca ggccagccgt gcctccaggg tgctccccat cctgcccatg 1920
aatgggaaga tgcatagcgc tgtggactgc aatggtgtgg tctccctggt cgggggccct 1980
tctaccctca catctgctgg gcagctccta ccagagggca caactactga aacagaaata 2040
agaaagagac ggtccagttc ttatcatgtt tccatggatt tattggaaga tcctacatca 2100
aggcaaagag caatgagtat agccagtatt ttgaccaaca ccatggaaga acttgaagaa 2160
tccagacaga aatgcccacc atgctggtat aaatttgcta atatgtgttt gatttgggac 2220
tgttgtaaac catggttaaa ggtgaaacac cttgtcaacc tggttgtaat ggacccattt 2280
gttgacctgg ccatcaccat ctgcattgtc ttaaatacac tcttcatggc tatggagcac 2340
tatcccatga cggagcagtt cagcagtgta ctgtctgttg gaaacctggt cttcacaggg 2400
atcttcacag cagaaatgtt tctcaagata attgccatgg atccatatta ttactttcaa 2460
gaaggctgga atatttttga tggttttatt gtgagcctta gtttaatgga acttggtttg 2520
gcaaatgtgg aaggattgtc agttctccga tcattccggc tgctccgagt tttcaagttg 2580
gcaaaatctt ggccaactct aaatatgcta attaagatca ttggcaattc tgtgggggct 2640
ctaggaaacc tcaccttggt attggccatc atcgtcttca tttttgctgt ggtcggcatg 2700
cagctctttg gtaagagcta caaagaatgt gtctgcaaga tttccaatga ttgtgaactc 2760

ccacgctggc acatgcatga ctttttccac tccttcctga tcgtgttccg cgtgctgtgt 2820
ggagagtgga tagagaccat gtgggactgt atggaggtcg ctggccaaac catgtgcctt 2880
actgtcttca tgatggtcat ggtgattgga aatctagtgg ttctgaacct cttcttggcc 2940
ttgcttttga gttccttcag ttctgacaat cttgctgcca ctgatgatga taacgaaatg 3000
aataatctcc agattgctgt gggaaggatg cagaaaggaa tcgattttgt taaaagaaaa 3060
atacgtgaat ttattcagaa agcctttgtt aggaagcaga aagctttaga tgaaattaaa 3120
ccgcttgaag atctaaataa taaaaaagac agctgtattt ccaaccatac caccatagaa 3180
ataggcaaag acctcaatta tctcaaagac ggaaatggaa ctactagtgg cataggcagc 3240
agtgtagaaa aatatgtcgt ggatgaaagt gattacatgt catttataaa caaccctagc 3300
ctcactgtga cagtaccaat tgctgttgga gaatctgact ttgaaaattt aaatactgaa 3360
gaattcagca gcgagtcaga tatggaggaa agcaaagaga agctaaatgc aactagttca 3420
tctgaaggca gcacggttga tattggagct cccgccgagg gagaacagcc tgaggttgaa 3480
cctgaggaat cccttgaacc tgaagcctgt tttacagaag actgtgtacg gaagttcaag 3540
tgttgtcaga taagcataga agaaggcaaa gggaaactct ggtggaattt gaggaaaaca 3600
tgctataaga tagtggagca caattggttc gaaaccttca ttgtcttcat gattctgctg 3660
agcagtgggg ctctggcctt tgaagatata tacattgagc agcgaaaaac cattaagacc 3720
atgttagaat atgctgacaa ggttttcact tacatattca ttctggaaat gctgctaaag 3780
tgggttgcat atggttttca agtgtatttt accaatgcct ggtgctggct agacttcctg 3840
attgttgatg tctcactggt tagcttaact gcaaatgcct tgggttactc agaacttggt 3900
gccatcaaat ccctcagaac actaagagct ctgaggccac tgagagcttt gtcccggttt 3960
gaaggaatga gggttgttgt aaatgctctt ttaggagcca ttccatctat catgaatgta 4020
cttctggttt gtctgatctt ttggctaata ttcagtatca tgggagtgaa tctctttgct 4080
ggcaagtttt accattgtat taattacacc actggagaga tgtttgatgt aagcgtggtc 4140
aacaactaca gtgagtgcaa agctctcatt gagagcaatc aaactgccag gtggaaaaat 4200
gtgaaagtaa actttgataa cgtaggactt ggatatctgt ctctacttca agtagccacg 4260
tttaagggat ggatggatat tatgtatgca gctgttgatt cacgaaatgt agaattacaa 4320
cccaagtatg aagacaacct gtacatgtat ctttattttg tcatctttat tatttttggt 4380
tcattcttta ccttgaatct tttcattggt gtcatcatag ataacttcaa ccaacagaaa 4440
aagaagtttg gaggtcaaga catttttatg acagaagaac agaagaaata ctacaatgca 4500
atgaaaaaac tgggttcaaa gaaaccacaa aaacccatac ctcgacctgc taacaaattc 4560
caaggaatgg tctttgattt tgtaaccaaa caagtctttg atatcagcat catgatcctc 4620
atctgcctta acatggtcac catgatggtg gaaaccgatg accagagtca agaaatgaca 4680
aacattctgt actggattaa tctggtgttt attgttctgt tcactggaga atgtgtgctg 4740

aaactgatct ctcttcgtta ctactatttc actattggat ggaatatttt tgattttgtg 4800

gtggtcattc tctccattgt aggaatgttt ctggctgaac tgatagaaaa gtattttgtg 4860

tcccctaccc tgttccgagt gatccgtctt gccaggattg gccgaatcct acgtctgatc 4920

aaaggagcaa aggggatccg cacgctgctc tttgctttga tgatgtccct tcctgcgttg 4980

tttaacatcg gcctccttct tttcctggtc atgttcatct acgccatctt tgggatgtcc 5040

aattttgcct atgttaagag ggaagttggg atcgatgaca tgttcaactt tgagaccttt 5100

ggcaacagca tgatctgcct gttccaaatt acaacctctg ctggctggga tggattgcta 5160

gcacctattc ttaatagtgg acctccagac tgtgaccctg acaaagatca ccctggaagc 5220

tcagttaaag gagactgtgg gaacccatct gttgggattt tctttttgt cagttacatc 5280

atcatatcct tcctggttgt ggtgaacatg tacatcgcgg tcatcctgga gaacttcagt 5340

gttgctactg aagaaagtgc agagcctctg agtgaggatg actttgagat gttctatgag 5400

gtttgggaga agtttgatcc cgatgcgacc cagtttatag agtttgccaa actttctgat 5460

tttgcagatg ccctggatcc tcctcttctc atagcaaaac ccaacaaagt ccagctcatt 5520

gccatggatc tgcccatggt gagtggtgac cggatccact gtcttgacat cttatttgct 5580

tttacaaagc gtgttttggg tgagagtgga gagatggatg cccttcgaat acagatggaa 5640

gagcgattca tggcatcaaa cccctccaaa gtctcttatg agcccattac gaccacgttg 5700

aaacgcaaac aagaggaggt gtctgctatt attatccaga gggcttacag acgctacctc 5760

ttgaagcaaa aagttaaaaa ggtatcaagt atatacaaga aagacaaagg caaagaatgt 5820

gatggaacac ccatcaaaga agatactctc attgataaac tgaatgagaa ttcaactcca 5880

gagaaaaccg atatgacgcc ttccaccacg tctccaccct cgtatgatag tgtgaccaaa 5940

ccagaaaaag aaaaatttga aaaagacaaa tcagaaaagg aagacaaagg gaaagatatc 6000

agggaaagta aaaagtaa 6018

<210> 8
<211> 6003
<212> DNA
<213> Homo sapiens

<400> 8

```
atggcacagg cactgttggt acccccagga cctgaaagct tccgcctttt tactagagaa      60
tctcttgctg ctatcgaaaa acgtgctgca gaagagaaag ccaagaagcc caaaaaggaa     120
caagataatg atgatgagaa caaaccaaag ccaaatagtg acttggaagc tggaaagaac     180
cttccattta tttatggaga cattcctcca gagatggtgt cagagcccct ggaggacctg     240
gatccctact atatcaataa gaaaactttt atagtaatga ataaaggaaa ggcaattttc     300
cgattcagtg ccacctctgc cttgtatatt ttaactccac taaaccctgt taggaaaatt     360
gctatcaaga ttttggtaca ttctttattc agcatgctta tcatgtgcac tattttgacc     420
```

aactgtgtat ttatgacctt gagcaaccct cctgactgga caaagaatgt agagtacaca 480
ttcactggaa tctatacctt tgagtcactt ataaaaatct tggcaagagg gttttgctta 540
gaagatttta cgtttcttcg tgatccatgg aactggctgg atttcagtgt cattgtgatg 600
gcatatgtga cagagtttgt ggacctgggc aatgtctcag cgttgagaac attcagagtt 660
ctccgagcac tgaaaacaat ttcagtcatt ccaggtttaa agaccattgt gggggccctg 720
atccagtcgg taaagaagct ttctgatgtg atgatcctga ctgtgttctg tctgagcgtg 780
tttgctctca ttgggctgca gctgttcatg ggcaatctga ggaataaatg tttgcagtgg 840
cccccaagcg attctgcttt tgaaaccaac accacttcct actttaatgg cacaatggat 900
tcaaatggga catttgttaa tgtaacaatg agcacattta actggaagga ttacattgga 960
gatgacagtc acttttatgt tttggatggg caaaaagacc ctttactctg tggaaatggc 1020
tcagatgcag gccagtgtcc agaaggatac atctgtgtga aggctggtcg aaaccccaac 1080
tatggctaca caagctttga cacctttagc tgggctttcc tgtctctatt tcgactcatg 1140
actcaagact actgggaaaa tctttaccag ttgacattac gtgctgctgg gaaaacatac 1200
atgatatttt ttgtcctggt cattttcttg ggctcatttt atttggtgaa tttgatcctg 1260
gctgtggtgg ccatggccta tgaggagcag aatcaggcca ccttggaaga agcagaacaa 1320
aaagaggccg aatttcagca gatgctcgaa cagcttaaaa agcaacagga agaagctcag 1380
gcagttgcgg cagcatcagc tgcttcaaga gatttcagtg gaataggtgg gttaggagag 1440
ctgttggaaa gttcttcaga agcatcaaag ttgagttcca aaagtgctaa agaatggagg 1500
aaccgaagga agaaaagaag acagagagag caccttgaag gaaacaacaa aggagagaga 1560
gacagctttc ccaaatccga atctgaagac agcgtcaaaa gaagcagctt ccttttctcc 1620
atggatggaa acagactgac cagtgacaaa aaattctgct cccctcatca gtctctcttg 1680
agtatccgtg gctccctgtt ttccccaaga cgcaatagca aaacaagcat tttcagtttc 1740
agaggtcggg caaaggatgt tggatctgaa aatgactttg ctgatgatga acacagcaca 1800
tttgaagaca gcgaaagcag gagagactca ctgtttgtgc cgcacagaca tggagagcga 1860
cgcaacagta acgttagtca ggccagtatg tcatccagga tggtgccagg gcttccagca 1920
aatgggaaga tgcacagcac tgtggattgc aatggtgtgg tttccttggt gggtggacct 1980
tcagctctaa cgtcacctac tggacaactt cccccagagg gcaccaccac agaaacggaa 2040
gtcagaaaga gaaggttaag ctcttaccag atttcaatgg agatgctgga ggattcctct 2100
ggaaggcaaa gagccgtgag catagccagc attctgacca acacaatgga agaacttgaa 2160
gaatctagac agaaatgtcc gccatgctgg tatagatttg ccaatgtgtt cttgatctgg 2220
gactgctgtg atgcatggtt aaaagtaaaa catcttgtga atttaattgt tatggatcca 2280
tttgttgatc ttgccatcac tatttgcatt gtcttaaata ccctctttat ggccatggag 2340

cactacccca tgactgagca attcagtagt gtgttgactg taggaaacct ggtctttact 2400
gggattttca cagcagaaat ggttctcaag atcattgcca tggatcctta ttactatttc 2460
caagaaggct ggaatatctt tgatggaatt attgtcagcc tcagtttaat ggagcttggt 2520
ctgtcaaatg tggagggatt gtctgtactg cgatcattca gactgcttag agttttcaag 2580
ttggcaaaat cctggcccac actaaatatg ctaattaaga tcattggcaa ttctgtgggg 2640
gctctaggaa acctcacctt ggtgttggcc atcatcgtct tcatttttgc tgtggtcggc 2700
atgcagctct ttggtaagag ctacaaagaa tgtgtctgca agatcaatga tgactgtacg 2760
ctcccacggt ggcacatgaa cgacttcttc cactccttcc tgattgtgtt ccgcgtgctg 2820
tgtggagagt ggatagagac catgtgggac tgtatggagg tcgctggcca aaccatgtgc 2880
cttattgttt tcatgttggt catggtcatt ggaaaccttg tggttctgaa cctctttctg 2940
gccttattgt tgagttcatt tagctcagac aaccttgctg ctactgatga tgacaatgaa 3000
atgaataatc tgcagattgc agtaggaaga atgcaaaagg gaattgatta tgtgaaaaat 3060
aagatgcggg agtgtttcca aaaagccttt tttagaaagc caaaagttat agaaatccat 3120
gaaggcaata agatagacag ctgcatgtcc aataatactg gaattgaaat aagcaaagag 3180
cttaattatc ttagagatgg gaatggaacc accagtggtg taggtactgg aagcagtgtt 3240
gaaaaatacg taatcgatga aaatgattat atgtcattca taaacaaccc cagcctcacc 3300
gtcacagtgc caattgctgt tggagagtct gactttgaaa acttaaatac tgaagagttc 3360
agcagtgagt cagaactaga agaaagcaaa gagaaattaa atgcaaccag ctcatctgaa 3420
ggaagcacag ttgatgttgt tctacccga gaaggtgaac aagctgaaac tgaacccgaa 3480
gaagacctta aaccggaagc ttgttttact gaaggatgta ttaaaaagtt tccattctgt 3540
caagtaagta cagaagaagg caaagggaag atctggtgga atcttcgaaa aacctgctac 3600
agtattgttg agcacaactg gtttgagact ttcattgtgt tcatgatcct tctcagtagt 3660
ggtgcattgg cctttgaaga tatatacatt gaacagcgaa agactatcaa aaccatgcta 3720
gaatatgctg acaaagtctt tacctatata ttcattctgg aaatgcttct caaatgggtt 3780
gcttatggat ttcaaacata tttcactaat gcctggtgct ggctagattt cttgatcgtt 3840
gatgtttctt tggttagcct ggtagccaat gctcttggct actcagaact cggtgccatc 3900
aaatcattac ggacattaag agctttaaga cctctaagag ccttatcccg gtttgaaggc 3960
atgagggtgg ttgtgaatgc tcttgttgga gcaattccct ctatcatgaa tgtgctgttg 4020
gtctgtctca tcttctggtt gatctttagc atcatgggtg tgaatttgtt tgctggcaag 4080
ttctaccact gtgttaacat gacaacgggt aacatgtttg acattagtga tgttaacaat 4140
ttgagtgact gtcaggctct tggcaagcaa gctcggtgga aaaacgtgaa agtaaacttt 4200
gataatgttg gcgctggcta tcttgcactg cttcaagtgg ccacatttaa aggctggatg 4260
gatattatgt atgcagctgt tgattcacga gatgttaaac ttcagcctgt atatgaagaa 4320

aatctgtaca tgtatttata ctttgtcatc tttatcatct ttgggtcatt cttcactctg 4380
aatctattca ttggtgtcat catagataac ttcaaccagc agaaaaagaa gtttggaggt 4440
caagacatct ttatgacaga ggaacagaaa aaatattaca atgcaatgaa gaaacttgga 4500
tccaagaaac ctcagaaacc catacctcgc ccagcaaaca aattccaagg aatggtcttt 4560
gattttgtaa ccagacaagt ctttgatatc agcatcatga tcctcatctg cctcaacatg 4620
gtcaccatga tggtggaaac ggatgaccag ggcaaataca tgaccctagt tttgtcccgg 4680
atcaacctag tgttcattgt tctgttcact ggagaatttg tgctgaagct cgtctccctc 4740
agacactact acttcactat aggctggaac atctttgact ttgtggtggt gattctctcc 4800
attgtaggta tgtttctggc tgagatgata gaaaagtatt ttgtgtcccc taccttgttc 4860
cgagtgatcc gtcttgccag gattggccga atcctacgtc tgatcaaagg agcaaagggg 4920
atccgcacgc tgctctttgc tttgatgatg tcccttcctg cgttgtttaa catcggcctc 4980
ctgctcttcc tggtcatgtt tatctatgcc atctttggga tgtccaactt tgcctatgtt 5040
aaaaaggaag ctggaattga tgacatgttc aactttgaga cctttggcaa cagcatgatc 5100
tgcttgttcc aaattacaac ctctgctggc tgggatggat tgctagcacc tattcttaat 5160
agtgcaccac ccgactgtga ccctgacaca attcaccctg gcagctcagt taagggagac 5220
tgtgggaacc catctgttgg gattttcttt tttgtcagtt acatcatcat atccttcctg 5280
gttgtggtga acatgtacat cgcggtcatc ctggagaact tcagtgttgc tactgaagaa 5340
agtgcagagc ccctgagtga ggatgacttt gagatgttct atgaggtttg ggaaaagttt 5400
gatcccgatg cgacccagtt tatagagttc tctaaactct ctgattttgc agctgccctg 5460
gatcctcctc ttctcatagc aaaacccaac aaagtccagc ttattgccat ggatctgccc 5520
atggtcagtg gtgaccggat ccactgtctt gatattttat ttgcctttac aaagcgtgtt 5580
ttgggtgaga gtggagagat ggatgccctt cgaatacaga tggaagacag gtttatggca 5640
tcaaacccct ccaaagtctc ttatgagcct attacaacca ctttgaaacg taaacaagag 5700
gaggtgtctg ccgctatcat tcagcgtaat ttcagatgtt atcttttaaa gcaaaggtta 5760
aaaaatatat caagtaacta taacaaagag gcaattaaag ggaggattga cttacctata 5820
aaacaagaca tgattattga caaactaaat gggaactcca ctccagaaaa aacagatggg 5880
agttcctcta ccacctctcc tccttcctat gatagtgtaa caaaaccaga caaggaaaag 5940
tttgagaaag acaaaccaga aaaagaaagc aaaggaaaag aggtcagaga aaatcaaaag 6000
taa 6003

<210> 9
<211> 5511
<212> DNA

<213> Homo sapiens

<400> 9

```
atggccagac catctctgtg caccctggtg cctctgggcc ctgagtgctt gcgccccttc      60
acccgggagt cactggcagc catagaacag cgggcggtgg aggaggaggc ccggctgcag     120
cggaataagc agatggagat tgaggagccc gaacggaagc cacgaagtga cttggaggct     180
ggcaagaacc tacccatgat ctacggagac cccccgccgg aggtcatcgg catccccctg     240
gaggacctgg atccctacta cagcaataag aagaccttca tcgtactcaa caagggcaag     300
gccatcttcc gcttctccgc cacacctgct ctctacctgc tgagcccctt cagcgtagtc     360
aggcgcgggg ccatcaaggt gctcatccat gcgctgttca gcatgttcat catgatcacc     420
atcttgacca actgcgtatt catgaccatg agtgacccgc ctccctggtc caagaatgtg     480
gagtacacct tcacagggat ctacaccttt gagtccctca tcaagatact ggcccgaggc     540
ttctgtgtcg acgacttcac attcctccgg gacccctgga actggctgga cttcagtgtc     600
atcatgatgg cgtacctgac agagtttgtg gacttgggca acatctcagc cctgaggacc     660
ttccgggtgc tgcgggccct caaaaccatc acggtcatcc cagggctgaa gacgatcgtg     720
ggggccctga tccagtcggt gaaaaagctg tcggatgtga tgatcctcac tgtcttctgc     780
ctgagcgtct ttgcgctggt aggactgcag ctcttcatgg gaaacctgag gcagaagtgt     840
gtgcgctggc cccgccgtt caacgacacc aacaccacgt ggtacagcaa tgacacgtgg      900
tacggcaatg acacatggta tggcaatgag atgtggtacg gcaatgactc atggtatgcc     960
aacgacacgt ggaacagcca tgcaagctgg gccaccaacg ataccttga ttgggacgcc     1020
tacatcagtg atgaagggaa cttctacttc ctggagggct ccaacgatgc cctgctctgt    1080
gggaacagca gtgatgctgg gcactgccct gagggttatg agtgcatcaa gaccgggcgg    1140
aaccccaact atggctacac cagctatgac accttcagct gggccttctt ggctctcttc    1200
cgcctcatga cacaggacta ttgggagaac ctcttccagc tgacccttcg agcagctggc    1260
aagacctaca tgatcttctt cgtggtcatc atcttcctgg gctctttcta cctcatcaat    1320
ctgatcctgg ccgtggtggc catggcatat gccgagcaga atgaggccac cctggccgag    1380
gataaggaga aagaggagga gtttcagcag atgcttgaga agttcaaaaa gcaccaggag    1440
gagctggaga aggccaaggc cgcccaagct ctggaaggtg gggaggcaga tggggaccca    1500
gcccatggca aagactgcaa tggcagcctg gacacatcgc aaggggagaa gggagccccg    1560
aggcagagca gcagcggaga cagcggcatc tccgacgcca tggaagaact ggaagaggcc    1620
caccaaaagt gcccaccatg gtggtacaag tgcgcccaca aagtgctcat atggaactgc    1680
tgcgccccgt ggctgaagtt caagaacatc atccacctga tcgtcatgga cccgttcgtg    1740
gacctgggca tcaccatctg catcgtgctc aacaccctct tcatggccat ggaacattac    1800
cccatgacgg agcactttga caacgtgctc actgtgggca acctggtctt cacaggcatc    1860
ttcacagcag agatggttct gaagctgatt gccatggacc cctacgagta tttccagcag    1920
```

ggttggaata tcttcgacag catcatcgtc accctcagcc tggtagagct aggcctggcc 1980
aacgtacagg gactgtctgt gctacgctcc ttccgtctgc tgcgggtctt caagctggcc 2040
aagtcgtggc caacgctgaa catgctcatc aagatcattg gcaattcagt gggggcgctg 2100
ggtaacctga cgctggtgct ggctatcatc gtgttcatct tcgccgtggt gggcatgcag 2160
ctgtttggca agagctacaa ggagtgcgtg tgcaagattg ccttggactg caacctgccg 2220
cgctggcaca tgcatgattt cttccactcc ttcctcatcg tcttccgcat cctgtgcggg 2280
gagtggatcg agaccatgtg ggactgcatg gaggtggccg gccaagccat gtgcctcacc 2340
gtcttcctca tggtcatggt catcggcaat cttgtggtcc tgaacctgtt cctggctctg 2400
ctgctgagct ccttcagcgc cgacagtctg gcagcctcgg atgaggatgg cgagatgaac 2460
aacctgcaga ttgccatcgg gcgcatcaag ttgggcatcg gctttgccaa ggccttcctc 2520
ctggggctgc tgcatggcaa gatcctgagc cccaaggaca tcatgctcag cctcggggag 2580
gctgacgggg ccggggaggc tggagaggcg ggggagactg cccccgagga tgagaagaag 2640
gagccgcccg aggaggacct gaagaaggac aatcacatcc tgaaccacat gggcctggct 2700
gacggccccc catccagcct cgagctggac caccttaact tcatcaacaa cccctacctg 2760
accatacagg tgcccatcgc ctccgaggag tccgacctgg agatgcccac cgaggaggaa 2820
accgacactt tctcagagcc tgaggatagc aagaagccgc cgcagcctct ctatgatggg 2880
aactcgtccg tctgcagcac agctgactac aagcccccg aggaggaccc tgaggagcag 2940
gcagaggaga accccgaggg ggagcagcct gaggagtgct tcactgaggc ctgcgtgcag 3000
cgctggccct gcctctacgt ggacatctcc cagggccgtg ggaagaagtg gtggactctg 3060
cgcagggcct gcttcaagat tgtcgagcac aactggttcg agaccttcat tgtcttcatg 3120
atcctgctca gcagtggggc tctggccttc gaggacatct acattgagca gcggcgagtc 3180
attcgcacca tcctagaata tgccgacaag gtcttcacct acatcttcat catggagatg 3240
ctgctcaaat gggtggccta cggctttaag gtgtacttca ccaacgcctg gtgctggctc 3300
gacttcctca tcgtggatgt ctccatcatc agcttggtgg ccaactggct gggctactcg 3360
gagctgggac ccatcaaatc cctgcggaca ctgcgggccc tgcgtcccct gagggcactg 3420
tcccgattcg agggcatgag ggtggtggtg aacgccctcc taggcgccat cccctccatc 3480
atgaatgtgc tgcttgtctg cctcatcttc tggctgatct tcagcatcat gggtgtcaac 3540
ctgtttgccg gcaagttcta ctactgcatc aacaccacca cctctgagag gttcgacatc 3600
tccgaggtca acaacaagtc tgagtgcgag agcctcatgc acacaggcca ggtccgctgg 3660
ctcaatgtca aggtcaacta cgacaacgtg ggtctgggct acctctccct cctgcaggtg 3720
gccaccttca agggttggat ggacatcatg tatgcagccg tggactcccg ggagaaggag 3780
gagcagccgc agtacgaggt gaacctctac atgtacctct actttgtcat cttcatcatc 3840
tttggctcct tcttcaccct caacctcttc attggcgtca tcattgacaa cttcaaccag 3900

cagaagaaga agttaggggg gaaagacatc tttatgacgg aggaacagaa gaaatactat 3960

aacgccatga agaagcttgg ctccaagaag cctcagaagc caattccccg gccccagaac 4020

aagatccagg gcatggtgta tgacctcgtg acgaagcagg ccttcgacat caccatcatg 4080

atcctcatct gcctcaacat ggtcaccatg atggtggaga cagacaacca gagccagctc 4140

aaggtggaca tcctgtacaa catcaacatg atcttcatca tcatcttcac aggggagtgc 4200

gtgctcaaga tgctcgccct gcgccagtac tacttcaccg ttggctggaa catctttgac 4260

ttcgtggtcg tcatcctgtc cattgtgggc cttgccctct ctgacctgat ccagaagtac 4320

ttcgtgtcac ccacgctgtt ccgtgtgatc cgcctggcgc ggattgggcg tgtcctgcgg 4380

ctgatccgcg gggccaaggg catccggacg ctgctgttcg ccctcatgat gtcgctgcct 4440

gccctcttca acatcggcct cctcctcttc ctggtcatgt tcatctactc catcttcggc 4500

atgtccaact ttgcctacgt caagaaggag tcgggcatcg atgatatgtt caacttcgag 4560

accttcggca acagcatcat ctgcctgttc gagatcacca cgtcggccgg ctgggacggg 4620

ctcctcaacc ccatcctcaa cagcgggccc ccagactgtg accccaacct ggagaacccg 4680

ggcaccagtg tcaagggtga ctgcggcaac ccctccatcg gcatctgctt cttctgcagc 4740

tatatcatca tctccttcct catcgtggtc aacatgtaca tcgccatcat cctggagaac 4800

ttcaatgtgg ccacagagga gagcagcgag ccccttggtg aagatgactt tgagatgttc 4860

tacgagacat gggagaagtt cgaccccgac gccacccagt tcatcgccta cagccgcctc 4920

tcagacttcg tggacaccct gcaggaaccg ctgaggattg ccaagcccaa caagatcaag 4980

ctcatcacac tggacttgcc catggtgcca ggggacaaga tccactgcct ggacatcctc 5040

tttgccctga ccaaagaggt cctgggtgac tctggggaaa tggacgccct caagcagacc 5100

atggaggaga agttcatggc agccaacccc tccaaggtgt cctacgagcc catcaccacc 5160

accctcaaga ggaagcacga ggaggtgtgc gccatcaaga tccagagggc ctaccgccgg 5220

cacctgctac agcgctccat gaagcaggca tcctacatgt accgccacag ccacgacggc 5280

agcggggatg acgcccctga gaaggagggg ctgcttgcca acaccatgag caagatgtat 5340

ggccacgaga atgggaacag cagctcgcca agcccggagg agaagggcga ggcaggggac 5400

gccggaccca ctatggggct gatgcccatc agcccctcag acactgcctg gcctcccgcc 5460

cctcccccag ggcagactgt gcgcccaggt gtcaaggagt ctcttgtcta g 5511

<210> 10
<211> 6051
<212> DNA
<213> Homo sapiens

<400> 10

atggcaaact tcctattacc tcggggcacc agcagcttcc gcaggttcac acgggagtcc 60

ctggcagcca tcgagaagcg catggcagag aagcaagccc gcggctcaac caccttgcag 120

gagagccgag aggggctgcc cgaggaggag gctccccggc cccagctgga cctgcaggcc 180
tccaaaaagc tgccagatct ctatggcaat ccaccccaag agctcatcgg agagcccctg 240
gaggacctgg accccttcta tagcacccaa aagactttca tcgtactgaa taaaggcaag 300
accatcttcc ggttcagtgc caccaacgcc ttgtatgtcc tcagtccctt ccaccccatc 360
cggagagcgg ctgtgaagat tctggttcac tcgctcttca acatgctcat catgtgcacc 420
atcctcacca actgcgtgtt catggcccag cacgaccctc caccctggac caagtatgtc 480
gagtacacct tcaccgccat ttacaccttt gagtctctgg tcaagattct ggctcgaggc 540
ttctgcctgc acgcgttcac tttccttcgg gacccatgga actggctgga ctttagtgtg 600
attatcatgg catacacaac tgaatttgtg gacctgggca atgtctcagc cttacgcacc 660
ttccgagtcc tccgggccct gaaaactata tcagtcattt cagggctgaa gaccatcgtg 720
ggggccctga tccagtctgt gaagaagctg gctgatgtga tggtcctcac agtcttctgc 780
ctcagcgtct ttgccctcat cggcctgcag ctcttcatgg gcaacctaag gcacaagtgc 840
gtgcgcaact tcacagcgct caacggcacc aacggctccg tggaggccga cggcttggtc 900
tgggaatccc tggaccttta cctcagtgat ccagaaaatt acctgctcaa gaacggcacc 960
tctgatgtgt tactgtgtgg gaacagctct gacgctggga catgtccgga gggctaccgg 1020
tgcctaaagg caggcgagaa ccccgaccac ggctacacca gcttcgattc ctttgcctgg 1080
gcctttcttg cactcttccg cctgatgacg caggactgct gggagcgcct ctatcagcag 1140
accctcaggt ccgcagggaa gatctacatg atcttcttca tgcttgtcat cttcctgggg 1200
tccttctacc tggtgaacct gatcctggcc gtggtcgcaa tggcctatga ggagcaaaac 1260
caagccacca tcgctgagac cgaggagaag gaaaagcgct tccaggaggc catggaaatg 1320
ctcaagaaag aacacgaggc cctcaccatc aggggtgtgg ataccgtgtc ccgtagctcc 1380
ttggagatgt cccctttggc cccagtaaac agccatgaga gaagaagcaa gaggagaaaa 1440
cggatgtctt caggaactga ggagtgtggg gaggacaggc tccccaagtc tgactcagaa 1500
gatggtccca gagcaatgaa tcatctcagc ctcacccgtg gcctcagcag gacttctatg 1560
aagccacgtt ccagccgcgg gagcattttc acctttcgca ggcgagacct gggttctgaa 1620
gcagattttg cagatgatga aaacagcaca gcgggggaga gcgagagcca ccacacatca 1680
ctgctggtgc cctggcccct gcgccggacc agtgcccagg gacagcccag tcccggaacc 1740
tcggctcctg gccacgccct ccatggcaaa aagaacagca ctgtggactg caatggggtg 1800
gtctcattac tgggggcagg cgacccagag gccacatccc caggaagcca cctcctccgc 1860
cctgtgatgc tagagcaccc gccagacacg accacgccat cggaggagcc aggcgggccc 1920
cagatgctga cctcccaggc tccgtgtgta gatggcttcg aggagccagg agcacggcag 1980
cgggccctca gcgcagtcag cgtcctcacc agcgcactgg aagagttaga ggagtctcgc 2040

cacaagtgtc caccatgctg gaaccgtctc gcccagcgct acctgatctg ggagtgctgc 2100

ccgctgtgga tgtccatcaa gcagggagtg aagttggtgg tcatggaccc gtttactgac 2160

ctcaccatca ctatgtgcat cgtactcaac acactcttca tggcgctgga gcactacaac 2220

atgacaagtg aattcgagga gatgctgcag gtcggaaacc tggtcttcac agggattttc 2280

acagcagaga tgaccttcaa gatcattgcc ctcgacccct actactactt ccaacagggc 2340

tggaacatct tcgacagcat catcgtcatc cttagcctca tggagctggg cctgtcccgc 2400

atgagcaact tgtcggtgct gcgctccttc cgcctgctgc gggtcttcaa gctggccaaa 2460

tcatggccca ccctgaacac actcatcaag atcatcggga actcagtggg ggcactgggg 2520

aacctgacac tggtgctagc catcatcgtg ttcatctttg ctgtggtggg catgcagctc 2580

tttggcaaga actactcgga gctgagggac agcgactcag gcctgctgcc tcgctggcac 2640

atgatggact tctttcatgc cttcctcatc atcttccgca tcctctgtgg agagtggatc 2700

gagaccatgt gggactgcat ggaggtgtcg gggcagtcat tatgcctgct ggtcttcttg 2760

cttgttatgg tcattggcaa ccttgtggtc ctgaatctct tcctggcctt gctgctcagc 2820

tccttcagtg cagacaacct cacagcccct gatgaggaca gagagatgaa caacctccag 2880

ctggccctgg cccgcatcca gaggggcctg cgctttgtca agcggaccac ctgggatttc 2940

tgctgtggtc tcctgcggca gcggcctcag aagcccgcag cccttgccgc ccagggccag 3000

ctgcccagct gcattgccac cccctactcc ccgccacccc cagagacgga gaaggtgcct 3060

cccacccgca aggaaacacg gtttgaggaa ggcgagcaac caggccaggg cacccccggg 3120

gatccagagc ccgtgtgtgt gcccatcgct gtggccgagt cagacacaga tgaccaagaa 3180

gaagatgagg agaacagcct gggcacggag gaggagtcca gcaagcagca ggaatcccag 3240

cctgtgtccg gtggcccaga ggcccctccg gattccagga cctggagcca ggtgtcagcg 3300

actgcctcct ctgaggccga ggccagtgca tctcaggccg actggcggca gcagtggaaa 3360

gcggaacccc aggccccagg gtgcggtgag accccagagg acagttgctc cgagggcagc 3420

acagcagaca tgaccaacac cgctgagctc ctggagcaga tccctgacct cggccaggat 3480

gtcaaggacc cagaggactg cttcactgaa ggctgtgtcc ggcgctgtcc ctgctgtgcg 3540

gtggacacca cacaggcccc agggaaggtc tggtggcggt tgcgcaagac ctgctaccac 3600

atcgtggagc acagctggtt cgagacattc atcatcttca tgatcctact cagcagtgga 3660

gcgctggcct tcgaggacat ctacctagag gagcggaaga ccatcaaggt tctgcttgag 3720

tatgccgaca agatgttcac atatgtcttc gtgctggaga tgctgctcaa gtgggtggcc 3780

tacggcttca agaagtactt caccaatgcc tggtgctggc tcgacttcct catcgtagac 3840

gtctctctgg tcagcctggt ggccaacacc ctgggctttg ccgagatggg ccccatcaag 3900

tcactgcgga cgctgcgtgc actccgtcct ctgagagctc tgtcacgatt tgagggcatg 3960

agggtggtgg tcaatgccct ggtgggcgcc atcccgtcca tcatgaacgt cctcctcgtc 4020

tgcctcatct tctggctcat cttcagcatc atgggcgtga acctctttgc ggggaagttt 4080
gggaggtgca tcaaccagac agagggagac ttgcctttga actacaccat cgtgaacaac 4140
aagagccagt gtgagtcctt gaacttgacc ggagaattgt actggaccaa ggtgaaagtc 4200
aactttgaca acgtgggggc cgggtacctg gcccttctgc aggtggcaac atttaaaggc 4260
tggatggaca ttatgtatgc agctgtggac tccagggggt atgaagagca gcctcagtgg 4320
gaatacaacc tctacatgta catctatttt gtcattttca tcatctttgg gtctttcttc 4380
accctgaacc tctttattgg tgtcatcatt gacaacttca accaacagaa gaaaaagtta 4440
gggggccagg acatcttcat gacagaggag cagaagaagt actacaatgc catgaagaag 4500
ctgggctcca agaagcccca gaagcccatc ccacggcccc tgaacaagta ccagggcttc 4560
atattcgaca ttgtgaccaa gcaggccttt gacgtcacca tcatgtttct gatctgcttg 4620
aatatggtga ccatgatggt ggagacagat gaccaaagtc ctgagaaaat caacatcttg 4680
gccaagatca acctgctctt tgtggccatc ttcacaggcg agtgtattgt caagctggct 4740
gccctgcgcc actactactt caccaacagc tggaatatct tcgacttcgt ggttgtcatc 4800
ctctccatcg tgggcactgt gctctcggac atcatccaga agtacttctt ctccccgacg 4860
ctcttccgag tcatccgcct ggcccgaata ggccgcatcc tcagactgat ccgaggggcc 4920
aaggggatcc gcacgctgct ctttgccctc atgatgtccc tgcctgccct cttcaacatc 4980
gggctgctgc tcttcctcgt catgttcatc tactccatct ttggcatggc caacttcgct 5040
tatgtcaagt gggaggctgg catcgacgac atgttcaact tccagacctt cgccaacagc 5100
atgctgtgcc tcttccagat caccacgtcg gccggctggg atggcctcct cagccccatc 5160
ctcaacactg ggccgcccta ctgcgacccc actctgccca acagcaatgg ctctcggggg 5220
gactgcggga gcccagccgt gggcatcctc ttcttcacca cctacatcat catctccttc 5280
ctcatcgtgg tcaacatgta cattgccatc atcctggaga acttcagcgt ggccacggag 5340
gagagcaccg agcccctgag tgaggacgac ttcgatatgt tctatgagat ctgggagaaa 5400
tttgacccag aggccactca gtttattgag tattcggtcc tgtctgactt tgccgatgcc 5460
ctgtctgagc cactccgtat cgccaagccc aaccagataa gcctcatcaa catggacctg 5520
cccatggtga gtggggaccg catccattgc atggacattc tctttgcctt caccaaaagg 5580
gtcctggggg agtctgggga gatggacgcc ctgaagatcc agatggagga gaagttcatg 5640
gcagccaacc catccaagat ctcctacgag cccatcacca ccacactccg gcgcaagcac 5700
gaagaggtgt cggccatggt tatccagaga gccttccgca ggcacctgct gcaacgctct 5760
ttgaagcatg cctccttcct cttccgtcag caggcgggca gcggcctctc cgaagaggat 5820
gcccctgagc gagagggcct catcgcctac gtgatgagtg agaacttctc ccgacccctt 5880
ggcccaccct ccagctcctc catctcctcc acttccttcc caccctccta tgacagtgtc 5940

actagagcca ccagcgataa cctccaggtg cgggggtctg actacagcca cagtgaagat 6000

ctcgccgact tcccccttc tccggacagg gaccgtgagt ccatcgtgtg a 6051

<210> 11
<211> 5049
<212> DNA
<213> Homo sapiens

<400> 11

```
atgttggctt caccagaacc taagggcctt gttcccttca ctaaagagtc ttttgaactt      60
ataaaacagc atattgctaa aacacataat gaagaccatg aagaagaaga cttaaagcca     120
actcctgatt tggaagttgg caaaaagctt ccatttattt atggaaacct ttctcaagga     180
atggtgtcag agcccttgga agatgtggac ccatattact acaagaaaaa aaatactttc     240
atagtattaa ataaaaatag aacaatcttc agattcaatg cggcttccat cttgtgtaca     300
ttgtctcctt tcaattgtat tagaagaaca actatcaagg ttttggtaca tccctttttc     360
caactgttta ttctaattag tgtcctgatt gattgcgtat tcatgtccct gactaatttg     420
ccaaaatgga gaccagtatt agagaatact ttgcttggaa tttacacatt tgaaatactt     480
gtaaaactct ttgcaagagg tgtctgggca ggatcatttt ccttcctcgg tgatccatgg     540
aactggctcg atttcagcgt aactgtgttt gaggttatta taagatactc acctctggac     600
ttcattccaa cgcttcaaac tgcaagaact ttgagaattt taaaaattat tcctttaaat     660
caaggtctga aatcccttgt aggggtcctg atccactgct tgaagcagct tattggtgtc     720
attatcctaa ctctgttttt tctgagcata ttttctctaa ttgggatggg gctcttcatg     780
ggcaacttga aacataaatg ttttcgatgg ccccaagaga atgaaaatga aaccctgcac     840
aacagaactg gaaacccata ttatattcga gaaacagaaa acttttatta tttggaagga     900
gaaagatatg ctctcctttg tggcaacagg acagatgctg gtcagtgtcc tgaaggatat     960
gtgtgtgtaa aagctggcat aaatcctgat caaggcttca caaattttga cagttttggc    1020
tgggccttat ttgccctatt tcggttaatg gctcaggatt accctgaagt actttatcac    1080
cagatacttt atgcttctgg gaaggtctac atgatatttt ttgtggtggt aagtttttttg   1140
ttttcctttt atatggcaag tttgttctta ggcatacttg ccatggccta tgaagaagaa    1200
aagcagagag ttggtgaaat atctaagaag attgaaccaa aatttcaaca gactggaaaa    1260
gaacttcaag aaggaaatga aacagatgag gccaagacca tacaaataga aatgaagaaa    1320
aggtcaccaa tttccacaga cacatcattg gatgtgttgg aagatgctac tctcagacat    1380
aaggaagaac ttgaaaaatc caagaagata tgcccattat actggtataa gtttgctaaa    1440
actttcttga tctggaattg ttctccctgt tggttaaaat tgaaagagtt tgtccatagg    1500
attataatgg caccatttac tgatcttttc cttatcatat gcataatttt aaacgtatgt    1560
tttctgacct tggagcatta tccaatgagt aaacaaacta acactcttct caacattgga    1620
```

```
aacctggttt tcattggaat tttcacagca gaaatgattt ttaaaataat tgcaatgcat     1680
ccatatgggt atttccaagt aggttggaac atttttgata gcatgatagt gttccatggt     1740
ttaatagaac tttgtctagc aaatgttgca ggaatggctc ttcttcgatt attcaggatg     1800
ttaagaattt tcaagttggg aaagtattgg ccaacattcc agattttgat gtggtctctt     1860
agtaactcat gggtggccct gaaagacttg gtcctgttgt tgttcacatt catcttcttt     1920
tctgctgcat tcggcatgaa gctgtttggt aagaattatg aagaatttgt ctgccacata     1980
gacaaagact gtcaactccc acgctggcac atgcatgact tttccactc cttcctgaat      2040
gtgttccgaa ttctctgtgg agagtgggta gagaccttgt gggactgtat ggaggttgca     2100
ggccaatcct ggtgtattcc tttttacctg atggtcattt taattggaaa tttactggta     2160
ctttacctgt ttctggcatt ggtgagctca tttagttcat gcaaggatgt aacagctgaa     2220
gagaataatg aagcaaaaaa tctccagctt gcagtggcaa gaattaaaaa aggaataaac     2280
tatgtgcttc ttaaaatact atgcaaaaca caaaatgtcc caaaggacac aatggaccat     2340
gtaaatgagg tatatgttaa agaagatatt tctgaccata ccctttctga attgagcaac     2400
acccaagatt ttctcaaaga taaggaaaaa agcagtggca cagagaaaaa cgctactgaa     2460
aatgagagcc aatcacttat ccccagtcct agtgtctcag aaactgtacc aattgcttca     2520
ggagaatctg atatagaaaa tctggataat aaggagattc agagtaagtc tggtgatgga     2580
ggcagcaaag agaaaataaa gcaatctagc tcatctgaat gcagtactgt tgatattgct     2640
atctctgaag aagaagaaat gttctatgga ggtgaaagat caaagcatct gaaaaatggt     2700
tgcagacgcg gatcttcact tggtcaaatc agtggagcat ccaagaaagg aaaaatctgg     2760
cagaacatca ggaaaacctg ctgcaagatt gtagagaaca attggtttaa gtgttttatt     2820
gggcttgtta ctctgctcag cactggcact ctggcttttg aagatatata tatggatcag     2880
agaaagacaa ttaaaatttt attagaatat gctgacatga tctttactta tatcttcatt     2940
ctggaaatgc ttctaaaatg gatggcatat ggttttaagg cctatttctc taatggctgg     3000
tacaggctgg acttcgtggt tgttattgtg ttttgtctta gcttaatagg caaaactcgg     3060
gaagaactaa aacctcttat ttccatgaaa ttccttcggc ccctcagagt tctatctcaa     3120
tttgaaagaa tgaaggtggt tgtgagagct ttgatcaaaa caaccttacc cactttgaat     3180
gtgtttcttg tctgcctgat gatctggctg atttttagta tcatgggagt agacttattt     3240
gctggcagat tctatgaatg cattgaccca acaagtggag aaaggtttcc ttcatctgaa     3300
gtcatgaata agagtcggtg tgaaagcctt ctgtttaacg aatccatgct atgggaaaat     3360
gcaaaaatga actttgataa tgttggaaat ggtttccttt ctctgcttca agtagcaaca     3420
tttaatggat ggatcactat tatgaattca gcaattgatt ctgttgctgt taatatacag     3480
cctcattttg aagtcaacat ctacatgtat tgttacttta tcaactttat tatatttgga     3540
gtatttctcc ctctgagtat gctgattact gttattattg ataatttcaa caagcataaa     3600
```

```
ataaagctgg gaggctcaaa tatctttata acggttaaac agagaaaaca gtaccgcagg      3660
ctgaagaagc taatgtatga ggattctcaa agaccagtac ctcgcccatt aaacaagctc      3720
caaggattca tctttgatgt ggtaacaagc caagctttta atgtcattgt tatggttctt      3780
atatgtttcc aagcaatagc catgatgata gacactgatg ttcagagtct acaaatgtcc      3840
attgctctct actggattaa ctcaattttt gttatgctat atactatgga atgtatactg      3900
aagctcatcg ctttccgttg tttttatttc accattgcgt ggaacatttt tgattttatg      3960
gtggttattt tctccatcac aggactatgt ctgcctatga cagtaggatc ctaccttgtg      4020
cctccttcac ttgtgcaact gatacttctc tcacggatca ttcacatgct gcgtcttgga      4080
aaaggaccaa aggtgtttca taatctgatg cttcctttga tgctgtccct cccagcatta      4140
ttgaacatca ttcttctcat cttcctggtc atgttcatct atgccgtatt tggaatgtat      4200
aattttgcct atgttaaaaa agaagctgga attaatgatg tgtctaattt tgaaaccttt      4260
ggcaacagta tgctctgtct tttcaagtt gcaatatttg ctggttggga tgggatgctt      4320
gatgcaattt tcaacagtaa atggtctgac tgtgatcctg ataaaattaa ccctgggact      4380
caagttagag gagattgtgg gaacccctct gttgggattt tttattttgt cagttatatc      4440
ctcatatcat ggctgatcat tgtaaatatg tacattgttg ttgtcatgga gtttttaaat      4500
attgcttcta agaagaaaaa caagaccttg agtgaagatg attttaggaa attctttcag      4560
gtatggaaaa ggtttgatcc tgataggacc cagtacatag actctagcaa gctttcagat      4620
tttgcagctg ctcttgatcc tcctcttttc atggcaaaac caaacaaggg ccagctcatt      4680
gctttggacc tccccatggc tgttggggac agaattcatt gcctcgatat cttacttgct      4740
tttacaaaga gagttatggg tcaagatgtg aggatggaga aagttgtttc agaaatagaa      4800
tcagggtttt tgttagccaa ccctttttaag atcacatgtg agccaattac gactactttg      4860
aaacgaaaac aagaggcagt ttcagcaacc atcattcaac gtgcttataa aaattaccgc      4920
ttgaggcgaa atgacaaaaa tacatcagat attcatatga tagatggtga cagagatgtt      4980
catgctacta aagaaggtgc ctattttgac aaagctaagg aaaagtcacc tattcaaagc      5040
cagatctaa                                                              5049
```

<210> 12
<211> 5943
<212> DNA
<213> Homo sapiens

<400> 12

```
atggcagcgc ggctgcttgc accaccaggc cctgatagtt tcaagccttt cacccctgag     60
tcactggcaa acattgagag gcgcattgct gagagcaagc tcaagaaacc accaaaggcc    120
gatggcagtc atcgggagga cgatgaggac agcaagccca agccaaacag cgacctggaa    180
gcagggaaga gtttgccttt catctacggg gacatccccc aaggcctggt tgcagttccc    240
```

```
ctggaggact ttgacccata ctatttgacg cagaaaacct ttgtagtatt aaacagaggg      300
aaaactctct tcagatttag tgccacgcct gccttgtaca ttttaagtcc ttttaacctg      360
ataagaagaa tagctattaa aattttgata cattcagtat ttagcatgat cattatgtgc      420
actattttga ccaactgtgt attcatgact tttagtaacc ctcctgactg gtcgaagaat      480
gtggagtaca cgttcacagg gatttataca tttgaatcac tagtgaaaat cattgcaaga      540
ggtttctgca tagatggctt tacctttta cgggacccat ggaactggtt agatttcagt      600
gtcatcatga tggcgtatat aacagagttt gtaaacctag gcaatgtttc agctctacgc      660
actttcaggg tactgagggc tttgaaaact atttcggtaa tcccaggcct gaagacaatt      720
gtgggtgccc tgattcagtc tgtgaagaaa ctgtcagatg tgatgatcct gacagtgttc      780
tgcctgagtg tttttgcctt gatcggactg cagctgttca tggggaacct tcgaaacaag      840
tgtgttgtgt ggcccataaa cttcaacgag agctatcttg aaaatggcac caaaggcttt      900
gattgggaag agtatatcaa caataaaaca aatttctaca cagttcctgg catgctggaa      960
cctttactct gtgggaacag ttctgatgct gggcaatgcc cagagggata ccagtgtatg     1020
aaagcaggaa ggaaccccaa ctatggttac acaagttttg acacttttag ctgggccttc     1080
ttggcattat ttcgccttat gacccaggac tattgggaaa acttgtatca attgacttta     1140
cgagcagccg ggaaaacata catgatcttc ttcgtcttgg tcatctttgt gggttctttc     1200
tatctggtga acttgatctt ggctgtggtg gccatggctt atgaagaaca gaatcaggca     1260
acactggagg aggcagaaca aaaagaggct gaatttaaag caatgttgga gcaacttaag     1320
aagcaacagg aagaggcaca ggctgctgcg atggccactt cagcaggaac tgtctcagaa     1380
gatgccatag aggaagaagg tgaagaagga gggggctccc ctcggagctc ttctgaaatc     1440
tctaaactca gctcaaagag tgcaaaggaa agacgtaaca ggagaaagaa gaggaagcaa     1500
aaggaactct ctgaaggaga ggagaaaggg gatcccgaga aggtgtttaa gtcagagtca     1560
gaagatggca tgagaaggaa ggcctttcgg ctgccagaca acagaatagg gaggaaattt     1620
tccatcatga atcagtcact gctcagcatc ccaggctcgc ccttcctctc ccgccacaac     1680
agcaagagca gcatcttcag tttcagggga cctggcggt tccgagaccc gggctccgag      1740
aatgagttcg cggatgacga gcacagcacg gtggaggaga gcgagggccg ccgggactcc     1800
ctcttcatcc ccatccgggc ccgcgagcgc cggagcagct acagcggcta cagcggctac     1860
agccagggca gccgctcctc gcgcatcttc cccagcctgc ggcgcagcgt gaagcgcaac     1920
agcacggtgg actgcaacgg cgtggtgtcc ctcatcggcg gccccggctc ccacatcggc     1980
gggcgtctcc tgccagaggc tacaactgag gtggaaatta agaagaaagg ccctggatct     2040
cttttagttt ccatggacca attagcctcc tacgggcgga aggacagaat caacagtata     2100
atgagtgttg ttacaaatac actagtagaa gaactggaag agtctcagag aaagtgcccg     2160
```

ccatgctggt ataaatttgc caacactttc ctcatctggg agtgccaccc ctactggata 2220
aaactgaaag agattgtgaa cttgatagtt atggaccctt ttgtggattt agccatcacc 2280
atctgcatcg tcctgaatac actgtttatg gcaatggagc accatcctat gacaccacaa 2340
tttgaacatg tcttggctgt aggaaatctg gttttcactg gaattttcac agcggaaatg 2400
ttcctgaagc tcatagccat ggatccctac tattatttcc aagaaggttg gaacattttt 2460
gacggattta ttgtctccct cagtttaatg gaactgagtc tagcagacgt ggaggggctt 2520
tcagtgctgc gatctttccg attgctccga gtcttcaaat tggccaaatc ctggcccacc 2580
ctgaacatgc taatcaagat tattggaaat tcagtgggtg ccctgggcaa cctgacactg 2640
gtgctggcca ttattgtctt catctttgcc gtggtgggga tgcaactctt tggaaaaagc 2700
tacaaagagt gtgtctgcaa gatcaaccag gactgtgaac tccctcgctg gcatatgcat 2760
gactttttcc attccttcct cattgtcttt cgagtgttgt gcggggagtg gattgagacc 2820
atgtgggact gcatggaagt ggcaggccag gccatgtgcc tcattgtctt tatgatggtc 2880
atggtgattg gcaacttggt ggtgctgaac ctgtttctgg ccttgctcct gagctccttc 2940
agtgcagaca acctggctgc cacagatgac gatggggaaa tgaacaacct ccagatctca 3000
gtgatccgta tcaagaaggg tgtggcctgg accaaactaa aggtgcacgc cttcatgcag 3060
gcccacttta agcagcgtga ggctgatgag gtgaagcctc tggatgagtt gtatgaaaag 3120
aaggccaact gtatcgccaa tcacaccggt gcagacatcc accggaatgg tgacttccag 3180
aagaatggca atggcacaac cagcggcatt ggcagcagcg tggagaagta catcattgat 3240
gaggaccaca tgtccttcat caacaacccc aacttgactg tacgggtacc cattgctgtg 3300
ggcgagtctg actttgagaa cctcaacaca gaggatgtta gcagcgagtc ggatcctgaa 3360
ggcagcaaag ataaactaga tgacaccagc tcctctgaag gaagcaccat tgatatcaaa 3420
ccagaagtag aagaggtccc tgtggaacag cctgaggaat acttggatcc agatgcctgc 3480
ttcacagaag gttgtgtcca gcggttcaag tgctgccagg tcaacatcga ggaagggcta 3540
ggcaagtctt ggtggatcct gcggaaaacc tgcttcctca tcgtggagca caactggttt 3600
gagaccttca tcatcttcat gattctgctg agcagtggcg ccctggcctt cgaggacatc 3660
tacattgagc agagaaagac catccgcacc atcctggaat atgctgacaa agtcttcacc 3720
tatatcttca tcctggagat gttgctcaag tggacagcct atggcttcgt caagttcttc 3780
accaatgcct ggtgttggct ggacttcctc attgtggctg tctctttagt cagccttata 3840
gctaatgccc tgggctactc ggaactaggt gccataaagt cccttaggac cctaagagct 3900
ttgagaccct taagagcctt atcacgattt gaagggatga gggtggtggt gaatgccttg 3960
gtgggcgcca tcccctccat catgaatgtg ctgctggtgt gtctcatctt ctggctgatt 4020
ttcagcatca tgggagttaa cttgtttgcg ggaaagtacc actactgctt taatgagact 4080
tctgaaatcc gatttgaaat tgaagatgtc aacaataaaa ctgaatgtga aaagcttatg 4140

gaggggaaca atacagagat cagatggaag aacgtgaaga tcaactttga caatgttggg 4200

gcaggatacc tggcccttct tcaagtagca accttcaaag gctggatgga catcatgtat 4260

gcagctgtag attcccggaa gcctgatgag cagcctaagt atgaggacaa tatctacatg 4320

tacatctatt ttgtcatctt catcatcttc ggctccttct tcaccctgaa cctgttcatt 4380

ggtgtcatca ttgataactt caatcaacaa aagaaaaagt tcggaggtca ggacatcttc 4440

atgaccgaag aacagaagaa gtactacaat gccatgaaaa agctgggctc aaagaagcca 4500

cagaaaccta ttccccgccc cttgaacaaa atccaaggaa tcgtctttga ttttgtcact 4560

cagcaagcct ttgacattgt tatcatgatg ctcatctgcc ttaacatggt gacaatgatg 4620

gtggagacag acactcaaag caagcagatg gagaacatcc tctactggat taacctggtg 4680

tttgttatct tcttcacctg tgagtgtgtg ctcaaaatgt ttgcgttgag gcactactac 4740

ttcaccattg gctggaacat cttcgacttc gtggtagtca tcctctccat tgtgggaatg 4800

ttcctggcag atataattga gaaatacttt gtttccccaa ccctattccg agtcatccga 4860

ttggcccgta ttgggcgcat cttgcgtctg atcaaaggcg ccaaagggat tcgtaccctg 4920

ctctttgcct taatgatgtc cttgcctgcc ctgttcaaca tcggccttct gctcttcctg 4980

gtcatgttca tcttctccat ttttgggatg tccaattttg catatgtgaa gcacgaggct 5040

ggtatcgatg acatgttcaa ctttgagaca tttggcaaca gcatgatctg cctgtttcaa 5100

atcacaacct cagctggttg ggatggcctg ctgctgccca tcctaaaccg ccccccctgac 5160

tgcagcctag ataaggaaca cccagggagt ggctttaagg gagattgtgg gaacccctca 5220

gtgggcatct tcttctttgt aagctacatc atcatctctt tcctaattgt cgtgaacatg 5280

tacattgcca tcatcctgga gaacttcagt gtagccacag aggaaagtgc agaccctctg 5340

agtgaggatg actttgagac cttctatgag atctgggaga agttcgaccc cgatgccacc 5400

cagttcattg agtactgtaa gctggcagac tttgcagatg ccttggagca tcctctccga 5460

gtgcccaagc ccaataccat tgagctcatc gctatggatc tgccaatggt gagcggggat 5520

cgcatccact gcttggacat cctttttgcc ttcaccaagc gggtcctggg agatagcggg 5580

gagttggaca tcctgcggca gcagatggaa gagcggttcg tggcatccaa tccttccaaa 5640

gtgtcttacg agccaatcac aaccacactg cgtcgcaagc aggaggaggt atctgcagtg 5700

gtcctgcagc gtgcctaccg gggacatttg gcaaggcggg gcttcatctg caaaaagaca 5760

acttctaata agctggagaa tggaggcaca caccgggaga aaaaagagag caccccatct 5820

acagcctccc tcccgtccta tgacagtgta actaaacctg aaaaggagaa acagcagcgg 5880

gcagaggaag gaagaaggga aagagccaaa agacaaaaag aggtcagaga atccaagtgt 5940

tag 5943

<210> 13
<211> 5934
<212> DNA
<213> Homo sapiens

<400> 13

atggcaatgt tgcctccccc aggacctcag agctttgtcc atttcacaaa acagtctctt 60

gccctcattg aacaacgcat tgctgaaaga aaatcaaagg aacccaaaga agaaaagaaa 120

gatgatgatg aagaagcccc aaagccaagc agtgacttgg aagctggcaa acaactgccc 180

ttcatctatg gggacattcc tcccggcatg gtgtcagagc ccctggagga cttggacccc 240

tactatgcag acaaaaagac tttcatagta ttgaacaaag ggaaaacaat cttccgtttc 300

aatgccacac ctgctttata tatgctttct cctttcagtc ctctaagaag aatatctatt 360

aagattttag tacactcctt attcagcatg ctcatcatgt gcactattct gacaaactgc 420

atatttatga ccatgaataa cccgccggac tggaccaaaa atgtcgagta cacttttact 480

ggaatatata cttttgaatc acttgtaaaa atccttgcaa gaggcttctg tgtaggagaa 540

ttcacttttc ttcgtgaccc gtggaactgg ctggattttg tcgtcattgt ttttgcgtat 600

ttaacagaat ttgtaaacct aggcaatgtt tcagctcttc gaactttcag agtattgaga 660

gctttgaaaa ctatttctgt aatcccaggc ctgaagacaa ttgtaggggc tttgatccag 720

tcagtgaaga agctttctga tgtcatgatc ctgactgtgt tctgtctgag tgtgtttgca 780

ctaattggac tacagctgtt catgggaaac ctgaagcata aatgttttcg aaattcactt 840

gaaaataatg aaacattaga aagcataatg aataccctag agagtgaaga agactttaga 900

aaatattttt attacttgga aggatccaaa gatgctctcc tttgtggttt cagcacagat 960

tcaggtcagt gtccagaggg gtacacctgt gtgaaaattg gcagaaaccc tgattatggc 1020

tacacgagct ttgacacttt cagctgggcc ttcttagcct tgtttaggct aatgacccaa 1080

gattactggg aaaaccttta ccaacagacg ctgcgtgctg ctggcaaaac ctacatgatc 1140

ttctttgtcg tagtgatttt cctgggctcc ttttatctaa taaacttgat cctggctgtg 1200

gttgccatgg catatgaaga acagaaccag gcaaacattg aagaagctaa acagaaagaa 1260

ttagaatttc aacagatgtt agaccgtctt aaaaaagagc aagaagaagc tgaggcaatt 1320

gcagcggcag cggctgaata tacaagtatt aggagaagca gaattatggg cctctcagag 1380

agttcttctg aaacatccaa actgagctct aaaagtgcta aagaaagaag aaacagaaga 1440

aagaaaaaga atcaaaagaa gctctccagt ggagaggaaa agggagatgc tgagaaattg 1500

tcgaaatcag aatcagagga cagcatcaga agaaaaagtt tccaccttgg tgtcgaaggg 1560

cataggcgag cacatgaaaa gaggttgtct acccccaatc agtcaccact cagcattcgt 1620

ggctccttgt tttctgcaag gcgaagcagc agaacaagtc tttttagttt caaaggcaga 1680

ggaagagata taggatctga gactgaattt gccgatgatg agcacagcat tttggagac 1740

aatgagagca gaaggggctc actgtttgtg ccccacagac cccaggagcg acgcagcagt 1800

aacatcagcc aagccagtag gtccccacca atgctgccgg tgaacgggaa aatgcacagt 1860
gctgtggact gcaacggtgt ggtctccctg gttgatggac gctcagccct catgctcccc 1920
aatggacagc ttctgccaga gggcacgacc aatcaaatac acaagaaaag gcgttgtagt 1980
tcctatctcc tttcagagga tatgctgaat gatcccaacc tcagacagag agcaatgagt 2040
agagcaagca tattaacaaa cactgtggaa gaacttgaag agtccagaca aaaatgtcca 2100
ccttggtggt acagatttgc acacaaattc ttgatctgga attgctctcc atattggata 2160
aaattcaaaa agtgtatcta ttttattgta atggatcctt ttgtagatct tgcaattacc 2220
atttgcatag ttttaaacac attatttatg gctatggaac accacccaat gactgaggaa 2280
ttcaaaaatg tacttgctat aggaaatttg gtctttactg gaatctttgc agctgaaatg 2340
gtattaaaac tgattgccat ggatccatat gagtatttcc aagtaggctg gaatattttt 2400
gacagcctta ttgtgacttt aagtttagtg gagctctttc tagcagatgt ggaaggattg 2460
tcagttctgc gatcattcag actgctccga gtcttcaagt tggcaaaatc ctggccaaca 2520
ttgaacatgc tgattaagat cattggtaac tcagtagggg ctctaggtaa cctcacctta 2580
gtgttggcca tcatcgtctt catttttgct gtggtcggca tgcagctctt tggtaagagc 2640
tacaaagaat gtgtctgcaa gatcaatgat gactgtacgc tcccacggtg gcacatgaac 2700
gacttcttcc actccttcct gattgtgttc cgcgtgctgt gtggagagtg gatagagacc 2760
atgtgggact gtatggaggt cgctggtcaa gctatgtgcc ttattgttta catgatggtc 2820
atggtcattg gaaacctggt ggtcctaaac ctatttctgg ccttattatt gagctcattt 2880
agttcagaca atcttacagc aattgaagaa gaccctgatg caaacaacct ccagattgca 2940
gtgactagaa ttaaaaaggg aataaattat gtgaaacaaa ccttacgtga atttattcta 3000
aaagcatttt ccaaaaagcc aaagatttcc agggagataa gacaagcaga agatctgaat 3060
actaagaagg aaaactatat ttctaaccat acacttgctg aaatgagcaa aggtcacaat 3120
ttcctcaagg aaaaagataa aatcagtggt tttggaagca gcgtggacaa acacttgatg 3180
gaagacagtg atggtcaatc atttattcac aatcccagcc tcacagtgac agtgccaatt 3240
gcacctgggg aatccgattt ggaaaatatg aatgctgagg aacttagcag tgattcggat 3300
agtgaataca gcaaagtgag attaaaccgg tcaagctcct cagagtgcag cacagttgat 3360
aacccctttgc ctggagaagg agaagaagca gaggctgaac ctatgaattc cgatgagcca 3420
gaggcctgtt tcacagatgg ttgtgtacgg aggttctcat gctgccaagt taacatagag 3480
tcagggaaag gaaaaatctg gtggaacatc aggaaaacct gctacaagat tgttgaacac 3540
agttggtttg aaagcttcat tgtcctcatg atcctgctca gcagtggtgc cctggctttt 3600
gaagatattt atattgaaag gaaaaagacc attaagatta tcctggagta tgcagacaag 3660
atcttcactt acatcttcat tctggaaatg cttctaaaat ggatagcata tggttataaa 3720
acatatttca ccaatgcctg gtgttggctg gatttcctaa ttgttgatgt ttctttggtt 3780

actttagtgg caaacactct tggctactca gatcttggcc ccattaaatc ccttcggaca 3840
ctgagagctt taagacctct aagagcctta tctagatttg aaggaatgag ggtcgttgtg 3900
aatgcactca taggagcaat tccttccatc atgaatgtgc tacttgtgtg tcttatattc 3960
tggctgatat tcagcatcat gggagtaaat ttgtttgctg gcaagttcta tgagtgtatt 4020
aacaccacag atgggtcacg gtttcctgca agtcaagttc caaatcgttc cgaatgtttt 4080
gcccttatga atgttagtca aaatgtgcga tggaaaaacc tgaaagtgaa ctttgataat 4140
gtcggacttg gttacctatc tctgcttcaa gttgcaactt ttaagggatg gacgattatt 4200
atgtatgcag cagtggattc tgttaatgta gacaagcagc ccaaatatga atatagcctc 4260
tacatgtata tttattttgt cgtctttatc atctttgggt cattcttcac tttgaacttg 4320
ttcattggtg tcatcataga taatttcaac caacagaaaa agaagcttgg aggtcaagac 4380
atctttatga cagaagaaca gaagaaatac tataatgcaa tgaaaaagct ggggtccaag 4440
aagccacaaa agccaattcc tcgaccaggg aacaaaatcc aaggatgtat atttgaccta 4500
gtgacaaatc aagcctttga tattagtatc atggttctta tctgtctcaa catggtaacc 4560
atgatggtag aaaaggaggg tcaaagtcaa catatgactg aagttttata ttggataaat 4620
gtggttttta taatcctttt cactggagaa tgtgtgctaa aactgatctc cctcagacac 4680
tactacttca ctgtaggatg gaatattttt gattttgtgg ttgtgattat ctccattgta 4740
ggtatgtttc tagctgattt gattgaaacg tattttgtgt cccctaccct gttccgagtg 4800
atccgtcttg ccaggattgg ccgaatccta cgtctagtca aggagcaaa ggggatccgc 4860
acgctgctct ttgctttgat gatgtccctt cctgcgttgt ttaacatcgg cctcctgctc 4920
ttcctggtca tgttcatcta cgccatcttt ggaatgtcca actttgccta tgttaaaaag 4980
gaagatggaa ttaatgacat gttcaatttt gagacctttg gcaacagtat gatttgcctg 5040
ttccaaatta caacctctgc tggctgggat ggattgctag cacctattct taacagtaag 5100
ccacccgact gtgacccaaa aaaagttcat cctggaagtt cagttgaagg agactgtggt 5160
aacccatctg ttggaatatt ctactttgtt agttatatca tcatatcctt cctggttgtg 5220
gtgaacatgt acattgcagt catactggag aattttagtg ttgccactga agaaagtact 5280
gaacctctga gtgaggatga ctttgagatg ttctatgagg tttgggagaa gtttgatccc 5340
gatgcgaccc agtttataga gttctctaaa ctctctgatt ttgcagctgc cctggatcct 5400
cctcttctca tagcaaaacc caacaaagtc cagctcattg ccatggatct gcccatggtt 5460
agtggtgacc ggatccattg tcttgacatc ttatttgctt ttacaaagcg tgttttgggt 5520
gagagtgggg agatggattc tcttcgttca cagatggaag aaaggttcat gtctgcaaat 5580
ccttccaaag tgtcctatga acccatcaca accacactaa aacggaaaca agaggatgtg 5640
tctgctactg tcattcagcg tgcttataga cgttaccgct taaggcaaaa tgtcaaaaat 5700

atatcaagta tatacataaa agatggagac agagatgatg atttactcaa taaaaaagat 5760

atggcttttg ataatgttaa tgagaactca agtccagaaa aaacagatgc cacttcatcc 5820

accacctctc caccttcata tgatagtgta acaaagccag acaaagagaa atatgaacaa 5880

gacagaacag aaaaggaaga caaagggaaa gacagcaagg aaagcaaaaa atag 5934

<210> 14
<211> 5871
<212> DNA
<213> Homo sapiens

<400> 14

```
atggaattcc ccattggatc cctcgaaact aacaacttcc gtcgctttac tccggagtca      60
ctggtggaga tagagaagca aattgctgcc aagcagggaa caaagaaagc cagagagaag     120
catagggagc agaaggacca agaagagaag cctcggcccc agctggactt gaaagcctgc     180
aaccagctgc ccaagttcta tggtgagctc ccagcagaac tgatcgggga gcccctggag     240
gatctagatc cgttctacag cacacaccgg acatttatgg tgctgaacaa agggaggacc     300
atttcccggt ttagtgccac tcgggccctg tggctattca gtcctttcaa cctgatcaga     360
agaacggcca tcaaagtgtc tgtccactcg tggttcagtt tatttattac ggtcactatt     420
ttggttaatt gtgtgtgcat gacccgaact gaccttccag agaaaattga atatgtcttc     480
actgtcattt acacctttga agccttgata aagatactgg caagaggatt ttgtctaaat     540
gagttcacgt acctgagaga tccttggaac tggctggatt ttagcgtcat taccctggca     600
tatgttggca cagcaataga tctccgtggg atctcaggcc tgcggacatt cagagttctt     660
agagcattaa aaacagtttc tgtgatccca ggcctgaagg tcattgtggg ggccctgatt     720
cactcagtga agaaactggc tgatgtgacc atcctcacca tcttctgcct aagtgttttt     780
gccttggtgg ggctgcaact cttcaagggc aacctcaaaa ataaatgtgt caagaatgac     840
atggctgtca atgagacaac caactactca tctcacagaa aaccagatat ctacataaat     900
aagcgaggca cttctgaccc cttactgtgt ggcaatggat ctgactcagg ccactgccct     960
gatggttata tctgccttaa aacttctgac aacccggatt ttaactacac cagctttgat    1020
tcctttgctt gggctttcct ctcactgttc cgcctcatga cacaggattc ctgggaacgc    1080
ctctaccagc agaccctgag gacttctggg aaaatctata tgatcttttt tgtgctcgta    1140
atcttcctgg gatctttcta cctggtcaac ttgatcttgg ctgtagtcac catggcgtat    1200
gaggagcaga accaggcaac cactgatgaa attgaagcaa aggagaagaa gttccaggag    1260
gccctcgaga tgctccggaa ggagcaggag gtgctagcag cactagggat tgacacaacc    1320
tctctccact cccacaatgg atcaccttta acctccaaaa atgccagtga gagaaggcat    1380
agaataaagc caagagtgtc agagggctcc acagaagaca acaaatcacc ccgctctgat    1440
ccttacaacc agcgcaggat gtcttttcta ggcctcgcct ctggaaaacg ccgggctagt    1500
```

catggcagtg tgttccattt ccggtcccct ggccgagata tctcactccc tgagggagtc 1560
acagatgatg gagtctttcc tggagaccac gaaagccatc ggggctctct gctgctgggt 1620
gggggtgctg gccagcaagg ccccctccct agaagccctc ttcctcaacc cagcaaccct 1680
gactccaggc atggagaaga tgaacaccaa ccgccgccca ctagtgagct tgcccctgga 1740
gctgtcgatg tctcggcatt cgatgcagga caaaagaaga ctttcttgtc agcagaatac 1800
ttagatgaac ctttccgggc ccaaagggca atgagtgttg tcagtatcat aacctccgtc 1860
cttgaggaac tcgaggagtc tgaacagaag tgcccaccct gcttgaccag cttgtctcag 1920
aagtatctga tctgggattg ctgccccatg tgggtgaagc tcaagacaat tctctttggg 1980
cttgtgacgg atccctttgc agagctcacc atcaccttgt gcatcgtggt gaacaccatc 2040
ttcatggcca tggagcacca tggcatgagc cctaccttcg aagccatgct ccagataggc 2100
aacatcgtct ttaccatatt ttttactgct gaaatggtct tcaaaatcat tgccttcgac 2160
ccatactatt atttccagaa gaagtggaat atctttgact gcatcatcgt cactgtgagt 2220
ctgctagagc tgggcgtggc caagaaggga agcctgtctg tgctgcggag cttccgcttg 2280
ctgcgcgtat tcaagctggc caaatcctgg cccaccttaa acacactcat caagatcatc 2340
ggaaactcag tgggggcact ggggaacctc accatcatcc tggccatcat tgtctttgtc 2400
tttgctctgg ttggcaagca gctcctaggg gaaaactacc gtaacaaccg aaaaaatatc 2460
tccgcgcccc atgaagactg gccccgctgg cacatgcacg acttcttcca ctctttcctc 2520
attgtcttcc gtatcctctg tggagagtgg attgagaaca tgtgggcctg catggaagtt 2580
ggccaaaaat ccatatgcct catccttttc ttgacggtga tggtgctagg gaacctggtg 2640
gtgcttaacc tgttcatcgc cctgctattg aactctttca gtgctgacaa cctcacagcc 2700
ccggaggacg atggggaggt gaacaacctg caggtggccc tggcacggat ccaggtcttt 2760
ggccatcgta ccaaacaggc tctttgcagc ttcttcagca ggtcctgccc attcccccag 2820
cccaaggcag agcctgagct ggtggtgaaa ctcccactct ccagctccaa ggctgagaac 2880
cacattgctg ccaacactgc caggggggagc tctggagggc tccaagctcc cagaggcccc 2940
agggatgagc acagtgactt catcgctaat ccgactgtgt gggtctctgt gcccattgct 3000
gagggtgaat ctgatcttga tgacttggag gatgatggtg gggaagatgc tcagagcttc 3060
cagcaggaag tgatccccaa aggacagcag gagcagctgc agcaagtcga gaggtgtggg 3120
gaccacctga cacccaggag cccaggcact ggaacatctt ctgaggacct ggctccatcc 3180
ctgggtgaga cgtggaaaga tgagtctgtt cctcaggtcc ctgctgaggg agtggacgac 3240
acaagctcct ctgagggcag cacggtggac tgcctagatc ctgaggaaat cctgaggaag 3300
atccctgagc tggcagatga cctggaagaa ccagatgact gcttcacaga aggatgcatt 3360
cgccactgtc cctgctgcaa actggatacc accaagagtc catgggatgt gggctggcag 3420
gtgcgcaaga cttgctaccg tatcgtggag cacagctggt ttgagagctt catcatcttc 3480

atgatcctgc tcagcagtgg atctctggcc tttgaagact attacctgga ccagaagccc 3540
acggtgaaag ctttgctgga gtacactgac agggtcttca cctttatctt tgtgttcgag 3600
atgctgctta agtgggtggc ctatggcttc aaaaagtact tcaccaatgc ctggtgctgg 3660
ctggacttcc tcattgtgaa tatctcactg ataagtctca cagcgaagat tctggaatat 3720
tctgaagtgg ctcccatcaa agcccttcga acccttcgcg ctctgcggcc actgcgggct 3780
ctttctcgat ttgaaggcat gcgggtggtg gtggatgccc tggtgggcgc catcccatcc 3840
atcatgaatg tcctcctcgt ctgcctcatc ttctggctca tcttcagcat catgggtgtg 3900
aacctcttcg cagggaagtt ttggaggtgc atcaactata ccgatggaga gttttccctt 3960
gtacctttgt cgattgtgaa taacaagtct gactgcaaga ttcaaaactc cactggcagc 4020
ttcttctggg tcaatgtgaa agtcaacttt gataatgttg caatgggtta ccttgcactt 4080
ctgcaggtgg caacctttaa aggctggatg gacattatgt atgcagctgt tgattcccgg 4140
gaggtcaaca tgcaacccaa gtgggaggac aacgtgtaca tgtatttgta ctttgtcatc 4200
ttcatcattt ttggaggctt cttcacactg aatctctttg ttggggtcat aattgacaac 4260
ttcaatcaac agaaaaaaaa gttagggggc caggacatct tcatgacaga ggagcagaag 4320
aaatactaca atgccatgaa gaagttgggc tccaagaagc cccagaagcc catcccacgg 4380
cccctgaaca agttccaggg ttttgtcttt gacatcgtga ccagacaagc ttttgacatc 4440
accatcatgg tcctcatctg cctcaacatg atcaccatga tggtggagac tgatgaccaa 4500
agtgaagaaa agacgaaaat tctgggcaaa atcaaccagt tctttgtggc cgtcttcaca 4560
ggcgaatgtg tcatgaagat gttcgctttg aggcagtact acttcacaaa tggctggaat 4620
gtgtttgact tcattgtggt ggttctctcc attgcgagcc tgattttttc tgcaattctt 4680
aagtcacttc aaagttactt ctccccaacg ctcttcagag tcatccgcct ggcccgaatt 4740
ggccgcatcc tcagactgat ccgagcggcc aaggggatcc gcacactgct ctttgccctc 4800
atgatgtccc tgcctgccct cttcaacatc gggctgttgc tattccttgt catgttcatc 4860
tactctatct tcggtatgtc cagctttccc catgtgaggt gggaggctgg catcgacgac 4920
atgttcaact tccagacctt cgccaacagc atgctgtgcc tcttccagat taccacgtcg 4980
gccggctggg atggcctcct cagccccatc ctcaacacag ggccccccta ctgtgacccc 5040
aatctgccca acagcaatgg caccagaggg gactgtggga gcccagccgt aggcatcatc 5100
ttcttcacca cctacatcat catctccttc ctcatcatgg tcaacatgta cattgcagtg 5160
attctggaga acttcaatgt ggccacggag gagagcactg agcccctgag tgaggacgac 5220
tttgacatgt tctatgagac ctgggagaag tttgacccag aggccactca gtttattacc 5280
ttttctgctc tctcggactt tgcagacact ctctctggtc ccctgagaat cccaaaaccc 5340
aatcgaaata tactgatcca gatggacctg cctttggtcc ctggagataa gatccactgc 5400

```
ttggacatcc tttttgcttt caccaagaat gtcctaggag aatccgggga gttggattct     5460
ctgaaggcaa atatggagga gaagtttatg gcaactaatc tttcaaaatc atcctatgaa     5520
ccaatagcaa ccactctccg atggaagcaa gaagacattt cagccactgt cattcaaaag     5580
gcctatcgga gctatgtgct gcaccgctcc atggcactct ctaacacccc atgtgtgccc     5640
agagctgagg aggaggctgc atcactccca gatgaaggtt ttgttgcatt cacagcaaat     5700
gaaaattgtg tactcccaga caaatctgaa actgcttctg ccacatcatt cccaccgtcc     5760
tatgagagtg tcactagagg ccttagtgat agagtcaaca tgaggacatc tagctcaata     5820
caaaatgaag atgaagccac cagtatggag ctgattgccc ctgggcccta g              5871
```

<210> 15
<211> 5376
<212> DNA
<213> Homo sapiens

<400> 15

```
atggatgaca gatgctaccc agtaatcttt ccagatgagc ggaatttccg ccccttcact      60
tccgactctc tggctgcaat tgagaagcgg attgccatcc aaaaggagaa aaagaagtct     120
aaagaccaga caggagaagt accccagcct cggcctcagc ttgacctaaa ggcctccagg     180
aagttgccca agctctatgg cgacattcct cgtgagctca taggaaagcc tctggaagac     240
ttggacccat tctaccgaaa tcataagaca tttatggtgt taaacagaaa gaggacaatc     300
taccgcttca gtgccaagca tgccttgttc atttttgggc ctttcaattc aatcagaagt     360
ttagccatta gagtctcagt ccattcattg ttcagcatgt tcattatcgg caccgttatc     420
atcaactgcg tgttcatggc tacagggcct gctaaaaaca gcaacagtaa caatactgac     480
attgcagagt gtgtcttcac tgggatttat atttttgaag ctttgattaa aatattggca     540
agaggtttca ttctggatga gttttctttc cttcgagatc catggaactg gctggactcc     600
attgtcattg gaatagcgat tgtgtcatat attccaggaa tcaccatcaa actattgccc     660
ctgcgtacct tccgtgtgtt cagagctttg aaagcaattt cagtagtttc acgtctgaag     720
gtcatcgtgg gggccttgct acgctctgtg aagaagctgg tcaacgtgat tatcctcacc     780
ttcttttgcc tcagcatctt tgccctggta ggtcagcagc tcttcatggg aagtctgaac     840
ctgaaatgca tctcgaggga ctgtaaaaat atcagtaacc cggaagctta tgaccattgc     900
tttgaaaaga aagaaaattc acctgaattc aaaatgtgtg gcatctggat gggtaacagt     960
gcctgttcca tacaatatga atgtaagcac accaaaatta atcctgacta taattatacg    1020
aattttgaca actttggctg gtcttttctt gccatgttcc ggctgatgac ccaagattcc    1080
tgggagaagc tttatcaaca gaccctgcgt actactgggc tctactcagt cttcttcttc    1140
attgtggtca ttttcctggg ctccttctac ctgattaact taaccctggc tgttgttacc    1200
atggcatatg aggagcagaa caagaatgta gctgcagaga tagaggccaa ggaaaagatg    1260
```

tttcaggaag cccagcagct gttaaaggag gaaaaggagg ctctggttgc catgggaatt 1320
gacagaagtt cacttacttc ccttgaaaca tcatatttta ccccaaaaaa gagaaagctc 1380
tttggtaata agaaaaggaa gtccttcttt ttgagagagt ctgggaaaga ccagcctcct 1440
gggtcagatt ctgatgaaga ttgccaaaaa aagccacagc tcctagagca aaccaaacga 1500
ctgtcccaga atctatcact ggaccacttt gatgagcatg gagatcctct ccaaaggcag 1560
agagcactga gtgctgtcag catcctcacc atcaccatga aggaacaaga aaaatcacaa 1620
gagccttgtc tcccttgtgg agaaaacctg gcatccaagt acctcgtgtg gaactgttgc 1680
ccccagtggc tgtgcgttaa gaaggtcctg agaactgtga tgactgaccc gtttactgag 1740
ctggccatca ccatctgcat catcatcaac actgtcttct tggccatgga gcatcacaag 1800
atggaggcca gttttgagaa gatgttgaat atagggaatt tggttttcac tagcattttt 1860
atagcagaaa tgtgcctaaa aatcattgcg ctcgatccct accactactt tcgccgaggc 1920
tggaacattt ttgacagcat tgttgctctt ctgagttttg cagatgtaat gaactgtgta 1980
cttcaaaaga gaagctggcc attcttgcgt tccttcagag tgctcagggt cttcaagtta 2040
gccaaatcct ggccaacttt gaacacacta attaagataa tcggcaactc tgtcggagcc 2100
cttggaagcc tgactgtggt cctggtcatt gtgatcttta ttttctcagt agttggcatg 2160
cagcttttttg gccgtagctt caattcccaa aagagtccaa aactctgtaa cccgacaggc 2220
ccgacagtct catgtttacg gcactggcac atgggggatt tctggcactc cttcctagtg 2280
gtattccgca tcctctgcgg ggaatggatc gaaaatatgt gggaatgtat gcaagaagcg 2340
aatgcatcat catcattgtg tgttattgtc ttcatattga tcacggtgat aggaaaactt 2400
gtggtgctca acctcttcat tgccttactg ctcaattcct ttagcaatga ggaaagaaat 2460
ggaaacttag aaggagaggc caggaaaact aaagtccagt tagcactgga tcgattccgc 2520
cgggcttttt gttttgtgag acacactctt gagcatttct gtcacaagtg gtgcaggaag 2580
caaaacttac cacagcaaaa agaggtggca ggaggctgtg ctgcacaaag caaagacatc 2640
attcccctgg tcatggagat gaaaaggggc tcagagaccc aggaggagct tggtatacta 2700
acctctgtac caaagaccct gggcgtcagg catgattgga cttggttggc accacttgcg 2760
gaggaggaag atgacgttga attttctggt gaagataatg cacagcgcat cacacaacct 2820
gagcctgaac aacaggccta tgagctccat caggagaaca agaagcccac gagccagaga 2880
gttcaaagtg tggaaattga catgttctct gaagatgagc ctcatctgac catacaggat 2940
ccccgaaaga agtctgatgt taccagtata ctatcagaat gtagcaccat tgatcttcag 3000
gatggctttg gatggttacc tgagatggtt cccaaaaagc aaccagagag atgtttgccc 3060
aaaggctttg gttgctgctt tccatgctgt agcgtggaca agagaaagcc tccctgggtc 3120
atttggtgga acctgcggaa aacctgctac caaatagtga aacacagctg gtttgagagc 3180
tttattatct ttgtgattct gctgagcagt ggggcactga tatttgaaga tgttcacctt 3240

gagaaccaac ccaaaatcca agaattacta aattgtactg acattatttt tacacatatt 3300
tttatcctgg agatggtact aaaatgggta gccttcggat ttggaaagta tttcaccagt 3360
gcctggtgct gccttgattt catcattgtg attgtctctg tgaccaccct cattaactta 3420
atggaattga agtccttccg gactctacga gcactgaggc ctcttcgtgc gctgtcccag 3480
tttgaaggaa tgaaggtggt ggtcaatgct ctcataggtg ccatacctgc cattctgaat 3540
gttttgcttg tctgcctcat tttctggctc gtattttgta ttctgggagt atacttcttt 3600
tctggaaaat ttgggaaatg cattaatgga acagactcag ttataaatta taccatcatt 3660
acaaataaaa gtcaatgtga aagtggcaat ttctcttgga tcaaccagaa agtcaacttt 3720
gacaatgtgg gaaatgctta cctcgctctg ctgcaagtgg caacatttaa gggctggatg 3780
gatattatat atgcagctgt tgattccaca gagaaagaac aacagccaga gtttgagagc 3840
aattcactcg gttacattta cttcgtagtc tttatcatct ttggctcatt cttcactctg 3900
aatctcttca ttggcgttat cattgacaac ttcaaccaac agcagaaaaa gttaggtggc 3960
caagacattt ttatgacaga agaacagaag aaatactata atgcaatgaa aaaattagga 4020
tccaaaaaac ctcaaaaacc cattccacgg cctctgaaca aatgtcaagg tctcgtgttc 4080
gacatagtca caagccagat ctttgacatc atcatcataa gtctcattat cctaaacatg 4140
attagcatga tggctgaatc atacaaccaa cccaaagcca tgaaatccat ccttgaccat 4200
ctcaactggg tctttgtggt catctttacg ttagaatgtc tcatcaaaat ctttgctttg 4260
aggcaatact acttcaccaa tggctggaat ttatttgact gtgtggtcgt gcttctttcc 4320
attgttagta caatgatttc taccttggaa aatcaggagc acattccttt ccctccgacg 4380
ctcttcagaa ttgtccgctt ggctcggatt ggccgaatcc tgaggcttgt ccgggctgca 4440
cgaggaatca ggactctcct ctttgctctg atgatgtcgc ttccttctct gttcaacatt 4500
ggtcttctac tctttctgat tatgtttatc tatgccattc tgggtatgaa ctggttttcc 4560
aaagtgaatc cagagtctgg aatcgatgac atattcaact tcaagacttt tgccagcagc 4620
atgctctgtc tcttccagat aagcacatca gcaggttggg attccctgct cagccccatg 4680
ctgcgatcaa aagaatcatg taactcttcc tcagaaaact gccacctccc tggcatagcc 4740
acatcctact ttgtcagtta cattatcatc tcctttctca ttgttgtcaa catgtacatt 4800
gctgtgattt tagagaactt caatacagcc actgaagaaa gtgaggaccc tttgggtgaa 4860
gatgactttg acatatttta tgaagtgtgg gaaaagtttg acccagaagc aacacaattt 4920
atcaaatatt ctgccctttc tgactttgct gatgccttgc ctgagccttt gcgtgtcgca 4980
aagccaaata aatatcaatt tctagtaatg gacttgccca tggtgagtga agatcgcctc 5040
cactgcatgg atattctttt cgccttcacc gctagggtac tcggtggctc tgatggccta 5100
gatagtatga aagcaatgat ggaagagaag ttcatggaag ccaatcctct caagaagttg 5160

tatgaaccca tagtcaccac caccaagaga aaggaagagg aaagaggtgc tgctattatt 5220

caaaaggcct ttcgaaagta catgatgaag gtgaccaagg gtgaccaagg tgaccaaaat 5280

gacttggaaa acgggcctca ttcaccactc cagactcttt gcaatggaga cttgtctagc 5340

tttggggtgg ccaagggcaa ggtccactgt gactga 5376

<210> 16
<211> 657
<212> DNA
<213> Homo sapiens

<400> 16

atggggaggc tgctggcctt agtggtcggc gcggcactgg tgtcctcagc ctgcgggggc 60

tgcgtggagg tggactcgga gaccgaggcc gtgtatggga tgaccttcaa aattctttgc 120

atctcctgca agcgccgcag cgagaccaac gctgagacct tcaccgagtg gaccttccgc 180

cagaagggca ctgaggagtt tgtcaagatc ctgcgctatg agaatgaggt gttgcagctg 240

gaggaggatg agcgcttcga gggccgcgtg gtgtggaatg gcagccgggg caccaaagac 300

ctgcaggatc tgtctatctt catcaccaat gtcacctaca accactcggg cgactacgag 360

tgccacgtct accgcctgct cttcttcgaa aactacgagc acaacaccag cgtcgtcaag 420

aagatccaca ttgaggtagt ggacaaagcc aacagagaca tggcatccat cgtgtctgag 480

atcatgatgt atgtgctcat tgtggtgttg accatatggc tcgtggcaga gatgatttac 540

tgctacaaga agatcgctgc cgccacggag actgctgcac aggagaatgc ctcggaatac 600

ctggccatca cctctgaaag caaagagaac tgcacgggcg tccaggtggc cgaatag 657

<210> 17
<211> 648
<212> DNA
<213> Homo sapiens

<400> 17

atgcacagag atgcctggct acctcgccct gccttcagcc tcacggggct cagtctcttt 60
ttctctttgg tgccaccagg acggagcatg gaggtcacag tacctgccac cctcaacgtc 120
ctcaatggct ctgacgcccg cctgccctgc accttcaact cctgctacac agtgaaccac 180
aaacagttct ccctgaactg gacttaccag gagtgcaaca actgctctga ggagatgttc 240
ctccagttcc gcatgaagat cattaacctg aagctggagc ggtttcaaga ccgcgtggag 300
ttctcaggga accccagcaa gtacgatgtg tcggtgatgc tgagaaacgt gcagccggag 360
gatgagggga tttacaactg ctacatcatg aacccccctg accgccaccg tggccatggc 420
aagatccatc tgcaggtcct catggaagag ccccctgagc gggactccac ggtggccgtg 480
attgtgggtg cctccgtcgg gggcttcctg gctgtggtca tcttggtgct gatggtggtc 540
aagtgtgtga ggagaaaaaa agagcagaag ctgagcacag atgacctgaa gaccgaggag 600
gagggcaaga cggacggtga aggcaacccg gatgatggcg ccaagtag 648

<210> 18
<211> 648
<212> DNA
<213> Homo sapiens

<400> 18

atgcctgcct tcaatagatt gtttcccctg gcttctctcg tgcttatcta ctgggtcagt 60
gtctgcttcc ctgtgtgtgt ggaagtgccc tcggagacgg aggccgtgca gggcaacccc 120
atgaagctgc gctgcatctc ctgcatgaag agagaggagg tggaggccac cacggtggtg 180
gaatggttct acaggcccga gggcggtaaa gatttcctta tttacgagta tcggaatggc 240
caccaggagg tggagagccc ctttcagggg cgcctgcagt ggaatggcag caaggacctg 300
caggacgtgt ccatcactgt gctcaacgtc actctgaacg actctggcct ctacacctgc 360
aatgtgtccc gggagtttga gtttgaggcg catcggccct ttgtgaagac gacgcggctg 420
atccccctaa gagtcaccga ggaggctgga gaggacttca cctctgtggt ctcagaaatc 480
atgatgtaca tccttctggt cttcctcacc ttgtggctgc tcatcgagat gatatattgc 540
tacagaaagg tctcaaaagc cgaagaggca gcccaagaaa acgcgtctga ctaccttgcc 600
atcccatctg agaacaagga gaactctgcg gtaccagtgg aggaatag 648

<210> 19
<211> 687
<212> DNA
<213> Homo sapiens

<400> 19

```
atgcccgggg ctggggacgg aggcaaagcc ccggcgagat ggctgggcac tgggcttttg        60
ggcctcttcc tgctccccgt aaccctgtcg ctggaggtgt ctgtgggaaa ggccaccgac       120
atctacgctg tcaatggcac ggagatcctg ctgccctgca ccttctccag ctgctttggc       180
ttcgaggacc tccacttccg gtggacctac aacagcagtg acgcattcaa gattctcata       240
gaggggactg tgaagaatga gaagtctgac cccaaggtga cgttgaaaga cgatgaccgc       300
atcactctgg taggctctac taaggagaag atgaacaaca tttccattgt gctgagggac       360
ctggagttca gcgacacggg caaatacacc tgccatgtga agaaccccaa ggagaataat       420
ctccagcacc acgccaccat cttcctccaa gtcgttgata gactggaaga agtggacaac       480
acagtgacac tcatcatcct ggctgtcgtg ggcggggtca tcgggctcct catcctcatc       540
ctgctgatca agaaactcat catcttcatc ctgaagaaga ctcgggagaa gaagaaggag       600
tgtctcgtga gctcctcggg gaatgacaac acggagaacg gcttgcctgg ctccaaggca       660
gaggagaaac caccttcaaa agtgtga                                           687
```

<210> 20
<211> 1998
<212> PRT
<213> Homo sapiens

<400> 20

```
Met Glu Gln Thr Val Leu Val Pro Pro Gly Pro Asp Ser Phe Asn Phe
1               5                   10                  15

Phe Thr Arg Glu Ser Leu Ala Ala Ile Glu Arg Arg Ile Ala Glu Glu
            20                  25                  30

Lys Ala Lys Asn Pro Lys Pro Asp Lys Lys Asp Asp Asp Glu Asn Gly
        35                  40                  45

Pro Lys Pro Asn Ser Asp Leu Glu Ala Gly Lys Asn Leu Pro Phe Ile
    50                  55                  60

Tyr Gly Asp Ile Pro Pro Glu Met Val Ser Glu Pro Leu Glu Asp Leu
65                  70                  75                  80

Asp Pro Tyr Tyr Ile Asn Lys Lys Thr Phe Ile Val Leu Asn Lys Gly
                85                  90                  95

Lys Ala Ile Phe Arg Phe Ser Ala Thr Ser Ala Leu Tyr Ile Leu Thr
            100                 105                 110

Pro Phe Asn Pro Leu Arg Lys Ile Ala Ile Lys Ile Leu Val His Ser
        115                 120                 125

Leu Phe Ser Met Leu Ile Met Cys Thr Ile Leu Thr Asn Cys Val Phe
    130                 135                 140

Met Thr Met Ser Asn Pro Pro Asp Trp Thr Lys Asn Val Glu Tyr Thr
145                 150                 155                 160

Phe Thr Gly Ile Tyr Thr Phe Glu Ser Leu Ile Lys Ile Ile Ala Arg
                165                 170                 175

Gly Phe Cys Leu Glu Asp Phe Thr Phe Leu Arg Asp Pro Trp Asn Trp
            180                 185                 190

Leu Asp Phe Thr Val Ile Thr Phe Ala Tyr Val Thr Glu Phe Val Asp
        195                 200                 205

Leu Gly Asn Val Ser Ala Leu Arg Thr Phe Arg Val Leu Arg Ala Leu
    210                 215                 220

Lys Thr Ile Ser Val Ile Pro Gly Leu Lys Thr Ile Val Gly Ala Leu
225                 230                 235                 240

Ile Gln Ser Val Lys Lys Leu Ser Asp Val Met Ile Leu Thr Val Phe
                245                 250                 255
```

```
Cys Leu Ser Val Phe Ala Leu Ile Gly Leu Gln Leu Phe Met Gly Asn
            260                 265                 270

Leu Arg Asn Lys Cys Ile Gln Trp Pro Pro Thr Asn Ala Ser Leu Glu
        275                 280                 285

Glu His Ser Ile Glu Lys Asn Ile Thr Val Asn Tyr Asn Gly Thr Leu
    290                 295                 300

Ile Asn Glu Thr Val Phe Glu Phe Asp Trp Lys Ser Tyr Ile Gln Asp
305                 310                 315                 320

Ser Arg Tyr His Tyr Phe Leu Glu Gly Phe Leu Asp Ala Leu Leu Cys
                325                 330                 335

Gly Asn Ser Ser Asp Ala Gly Gln Cys Pro Glu Gly Tyr Met Cys Val
            340                 345                 350

Lys Ala Gly Arg Asn Pro Asn Tyr Gly Tyr Thr Ser Phe Asp Thr Phe
        355                 360                 365

Ser Trp Ala Phe Leu Ser Leu Phe Arg Leu Met Thr Gln Asp Phe Trp
    370                 375                 380

Glu Asn Leu Tyr Gln Leu Thr Leu Arg Ala Ala Gly Lys Thr Tyr Met
385                 390                 395                 400

Ile Phe Phe Val Leu Val Ile Phe Leu Gly Ser Phe Tyr Leu Ile Asn
                405                 410                 415

Leu Ile Leu Ala Val Val Ala Met Ala Tyr Glu Glu Gln Asn Gln Ala
            420                 425                 430

Thr Leu Glu Glu Ala Glu Gln Lys Glu Ala Glu Phe Gln Gln Met Ile
        435                 440                 445

Glu Gln Leu Lys Lys Gln Gln Glu Ala Ala Gln Gln Ala Ala Thr Ala
    450                 455                 460

Thr Ala Ser Glu His Ser Arg Glu Pro Ser Ala Ala Gly Arg Leu Ser
465                 470                 475                 480

Asp Ser Ser Ser Glu Ala Ser Lys Leu Ser Ser Lys Ser Ala Lys Glu
                485                 490                 495

Arg Arg Asn Arg Arg Lys Lys Arg Lys Gln Lys Glu Gln Ser Gly Gly
            500                 505                 510
```

Glu Glu Lys Asp Glu Asp Glu Phe Gln Lys Ser Glu Ser Glu Asp Ser
515 520 525

Ile Arg Arg Lys Gly Phe Arg Phe Ser Ile Glu Gly Asn Arg Leu Thr
530 535 540

Tyr Glu Lys Arg Tyr Ser Ser Pro His Gln Ser Leu Leu Ser Ile Arg
545 550 555 560

Gly Ser Leu Phe Ser Pro Arg Arg Asn Ser Arg Thr Ser Leu Phe Ser
565 570 575

Phe Arg Gly Arg Ala Lys Asp Val Gly Ser Glu Asn Asp Phe Ala Asp
580 585 590

Asp Glu His Ser Thr Phe Glu Asp Asn Glu Ser Arg Arg Asp Ser Leu
595 600 605

Phe Val Pro Arg Arg His Gly Glu Arg Arg Asn Ser Asn Leu Ser Gln
610 615 620

Thr Ser Arg Ser Ser Arg Met Leu Ala Val Phe Pro Ala Asn Gly Lys
625 630 635 640

Met His Ser Thr Val Asp Cys Asn Gly Val Val Ser Leu Val Gly Gly
645 650 655

Pro Ser Val Pro Thr Ser Pro Val Gly Gln Leu Leu Pro Glu Gly Thr
660 665 670

Thr Thr Glu Thr Glu Met Arg Lys Arg Arg Ser Ser Ser Phe His Val
675 680 685

Ser Met Asp Phe Leu Glu Asp Pro Ser Gln Arg Gln Arg Ala Met Ser
690 695 700

Ile Ala Ser Ile Leu Thr Asn Thr Val Glu Glu Leu Glu Glu Ser Arg
705 710 715 720

Gln Lys Cys Pro Pro Cys Trp Tyr Lys Phe Ser Asn Ile Phe Leu Ile
725 730 735

Trp Asp Cys Ser Pro Tyr Trp Leu Lys Val Lys His Val Val Asn Leu
740 745 750

Val Val Met Asp Pro Phe Val Asp Leu Ala Ile Thr Ile Cys Ile Val
755 760 765

```
Leu Asn Thr Leu Phe Met Ala Met Glu His Tyr Pro Met Thr Asp His
    770                 775                 780

Phe Asn Asn Val Leu Thr Val Gly Asn Leu Val Phe Thr Gly Ile Phe
785                 790                 795                 800

Thr Ala Glu Met Phe Leu Lys Ile Ile Ala Met Asp Pro Tyr Tyr Tyr
                805                 810                 815

Phe Gln Glu Gly Trp Asn Ile Phe Asp Gly Phe Ile Val Thr Leu Ser
            820                 825                 830

Leu Val Glu Leu Gly Leu Ala Asn Val Glu Gly Leu Ser Val Leu Arg
        835                 840                 845

Ser Phe Arg Leu Leu Arg Val Phe Lys Leu Ala Lys Ser Trp Pro Thr
    850                 855                 860

Leu Asn Met Leu Ile Lys Ile Ile Gly Asn Ser Val Gly Ala Leu Gly
865                 870                 875                 880

Asn Leu Thr Leu Val Leu Ala Ile Ile Val Phe Ile Phe Ala Val Val
                885                 890                 895

Gly Met Gln Leu Phe Gly Lys Ser Tyr Lys Asp Cys Val Cys Lys Ile
            900                 905                 910

Ala Ser Asp Cys Gln Leu Pro Arg Trp His Met Asn Asp Phe Phe His
        915                 920                 925

Ser Phe Leu Ile Val Phe Arg Val Leu Cys Gly Glu Trp Ile Glu Thr
    930                 935                 940

Met Trp Asp Cys Met Glu Val Ala Gly Gln Ala Met Cys Leu Thr Val
945                 950                 955                 960

Phe Met Met Val Met Val Ile Gly Asn Leu Val Val Leu Asn Leu Phe
                965                 970                 975

Leu Ala Leu Leu Leu Ser Ser Phe Ser Ala Asp Asn Leu Ala Ala Thr
            980                 985                 990

Asp Asp Asp Asn Glu Met Asn Asn  Leu Gln Ile Ala Val  Asp Arg Met
        995                 1000                1005

His Lys  Gly Val Ala Tyr Val  Lys Arg Lys Ile Tyr  Glu Phe Ile
    1010                1015                1020

Gln Gln  Ser Phe Ile Arg Lys  Gln Lys Ile Leu Asp  Glu Ile Lys
```

```
    1025                1030                1035

Pro Leu  Asp Asp Leu Asn Asn  Lys Lys Asp Ser Cys  Met Ser Asn
    1040                1045                1050

His Thr  Ala Glu Ile Gly Lys  Asp Leu Asp Tyr Leu  Lys Asp Val
    1055                1060                1065

Asn Gly  Thr Thr Ser Gly Ile  Gly Thr Gly Ser Ser  Val Glu Lys
    1070                1075                1080

Tyr Ile  Ile Asp Glu Ser Asp  Tyr Met Ser Phe Ile  Asn Asn Pro
    1085                1090                1095

Ser Leu  Thr Val Thr Val Pro  Ile Ala Val Gly Glu  Ser Asp Phe
    1100                1105                1110

Glu Asn  Leu Asn Thr Glu Asp  Phe Ser Ser Glu Ser  Asp Leu Glu
    1115                1120                1125

Glu Ser  Lys Glu Lys Leu Asn  Glu Ser Ser Ser Ser  Ser Glu Gly
    1130                1135                1140

Ser Thr  Val Asp Ile Gly Ala  Pro Val Glu Glu Gln  Pro Val Val
    1145                1150                1155

Glu Pro  Glu Glu Thr Leu Glu  Pro Glu Ala Cys Phe  Thr Glu Gly
    1160                1165                1170

Cys Val  Gln Arg Phe Lys Cys  Cys Gln Ile Asn Val  Glu Glu Gly
    1175                1180                1185

Arg Gly  Lys Gln Trp Trp Asn  Leu Arg Arg Thr Cys  Phe Arg Ile
    1190                1195                1200

Val Glu  His Asn Trp Phe Glu  Thr Phe Ile Val Phe  Met Ile Leu
    1205                1210                1215

Leu Ser  Ser Gly Ala Leu Ala  Phe Glu Asp Ile Tyr  Ile Asp Gln
    1220                1225                1230

Arg Lys  Thr Ile Lys Thr Met  Leu Glu Tyr Ala Asp  Lys Val Phe
    1235                1240                1245

Thr Tyr  Ile Phe Ile Leu Glu  Met Leu Leu Lys Trp  Val Ala Tyr
    1250                1255                1260

Gly Tyr  Gln Thr Tyr Phe Thr  Asn Ala Trp Cys Trp  Leu Asp Phe
    1265                1270                1275
```

Leu Ile Val Asp Val Ser Leu Val Ser Leu Thr Ala Asn Ala Leu
1280 1285 1290

Gly Tyr Ser Glu Leu Gly Ala Ile Lys Ser Leu Arg Thr Leu Arg
1295 1300 1305

Ala Leu Arg Pro Leu Arg Ala Leu Ser Arg Phe Glu Gly Met Arg
1310 1315 1320

Val Val Val Asn Ala Leu Leu Gly Ala Ile Pro Ser Ile Met Asn
1325 1330 1335

Val Leu Leu Val Cys Leu Ile Phe Trp Leu Ile Phe Ser Ile Met
1340 1345 1350

Gly Val Asn Leu Phe Ala Gly Lys Phe Tyr His Cys Ile Asn Thr
1355 1360 1365

Thr Thr Gly Asp Arg Phe Asp Ile Glu Asp Val Asn Asn His Thr
1370 1375 1380

Asp Cys Leu Lys Leu Ile Glu Arg Asn Glu Thr Ala Arg Trp Lys
1385 1390 1395

Asn Val Lys Val Asn Phe Asp Asn Val Gly Phe Gly Tyr Leu Ser
1400 1405 1410

Leu Leu Gln Val Ala Thr Phe Lys Gly Trp Met Asp Ile Met Tyr
1415 1420 1425

Ala Ala Val Asp Ser Arg Asn Val Glu Leu Gln Pro Lys Tyr Glu
1430 1435 1440

Glu Ser Leu Tyr Met Tyr Leu Tyr Phe Val Ile Phe Ile Ile Phe
1445 1450 1455

Gly Ser Phe Phe Thr Leu Asn Leu Phe Ile Gly Val Ile Ile Asp
1460 1465 1470

Asn Phe Asn Gln Gln Lys Lys Lys Phe Gly Gly Gln Asp Ile Phe
1475 1480 1485

Met Thr Glu Glu Gln Lys Lys Tyr Tyr Asn Ala Met Lys Lys Leu
1490 1495 1500

Gly Ser Lys Lys Pro Gln Lys Pro Ile Pro Arg Pro Gly Asn Lys
1505 1510 1515

```
Phe Gln  Gly Met Val Phe Asp  Phe Val Thr Arg Gln  Val Phe Asp
    1520                 1525                 1530

Ile Ser  Ile Met Ile Leu Ile  Cys Leu Asn Met Val  Thr Met Met
    1535                 1540                 1545

Val Glu  Thr Asp Asp Gln Ser  Glu Tyr Val Thr Thr  Ile Leu Ser
    1550                 1555                 1560

Arg Ile  Asn Leu Val Phe Ile  Val Leu Phe Thr Gly  Glu Cys Val
    1565                 1570                 1575

Leu Lys  Leu Ile Ser Leu Arg  His Tyr Tyr Phe Thr  Ile Gly Trp
    1580                 1585                 1590

Asn Ile  Phe Asp Phe Val Val  Val Ile Leu Ser Ile  Val Gly Met
    1595                 1600                 1605

Phe Leu  Ala Glu Leu Ile Glu  Lys Tyr Phe Val Ser  Pro Thr Leu
    1610                 1615                 1620

Phe Arg  Val Ile Arg Leu Ala  Arg Ile Gly Arg Ile  Leu Arg Leu
    1625                 1630                 1635

Ile Lys  Gly Ala Lys Gly Ile  Arg Thr Leu Leu Phe  Ala Leu Met
    1640                 1645                 1650

Met Ser  Leu Pro Ala Leu Phe  Asn Ile Gly Leu Leu  Leu Phe Leu
    1655                 1660                 1665

Val Met  Phe Ile Tyr Ala Ile  Phe Gly Met Ser Asn  Phe Ala Tyr
    1670                 1675                 1680

Val Lys  Arg Glu Val Gly Ile  Asp Asp Met Phe Asn  Phe Glu Thr
    1685                 1690                 1695

Phe Gly  Asn Ser Met Ile Cys  Leu Phe Gln Ile Thr  Thr Ser Ala
    1700                 1705                 1710

Gly Trp  Asp Gly Leu Leu Ala  Pro Ile Leu Asn Ser  Lys Pro Pro
    1715                 1720                 1725

Asp Cys  Asp Pro Asn Lys Val  Asn Pro Gly Ser Ser  Val Lys Gly
    1730                 1735                 1740

Asp Cys  Gly Asn Pro Ser Val  Gly Ile Phe Phe Phe  Val Ser Tyr
    1745                 1750                 1755
```

```
Ile Ile  Ile Ser Phe Leu Val  Val Val Asn Met Tyr  Ile Ala Val
    1760                 1765                 1770

Ile Leu  Glu Asn Phe Ser Val  Ala Thr Glu Glu Ser  Ala Glu Pro
    1775                 1780                 1785

Leu Ser  Glu Asp Asp Phe Glu  Met Phe Tyr Glu Val  Trp Glu Lys
    1790                 1795                 1800

Phe Asp  Pro Asp Ala Thr Gln  Phe Met Glu Phe Glu  Lys Leu Ser
    1805                 1810                 1815

Gln Phe  Ala Ala Ala Leu Glu  Pro Pro Leu Asn Leu  Pro Gln Pro
    1820                 1825                 1830

Asn Lys  Leu Gln Leu Ile Ala  Met Asp Leu Pro Met  Val Ser Gly
    1835                 1840                 1845

Asp Arg  Ile His Cys Leu Asp  Ile Leu Phe Ala Phe  Thr Lys Arg
    1850                 1855                 1860

Val Leu  Gly Glu Ser Gly Glu  Met Asp Ala Leu Arg  Ile Gln Met
    1865                 1870                 1875

Glu Glu  Arg Phe Met Ala Ser  Asn Pro Ser Lys Val  Ser Tyr Gln
    1880                 1885                 1890

Pro Ile  Thr Thr Thr Leu Lys  Arg Lys Gln Glu Glu  Val Ser Ala
    1895                 1900                 1905

Val Ile  Ile Gln Arg Ala Tyr  Arg Arg His Leu Leu  Lys Arg Thr
    1910                 1915                 1920

Val Lys  Gln Ala Ser Phe Thr  Tyr Asn Lys Asn Lys  Ile Lys Gly
    1925                 1930                 1935

Gly Ala  Asn Leu Leu Ile Lys  Glu Asp Met Ile Ile  Asp Arg Ile
    1940                 1945                 1950

Asn Glu  Asn Ser Ile Thr Glu  Lys Thr Asp Leu Thr  Met Ser Thr
    1955                 1960                 1965

Ala Ala  Cys Pro Pro Ser Tyr  Asp Arg Val Thr Lys  Pro Ile Val
    1970                 1975                 1980

Glu Lys  His Glu Gln Glu Gly  Lys Asp Glu Lys Ala  Lys Gly Lys
    1985                 1990                 1995
```

<210> 21

<211> 2005
<212> PRT
<213> Homo sapiens

<400> 21

```
Met Ala Gln Ser Val Leu Val Pro Pro Gly Pro Asp Ser Phe Arg Phe
1               5                   10                  15

Phe Thr Arg Glu Ser Leu Ala Ala Ile Glu Gln Arg Ile Ala Glu Glu
            20                  25                  30

Lys Ala Lys Arg Pro Lys Gln Glu Arg Lys Asp Glu Asp Asp Glu Asn
        35                  40                  45

Gly Pro Lys Pro Asn Ser Asp Leu Glu Ala Gly Lys Ser Leu Pro Phe
    50                  55                  60

Ile Tyr Gly Asp Ile Pro Pro Glu Met Val Ser Val Pro Leu Glu Asp
65                  70                  75                  80

Leu Asp Pro Tyr Tyr Ile Asn Lys Lys Thr Phe Ile Val Leu Asn Lys
                85                  90                  95

Gly Lys Ala Ile Ser Arg Phe Ser Ala Thr Pro Ala Leu Tyr Ile Leu
            100                 105                 110

Thr Pro Phe Asn Pro Ile Arg Lys Leu Ala Ile Lys Ile Leu Val His
        115                 120                 125

Ser Leu Phe Asn Met Leu Ile Met Cys Thr Ile Leu Thr Asn Cys Val
    130                 135                 140

Phe Met Thr Met Ser Asn Pro Pro Asp Trp Thr Lys Asn Val Glu Tyr
145                 150                 155                 160

Thr Phe Thr Gly Ile Tyr Thr Phe Glu Ser Leu Ile Lys Ile Leu Ala
                165                 170                 175

Arg Gly Phe Cys Leu Glu Asp Phe Thr Phe Leu Arg Asp Pro Trp Asn
            180                 185                 190

Trp Leu Asp Phe Thr Val Ile Thr Phe Ala Tyr Val Thr Glu Phe Val
        195                 200                 205

Asp Leu Gly Asn Val Ser Ala Leu Arg Thr Phe Arg Val Leu Arg Ala
    210                 215                 220

Leu Lys Thr Ile Ser Val Ile Pro Gly Leu Lys Thr Ile Val Gly Ala
225                 230                 235                 240
```

```
Leu Ile Gln Ser Val Lys Lys Leu Ser Asp Val Met Ile Leu Thr Val
                245                 250                 255

Phe Cys Leu Ser Val Phe Ala Leu Ile Gly Leu Gln Leu Phe Met Gly
            260                 265                 270

Asn Leu Arg Asn Lys Cys Leu Gln Trp Pro Pro Asp Asn Ser Ser Phe
        275                 280                 285

Glu Ile Asn Ile Thr Ser Phe Phe Asn Asn Ser Leu Asp Gly Asn Gly
    290                 295                 300

Thr Thr Phe Asn Arg Thr Val Ser Ile Phe Asn Trp Asp Glu Tyr Ile
305                 310                 315                 320

Glu Asp Lys Ser His Phe Tyr Phe Leu Glu Gly Gln Asn Asp Ala Leu
                325                 330                 335

Leu Cys Gly Asn Ser Ser Asp Ala Gly Gln Cys Pro Glu Gly Tyr Ile
            340                 345                 350

Cys Val Lys Ala Gly Arg Asn Pro Asn Tyr Gly Tyr Thr Ser Phe Asp
        355                 360                 365

Thr Phe Ser Trp Ala Phe Leu Ser Leu Phe Arg Leu Met Thr Gln Asp
    370                 375                 380

Phe Trp Glu Asn Leu Tyr Gln Leu Thr Leu Arg Ala Ala Gly Lys Thr
385                 390                 395                 400

Tyr Met Ile Phe Phe Val Leu Val Ile Phe Leu Gly Ser Phe Tyr Leu
                405                 410                 415

Ile Asn Leu Ile Leu Ala Val Val Ala Met Ala Tyr Glu Glu Gln Asn
            420                 425                 430

Gln Ala Thr Leu Glu Glu Ala Glu Gln Lys Glu Ala Glu Phe Gln Gln
        435                 440                 445

Met Leu Glu Gln Leu Lys Lys Gln Gln Glu Glu Ala Gln Ala Ala Ala
    450                 455                 460

Ala Ala Ala Ser Ala Glu Ser Arg Asp Phe Ser Gly Ala Gly Gly Ile
465                 470                 475                 480

Gly Val Phe Ser Glu Ser Ser Ser Val Ala Ser Lys Leu Ser Ser Lys
                485                 490                 495

Ser Glu Lys Glu Leu Lys Asn Arg Arg Lys Lys Lys Lys Gln Lys Glu
```

```
            500                 505                 510

Gln Ser Gly Glu Glu Glu Lys Asn Asp Arg Val Arg Lys Ser Glu Ser
        515                 520                 525

Glu Asp Ser Ile Arg Arg Lys Gly Phe Arg Phe Ser Leu Glu Gly Ser
    530                 535                 540

Arg Leu Thr Tyr Glu Lys Arg Phe Ser Ser Pro His Gln Ser Leu Leu
545                 550                 555                 560

Ser Ile Arg Gly Ser Leu Phe Ser Pro Arg Arg Asn Ser Arg Ala Ser
                565                 570                 575

Leu Phe Ser Phe Arg Gly Arg Ala Lys Asp Ile Gly Ser Glu Asn Asp
            580                 585                 590

Phe Ala Asp Asp Glu His Ser Thr Phe Glu Asp Asn Asp Ser Arg Arg
        595                 600                 605

Asp Ser Leu Phe Val Pro His Arg His Gly Glu Arg Arg His Ser Asn
    610                 615                 620

Val Ser Gln Ala Ser Arg Ala Ser Arg Val Leu Pro Ile Leu Pro Met
625                 630                 635                 640

Asn Gly Lys Met His Ser Ala Val Asp Cys Asn Gly Val Val Ser Leu
                645                 650                 655

Val Gly Gly Pro Ser Thr Leu Thr Ser Ala Gly Gln Leu Leu Pro Glu
            660                 665                 670

Gly Thr Thr Thr Glu Thr Glu Ile Arg Lys Arg Arg Ser Ser Ser Tyr
        675                 680                 685

His Val Ser Met Asp Leu Leu Glu Asp Pro Thr Ser Arg Gln Arg Ala
    690                 695                 700

Met Ser Ile Ala Ser Ile Leu Thr Asn Thr Met Glu Glu Leu Glu Glu
705                 710                 715                 720

Ser Arg Gln Lys Cys Pro Pro Cys Trp Tyr Lys Phe Ala Asn Met Cys
                725                 730                 735

Leu Ile Trp Asp Cys Cys Lys Pro Trp Leu Lys Val Lys His Leu Val
            740                 745                 750

Asn Leu Val Val Met Asp Pro Phe Val Asp Leu Ala Ile Thr Ile Cys
        755                 760                 765
```

```
Ile Val Leu Asn Thr Leu Phe Met Ala Met Glu His Tyr Pro Met Thr
    770                 775                 780

Glu Gln Phe Ser Ser Val Leu Ser Val Gly Asn Leu Val Phe Thr Gly
785                 790                 795                 800

Ile Phe Thr Ala Glu Met Phe Leu Lys Ile Ile Ala Met Asp Pro Tyr
                805                 810                 815

Tyr Tyr Phe Gln Glu Gly Trp Asn Ile Phe Asp Gly Phe Ile Val Ser
            820                 825                 830

Leu Ser Leu Met Glu Leu Gly Leu Ala Asn Val Glu Gly Leu Ser Val
        835                 840                 845

Leu Arg Ser Phe Arg Leu Leu Arg Val Phe Lys Leu Ala Lys Ser Trp
    850                 855                 860

Pro Thr Leu Asn Met Leu Ile Lys Ile Ile Gly Asn Ser Val Gly Ala
865                 870                 875                 880

Leu Gly Asn Leu Thr Leu Val Leu Ala Ile Ile Val Phe Ile Phe Ala
                885                 890                 895

Val Val Gly Met Gln Leu Phe Gly Lys Ser Tyr Lys Glu Cys Val Cys
            900                 905                 910

Lys Ile Ser Asn Asp Cys Glu Leu Pro Arg Trp His Met His Asp Phe
        915                 920                 925

Phe His Ser Phe Leu Ile Val Phe Arg Val Leu Cys Gly Glu Trp Ile
    930                 935                 940

Glu Thr Met Trp Asp Cys Met Glu Val Ala Gly Gln Thr Met Cys Leu
945                 950                 955                 960

Thr Val Phe Met Met Val Met Val Ile Gly Asn Leu Val Val Leu Asn
                965                 970                 975

Leu Phe Leu Ala Leu Leu Leu Ser Ser Phe Ser Ser Asp Asn Leu Ala
            980                 985                 990

Ala Thr Asp Asp Asp Asn Glu Met  Asn Asn Leu Gln Ile  Ala Val Gly
        995                 1000                1005

Arg Met  Gln Lys Gly Ile Asp  Phe Val Lys Arg Lys  Ile Arg Glu
    1010                1015                1020
```

```
Phe Ile  Gln Lys Ala Phe Val  Arg Lys Gln Lys Ala  Leu Asp Glu
    1025                 1030                 1035

Ile Lys  Pro Leu Glu Asp Leu  Asn Asn Lys Lys Asp  Ser Cys Ile
    1040                 1045                 1050

Ser Asn  His Thr Thr Ile Glu  Ile Gly Lys Asp Leu  Asn Tyr Leu
    1055                 1060                 1065

Lys Asp  Gly Asn Gly Thr Thr  Ser Gly Ile Gly Ser  Ser Val Glu
    1070                 1075                 1080

Lys Tyr  Val Val Asp Glu Ser  Asp Tyr Met Ser Phe  Ile Asn Asn
    1085                 1090                 1095

Pro Ser  Leu Thr Val Thr Val  Pro Ile Ala Val Gly  Glu Ser Asp
    1100                 1105                 1110

Phe Glu  Asn Leu Asn Thr Glu  Glu Phe Ser Ser Glu  Ser Asp Met
    1115                 1120                 1125

Glu Glu  Ser Lys Glu Lys Leu  Asn Ala Thr Ser Ser  Ser Glu Gly
    1130                 1135                 1140

Ser Thr  Val Asp Ile Gly Ala  Pro Ala Glu Gly Glu  Gln Pro Glu
    1145                 1150                 1155

Val Glu  Pro Glu Glu Ser Leu  Glu Pro Glu Ala Cys  Phe Thr Glu
    1160                 1165                 1170

Asp Cys  Val Arg Lys Phe Lys  Cys Cys Gln Ile Ser  Ile Glu Glu
    1175                 1180                 1185

Gly Lys  Gly Lys Leu Trp Trp  Asn Leu Arg Lys Thr  Cys Tyr Lys
    1190                 1195                 1200

Ile Val  Glu His Asn Trp Phe  Glu Thr Phe Ile Val  Phe Met Ile
    1205                 1210                 1215

Leu Leu  Ser Ser Gly Ala Leu  Ala Phe Glu Asp Ile  Tyr Ile Glu
    1220                 1225                 1230

Gln Arg  Lys Thr Ile Lys Thr  Met Leu Glu Tyr Ala  Asp Lys Val
    1235                 1240                 1245

Phe Thr  Tyr Ile Phe Ile Leu  Glu Met Leu Leu Lys  Trp Val Ala
    1250                 1255                 1260
```

Tyr Gly Phe Gln Val Tyr Phe Thr Asn Ala Trp Cys Trp Leu Asp
1265 1270 1275

Phe Leu Ile Val Asp Val Ser Leu Val Ser Leu Thr Ala Asn Ala
1280 1285 1290

Leu Gly Tyr Ser Glu Leu Gly Ala Ile Lys Ser Leu Arg Thr Leu
1295 1300 1305

Arg Ala Leu Arg Pro Leu Arg Ala Leu Ser Arg Phe Glu Gly Met
1310 1315 1320

Arg Val Val Val Asn Ala Leu Leu Gly Ala Ile Pro Ser Ile Met
1325 1330 1335

Asn Val Leu Leu Val Cys Leu Ile Phe Trp Leu Ile Phe Ser Ile
1340 1345 1350

Met Gly Val Asn Leu Phe Ala Gly Lys Phe Tyr His Cys Ile Asn
1355 1360 1365

Tyr Thr Thr Gly Glu Met Phe Asp Val Ser Val Val Asn Asn Tyr
1370 1375 1380

Ser Glu Cys Lys Ala Leu Ile Glu Ser Asn Gln Thr Ala Arg Trp
1385 1390 1395

Lys Asn Val Lys Val Asn Phe Asp Asn Val Gly Leu Gly Tyr Leu
1400 1405 1410

Ser Leu Leu Gln Val Ala Thr Phe Lys Gly Trp Met Asp Ile Met
1415 1420 1425

Tyr Ala Ala Val Asp Ser Arg Asn Val Glu Leu Gln Pro Lys Tyr
1430 1435 1440

Glu Asp Asn Leu Tyr Met Tyr Leu Tyr Phe Val Ile Phe Ile Ile
1445 1450 1455

Phe Gly Ser Phe Phe Thr Leu Asn Leu Phe Ile Gly Val Ile Ile
1460 1465 1470

Asp Asn Phe Asn Gln Gln Lys Lys Lys Phe Gly Gly Gln Asp Ile
1475 1480 1485

Phe Met Thr Glu Glu Gln Lys Lys Tyr Tyr Asn Ala Met Lys Lys
1490 1495 1500

Leu Gly Ser Lys Lys Pro Gln Lys Pro Ile Pro Arg Pro Ala Asn

1505 1510 1515

Lys Phe Gln Gly Met Val Phe Asp Phe Val Thr Lys Gln Val Phe
1520 1525 1530

Asp Ile Ser Ile Met Ile Leu Ile Cys Leu Asn Met Val Thr Met
1535 1540 1545

Met Val Glu Thr Asp Asp Gln Ser Gln Glu Met Thr Asn Ile Leu
1550 1555 1560

Tyr Trp Ile Asn Leu Val Phe Ile Val Leu Phe Thr Gly Glu Cys
1565 1570 1575

Val Leu Lys Leu Ile Ser Leu Arg Tyr Tyr Tyr Phe Thr Ile Gly
1580 1585 1590

Trp Asn Ile Phe Asp Phe Val Val Val Ile Leu Ser Ile Val Gly
1595 1600 1605

Met Phe Leu Ala Glu Leu Ile Glu Lys Tyr Phe Val Ser Pro Thr
1610 1615 1620

Leu Phe Arg Val Ile Arg Leu Ala Arg Ile Gly Arg Ile Leu Arg
1625 1630 1635

Leu Ile Lys Gly Ala Lys Gly Ile Arg Thr Leu Leu Phe Ala Leu
1640 1645 1650

Met Met Ser Leu Pro Ala Leu Phe Asn Ile Gly Leu Leu Leu Phe
1655 1660 1665

Leu Val Met Phe Ile Tyr Ala Ile Phe Gly Met Ser Asn Phe Ala
1670 1675 1680

Tyr Val Lys Arg Glu Val Gly Ile Asp Asp Met Phe Asn Phe Glu
1685 1690 1695

Thr Phe Gly Asn Ser Met Ile Cys Leu Phe Gln Ile Thr Thr Ser
1700 1705 1710

Ala Gly Trp Asp Gly Leu Leu Ala Pro Ile Leu Asn Ser Gly Pro
1715 1720 1725

Pro Asp Cys Asp Pro Asp Lys Asp His Pro Gly Ser Ser Val Lys
1730 1735 1740

Gly Asp Cys Gly Asn Pro Ser Val Gly Ile Phe Phe Phe Val Ser
1745 1750 1755

```
Tyr Ile  Ile Ile Ser Phe Leu  Val Val Val Asn Met  Tyr Ile Ala
    1760                 1765                 1770

Val Ile  Leu Glu Asn Phe Ser  Val Ala Thr Glu Glu  Ser Ala Glu
    1775                 1780                 1785

Pro Leu  Ser Glu Asp Asp Phe  Glu Met Phe Tyr Glu  Val Trp Glu
    1790                 1795                 1800

Lys Phe  Asp Pro Asp Ala Thr  Gln Phe Ile Glu Phe  Ala Lys Leu
    1805                 1810                 1815

Ser Asp  Phe Ala Asp Ala Leu  Asp Pro Pro Leu Leu  Ile Ala Lys
    1820                 1825                 1830

Pro Asn  Lys Val Gln Leu Ile  Ala Met Asp Leu Pro  Met Val Ser
    1835                 1840                 1845

Gly Asp  Arg Ile His Cys Leu  Asp Ile Leu Phe Ala  Phe Thr Lys
    1850                 1855                 1860

Arg Val  Leu Gly Glu Ser Gly  Glu Met Asp Ala Leu  Arg Ile Gln
    1865                 1870                 1875

Met Glu  Glu Arg Phe Met Ala  Ser Asn Pro Ser Lys  Val Ser Tyr
    1880                 1885                 1890

Glu Pro  Ile Thr Thr Thr Leu  Lys Arg Lys Gln Glu  Glu Val Ser
    1895                 1900                 1905

Ala Ile  Ile Ile Gln Arg Ala  Tyr Arg Arg Tyr Leu  Leu Lys Gln
    1910                 1915                 1920

Lys Val  Lys Lys Val Ser Ser  Ile Tyr Lys Lys Asp  Lys Gly Lys
    1925                 1930                 1935

Glu Cys  Asp Gly Thr Pro Ile  Lys Glu Asp Thr Leu  Ile Asp Lys
    1940                 1945                 1950

Leu Asn  Glu Asn Ser Thr Pro  Glu Lys Thr Asp Met  Thr Pro Ser
    1955                 1960                 1965

Thr Thr  Ser Pro Pro Ser Tyr  Asp Ser Val Thr Lys  Pro Glu Lys
    1970                 1975                 1980

Glu Lys  Phe Glu Lys Asp Lys  Ser Glu Lys Glu Asp  Lys Gly Lys
    1985                 1990                 1995
```

Asp Ile Arg Glu Ser Lys Lys
2000 2005

<210> 22
<211> 2000
<212> PRT
<213> Homo sapiens

<400> 22

```
Met Ala Gln Ala Leu Leu Val Pro Pro Gly Pro Glu Ser Phe Arg Leu
1               5                   10                  15

Phe Thr Arg Glu Ser Leu Ala Ala Ile Glu Lys Arg Ala Ala Glu Glu
            20                  25                  30

Lys Ala Lys Lys Pro Lys Lys Glu Gln Asp Asn Asp Asp Glu Asn Lys
        35                  40                  45

Pro Lys Pro Asn Ser Asp Leu Glu Ala Gly Lys Asn Leu Pro Phe Ile
    50                  55                  60

Tyr Gly Asp Ile Pro Pro Glu Met Val Ser Glu Pro Leu Glu Asp Leu
65                  70                  75                  80

Asp Pro Tyr Tyr Ile Asn Lys Lys Thr Phe Ile Val Met Asn Lys Gly
                85                  90                  95

Lys Ala Ile Phe Arg Phe Ser Ala Thr Ser Ala Leu Tyr Ile Leu Thr
            100                 105                 110

Pro Leu Asn Pro Val Arg Lys Ile Ala Ile Lys Ile Leu Val His Ser
        115                 120                 125

Leu Phe Ser Met Leu Ile Met Cys Thr Ile Leu Thr Asn Cys Val Phe
    130                 135                 140

Met Thr Leu Ser Asn Pro Pro Asp Trp Thr Lys Asn Val Glu Tyr Thr
145                 150                 155                 160

Phe Thr Gly Ile Tyr Thr Phe Glu Ser Leu Ile Lys Ile Leu Ala Arg
                165                 170                 175

Gly Phe Cys Leu Glu Asp Phe Thr Phe Leu Arg Asp Pro Trp Asn Trp
            180                 185                 190

Leu Asp Phe Ser Val Ile Val Met Ala Tyr Val Thr Glu Phe Val Asp
        195                 200                 205

Leu Gly Asn Val Ser Ala Leu Arg Thr Phe Arg Val Leu Arg Ala Leu
    210                 215                 220
```

```
Lys Thr Ile Ser Val Ile Pro Gly Leu Lys Thr Ile Val Gly Ala Leu
225                 230                 235                 240

Ile Gln Ser Val Lys Lys Leu Ser Asp Val Met Ile Leu Thr Val Phe
                245                 250                 255

Cys Leu Ser Val Phe Ala Leu Ile Gly Leu Gln Leu Phe Met Gly Asn
            260                 265                 270

Leu Arg Asn Lys Cys Leu Gln Trp Pro Pro Ser Asp Ser Ala Phe Glu
        275                 280                 285

Thr Asn Thr Thr Ser Tyr Phe Asn Gly Thr Met Asp Ser Asn Gly Thr
    290                 295                 300

Phe Val Asn Val Thr Met Ser Thr Phe Asn Trp Lys Asp Tyr Ile Gly
305                 310                 315                 320

Asp Asp Ser His Phe Tyr Val Leu Asp Gly Gln Lys Asp Pro Leu Leu
                325                 330                 335

Cys Gly Asn Gly Ser Asp Ala Gly Gln Cys Pro Glu Gly Tyr Ile Cys
            340                 345                 350

Val Lys Ala Gly Arg Asn Pro Asn Tyr Gly Tyr Thr Ser Phe Asp Thr
        355                 360                 365

Phe Ser Trp Ala Phe Leu Ser Leu Phe Arg Leu Met Thr Gln Asp Tyr
    370                 375                 380

Trp Glu Asn Leu Tyr Gln Leu Thr Leu Arg Ala Ala Gly Lys Thr Tyr
385                 390                 395                 400

Met Ile Phe Phe Val Leu Val Ile Phe Leu Gly Ser Phe Tyr Leu Val
                405                 410                 415

Asn Leu Ile Leu Ala Val Val Ala Met Ala Tyr Glu Glu Gln Asn Gln
            420                 425                 430

Ala Thr Leu Glu Glu Ala Glu Gln Lys Glu Ala Glu Phe Gln Gln Met
        435                 440                 445

Leu Glu Gln Leu Lys Lys Gln Gln Glu Glu Ala Gln Ala Val Ala Ala
    450                 455                 460

Ala Ser Ala Ala Ser Arg Asp Phe Ser Gly Ile Gly Gly Leu Gly Glu
465                 470                 475                 480
```

```
Leu Leu Glu Ser Ser Ser Glu Ala Ser Lys Leu Ser Ser Lys Ser Ala
                485                 490                 495

Lys Glu Trp Arg Asn Arg Arg Lys Lys Arg Arg Gln Arg Glu His Leu
            500                 505                 510

Glu Gly Asn Asn Lys Gly Glu Arg Asp Ser Phe Pro Lys Ser Glu Ser
        515                 520                 525

Glu Asp Ser Val Lys Arg Ser Ser Phe Leu Phe Ser Met Asp Gly Asn
    530                 535                 540

Arg Leu Thr Ser Asp Lys Lys Phe Cys Ser Pro His Gln Ser Leu Leu
545                 550                 555                 560

Ser Ile Arg Gly Ser Leu Phe Ser Pro Arg Arg Asn Ser Lys Thr Ser
                565                 570                 575

Ile Phe Ser Phe Arg Gly Arg Ala Lys Asp Val Gly Ser Glu Asn Asp
            580                 585                 590

Phe Ala Asp Asp Glu His Ser Thr Phe Glu Asp Ser Glu Ser Arg Arg
        595                 600                 605

Asp Ser Leu Phe Val Pro His Arg His Gly Glu Arg Arg Asn Ser Asn
    610                 615                 620

Val Ser Gln Ala Ser Met Ser Ser Arg Met Val Pro Gly Leu Pro Ala
625                 630                 635                 640

Asn Gly Lys Met His Ser Thr Val Asp Cys Asn Gly Val Val Ser Leu
                645                 650                 655

Val Gly Gly Pro Ser Ala Leu Thr Ser Pro Thr Gly Gln Leu Pro Pro
            660                 665                 670

Glu Gly Thr Thr Thr Glu Thr Glu Val Arg Lys Arg Arg Leu Ser Ser
        675                 680                 685

Tyr Gln Ile Ser Met Glu Met Leu Glu Asp Ser Ser Gly Arg Gln Arg
    690                 695                 700

Ala Val Ser Ile Ala Ser Ile Leu Thr Asn Thr Met Glu Glu Leu Glu
705                 710                 715                 720

Glu Ser Arg Gln Lys Cys Pro Pro Cys Trp Tyr Arg Phe Ala Asn Val
                725                 730                 735
```

Phe Leu Ile Trp Asp Cys Cys Asp Ala Trp Leu Lys Val Lys His Leu
            740                 745                 750

Val Asn Leu Ile Val Met Asp Pro Phe Val Asp Leu Ala Ile Thr Ile
        755                 760                 765

Cys Ile Val Leu Asn Thr Leu Phe Met Ala Met Glu His Tyr Pro Met
    770                 775                 780

Thr Glu Gln Phe Ser Ser Val Leu Thr Val Gly Asn Leu Val Phe Thr
785                 790                 795                 800

Gly Ile Phe Thr Ala Glu Met Val Leu Lys Ile Ile Ala Met Asp Pro
                805                 810                 815

Tyr Tyr Tyr Phe Gln Glu Gly Trp Asn Ile Phe Asp Gly Ile Ile Val
            820                 825                 830

Ser Leu Ser Leu Met Glu Leu Gly Leu Ser Asn Val Glu Gly Leu Ser
        835                 840                 845

Val Leu Arg Ser Phe Arg Leu Leu Arg Val Phe Lys Leu Ala Lys Ser
    850                 855                 860

Trp Pro Thr Leu Asn Met Leu Ile Lys Ile Ile Gly Asn Ser Val Gly
865                 870                 875                 880

Ala Leu Gly Asn Leu Thr Leu Val Leu Ala Ile Ile Val Phe Ile Phe
                885                 890                 895

Ala Val Val Gly Met Gln Leu Phe Gly Lys Ser Tyr Lys Glu Cys Val
            900                 905                 910

Cys Lys Ile Asn Asp Asp Cys Thr Leu Pro Arg Trp His Met Asn Asp
        915                 920                 925

Phe Phe His Ser Phe Leu Ile Val Phe Arg Val Leu Cys Gly Glu Trp
    930                 935                 940

Ile Glu Thr Met Trp Asp Cys Met Glu Val Ala Gly Gln Thr Met Cys
945                 950                 955                 960

Leu Ile Val Phe Met Leu Val Met Val Ile Gly Asn Leu Val Val Leu
                965                 970                 975

Asn Leu Phe Leu Ala Leu Leu Leu Ser Ser Phe Ser Ser Asp Asn Leu
            980                 985                 990

Ala Ala Thr Asp Asp Asp Asn Glu  Met Asn Asn Leu Gln  Ile Ala Val

995 1000 1005

Gly Arg Met Gln Lys Gly Ile Asp Tyr Val Lys Asn Lys Met Arg
1010 1015 1020

Glu Cys Phe Gln Lys Ala Phe Phe Arg Lys Pro Lys Val Ile Glu
1025 1030 1035

Ile His Glu Gly Asn Lys Ile Asp Ser Cys Met Ser Asn Asn Thr
1040 1045 1050

Gly Ile Glu Ile Ser Lys Glu Leu Asn Tyr Leu Arg Asp Gly Asn
1055 1060 1065

Gly Thr Thr Ser Gly Val Gly Thr Gly Ser Ser Val Glu Lys Tyr
1070 1075 1080

Val Ile Asp Glu Asn Asp Tyr Met Ser Phe Ile Asn Asn Pro Ser
1085 1090 1095

Leu Thr Val Thr Val Pro Ile Ala Val Gly Glu Ser Asp Phe Glu
1100 1105 1110

Asn Leu Asn Thr Glu Glu Phe Ser Ser Glu Ser Glu Leu Glu Glu
1115 1120 1125

Ser Lys Glu Lys Leu Asn Ala Thr Ser Ser Ser Glu Gly Ser Thr
1130 1135 1140

Val Asp Val Val Leu Pro Arg Glu Gly Glu Gln Ala Glu Thr Glu
1145 1150 1155

Pro Glu Glu Asp Leu Lys Pro Glu Ala Cys Phe Thr Glu Gly Cys
1160 1165 1170

Ile Lys Lys Phe Pro Phe Cys Gln Val Ser Thr Glu Glu Gly Lys
1175 1180 1185

Gly Lys Ile Trp Trp Asn Leu Arg Lys Thr Cys Tyr Ser Ile Val
1190 1195 1200

Glu His Asn Trp Phe Glu Thr Phe Ile Val Phe Met Ile Leu Leu
1205 1210 1215

Ser Ser Gly Ala Leu Ala Phe Glu Asp Ile Tyr Ile Glu Gln Arg
1220 1225 1230

Lys Thr Ile Lys Thr Met Leu Glu Tyr Ala Asp Lys Val Phe Thr
1235 1240 1245

```
Tyr Ile  Phe Ile Leu Glu Met  Leu Leu Lys Trp Val  Ala Tyr Gly
    1250                 1255                 1260

Phe Gln  Thr Tyr Phe Thr Asn  Ala Trp Cys Trp Leu  Asp Phe Leu
    1265                 1270                 1275

Ile Val  Asp Val Ser Leu Val  Ser Leu Val Ala Asn  Ala Leu Gly
    1280                 1285                 1290

Tyr Ser  Glu Leu Gly Ala Ile  Lys Ser Leu Arg Thr  Leu Arg Ala
    1295                 1300                 1305

Leu Arg  Pro Leu Arg Ala Leu  Ser Arg Phe Glu Gly  Met Arg Val
    1310                 1315                 1320

Val Val  Asn Ala Leu Val Gly  Ala Ile Pro Ser Ile  Met Asn Val
    1325                 1330                 1335

Leu Leu  Val Cys Leu Ile Phe  Trp Leu Ile Phe Ser  Ile Met Gly
    1340                 1345                 1350

Val Asn  Leu Phe Ala Gly Lys  Phe Tyr His Cys Val  Asn Met Thr
    1355                 1360                 1365

Thr Gly  Asn Met Phe Asp Ile  Ser Asp Val Asn Asn  Leu Ser Asp
    1370                 1375                 1380

Cys Gln  Ala Leu Gly Lys Gln  Ala Arg Trp Lys Asn  Val Lys Val
    1385                 1390                 1395

Asn Phe  Asp Asn Val Gly Ala  Gly Tyr Leu Ala Leu  Leu Gln Val
    1400                 1405                 1410

Ala Thr  Phe Lys Gly Trp Met  Asp Ile Met Tyr Ala  Ala Val Asp
    1415                 1420                 1425

Ser Arg  Asp Val Lys Leu Gln  Pro Val Tyr Glu Glu  Asn Leu Tyr
    1430                 1435                 1440

Met Tyr  Leu Tyr Phe Val Ile  Phe Ile Ile Phe Gly  Ser Phe Phe
    1445                 1450                 1455

Thr Leu  Asn Leu Phe Ile Gly  Val Ile Ile Asp Asn  Phe Asn Gln
    1460                 1465                 1470

Gln Lys  Lys Lys Phe Gly Gly  Gln Asp Ile Phe Met  Thr Glu Glu
    1475                 1480                 1485
```

Gln Lys Lys Tyr Tyr Asn Ala Met Lys Lys Leu Gly Ser Lys Lys
1490 1495 1500

Pro Gln Lys Pro Ile Pro Arg Pro Ala Asn Lys Phe Gln Gly Met
1505 1510 1515

Val Phe Asp Phe Val Thr Arg Gln Val Phe Asp Ile Ser Ile Met
1520 1525 1530

Ile Leu Ile Cys Leu Asn Met Val Thr Met Met Val Glu Thr Asp
1535 1540 1545

Asp Gln Gly Lys Tyr Met Thr Leu Val Leu Ser Arg Ile Asn Leu
1550 1555 1560

Val Phe Ile Val Leu Phe Thr Gly Glu Phe Val Leu Lys Leu Val
1565 1570 1575

Ser Leu Arg His Tyr Tyr Phe Thr Ile Gly Trp Asn Ile Phe Asp
1580 1585 1590

Phe Val Val Val Ile Leu Ser Ile Val Gly Met Phe Leu Ala Glu
1595 1600 1605

Met Ile Glu Lys Tyr Phe Val Ser Pro Thr Leu Phe Arg Val Ile
1610 1615 1620

Arg Leu Ala Arg Ile Gly Arg Ile Leu Arg Leu Ile Lys Gly Ala
1625 1630 1635

Lys Gly Ile Arg Thr Leu Leu Phe Ala Leu Met Met Ser Leu Pro
1640 1645 1650

Ala Leu Phe Asn Ile Gly Leu Leu Leu Phe Leu Val Met Phe Ile
1655 1660 1665

Tyr Ala Ile Phe Gly Met Ser Asn Phe Ala Tyr Val Lys Lys Glu
1670 1675 1680

Ala Gly Ile Asp Asp Met Phe Asn Phe Glu Thr Phe Gly Asn Ser
1685 1690 1695

Met Ile Cys Leu Phe Gln Ile Thr Thr Ser Ala Gly Trp Asp Gly
1700 1705 1710

Leu Leu Ala Pro Ile Leu Asn Ser Ala Pro Pro Asp Cys Asp Pro
1715 1720 1725

```
Asp Thr  Ile His Pro Gly Ser  Ser Val Lys Gly Asp  Cys Gly Asn
    1730                 1735                 1740

Pro Ser  Val Gly Ile Phe Phe  Phe Val Ser Tyr Ile  Ile Ile Ser
    1745                 1750                 1755

Phe Leu  Val Val Val Asn Met  Tyr Ile Ala Val Ile  Leu Glu Asn
    1760                 1765                 1770

Phe Ser  Val Ala Thr Glu Glu  Ser Ala Glu Pro Leu  Ser Glu Asp
    1775                 1780                 1785

Asp Phe  Glu Met Phe Tyr Glu  Val Trp Glu Lys Phe  Asp Pro Asp
    1790                 1795                 1800

Ala Thr  Gln Phe Ile Glu Phe  Ser Lys Leu Ser Asp  Phe Ala Ala
    1805                 1810                 1815

Ala Leu  Asp Pro Pro Leu Leu  Ile Ala Lys Pro Asn  Lys Val Gln
    1820                 1825                 1830

Leu Ile  Ala Met Asp Leu Pro  Met Val Ser Gly Asp  Arg Ile His
    1835                 1840                 1845

Cys Leu  Asp Ile Leu Phe Ala  Phe Thr Lys Arg Val  Leu Gly Glu
    1850                 1855                 1860

Ser Gly  Glu Met Asp Ala Leu  Arg Ile Gln Met Glu  Asp Arg Phe
    1865                 1870                 1875

Met Ala  Ser Asn Pro Ser Lys  Val Ser Tyr Glu Pro  Ile Thr Thr
    1880                 1885                 1890

Thr Leu  Lys Arg Lys Gln Glu  Glu Val Ser Ala Ala  Ile Ile Gln
    1895                 1900                 1905

Arg Asn  Phe Arg Cys Tyr Leu  Leu Lys Gln Arg Leu  Lys Asn Ile
    1910                 1915                 1920

Ser Ser  Asn Tyr Asn Lys Glu  Ala Ile Lys Gly Arg  Ile Asp Leu
    1925                 1930                 1935

Pro Ile  Lys Gln Asp Met Ile  Ile Asp Lys Leu Asn  Gly Asn Ser
    1940                 1945                 1950

Thr Pro  Glu Lys Thr Asp Gly  Ser Ser Ser Thr Thr  Ser Pro Pro
    1955                 1960                 1965

Ser Tyr  Asp Ser Val Thr Lys  Pro Asp Lys Glu Lys  Phe Glu Lys
```

1970 1975 1980

Asp Lys Pro Glu Lys Glu Ser Lys Gly Lys Glu Val Arg Glu Asn
1985 1990 1995

Gln Lys
2000

<210> 23
<211> 1836
<212> PRT
<213> Homo sapiens

<400> 23

```
Met Ala Arg Pro Ser Leu Cys Thr Leu Val Pro Leu Gly Pro Glu Cys
1               5                   10                  15

Leu Arg Pro Phe Thr Arg Glu Ser Leu Ala Ala Ile Glu Gln Arg Ala
            20                  25                  30

Val Glu Glu Glu Ala Arg Leu Gln Arg Asn Lys Gln Met Glu Ile Glu
        35                  40                  45

Glu Pro Glu Arg Lys Pro Arg Ser Asp Leu Glu Ala Gly Lys Asn Leu
    50                  55                  60

Pro Met Ile Tyr Gly Asp Pro Pro Pro Glu Val Ile Gly Ile Pro Leu
65                  70                  75                  80

Glu Asp Leu Asp Pro Tyr Tyr Ser Asn Lys Lys Thr Phe Ile Val Leu
                85                  90                  95

Asn Lys Gly Lys Ala Ile Phe Arg Phe Ser Ala Thr Pro Ala Leu Tyr
            100                 105                 110

Leu Leu Ser Pro Phe Ser Val Val Arg Arg Gly Ala Ile Lys Val Leu
        115                 120                 125

Ile His Ala Leu Phe Ser Met Phe Ile Met Ile Thr Ile Leu Thr Asn
    130                 135                 140

Cys Val Phe Met Thr Met Ser Asp Pro Pro Pro Trp Ser Lys Asn Val
145                 150                 155                 160

Glu Tyr Thr Phe Thr Gly Ile Tyr Thr Phe Glu Ser Leu Ile Lys Ile
                165                 170                 175

Leu Ala Arg Gly Phe Cys Val Asp Asp Phe Thr Phe Leu Arg Asp Pro
            180                 185                 190
```

```
Trp Asn Trp Leu Asp Phe Ser Val Ile Met Met Ala Tyr Leu Thr Glu
          195                 200                 205

Phe Val Asp Leu Gly Asn Ile Ser Ala Leu Arg Thr Phe Arg Val Leu
    210                 215                 220

Arg Ala Leu Lys Thr Ile Thr Val Ile Pro Gly Leu Lys Thr Ile Val
225                 230                 235                 240

Gly Ala Leu Ile Gln Ser Val Lys Lys Leu Ser Asp Val Met Ile Leu
                245                 250                 255

Thr Val Phe Cys Leu Ser Val Phe Ala Leu Val Gly Leu Gln Leu Phe
            260                 265                 270

Met Gly Asn Leu Arg Gln Lys Cys Val Arg Trp Pro Pro Pro Phe Asn
        275                 280                 285

Asp Thr Asn Thr Thr Trp Tyr Ser Asn Asp Thr Trp Tyr Gly Asn Asp
    290                 295                 300

Thr Trp Tyr Gly Asn Glu Met Trp Tyr Gly Asn Asp Ser Trp Tyr Ala
305                 310                 315                 320

Asn Asp Thr Trp Asn Ser His Ala Ser Trp Ala Thr Asn Asp Thr Phe
                325                 330                 335

Asp Trp Asp Ala Tyr Ile Ser Asp Glu Gly Asn Phe Tyr Phe Leu Glu
            340                 345                 350

Gly Ser Asn Asp Ala Leu Leu Cys Gly Asn Ser Ser Asp Ala Gly His
        355                 360                 365

Cys Pro Glu Gly Tyr Glu Cys Ile Lys Thr Gly Arg Asn Pro Asn Tyr
    370                 375                 380

Gly Tyr Thr Ser Tyr Asp Thr Phe Ser Trp Ala Phe Leu Ala Leu Phe
385                 390                 395                 400

Arg Leu Met Thr Gln Asp Tyr Trp Glu Asn Leu Phe Gln Leu Thr Leu
                405                 410                 415

Arg Ala Ala Gly Lys Thr Tyr Met Ile Phe Phe Val Val Ile Ile Phe
            420                 425                 430

Leu Gly Ser Phe Tyr Leu Ile Asn Leu Ile Leu Ala Val Val Ala Met
        435                 440                 445

Ala Tyr Ala Glu Gln Asn Glu Ala Thr Leu Ala Glu Asp Lys Glu Lys
```

```
    450                 455                 460

Glu Glu Glu Phe Gln Gln Met Leu Glu Lys Phe Lys Lys His Gln Glu
465                 470                 475                 480

Glu Leu Glu Lys Ala Lys Ala Ala Gln Ala Leu Glu Gly Gly Glu Ala
                485                 490                 495

Asp Gly Asp Pro Ala His Gly Lys Asp Cys Asn Gly Ser Leu Asp Thr
            500                 505                 510

Ser Gln Gly Glu Lys Gly Ala Pro Arg Gln Ser Ser Ser Gly Asp Ser
        515                 520                 525

Gly Ile Ser Asp Ala Met Glu Glu Leu Glu Glu Ala His Gln Lys Cys
    530                 535                 540

Pro Pro Trp Trp Tyr Lys Cys Ala His Lys Val Leu Ile Trp Asn Cys
545                 550                 555                 560

Cys Ala Pro Trp Leu Lys Phe Lys Asn Ile Ile His Leu Ile Val Met
                565                 570                 575

Asp Pro Phe Val Asp Leu Gly Ile Thr Ile Cys Ile Val Leu Asn Thr
            580                 585                 590

Leu Phe Met Ala Met Glu His Tyr Pro Met Thr Glu His Phe Asp Asn
        595                 600                 605

Val Leu Thr Val Gly Asn Leu Val Phe Thr Gly Ile Phe Thr Ala Glu
    610                 615                 620

Met Val Leu Lys Leu Ile Ala Met Asp Pro Tyr Glu Tyr Phe Gln Gln
625                 630                 635                 640

Gly Trp Asn Ile Phe Asp Ser Ile Ile Val Thr Leu Ser Leu Val Glu
                645                 650                 655

Leu Gly Leu Ala Asn Val Gln Gly Leu Ser Val Leu Arg Ser Phe Arg
            660                 665                 670

Leu Leu Arg Val Phe Lys Leu Ala Lys Ser Trp Pro Thr Leu Asn Met
        675                 680                 685

Leu Ile Lys Ile Ile Gly Asn Ser Val Gly Ala Leu Gly Asn Leu Thr
    690                 695                 700

Leu Val Leu Ala Ile Ile Val Phe Ile Phe Ala Val Val Gly Met Gln
705                 710                 715                 720
```

Leu Phe Gly Lys Ser Tyr Lys Glu Cys Val Cys Lys Ile Ala Leu Asp
725 730 735

Cys Asn Leu Pro Arg Trp His Met His Asp Phe Phe His Ser Phe Leu
740 745 750

Ile Val Phe Arg Ile Leu Cys Gly Glu Trp Ile Glu Thr Met Trp Asp
755 760 765

Cys Met Glu Val Ala Gly Gln Ala Met Cys Leu Thr Val Phe Leu Met
770 775 780

Val Met Val Ile Gly Asn Leu Val Val Leu Asn Leu Phe Leu Ala Leu
785 790 795 800

Leu Leu Ser Ser Phe Ser Ala Asp Ser Leu Ala Ala Ser Asp Glu Asp
805 810 815

Gly Glu Met Asn Asn Leu Gln Ile Ala Ile Gly Arg Ile Lys Leu Gly
820 825 830

Ile Gly Phe Ala Lys Ala Phe Leu Leu Gly Leu Leu His Gly Lys Ile
835 840 845

Leu Ser Pro Lys Asp Ile Met Leu Ser Leu Gly Glu Ala Asp Gly Ala
850 855 860

Gly Glu Ala Gly Glu Ala Gly Glu Thr Ala Pro Glu Asp Glu Lys Lys
865 870 875 880

Glu Pro Pro Glu Glu Asp Leu Lys Lys Asp Asn His Ile Leu Asn His
885 890 895

Met Gly Leu Ala Asp Gly Pro Pro Ser Ser Leu Glu Leu Asp His Leu
900 905 910

Asn Phe Ile Asn Asn Pro Tyr Leu Thr Ile Gln Val Pro Ile Ala Ser
915 920 925

Glu Glu Ser Asp Leu Glu Met Pro Thr Glu Glu Glu Thr Asp Thr Phe
930 935 940

Ser Glu Pro Glu Asp Ser Lys Lys Pro Pro Gln Pro Leu Tyr Asp Gly
945 950 955 960

Asn Ser Ser Val Cys Ser Thr Ala Asp Tyr Lys Pro Pro Glu Glu Asp
965 970 975

```
Pro Glu Glu Gln Ala Glu Glu Asn Pro Glu Gly Glu Gln Pro Glu Glu
            980                 985                 990

Cys Phe Thr Glu Ala Cys Val Gln  Arg Trp Pro Cys Leu  Tyr Val Asp
        995                 1000                 1005

Ile Ser  Gln Gly Arg Gly Lys  Lys Trp Trp Thr Leu  Arg Arg Ala
    1010                 1015                 1020

Cys Phe  Lys Ile Val Glu His  Asn Trp Phe Glu Thr  Phe Ile Val
    1025                 1030                 1035

Phe Met  Ile Leu Leu Ser Ser  Gly Ala Leu Ala Phe  Glu Asp Ile
    1040                 1045                 1050

Tyr Ile  Glu Gln Arg Arg Val  Ile Arg Thr Ile Leu  Glu Tyr Ala
    1055                 1060                 1065

Asp Lys  Val Phe Thr Tyr Ile  Phe Ile Met Glu Met  Leu Leu Lys
    1070                 1075                 1080

Trp Val  Ala Tyr Gly Phe Lys  Val Tyr Phe Thr Asn  Ala Trp Cys
    1085                 1090                 1095

Trp Leu  Asp Phe Leu Ile Val  Asp Val Ser Ile Ile  Ser Leu Val
    1100                 1105                 1110

Ala Asn  Trp Leu Gly Tyr Ser  Glu Leu Gly Pro Ile  Lys Ser Leu
    1115                 1120                 1125

Arg Thr  Leu Arg Ala Leu Arg  Pro Leu Arg Ala Leu  Ser Arg Phe
    1130                 1135                 1140

Glu Gly  Met Arg Val Val Val  Asn Ala Leu Leu Gly  Ala Ile Pro
    1145                 1150                 1155

Ser Ile  Met Asn Val Leu Leu  Val Cys Leu Ile Phe  Trp Leu Ile
    1160                 1165                 1170

Phe Ser  Ile Met Gly Val Asn  Leu Phe Ala Gly Lys  Phe Tyr Tyr
    1175                 1180                 1185

Cys Ile  Asn Thr Thr Thr Ser  Glu Arg Phe Asp Ile  Ser Glu Val
    1190                 1195                 1200

Asn Asn  Lys Ser Glu Cys Glu  Ser Leu Met His Thr  Gly Gln Val
    1205                 1210                 1215
```

```
Arg Trp  Leu Asn Val Lys Val  Asn Tyr Asp Asn Val  Gly Leu Gly
    1220                 1225                 1230

Tyr Leu  Ser Leu Leu Gln Val  Ala Thr Phe Lys Gly  Trp Met Asp
    1235                 1240                 1245

Ile Met  Tyr Ala Ala Val Asp  Ser Arg Glu Lys Glu  Glu Gln Pro
    1250                 1255                 1260

Gln Tyr  Glu Val Asn Leu Tyr  Met Tyr Leu Tyr Phe  Val Ile Phe
    1265                 1270                 1275

Ile Ile  Phe Gly Ser Phe Phe  Thr Leu Asn Leu Phe  Ile Gly Val
    1280                 1285                 1290

Ile Ile  Asp Asn Phe Asn Gln  Gln Lys Lys Lys Leu  Gly Gly Lys
    1295                 1300                 1305

Asp Ile  Phe Met Thr Glu Glu  Gln Lys Lys Tyr Tyr  Asn Ala Met
    1310                 1315                 1320

Lys Lys  Leu Gly Ser Lys Lys  Pro Gln Lys Pro Ile  Pro Arg Pro
    1325                 1330                 1335

Gln Asn  Lys Ile Gln Gly Met  Val Tyr Asp Leu Val  Thr Lys Gln
    1340                 1345                 1350

Ala Phe  Asp Ile Thr Ile Met  Ile Leu Ile Cys Leu  Asn Met Val
    1355                 1360                 1365

Thr Met  Met Val Glu Thr Asp  Asn Gln Ser Gln Leu  Lys Val Asp
    1370                 1375                 1380

Ile Leu  Tyr Asn Ile Asn Met  Ile Phe Ile Ile Ile  Phe Thr Gly
    1385                 1390                 1395

Glu Cys  Val Leu Lys Met Leu  Ala Leu Arg Gln Tyr  Tyr Phe Thr
    1400                 1405                 1410

Val Gly  Trp Asn Ile Phe Asp  Phe Val Val Val Ile  Leu Ser Ile
    1415                 1420                 1425

Val Gly  Leu Ala Leu Ser Asp  Leu Ile Gln Lys Tyr  Phe Val Ser
    1430                 1435                 1440

Pro Thr  Leu Phe Arg Val Ile  Arg Leu Ala Arg Ile  Gly Arg Val
    1445                 1450                 1455

Leu Arg  Leu Ile Arg Gly Ala  Lys Gly Ile Arg Thr  Leu Leu Phe
```

1460 1465 1470

Ala Leu Met Met Ser Leu Pro Ala Leu Phe Asn Ile Gly Leu Leu
1475 1480 1485

Leu Phe Leu Val Met Phe Ile Tyr Ser Ile Phe Gly Met Ser Asn
1490 1495 1500

Phe Ala Tyr Val Lys Lys Glu Ser Gly Ile Asp Asp Met Phe Asn
1505 1510 1515

Phe Glu Thr Phe Gly Asn Ser Ile Ile Cys Leu Phe Glu Ile Thr
1520 1525 1530

Thr Ser Ala Gly Trp Asp Gly Leu Leu Asn Pro Ile Leu Asn Ser
1535 1540 1545

Gly Pro Pro Asp Cys Asp Pro Asn Leu Glu Asn Pro Gly Thr Ser
1550 1555 1560

Val Lys Gly Asp Cys Gly Asn Pro Ser Ile Gly Ile Cys Phe Phe
1565 1570 1575

Cys Ser Tyr Ile Ile Ile Ser Phe Leu Ile Val Val Asn Met Tyr
1580 1585 1590

Ile Ala Ile Ile Leu Glu Asn Phe Asn Val Ala Thr Glu Glu Ser
1595 1600 1605

Ser Glu Pro Leu Gly Glu Asp Asp Phe Glu Met Phe Tyr Glu Thr
1610 1615 1620

Trp Glu Lys Phe Asp Pro Asp Ala Thr Gln Phe Ile Ala Tyr Ser
1625 1630 1635

Arg Leu Ser Asp Phe Val Asp Thr Leu Gln Glu Pro Leu Arg Ile
1640 1645 1650

Ala Lys Pro Asn Lys Ile Lys Leu Ile Thr Leu Asp Leu Pro Met
1655 1660 1665

Val Pro Gly Asp Lys Ile His Cys Leu Asp Ile Leu Phe Ala Leu
1670 1675 1680

Thr Lys Glu Val Leu Gly Asp Ser Gly Glu Met Asp Ala Leu Lys
1685 1690 1695

Gln Thr Met Glu Glu Lys Phe Met Ala Ala Asn Pro Ser Lys Val
1700 1705 1710

```
Ser Tyr  Glu Pro Ile Thr Thr  Thr Leu Lys Arg Lys  His Glu Glu
    1715                 1720                 1725

Val Cys  Ala Ile Lys Ile Gln  Arg Ala Tyr Arg Arg  His Leu Leu
    1730                 1735                 1740

Gln Arg  Ser Met Lys Gln Ala  Ser Tyr Met Tyr Arg  His Ser His
    1745                 1750                 1755

Asp Gly  Ser Gly Asp Asp Ala  Pro Glu Lys Glu Gly  Leu Leu Ala
    1760                 1765                 1770

Asn Thr  Met Ser Lys Met Tyr  Gly His Glu Asn Gly  Asn Ser Ser
    1775                 1780                 1785

Ser Pro  Ser Pro Glu Glu Lys  Gly Glu Ala Gly Asp  Ala Gly Pro
    1790                 1795                 1800

Thr Met  Gly Leu Met Pro Ile  Ser Pro Ser Asp Thr  Ala Trp Pro
    1805                 1810                 1815

Pro Ala  Pro Pro Pro Gly Gln  Thr Val Arg Pro Gly  Val Lys Glu
    1820                 1825                 1830

Ser Leu  Val
    1835
```

<210> 24
<211> 2016
<212> PRT
<213> Homo sapiens

<400> 24

```
Met Ala Asn Phe Leu Leu Pro Arg Gly Thr Ser Ser Phe Arg Arg Phe
1               5                   10                  15

Thr Arg Glu Ser Leu Ala Ala Ile Glu Lys Arg Met Ala Glu Lys Gln
            20                  25                  30

Ala Arg Gly Ser Thr Thr Leu Gln Glu Ser Arg Glu Gly Leu Pro Glu
        35                  40                  45

Glu Glu Ala Pro Arg Pro Gln Leu Asp Leu Gln Ala Ser Lys Lys Leu
    50                  55                  60

Pro Asp Leu Tyr Gly Asn Pro Pro Gln Glu Leu Ile Gly Glu Pro Leu
65                  70                  75                  80

Glu Asp Leu Asp Pro Phe Tyr Ser Thr Gln Lys Thr Phe Ile Val Leu
```

85 90 95

Asn Lys Gly Lys Thr Ile Phe Arg Phe Ser Ala Thr Asn Ala Leu Tyr
100 105 110

Val Leu Ser Pro Phe His Pro Ile Arg Arg Ala Ala Val Lys Ile Leu
115 120 125

Val His Ser Leu Phe Asn Met Leu Ile Met Cys Thr Ile Leu Thr Asn
130 135 140

Cys Val Phe Met Ala Gln His Asp Pro Pro Pro Trp Thr Lys Tyr Val
145 150 155 160

Glu Tyr Thr Phe Thr Ala Ile Tyr Thr Phe Glu Ser Leu Val Lys Ile
165 170 175

Leu Ala Arg Gly Phe Cys Leu His Ala Phe Thr Phe Leu Arg Asp Pro
180 185 190

Trp Asn Trp Leu Asp Phe Ser Val Ile Ile Met Ala Tyr Thr Thr Glu
195 200 205

Phe Val Asp Leu Gly Asn Val Ser Ala Leu Arg Thr Phe Arg Val Leu
210 215 220

Arg Ala Leu Lys Thr Ile Ser Val Ile Ser Gly Leu Lys Thr Ile Val
225 230 235 240

Gly Ala Leu Ile Gln Ser Val Lys Lys Leu Ala Asp Val Met Val Leu
245 250 255

Thr Val Phe Cys Leu Ser Val Phe Ala Leu Ile Gly Leu Gln Leu Phe
260 265 270

Met Gly Asn Leu Arg His Lys Cys Val Arg Asn Phe Thr Ala Leu Asn
275 280 285

Gly Thr Asn Gly Ser Val Glu Ala Asp Gly Leu Val Trp Glu Ser Leu
290 295 300

Asp Leu Tyr Leu Ser Asp Pro Glu Asn Tyr Leu Leu Lys Asn Gly Thr
305 310 315 320

Ser Asp Val Leu Leu Cys Gly Asn Ser Ser Asp Ala Gly Thr Cys Pro
325 330 335

Glu Gly Tyr Arg Cys Leu Lys Ala Gly Glu Asn Pro Asp His Gly Tyr
340 345 350

Thr Ser Phe Asp Ser Phe Ala Trp Ala Phe Leu Ala Leu Phe Arg Leu
355 360 365

Met Thr Gln Asp Cys Trp Glu Arg Leu Tyr Gln Gln Thr Leu Arg Ser
370 375 380

Ala Gly Lys Ile Tyr Met Ile Phe Phe Met Leu Val Ile Phe Leu Gly
385 390 395 400

Ser Phe Tyr Leu Val Asn Leu Ile Leu Ala Val Val Ala Met Ala Tyr
405 410 415

Glu Glu Gln Asn Gln Ala Thr Ile Ala Glu Thr Glu Glu Lys Glu Lys
420 425 430

Arg Phe Gln Glu Ala Met Glu Met Leu Lys Lys Glu His Glu Ala Leu
435 440 445

Thr Ile Arg Gly Val Asp Thr Val Ser Arg Ser Ser Leu Glu Met Ser
450 455 460

Pro Leu Ala Pro Val Asn Ser His Glu Arg Arg Ser Lys Arg Arg Lys
465 470 475 480

Arg Met Ser Ser Gly Thr Glu Glu Cys Gly Glu Asp Arg Leu Pro Lys
485 490 495

Ser Asp Ser Glu Asp Gly Pro Arg Ala Met Asn His Leu Ser Leu Thr
500 505 510

Arg Gly Leu Ser Arg Thr Ser Met Lys Pro Arg Ser Ser Arg Gly Ser
515 520 525

Ile Phe Thr Phe Arg Arg Arg Asp Leu Gly Ser Glu Ala Asp Phe Ala
530 535 540

Asp Asp Glu Asn Ser Thr Ala Gly Glu Ser Glu Ser His His Thr Ser
545 550 555 560

Leu Leu Val Pro Trp Pro Leu Arg Arg Thr Ser Ala Gln Gly Gln Pro
565 570 575

Ser Pro Gly Thr Ser Ala Pro Gly His Ala Leu His Gly Lys Lys Asn
580 585 590

Ser Thr Val Asp Cys Asn Gly Val Val Ser Leu Leu Gly Ala Gly Asp
595 600 605

Pro Glu Ala Thr Ser Pro Gly Ser His Leu Leu Arg Pro Val Met Leu
    610                 615                 620

Glu His Pro Pro Asp Thr Thr Thr Pro Ser Glu Glu Pro Gly Gly Pro
625                 630                 635                 640

Gln Met Leu Thr Ser Gln Ala Pro Cys Val Asp Gly Phe Glu Glu Pro
                645                 650                 655

Gly Ala Arg Gln Arg Ala Leu Ser Ala Val Ser Val Leu Thr Ser Ala
            660                 665                 670

Leu Glu Glu Leu Glu Glu Ser Arg His Lys Cys Pro Pro Cys Trp Asn
        675                 680                 685

Arg Leu Ala Gln Arg Tyr Leu Ile Trp Glu Cys Cys Pro Leu Trp Met
    690                 695                 700

Ser Ile Lys Gln Gly Val Lys Leu Val Val Met Asp Pro Phe Thr Asp
705                 710                 715                 720

Leu Thr Ile Thr Met Cys Ile Val Leu Asn Thr Leu Phe Met Ala Leu
                725                 730                 735

Glu His Tyr Asn Met Thr Ser Glu Phe Glu Glu Met Leu Gln Val Gly
            740                 745                 750

Asn Leu Val Phe Thr Gly Ile Phe Thr Ala Glu Met Thr Phe Lys Ile
        755                 760                 765

Ile Ala Leu Asp Pro Tyr Tyr Tyr Phe Gln Gln Gly Trp Asn Ile Phe
    770                 775                 780

Asp Ser Ile Ile Val Ile Leu Ser Leu Met Glu Leu Gly Leu Ser Arg
785                 790                 795                 800

Met Ser Asn Leu Ser Val Leu Arg Ser Phe Arg Leu Leu Arg Val Phe
                805                 810                 815

Lys Leu Ala Lys Ser Trp Pro Thr Leu Asn Thr Leu Ile Lys Ile Ile
            820                 825                 830

Gly Asn Ser Val Gly Ala Leu Gly Asn Leu Thr Leu Val Leu Ala Ile
        835                 840                 845

Ile Val Phe Ile Phe Ala Val Val Gly Met Gln Leu Phe Gly Lys Asn
    850                 855                 860

```
Tyr Ser Glu Leu Arg Asp Ser Asp Ser Gly Leu Leu Pro Arg Trp His
865                 870                 875                 880

Met Met Asp Phe Phe His Ala Phe Leu Ile Ile Phe Arg Ile Leu Cys
                885                 890                 895

Gly Glu Trp Ile Glu Thr Met Trp Asp Cys Met Glu Val Ser Gly Gln
            900                 905                 910

Ser Leu Cys Leu Leu Val Phe Leu Leu Val Met Val Ile Gly Asn Leu
        915                 920                 925

Val Val Leu Asn Leu Phe Leu Ala Leu Leu Leu Ser Ser Phe Ser Ala
    930                 935                 940

Asp Asn Leu Thr Ala Pro Asp Glu Asp Arg Glu Met Asn Asn Leu Gln
945                 950                 955                 960

Leu Ala Leu Ala Arg Ile Gln Arg Gly Leu Arg Phe Val Lys Arg Thr
                965                 970                 975

Thr Trp Asp Phe Cys Cys Gly Leu Leu Arg Gln Arg Pro Gln Lys Pro
            980                 985                 990

Ala Ala Leu Ala Ala Gln Gly Gln  Leu Pro Ser Cys Ile  Ala Thr Pro
        995                 1000                1005

Tyr Ser  Pro Pro Pro Pro Glu  Thr Glu Lys Val Pro  Pro Thr Arg
    1010                 1015                 1020

Lys Glu  Thr Arg Phe Glu Glu  Gly Glu Gln Pro Gly  Gln Gly Thr
    1025                 1030                 1035

Pro Gly  Asp Pro Glu Pro Val  Cys Val Pro Ile Ala  Val Ala Glu
    1040                 1045                 1050

Ser Asp  Thr Asp Asp Gln Glu  Glu Asp Glu Glu Asn  Ser Leu Gly
    1055                 1060                 1065

Thr Glu  Glu Glu Ser Ser Lys  Gln Gln Glu Ser Gln  Pro Val Ser
    1070                 1075                 1080

Gly Gly  Pro Glu Ala Pro Pro  Asp Ser Arg Thr Trp  Ser Gln Val
    1085                 1090                 1095

Ser Ala  Thr Ala Ser Ser Glu  Ala Glu Ala Ser Ala  Ser Gln Ala
    1100                 1105                 1110

Asp Trp  Arg Gln Gln Trp Lys  Ala Glu Pro Gln Ala  Pro Gly Cys
```

```
    1115                1120                1125

Gly Glu  Thr Pro Glu Asp Ser  Cys Ser Glu Gly Ser  Thr Ala Asp
    1130                1135                1140

Met Thr  Asn Thr Ala Glu Leu  Leu Glu Gln Ile Pro  Asp Leu Gly
    1145                1150                1155

Gln Asp  Val Lys Asp Pro Glu  Asp Cys Phe Thr Glu  Gly Cys Val
    1160                1165                1170

Arg Arg  Cys Pro Cys Cys Ala  Val Asp Thr Thr Gln  Ala Pro Gly
    1175                1180                1185

Lys Val  Trp Trp Arg Leu Arg  Lys Thr Cys Tyr His  Ile Val Glu
    1190                1195                1200

His Ser  Trp Phe Glu Thr Phe  Ile Ile Phe Met Ile  Leu Leu Ser
    1205                1210                1215

Ser Gly  Ala Leu Ala Phe Glu  Asp Ile Tyr Leu Glu  Glu Arg Lys
    1220                1225                1230

Thr Ile  Lys Val Leu Leu Glu  Tyr Ala Asp Lys Met  Phe Thr Tyr
    1235                1240                1245

Val Phe  Val Leu Glu Met Leu  Leu Lys Trp Val Ala  Tyr Gly Phe
    1250                1255                1260

Lys Lys  Tyr Phe Thr Asn Ala  Trp Cys Trp Leu Asp  Phe Leu Ile
    1265                1270                1275

Val Asp  Val Ser Leu Val Ser  Leu Val Ala Asn Thr  Leu Gly Phe
    1280                1285                1290

Ala Glu  Met Gly Pro Ile Lys  Ser Leu Arg Thr Leu  Arg Ala Leu
    1295                1300                1305

Arg Pro  Leu Arg Ala Leu Ser  Arg Phe Glu Gly Met  Arg Val Val
    1310                1315                1320

Val Asn  Ala Leu Val Gly Ala  Ile Pro Ser Ile Met  Asn Val Leu
    1325                1330                1335

Leu Val  Cys Leu Ile Phe Trp  Leu Ile Phe Ser Ile  Met Gly Val
    1340                1345                1350

Asn Leu  Phe Ala Gly Lys Phe  Gly Arg Cys Ile Asn  Gln Thr Glu
    1355                1360                1365
```

Gly Asp Leu Pro Leu Asn Tyr Thr Ile Val Asn Asn Lys Ser Gln
1370 1375 1380

Cys Glu Ser Leu Asn Leu Thr Gly Glu Leu Tyr Trp Thr Lys Val
1385 1390 1395

Lys Val Asn Phe Asp Asn Val Gly Ala Gly Tyr Leu Ala Leu Leu
1400 1405 1410

Gln Val Ala Thr Phe Lys Gly Trp Met Asp Ile Met Tyr Ala Ala
1415 1420 1425

Val Asp Ser Arg Gly Tyr Glu Glu Gln Pro Gln Trp Glu Tyr Asn
1430 1435 1440

Leu Tyr Met Tyr Ile Tyr Phe Val Ile Phe Ile Ile Phe Gly Ser
1445 1450 1455

Phe Phe Thr Leu Asn Leu Phe Ile Gly Val Ile Ile Asp Asn Phe
1460 1465 1470

Asn Gln Gln Lys Lys Lys Leu Gly Gly Gln Asp Ile Phe Met Thr
1475 1480 1485

Glu Glu Gln Lys Lys Tyr Tyr Asn Ala Met Lys Lys Leu Gly Ser
1490 1495 1500

Lys Lys Pro Gln Lys Pro Ile Pro Arg Pro Leu Asn Lys Tyr Gln
1505 1510 1515

Gly Phe Ile Phe Asp Ile Val Thr Lys Gln Ala Phe Asp Val Thr
1520 1525 1530

Ile Met Phe Leu Ile Cys Leu Asn Met Val Thr Met Met Val Glu
1535 1540 1545

Thr Asp Asp Gln Ser Pro Glu Lys Ile Asn Ile Leu Ala Lys Ile
1550 1555 1560

Asn Leu Leu Phe Val Ala Ile Phe Thr Gly Glu Cys Ile Val Lys
1565 1570 1575

Leu Ala Ala Leu Arg His Tyr Tyr Phe Thr Asn Ser Trp Asn Ile
1580 1585 1590

Phe Asp Phe Val Val Val Ile Leu Ser Ile Val Gly Thr Val Leu
1595 1600 1605

Ser Asp Ile Ile Gln Lys Tyr Phe Phe Ser Pro Thr Leu Phe Arg
1610 1615 1620

Val Ile Arg Leu Ala Arg Ile Gly Arg Ile Leu Arg Leu Ile Arg
1625 1630 1635

Gly Ala Lys Gly Ile Arg Thr Leu Leu Phe Ala Leu Met Met Ser
1640 1645 1650

Leu Pro Ala Leu Phe Asn Ile Gly Leu Leu Leu Phe Leu Val Met
1655 1660 1665

Phe Ile Tyr Ser Ile Phe Gly Met Ala Asn Phe Ala Tyr Val Lys
1670 1675 1680

Trp Glu Ala Gly Ile Asp Asp Met Phe Asn Phe Gln Thr Phe Ala
1685 1690 1695

Asn Ser Met Leu Cys Leu Phe Gln Ile Thr Thr Ser Ala Gly Trp
1700 1705 1710

Asp Gly Leu Leu Ser Pro Ile Leu Asn Thr Gly Pro Pro Tyr Cys
1715 1720 1725

Asp Pro Thr Leu Pro Asn Ser Asn Gly Ser Arg Gly Asp Cys Gly
1730 1735 1740

Ser Pro Ala Val Gly Ile Leu Phe Phe Thr Thr Tyr Ile Ile Ile
1745 1750 1755

Ser Phe Leu Ile Val Val Asn Met Tyr Ile Ala Ile Ile Leu Glu
1760 1765 1770

Asn Phe Ser Val Ala Thr Glu Glu Ser Thr Glu Pro Leu Ser Glu
1775 1780 1785

Asp Asp Phe Asp Met Phe Tyr Glu Ile Trp Glu Lys Phe Asp Pro
1790 1795 1800

Glu Ala Thr Gln Phe Ile Glu Tyr Ser Val Leu Ser Asp Phe Ala
1805 1810 1815

Asp Ala Leu Ser Glu Pro Leu Arg Ile Ala Lys Pro Asn Gln Ile
1820 1825 1830

Ser Leu Ile Asn Met Asp Leu Pro Met Val Ser Gly Asp Arg Ile
1835 1840 1845

```
His Cys Met Asp Ile Leu Phe Ala Phe Thr Lys Arg Val Leu Gly
    1850                1855                1860

Glu Ser Gly Glu Met Asp Ala Leu Lys Ile Gln Met Glu Glu Lys
    1865                1870                1875

Phe Met Ala Ala Asn Pro Ser Lys Ile Ser Tyr Glu Pro Ile Thr
    1880                1885                1890

Thr Thr Leu Arg Arg Lys His Glu Glu Val Ser Ala Met Val Ile
    1895                1900                1905

Gln Arg Ala Phe Arg Arg His Leu Leu Gln Arg Ser Leu Lys His
    1910                1915                1920

Ala Ser Phe Leu Phe Arg Gln Gln Ala Gly Ser Gly Leu Ser Glu
    1925                1930                1935

Glu Asp Ala Pro Glu Arg Glu Gly Leu Ile Ala Tyr Val Met Ser
    1940                1945                1950

Glu Asn Phe Ser Arg Pro Leu Gly Pro Pro Ser Ser Ser Ser Ile
    1955                1960                1965

Ser Ser Thr Ser Phe Pro Pro Ser Tyr Asp Ser Val Thr Arg Ala
    1970                1975                1980

Thr Ser Asp Asn Leu Gln Val Arg Gly Ser Asp Tyr Ser His Ser
    1985                1990                1995

Glu Asp Leu Ala Asp Phe Pro Pro Ser Pro Asp Arg Asp Arg Glu
    2000                2005                2010

Ser Ile Val
    2015
```

<210> 25
<211> 1682
<212> PRT
<213> Homo sapiens

<400> 25

```
Met Leu Ala Ser Pro Glu Pro Lys Gly Leu Val Pro Phe Thr Lys Glu
1               5                   10                  15

Ser Phe Glu Leu Ile Lys Gln His Ile Ala Lys Thr His Asn Glu Asp
            20                  25                  30

His Glu Glu Glu Asp Leu Lys Pro Thr Pro Asp Leu Glu Val Gly Lys
        35                  40                  45
```

```
Lys Leu Pro Phe Ile Tyr Gly Asn Leu Ser Gln Gly Met Val Ser Glu
    50                  55                  60

Pro Leu Glu Asp Val Asp Pro Tyr Tyr Tyr Lys Lys Lys Asn Thr Phe
65                  70                  75                  80

Ile Val Leu Asn Lys Asn Arg Thr Ile Phe Arg Phe Asn Ala Ala Ser
                85                  90                  95

Ile Leu Cys Thr Leu Ser Pro Phe Asn Cys Ile Arg Arg Thr Thr Ile
            100                 105                 110

Lys Val Leu Val His Pro Phe Phe Gln Leu Phe Ile Leu Ile Ser Val
        115                 120                 125

Leu Ile Asp Cys Val Phe Met Ser Leu Thr Asn Leu Pro Lys Trp Arg
    130                 135                 140

Pro Val Leu Glu Asn Thr Leu Leu Gly Ile Tyr Thr Phe Glu Ile Leu
145                 150                 155                 160

Val Lys Leu Phe Ala Arg Gly Val Trp Ala Gly Ser Phe Ser Phe Leu
                165                 170                 175

Gly Asp Pro Trp Asn Trp Leu Asp Phe Ser Val Thr Val Phe Glu Val
            180                 185                 190

Ile Ile Arg Tyr Ser Pro Leu Asp Phe Ile Pro Thr Leu Gln Thr Ala
        195                 200                 205

Arg Thr Leu Arg Ile Leu Lys Ile Ile Pro Leu Asn Gln Gly Leu Lys
    210                 215                 220

Ser Leu Val Gly Val Leu Ile His Cys Leu Lys Gln Leu Ile Gly Val
225                 230                 235                 240

Ile Ile Leu Thr Leu Phe Phe Leu Ser Ile Phe Ser Leu Ile Gly Met
                245                 250                 255

Gly Leu Phe Met Gly Asn Leu Lys His Lys Cys Phe Arg Trp Pro Gln
            260                 265                 270

Glu Asn Glu Asn Glu Thr Leu His Asn Arg Thr Gly Asn Pro Tyr Tyr
        275                 280                 285

Ile Arg Glu Thr Glu Asn Phe Tyr Tyr Leu Glu Gly Glu Arg Tyr Ala
    290                 295                 300
```

Leu Leu Cys Gly Asn Arg Thr Asp Ala Gly Gln Cys Pro Glu Gly Tyr
305                 310                 315                 320

Val Cys Val Lys Ala Gly Ile Asn Pro Asp Gln Gly Phe Thr Asn Phe
                325                 330                 335

Asp Ser Phe Gly Trp Ala Leu Phe Ala Leu Phe Arg Leu Met Ala Gln
            340                 345                 350

Asp Tyr Pro Glu Val Leu Tyr His Gln Ile Leu Tyr Ala Ser Gly Lys
        355                 360                 365

Val Tyr Met Ile Phe Phe Val Val Val Ser Phe Leu Phe Ser Phe Tyr
    370                 375                 380

Met Ala Ser Leu Phe Leu Gly Ile Leu Ala Met Ala Tyr Glu Glu Glu
385                 390                 395                 400

Lys Gln Arg Val Gly Glu Ile Ser Lys Lys Ile Glu Pro Lys Phe Gln
                405                 410                 415

Gln Thr Gly Lys Glu Leu Gln Glu Gly Asn Glu Thr Asp Glu Ala Lys
            420                 425                 430

Thr Ile Gln Ile Glu Met Lys Lys Arg Ser Pro Ile Ser Thr Asp Thr
        435                 440                 445

Ser Leu Asp Val Leu Glu Asp Ala Thr Leu Arg His Lys Glu Glu Leu
    450                 455                 460

Glu Lys Ser Lys Lys Ile Cys Pro Leu Tyr Trp Tyr Lys Phe Ala Lys
465                 470                 475                 480

Thr Phe Leu Ile Trp Asn Cys Ser Pro Cys Trp Leu Lys Leu Lys Glu
                485                 490                 495

Phe Val His Arg Ile Ile Met Ala Pro Phe Thr Asp Leu Phe Leu Ile
            500                 505                 510

Ile Cys Ile Ile Leu Asn Val Cys Phe Leu Thr Leu Glu His Tyr Pro
        515                 520                 525

Met Ser Lys Gln Thr Asn Thr Leu Leu Asn Ile Gly Asn Leu Val Phe
    530                 535                 540

Ile Gly Ile Phe Thr Ala Glu Met Ile Phe Lys Ile Ile Ala Met His
545                 550                 555                 560

Pro Tyr Gly Tyr Phe Gln Val Gly Trp Asn Ile Phe Asp Ser Met Ile

```
                565                 570                 575

Val Phe His Gly Leu Ile Glu Leu Cys Leu Ala Asn Val Ala Gly Met
            580                 585                 590

Ala Leu Leu Arg Leu Phe Arg Met Leu Arg Ile Phe Lys Leu Gly Lys
        595                 600                 605

Tyr Trp Pro Thr Phe Gln Ile Leu Met Trp Ser Leu Ser Asn Ser Trp
    610                 615                 620

Val Ala Leu Lys Asp Leu Val Leu Leu Leu Phe Thr Phe Ile Phe Phe
625                 630                 635                 640

Ser Ala Ala Phe Gly Met Lys Leu Phe Gly Lys Asn Tyr Glu Glu Phe
                645                 650                 655

Val Cys His Ile Asp Lys Asp Cys Gln Leu Pro Arg Trp His Met His
            660                 665                 670

Asp Phe Phe His Ser Phe Leu Asn Val Phe Arg Ile Leu Cys Gly Glu
        675                 680                 685

Trp Val Glu Thr Leu Trp Asp Cys Met Glu Val Ala Gly Gln Ser Trp
    690                 695                 700

Cys Ile Pro Phe Tyr Leu Met Val Ile Leu Ile Gly Asn Leu Leu Val
705                 710                 715                 720

Leu Tyr Leu Phe Leu Ala Leu Val Ser Ser Phe Ser Ser Cys Lys Asp
                725                 730                 735

Val Thr Ala Glu Glu Asn Asn Glu Ala Lys Asn Leu Gln Leu Ala Val
            740                 745                 750

Ala Arg Ile Lys Lys Gly Ile Asn Tyr Val Leu Leu Lys Ile Leu Cys
        755                 760                 765

Lys Thr Gln Asn Val Pro Lys Asp Thr Met Asp His Val Asn Glu Val
    770                 775                 780

Tyr Val Lys Glu Asp Ile Ser Asp His Thr Leu Ser Glu Leu Ser Asn
785                 790                 795                 800

Thr Gln Asp Phe Leu Lys Asp Lys Glu Lys Ser Ser Gly Thr Glu Lys
                805                 810                 815

Asn Ala Thr Glu Asn Glu Ser Gln Ser Leu Ile Pro Ser Pro Ser Val
            820                 825                 830
```

```
Ser Glu Thr Val Pro Ile Ala Ser Gly Glu Ser Asp Ile Glu Asn Leu
        835                 840                 845

Asp Asn Lys Glu Ile Gln Ser Lys Ser Gly Asp Gly Gly Ser Lys Glu
    850                 855                 860

Lys Ile Lys Gln Ser Ser Ser Ser Glu Cys Ser Thr Val Asp Ile Ala
865                 870                 875                 880

Ile Ser Glu Glu Glu Glu Met Phe Tyr Gly Gly Glu Arg Ser Lys His
                885                 890                 895

Leu Lys Asn Gly Cys Arg Arg Gly Ser Ser Leu Gly Gln Ile Ser Gly
            900                 905                 910

Ala Ser Lys Lys Gly Lys Ile Trp Gln Asn Ile Arg Lys Thr Cys Cys
        915                 920                 925

Lys Ile Val Glu Asn Asn Trp Phe Lys Cys Phe Ile Gly Leu Val Thr
    930                 935                 940

Leu Leu Ser Thr Gly Thr Leu Ala Phe Glu Asp Ile Tyr Met Asp Gln
945                 950                 955                 960

Arg Lys Thr Ile Lys Ile Leu Leu Glu Tyr Ala Asp Met Ile Phe Thr
                965                 970                 975

Tyr Ile Phe Ile Leu Glu Met Leu Leu Lys Trp Met Ala Tyr Gly Phe
            980                 985                 990

Lys Ala Tyr Phe Ser Asn Gly Trp  Tyr Arg Leu Asp Phe  Val Val Val
        995                 1000                1005

Ile Val  Phe Cys Leu Ser Leu  Ile Gly Lys Thr Arg  Glu Glu Leu
    1010                 1015                 1020

Lys Pro  Leu Ile Ser Met Lys  Phe Leu Arg Pro Leu  Arg Val Leu
    1025                 1030                 1035

Ser Gln  Phe Glu Arg Met Lys  Val Val Val Arg Ala  Leu Ile Lys
    1040                 1045                 1050

Thr Thr  Leu Pro Thr Leu Asn  Val Phe Leu Val Cys  Leu Met Ile
    1055                 1060                 1065

Trp Leu  Ile Phe Ser Ile Met  Gly Val Asp Leu Phe  Ala Gly Arg
    1070                 1075                 1080
```

```
Phe Tyr  Glu Cys Ile Asp Pro  Thr Ser Gly Glu Arg  Phe Pro Ser
    1085                 1090                 1095

Ser Glu  Val Met Asn Lys Ser  Arg Cys Glu Ser Leu  Leu Phe Asn
    1100                 1105                 1110

Glu Ser  Met Leu Trp Glu Asn  Ala Lys Met Asn Phe  Asp Asn Val
    1115                 1120                 1125

Gly Asn  Gly Phe Leu Ser Leu  Leu Gln Val Ala Thr  Phe Asn Gly
    1130                 1135                 1140

Trp Ile  Thr Ile Met Asn Ser  Ala Ile Asp Ser Val  Ala Val Asn
    1145                 1150                 1155

Ile Gln  Pro His Phe Glu Val  Asn Ile Tyr Met Tyr  Cys Tyr Phe
    1160                 1165                 1170

Ile Asn  Phe Ile Ile Phe Gly  Val Phe Leu Pro Leu  Ser Met Leu
    1175                 1180                 1185

Ile Thr  Val Ile Ile Asp Asn  Phe Asn Lys His Lys  Ile Lys Leu
    1190                 1195                 1200

Gly Gly  Ser Asn Ile Phe Ile  Thr Val Lys Gln Arg  Lys Gln Tyr
    1205                 1210                 1215

Arg Arg  Leu Lys Lys Leu Met  Tyr Glu Asp Ser Gln  Arg Pro Val
    1220                 1225                 1230

Pro Arg  Pro Leu Asn Lys Leu  Gln Gly Phe Ile Phe  Asp Val Val
    1235                 1240                 1245

Thr Ser  Gln Ala Phe Asn Val  Ile Val Met Val Leu  Ile Cys Phe
    1250                 1255                 1260

Gln Ala  Ile Ala Met Met Ile  Asp Thr Asp Val Gln  Ser Leu Gln
    1265                 1270                 1275

Met Ser  Ile Ala Leu Tyr Trp  Ile Asn Ser Ile Phe  Val Met Leu
    1280                 1285                 1290

Tyr Thr  Met Glu Cys Ile Leu  Lys Leu Ile Ala Phe  Arg Cys Phe
    1295                 1300                 1305

Tyr Phe  Thr Ile Ala Trp Asn  Ile Phe Asp Phe Met  Val Val Ile
    1310                 1315                 1320
```

Phe Ser Ile Thr Gly Leu Cys Leu Pro Met Thr Val Gly Ser Tyr
1325 1330 1335

Leu Val Pro Pro Ser Leu Val Gln Leu Ile Leu Leu Ser Arg Ile
1340 1345 1350

Ile His Met Leu Arg Leu Gly Lys Gly Pro Lys Val Phe His Asn
1355 1360 1365

Leu Met Leu Pro Leu Met Leu Ser Leu Pro Ala Leu Leu Asn Ile
1370 1375 1380

Ile Leu Leu Ile Phe Leu Val Met Phe Ile Tyr Ala Val Phe Gly
1385 1390 1395

Met Tyr Asn Phe Ala Tyr Val Lys Lys Glu Ala Gly Ile Asn Asp
1400 1405 1410

Val Ser Asn Phe Glu Thr Phe Gly Asn Ser Met Leu Cys Leu Phe
1415 1420 1425

Gln Val Ala Ile Phe Ala Gly Trp Asp Gly Met Leu Asp Ala Ile
1430 1435 1440

Phe Asn Ser Lys Trp Ser Asp Cys Asp Pro Asp Lys Ile Asn Pro
1445 1450 1455

Gly Thr Gln Val Arg Gly Asp Cys Gly Asn Pro Ser Val Gly Ile
1460 1465 1470

Phe Tyr Phe Val Ser Tyr Ile Leu Ile Ser Trp Leu Ile Ile Val
1475 1480 1485

Asn Met Tyr Ile Val Val Val Met Glu Phe Leu Asn Ile Ala Ser
1490 1495 1500

Lys Lys Lys Asn Lys Thr Leu Ser Glu Asp Asp Phe Arg Lys Phe
1505 1510 1515

Phe Gln Val Trp Lys Arg Phe Asp Pro Asp Arg Thr Gln Tyr Ile
1520 1525 1530

Asp Ser Ser Lys Leu Ser Asp Phe Ala Ala Ala Leu Asp Pro Pro
1535 1540 1545

Leu Phe Met Ala Lys Pro Asn Lys Gly Gln Leu Ile Ala Leu Asp
1550 1555 1560

Leu Pro Met Ala Val Gly Asp Arg Ile His Cys Leu Asp Ile Leu

```
    1565                1570                1575

Leu Ala  Phe Thr Lys Arg Val  Met Gly Gln Asp Val  Arg Met Glu
    1580                1585                1590

Lys Val  Val Ser Glu Ile Glu  Ser Gly Phe Leu Leu  Ala Asn Pro
    1595                1600                1605

Phe Lys  Ile Thr Cys Glu Pro  Ile Thr Thr Thr Leu  Lys Arg Lys
    1610                1615                1620

Gln Glu  Ala Val Ser Ala Thr  Ile Ile Gln Arg Ala  Tyr Lys Asn
    1625                1630                1635

Tyr Arg  Leu Arg Arg Asn Asp  Lys Asn Thr Ser Asp  Ile His Met
    1640                1645                1650

Ile Asp  Gly Asp Arg Asp Val  His Ala Thr Lys Glu  Gly Ala Tyr
    1655                1660                1665

Phe Asp  Lys Ala Lys Glu Lys  Ser Pro Ile Gln Ser  Gln Ile
    1670                1675                1680
```

<210> 26
<211> 1980
<212> PRT
<213> Homo sapiens

<400> 26

Met Ala Ala Arg Leu Leu Ala Pro Pro Gly Pro Asp Ser Phe Lys Pro
1               5                   10                  15

Phe Thr Pro Glu Ser Leu Ala Asn Ile Glu Arg Arg Ile Ala Glu Ser
            20                  25                  30

Lys Leu Lys Lys Pro Pro Lys Ala Asp Gly Ser His Arg Glu Asp Asp
        35                  40                  45

Glu Asp Ser Lys Pro Lys Pro Asn Ser Asp Leu Glu Ala Gly Lys Ser
    50                  55                  60

Leu Pro Phe Ile Tyr Gly Asp Ile Pro Gln Gly Leu Val Ala Val Pro
65                  70                  75                  80

Leu Glu Asp Phe Asp Pro Tyr Tyr Leu Thr Gln Lys Thr Phe Val Val
                85                  90                  95

Leu Asn Arg Gly Lys Thr Leu Phe Arg Phe Ser Ala Thr Pro Ala Leu
            100                 105                 110

Tyr Ile Leu Ser Pro Phe Asn Leu Ile Arg Arg Ile Ala Ile Lys Ile
115 120 125

Leu Ile His Ser Val Phe Ser Met Ile Ile Met Cys Thr Ile Leu Thr
130 135 140

Asn Cys Val Phe Met Thr Phe Ser Asn Pro Pro Asp Trp Ser Lys Asn
145 150 155 160

Val Glu Tyr Thr Phe Thr Gly Ile Tyr Thr Phe Glu Ser Leu Val Lys
165 170 175

Ile Ile Ala Arg Gly Phe Cys Ile Asp Gly Phe Thr Phe Leu Arg Asp
180 185 190

Pro Trp Asn Trp Leu Asp Phe Ser Val Ile Met Met Ala Tyr Ile Thr
195 200 205

Glu Phe Val Asn Leu Gly Asn Val Ser Ala Leu Arg Thr Phe Arg Val
210 215 220

Leu Arg Ala Leu Lys Thr Ile Ser Val Ile Pro Gly Leu Lys Thr Ile
225 230 235 240

Val Gly Ala Leu Ile Gln Ser Val Lys Lys Leu Ser Asp Val Met Ile
245 250 255

Leu Thr Val Phe Cys Leu Ser Val Phe Ala Leu Ile Gly Leu Gln Leu
260 265 270

Phe Met Gly Asn Leu Arg Asn Lys Cys Val Val Trp Pro Ile Asn Phe
275 280 285

Asn Glu Ser Tyr Leu Glu Asn Gly Thr Lys Gly Phe Asp Trp Glu Glu
290 295 300

Tyr Ile Asn Asn Lys Thr Asn Phe Tyr Thr Val Pro Gly Met Leu Glu
305 310 315 320

Pro Leu Leu Cys Gly Asn Ser Ser Asp Ala Gly Gln Cys Pro Glu Gly
325 330 335

Tyr Gln Cys Met Lys Ala Gly Arg Asn Pro Asn Tyr Gly Tyr Thr Ser
340 345 350

Phe Asp Thr Phe Ser Trp Ala Phe Leu Ala Leu Phe Arg Leu Met Thr
355 360 365

Gln Asp Tyr Trp Glu Asn Leu Tyr Gln Leu Thr Leu Arg Ala Ala Gly

370 375 380

Lys Thr Tyr Met Ile Phe Phe Val Leu Val Ile Phe Val Gly Ser Phe
385 390 395 400

Tyr Leu Val Asn Leu Ile Leu Ala Val Val Ala Met Ala Tyr Glu Glu
405 410 415

Gln Asn Gln Ala Thr Leu Glu Glu Ala Glu Gln Lys Glu Ala Glu Phe
420 425 430

Lys Ala Met Leu Glu Gln Leu Lys Lys Gln Gln Glu Glu Ala Gln Ala
435 440 445

Ala Ala Met Ala Thr Ser Ala Gly Thr Val Ser Glu Asp Ala Ile Glu
450 455 460

Glu Glu Gly Glu Glu Gly Gly Gly Ser Pro Arg Ser Ser Ser Glu Ile
465 470 475 480

Ser Lys Leu Ser Ser Lys Ser Ala Lys Glu Arg Arg Asn Arg Arg Lys
485 490 495

Lys Arg Lys Gln Lys Glu Leu Ser Glu Gly Glu Glu Lys Gly Asp Pro
500 505 510

Glu Lys Val Phe Lys Ser Glu Ser Glu Asp Gly Met Arg Arg Lys Ala
515 520 525

Phe Arg Leu Pro Asp Asn Arg Ile Gly Arg Lys Phe Ser Ile Met Asn
530 535 540

Gln Ser Leu Leu Ser Ile Pro Gly Ser Pro Phe Leu Ser Arg His Asn
545 550 555 560

Ser Lys Ser Ser Ile Phe Ser Phe Arg Gly Pro Gly Arg Phe Arg Asp
565 570 575

Pro Gly Ser Glu Asn Glu Phe Ala Asp Asp Glu His Ser Thr Val Glu
580 585 590

Glu Ser Glu Gly Arg Arg Asp Ser Leu Phe Ile Pro Ile Arg Ala Arg
595 600 605

Glu Arg Arg Ser Ser Tyr Ser Gly Tyr Ser Gly Tyr Ser Gln Gly Ser
610 615 620

Arg Ser Ser Arg Ile Phe Pro Ser Leu Arg Arg Ser Val Lys Arg Asn
625 630 635 640

Ser Thr Val Asp Cys Asn Gly Val Val Ser Leu Ile Gly Gly Pro Gly
645 650 655

Ser His Ile Gly Gly Arg Leu Leu Pro Glu Ala Thr Thr Glu Val Glu
660 665 670

Ile Lys Lys Lys Gly Pro Gly Ser Leu Leu Val Ser Met Asp Gln Leu
675 680 685

Ala Ser Tyr Gly Arg Lys Asp Arg Ile Asn Ser Ile Met Ser Val Val
690 695 700

Thr Asn Thr Leu Val Glu Glu Leu Glu Glu Ser Gln Arg Lys Cys Pro
705 710 715 720

Pro Cys Trp Tyr Lys Phe Ala Asn Thr Phe Leu Ile Trp Glu Cys His
725 730 735

Pro Tyr Trp Ile Lys Leu Lys Glu Ile Val Asn Leu Ile Val Met Asp
740 745 750

Pro Phe Val Asp Leu Ala Ile Thr Ile Cys Ile Val Leu Asn Thr Leu
755 760 765

Phe Met Ala Met Glu His His Pro Met Thr Pro Gln Phe Glu His Val
770 775 780

Leu Ala Val Gly Asn Leu Val Phe Thr Gly Ile Phe Thr Ala Glu Met
785 790 795 800

Phe Leu Lys Leu Ile Ala Met Asp Pro Tyr Tyr Tyr Phe Gln Glu Gly
805 810 815

Trp Asn Ile Phe Asp Gly Phe Ile Val Ser Leu Ser Leu Met Glu Leu
820 825 830

Ser Leu Ala Asp Val Glu Gly Leu Ser Val Leu Arg Ser Phe Arg Leu
835 840 845

Leu Arg Val Phe Lys Leu Ala Lys Ser Trp Pro Thr Leu Asn Met Leu
850 855 860

Ile Lys Ile Ile Gly Asn Ser Val Gly Ala Leu Gly Asn Leu Thr Leu
865 870 875 880

Val Leu Ala Ile Ile Val Phe Ile Phe Ala Val Val Gly Met Gln Leu
885 890 895

```
Phe Gly Lys Ser Tyr Lys Glu Cys Val Cys Lys Ile Asn Gln Asp Cys
            900                 905                 910

Glu Leu Pro Arg Trp His Met His Asp Phe Phe His Ser Phe Leu Ile
        915                 920                 925

Val Phe Arg Val Leu Cys Gly Glu Trp Ile Glu Thr Met Trp Asp Cys
    930                 935                 940

Met Glu Val Ala Gly Gln Ala Met Cys Leu Ile Val Phe Met Met Val
945                 950                 955                 960

Met Val Ile Gly Asn Leu Val Val Leu Asn Leu Phe Leu Ala Leu Leu
                965                 970                 975

Leu Ser Ser Phe Ser Ala Asp Asn Leu Ala Ala Thr Asp Asp Asp Gly
            980                 985                 990

Glu Met Asn Asn Leu Gln Ile Ser  Val Ile Arg Ile Lys  Lys Gly Val
        995                 1000                1005

Ala Trp  Thr Lys Leu Lys Val  His Ala Phe Met Gln  Ala His Phe
    1010                 1015                 1020

Lys Gln  Arg Glu Ala Asp Glu  Val Lys Pro Leu Asp  Glu Leu Tyr
    1025                 1030                 1035

Glu Lys  Lys Ala Asn Cys Ile  Ala Asn His Thr Gly  Ala Asp Ile
    1040                 1045                 1050

His Arg  Asn Gly Asp Phe Gln  Lys Asn Gly Asn Gly  Thr Thr Ser
    1055                 1060                 1065

Gly Ile  Gly Ser Ser Val Glu  Lys Tyr Ile Ile Asp  Glu Asp His
    1070                 1075                 1080

Met Ser  Phe Ile Asn Asn Pro  Asn Leu Thr Val Arg  Val Pro Ile
    1085                 1090                 1095

Ala Val  Gly Glu Ser Asp Phe  Glu Asn Leu Asn Thr  Glu Asp Val
    1100                 1105                 1110

Ser Ser  Glu Ser Asp Pro Glu  Gly Ser Lys Asp Lys  Leu Asp Asp
    1115                 1120                 1125

Thr Ser  Ser Ser Glu Gly Ser  Thr Ile Asp Ile Lys  Pro Glu Val
    1130                 1135                 1140
```

Glu Glu Val Pro Val Glu Gln Pro Glu Glu Tyr Leu Asp Pro Asp
1145 1150 1155

Ala Cys Phe Thr Glu Gly Cys Val Gln Arg Phe Lys Cys Cys Gln
1160 1165 1170

Val Asn Ile Glu Glu Gly Leu Gly Lys Ser Trp Trp Ile Leu Arg
1175 1180 1185

Lys Thr Cys Phe Leu Ile Val Glu His Asn Trp Phe Glu Thr Phe
1190 1195 1200

Ile Ile Phe Met Ile Leu Leu Ser Ser Gly Ala Leu Ala Phe Glu
1205 1210 1215

Asp Ile Tyr Ile Glu Gln Arg Lys Thr Ile Arg Thr Ile Leu Glu
1220 1225 1230

Tyr Ala Asp Lys Val Phe Thr Tyr Ile Phe Ile Leu Glu Met Leu
1235 1240 1245

Leu Lys Trp Thr Ala Tyr Gly Phe Val Lys Phe Phe Thr Asn Ala
1250 1255 1260

Trp Cys Trp Leu Asp Phe Leu Ile Val Ala Val Ser Leu Val Ser
1265 1270 1275

Leu Ile Ala Asn Ala Leu Gly Tyr Ser Glu Leu Gly Ala Ile Lys
1280 1285 1290

Ser Leu Arg Thr Leu Arg Ala Leu Arg Pro Leu Arg Ala Leu Ser
1295 1300 1305

Arg Phe Glu Gly Met Arg Val Val Val Asn Ala Leu Val Gly Ala
1310 1315 1320

Ile Pro Ser Ile Met Asn Val Leu Leu Val Cys Leu Ile Phe Trp
1325 1330 1335

Leu Ile Phe Ser Ile Met Gly Val Asn Leu Phe Ala Gly Lys Tyr
1340 1345 1350

His Tyr Cys Phe Asn Glu Thr Ser Glu Ile Arg Phe Glu Ile Glu
1355 1360 1365

Asp Val Asn Asn Lys Thr Glu Cys Glu Lys Leu Met Glu Gly Asn
1370 1375 1380

Asn Thr Glu Ile Arg Trp Lys Asn Val Lys Ile Asn Phe Asp Asn

1385 1390 1395

Val Gly Ala Gly Tyr Leu Ala Leu Leu Gln Val Ala Thr Phe Lys
1400 1405 1410

Gly Trp Met Asp Ile Met Tyr Ala Ala Val Asp Ser Arg Lys Pro
1415 1420 1425

Asp Glu Gln Pro Lys Tyr Glu Asp Asn Ile Tyr Met Tyr Ile Tyr
1430 1435 1440

Phe Val Ile Phe Ile Ile Phe Gly Ser Phe Phe Thr Leu Asn Leu
1445 1450 1455

Phe Ile Gly Val Ile Ile Asp Asn Phe Asn Gln Gln Lys Lys Lys
1460 1465 1470

Phe Gly Gly Gln Asp Ile Phe Met Thr Glu Glu Gln Lys Lys Tyr
1475 1480 1485

Tyr Asn Ala Met Lys Lys Leu Gly Ser Lys Lys Pro Gln Lys Pro
1490 1495 1500

Ile Pro Arg Pro Leu Asn Lys Ile Gln Gly Ile Val Phe Asp Phe
1505 1510 1515

Val Thr Gln Gln Ala Phe Asp Ile Val Ile Met Met Leu Ile Cys
1520 1525 1530

Leu Asn Met Val Thr Met Met Val Glu Thr Asp Thr Gln Ser Lys
1535 1540 1545

Gln Met Glu Asn Ile Leu Tyr Trp Ile Asn Leu Val Phe Val Ile
1550 1555 1560

Phe Phe Thr Cys Glu Cys Val Leu Lys Met Phe Ala Leu Arg His
1565 1570 1575

Tyr Tyr Phe Thr Ile Gly Trp Asn Ile Phe Asp Phe Val Val Val
1580 1585 1590

Ile Leu Ser Ile Val Gly Met Phe Leu Ala Asp Ile Ile Glu Lys
1595 1600 1605

Tyr Phe Val Ser Pro Thr Leu Phe Arg Val Ile Arg Leu Ala Arg
1610 1615 1620

Ile Gly Arg Ile Leu Arg Leu Ile Lys Gly Ala Lys Gly Ile Arg
1625 1630 1635

```
Thr Leu  Leu Phe Ala Leu Met  Met Ser Leu Pro Ala  Leu Phe Asn
    1640                 1645                 1650

Ile Gly  Leu Leu Leu Phe Leu  Val Met Phe Ile Phe  Ser Ile Phe
    1655                 1660                 1665

Gly Met  Ser Asn Phe Ala Tyr  Val Lys His Glu Ala  Gly Ile Asp
    1670                 1675                 1680

Asp Met  Phe Asn Phe Glu Thr  Phe Gly Asn Ser Met  Ile Cys Leu
    1685                 1690                 1695

Phe Gln  Ile Thr Thr Ser Ala  Gly Trp Asp Gly Leu  Leu Leu Pro
    1700                 1705                 1710

Ile Leu  Asn Arg Pro Pro Asp  Cys Ser Leu Asp Lys  Glu His Pro
    1715                 1720                 1725

Gly Ser  Gly Phe Lys Gly Asp  Cys Gly Asn Pro Ser  Val Gly Ile
    1730                 1735                 1740

Phe Phe  Phe Val Ser Tyr Ile  Ile Ile Ser Phe Leu  Ile Val Val
    1745                 1750                 1755

Asn Met  Tyr Ile Ala Ile Ile  Leu Glu Asn Phe Ser  Val Ala Thr
    1760                 1765                 1770

Glu Glu  Ser Ala Asp Pro Leu  Ser Glu Asp Asp Phe  Glu Thr Phe
    1775                 1780                 1785

Tyr Glu  Ile Trp Glu Lys Phe  Asp Pro Asp Ala Thr  Gln Phe Ile
    1790                 1795                 1800

Glu Tyr  Cys Lys Leu Ala Asp  Phe Ala Asp Ala Leu  Glu His Pro
    1805                 1810                 1815

Leu Arg  Val Pro Lys Pro Asn  Thr Ile Glu Leu Ile  Ala Met Asp
    1820                 1825                 1830

Leu Pro  Met Val Ser Gly Asp  Arg Ile His Cys Leu  Asp Ile Leu
    1835                 1840                 1845

Phe Ala  Phe Thr Lys Arg Val  Leu Gly Asp Ser Gly  Glu Leu Asp
    1850                 1855                 1860

Ile Leu  Arg Gln Gln Met Glu  Glu Arg Phe Val Ala  Ser Asn Pro
    1865                 1870                 1875
```

```
Ser Lys  Val Ser Tyr Glu Pro  Ile Thr Thr Thr Leu  Arg Arg Lys
    1880                 1885                 1890

Gln Glu  Glu Val Ser Ala Val  Val Leu Gln Arg Ala  Tyr Arg Gly
    1895                 1900                 1905

His Leu  Ala Arg Arg Gly Phe  Ile Cys Lys Lys Thr  Thr Ser Asn
    1910                 1915                 1920

Lys Leu  Glu Asn Gly Gly Thr  His Arg Glu Lys Lys  Glu Ser Thr
    1925                 1930                 1935

Pro Ser  Thr Ala Ser Leu Pro  Ser Tyr Asp Ser Val  Thr Lys Pro
    1940                 1945                 1950

Glu Lys  Glu Lys Gln Gln Arg  Ala Glu Glu Gly Arg  Arg Glu Arg
    1955                 1960                 1965

Ala Lys  Arg Gln Lys Glu Val  Arg Glu Ser Lys Cys
    1970                 1975                 1980
```

<210> 27
<211> 1977
<212> PRT
<213> Homo sapiens

<400> 27

```
Met Ala Met Leu Pro Pro Pro Gly Pro Gln Ser Phe Val His Phe Thr
1               5                   10                  15

Lys Gln Ser Leu Ala Leu Ile Glu Gln Arg Ile Ala Glu Arg Lys Ser
            20                  25                  30

Lys Glu Pro Lys Glu Glu Lys Lys Asp Asp Asp Glu Glu Ala Pro Lys
        35                  40                  45

Pro Ser Ser Asp Leu Glu Ala Gly Lys Gln Leu Pro Phe Ile Tyr Gly
    50                  55                  60

Asp Ile Pro Pro Gly Met Val Ser Glu Pro Leu Glu Asp Leu Asp Pro
65                  70                  75                  80

Tyr Tyr Ala Asp Lys Lys Thr Phe Ile Val Leu Asn Lys Gly Lys Thr
                85                  90                  95

Ile Phe Arg Phe Asn Ala Thr Pro Ala Leu Tyr Met Leu Ser Pro Phe
            100                 105                 110

Ser Pro Leu Arg Arg Ile Ser Ile Lys Ile Leu Val His Ser Leu Phe
        115                 120                 125
```

```
Ser Met Leu Ile Met Cys Thr Ile Leu Thr Asn Cys Ile Phe Met Thr
    130                 135                 140

Met Asn Asn Pro Pro Asp Trp Thr Lys Asn Val Glu Tyr Thr Phe Thr
145                 150                 155                 160

Gly Ile Tyr Thr Phe Glu Ser Leu Val Lys Ile Leu Ala Arg Gly Phe
                165                 170                 175

Cys Val Gly Glu Phe Thr Phe Leu Arg Asp Pro Trp Asn Trp Leu Asp
            180                 185                 190

Phe Val Val Ile Val Phe Ala Tyr Leu Thr Glu Phe Val Asn Leu Gly
        195                 200                 205

Asn Val Ser Ala Leu Arg Thr Phe Arg Val Leu Arg Ala Leu Lys Thr
    210                 215                 220

Ile Ser Val Ile Pro Gly Leu Lys Thr Ile Val Gly Ala Leu Ile Gln
225                 230                 235                 240

Ser Val Lys Lys Leu Ser Asp Val Met Ile Leu Thr Val Phe Cys Leu
                245                 250                 255

Ser Val Phe Ala Leu Ile Gly Leu Gln Leu Phe Met Gly Asn Leu Lys
            260                 265                 270

His Lys Cys Phe Arg Asn Ser Leu Glu Asn Asn Glu Thr Leu Glu Ser
        275                 280                 285

Ile Met Asn Thr Leu Glu Ser Glu Glu Asp Phe Arg Lys Tyr Phe Tyr
    290                 295                 300

Tyr Leu Glu Gly Ser Lys Asp Ala Leu Leu Cys Gly Phe Ser Thr Asp
305                 310                 315                 320

Ser Gly Gln Cys Pro Glu Gly Tyr Thr Cys Val Lys Ile Gly Arg Asn
                325                 330                 335

Pro Asp Tyr Gly Tyr Thr Ser Phe Asp Thr Phe Ser Trp Ala Phe Leu
            340                 345                 350

Ala Leu Phe Arg Leu Met Thr Gln Asp Tyr Trp Glu Asn Leu Tyr Gln
        355                 360                 365

Gln Thr Leu Arg Ala Ala Gly Lys Thr Tyr Met Ile Phe Phe Val Val
    370                 375                 380
```

Val Ile Phe Leu Gly Ser Phe Tyr Leu Ile Asn Leu Ile Leu Ala Val
385 390 395 400

Val Ala Met Ala Tyr Glu Glu Gln Asn Gln Ala Asn Ile Glu Glu Ala
405 410 415

Lys Gln Lys Glu Leu Glu Phe Gln Gln Met Leu Asp Arg Leu Lys Lys
420 425 430

Glu Gln Glu Glu Ala Glu Ala Ile Ala Ala Ala Ala Ala Glu Tyr Thr
435 440 445

Ser Ile Arg Arg Ser Arg Ile Met Gly Leu Ser Glu Ser Ser Ser Glu
450 455 460

Thr Ser Lys Leu Ser Ser Lys Ser Ala Lys Glu Arg Arg Asn Arg Arg
465 470 475 480

Lys Lys Lys Asn Gln Lys Lys Leu Ser Ser Gly Glu Glu Lys Gly Asp
485 490 495

Ala Glu Lys Leu Ser Lys Ser Glu Ser Glu Asp Ser Ile Arg Arg Lys
500 505 510

Ser Phe His Leu Gly Val Glu Gly His Arg Arg Ala His Glu Lys Arg
515 520 525

Leu Ser Thr Pro Asn Gln Ser Pro Leu Ser Ile Arg Gly Ser Leu Phe
530 535 540

Ser Ala Arg Arg Ser Ser Arg Thr Ser Leu Phe Ser Phe Lys Gly Arg
545 550 555 560

Gly Arg Asp Ile Gly Ser Glu Thr Glu Phe Ala Asp Asp Glu His Ser
565 570 575

Ile Phe Gly Asp Asn Glu Ser Arg Arg Gly Ser Leu Phe Val Pro His
580 585 590

Arg Pro Gln Glu Arg Arg Ser Ser Asn Ile Ser Gln Ala Ser Arg Ser
595 600 605

Pro Pro Met Leu Pro Val Asn Gly Lys Met His Ser Ala Val Asp Cys
610 615 620

Asn Gly Val Val Ser Leu Val Asp Gly Arg Ser Ala Leu Met Leu Pro
625 630 635 640

```
Asn Gly Gln Leu Leu Pro Glu Gly Thr Thr Asn Gln Ile His Lys Lys
                645                 650                 655

Arg Arg Cys Ser Ser Tyr Leu Leu Ser Glu Asp Met Leu Asn Asp Pro
            660                 665                 670

Asn Leu Arg Gln Arg Ala Met Ser Arg Ala Ser Ile Leu Thr Asn Thr
        675                 680                 685

Val Glu Glu Leu Glu Glu Ser Arg Gln Lys Cys Pro Pro Trp Trp Tyr
    690                 695                 700

Arg Phe Ala His Lys Phe Leu Ile Trp Asn Cys Ser Pro Tyr Trp Ile
705                 710                 715                 720

Lys Phe Lys Lys Cys Ile Tyr Phe Ile Val Met Asp Pro Phe Val Asp
                725                 730                 735

Leu Ala Ile Thr Ile Cys Ile Val Leu Asn Thr Leu Phe Met Ala Met
            740                 745                 750

Glu His His Pro Met Thr Glu Glu Phe Lys Asn Val Leu Ala Ile Gly
        755                 760                 765

Asn Leu Val Phe Thr Gly Ile Phe Ala Ala Glu Met Val Leu Lys Leu
    770                 775                 780

Ile Ala Met Asp Pro Tyr Glu Tyr Phe Gln Val Gly Trp Asn Ile Phe
785                 790                 795                 800

Asp Ser Leu Ile Val Thr Leu Ser Leu Val Glu Leu Phe Leu Ala Asp
                805                 810                 815

Val Glu Gly Leu Ser Val Leu Arg Ser Phe Arg Leu Leu Arg Val Phe
            820                 825                 830

Lys Leu Ala Lys Ser Trp Pro Thr Leu Asn Met Leu Ile Lys Ile Ile
        835                 840                 845

Gly Asn Ser Val Gly Ala Leu Gly Asn Leu Thr Leu Val Leu Ala Ile
    850                 855                 860

Ile Val Phe Ile Phe Ala Val Val Gly Met Gln Leu Phe Gly Lys Ser
865                 870                 875                 880

Tyr Lys Glu Cys Val Cys Lys Ile Asn Asp Asp Cys Thr Leu Pro Arg
                885                 890                 895

Trp His Met Asn Asp Phe Phe His Ser Phe Leu Ile Val Phe Arg Val
```

```
            900                 905                 910

Leu Cys Gly Glu Trp Ile Glu Thr Met Trp Asp Cys Met Glu Val Ala
        915                 920                 925

Gly Gln Ala Met Cys Leu Ile Val Tyr Met Met Val Met Val Ile Gly
    930                 935                 940

Asn Leu Val Val Leu Asn Leu Phe Leu Ala Leu Leu Leu Ser Ser Phe
945                 950                 955                 960

Ser Ser Asp Asn Leu Thr Ala Ile Glu Glu Asp Pro Asp Ala Asn Asn
                965                 970                 975

Leu Gln Ile Ala Val Thr Arg Ile Lys Lys Gly Ile Asn Tyr Val Lys
            980                 985                 990

Gln Thr Leu Arg Glu Phe Ile Leu  Lys Ala Phe Ser Lys  Lys Pro Lys
        995                 1000                 1005

Ile Ser  Arg Glu Ile Arg Gln  Ala Glu Asp Leu Asn  Thr Lys Lys
    1010                 1015                 1020

Glu Asn  Tyr Ile Ser Asn His  Thr Leu Ala Glu Met  Ser Lys Gly
    1025                 1030                 1035

His Asn  Phe Leu Lys Glu Lys  Asp Lys Ile Ser Gly  Phe Gly Ser
    1040                 1045                 1050

Ser Val  Asp Lys His Leu Met  Glu Asp Ser Asp Gly  Gln Ser Phe
    1055                 1060                 1065

Ile His  Asn Pro Ser Leu Thr  Val Thr Val Pro Ile  Ala Pro Gly
    1070                 1075                 1080

Glu Ser  Asp Leu Glu Asn Met  Asn Ala Glu Glu Leu  Ser Ser Asp
    1085                 1090                 1095

Ser Asp  Ser Glu Tyr Ser Lys  Val Arg Leu Asn Arg  Ser Ser Ser
    1100                 1105                 1110

Ser Glu  Cys Ser Thr Val Asp  Asn Pro Leu Pro Gly  Glu Gly Glu
    1115                 1120                 1125

Glu Ala  Glu Ala Glu Pro Met  Asn Ser Asp Glu Pro  Glu Ala Cys
    1130                 1135                 1140

Phe Thr  Asp Gly Cys Val Arg  Arg Phe Ser Cys Cys  Gln Val Asn
    1145                 1150                 1155
```

```
Ile Glu  Ser Gly Lys Gly Lys  Ile Trp Trp Asn Ile  Arg Lys Thr
    1160                 1165                 1170

Cys Tyr  Lys Ile Val Glu His  Ser Trp Phe Glu Ser  Phe Ile Val
    1175                 1180                 1185

Leu Met  Ile Leu Leu Ser Ser  Gly Ala Leu Ala Phe  Glu Asp Ile
    1190                 1195                 1200

Tyr Ile  Glu Arg Lys Lys Thr  Ile Lys Ile Ile Leu  Glu Tyr Ala
    1205                 1210                 1215

Asp Lys  Ile Phe Thr Tyr Ile  Phe Ile Leu Glu Met  Leu Leu Lys
    1220                 1225                 1230

Trp Ile  Ala Tyr Gly Tyr Lys  Thr Tyr Phe Thr Asn  Ala Trp Cys
    1235                 1240                 1245

Trp Leu  Asp Phe Leu Ile Val  Asp Val Ser Leu Val  Thr Leu Val
    1250                 1255                 1260

Ala Asn  Thr Leu Gly Tyr Ser  Asp Leu Gly Pro Ile  Lys Ser Leu
    1265                 1270                 1275

Arg Thr  Leu Arg Ala Leu Arg  Pro Leu Arg Ala Leu  Ser Arg Phe
    1280                 1285                 1290

Glu Gly  Met Arg Val Val Val  Asn Ala Leu Ile Gly  Ala Ile Pro
    1295                 1300                 1305

Ser Ile  Met Asn Val Leu Leu  Val Cys Leu Ile Phe  Trp Leu Ile
    1310                 1315                 1320

Phe Ser  Ile Met Gly Val Asn  Leu Phe Ala Gly Lys  Phe Tyr Glu
    1325                 1330                 1335

Cys Ile  Asn Thr Thr Asp Gly  Ser Arg Phe Pro Ala  Ser Gln Val
    1340                 1345                 1350

Pro Asn  Arg Ser Glu Cys Phe  Ala Leu Met Asn Val  Ser Gln Asn
    1355                 1360                 1365

Val Arg  Trp Lys Asn Leu Lys  Val Asn Phe Asp Asn  Val Gly Leu
    1370                 1375                 1380

Gly Tyr  Leu Ser Leu Leu Gln  Val Ala Thr Phe Lys  Gly Trp Thr
    1385                 1390                 1395
```

```
Ile Ile Met Tyr Ala Ala Val Asp Ser Val Asn Val Asp Lys Gln
    1400                1405                1410

Pro Lys Tyr Glu Tyr Ser Leu Tyr Met Tyr Ile Tyr Phe Val Val
    1415                1420                1425

Phe Ile Ile Phe Gly Ser Phe Phe Thr Leu Asn Leu Phe Ile Gly
    1430                1435                1440

Val Ile Ile Asp Asn Phe Asn Gln Gln Lys Lys Lys Leu Gly Gly
    1445                1450                1455

Gln Asp Ile Phe Met Thr Glu Glu Gln Lys Lys Tyr Tyr Asn Ala
    1460                1465                1470

Met Lys Lys Leu Gly Ser Lys Lys Pro Gln Lys Pro Ile Pro Arg
    1475                1480                1485

Pro Gly Asn Lys Ile Gln Gly Cys Ile Phe Asp Leu Val Thr Asn
    1490                1495                1500

Gln Ala Phe Asp Ile Ser Ile Met Val Leu Ile Cys Leu Asn Met
    1505                1510                1515

Val Thr Met Met Val Glu Lys Glu Gly Gln Ser Gln His Met Thr
    1520                1525                1530

Glu Val Leu Tyr Trp Ile Asn Val Val Phe Ile Ile Leu Phe Thr
    1535                1540                1545

Gly Glu Cys Val Leu Lys Leu Ile Ser Leu Arg His Tyr Tyr Phe
    1550                1555                1560

Thr Val Gly Trp Asn Ile Phe Asp Phe Val Val Val Ile Ile Ser
    1565                1570                1575

Ile Val Gly Met Phe Leu Ala Asp Leu Ile Glu Thr Tyr Phe Val
    1580                1585                1590

Ser Pro Thr Leu Phe Arg Val Ile Arg Leu Ala Arg Ile Gly Arg
    1595                1600                1605

Ile Leu Arg Leu Val Lys Gly Ala Lys Gly Ile Arg Thr Leu Leu
    1610                1615                1620

Phe Ala Leu Met Met Ser Leu Pro Ala Leu Phe Asn Ile Gly Leu
    1625                1630                1635
```

Leu Leu Phe Leu Val Met Phe Ile Tyr Ala Ile Phe Gly Met Ser
1640 1645 1650

Asn Phe Ala Tyr Val Lys Lys Glu Asp Gly Ile Asn Asp Met Phe
1655 1660 1665

Asn Phe Glu Thr Phe Gly Asn Ser Met Ile Cys Leu Phe Gln Ile
1670 1675 1680

Thr Thr Ser Ala Gly Trp Asp Gly Leu Leu Ala Pro Ile Leu Asn
1685 1690 1695

Ser Lys Pro Pro Asp Cys Asp Pro Lys Lys Val His Pro Gly Ser
1700 1705 1710

Ser Val Glu Gly Asp Cys Gly Asn Pro Ser Val Gly Ile Phe Tyr
1715 1720 1725

Phe Val Ser Tyr Ile Ile Ile Ser Phe Leu Val Val Val Asn Met
1730 1735 1740

Tyr Ile Ala Val Ile Leu Glu Asn Phe Ser Val Ala Thr Glu Glu
1745 1750 1755

Ser Thr Glu Pro Leu Ser Glu Asp Asp Phe Glu Met Phe Tyr Glu
1760 1765 1770

Val Trp Glu Lys Phe Asp Pro Asp Ala Thr Gln Phe Ile Glu Phe
1775 1780 1785

Ser Lys Leu Ser Asp Phe Ala Ala Ala Leu Asp Pro Pro Leu Leu
1790 1795 1800

Ile Ala Lys Pro Asn Lys Val Gln Leu Ile Ala Met Asp Leu Pro
1805 1810 1815

Met Val Ser Gly Asp Arg Ile His Cys Leu Asp Ile Leu Phe Ala
1820 1825 1830

Phe Thr Lys Arg Val Leu Gly Glu Ser Gly Glu Met Asp Ser Leu
1835 1840 1845

Arg Ser Gln Met Glu Glu Arg Phe Met Ser Ala Asn Pro Ser Lys
1850 1855 1860

Val Ser Tyr Glu Pro Ile Thr Thr Thr Leu Lys Arg Lys Gln Glu
1865 1870 1875

Asp Val Ser Ala Thr Val Ile Gln Arg Ala Tyr Arg Arg Tyr Arg

1880 1885 1890

Leu Arg Gln Asn Val Lys Asn Ile Ser Ser Ile Tyr Ile Lys Asp
1895 1900 1905

Gly Asp Arg Asp Asp Asp Leu Leu Asn Lys Lys Asp Met Ala Phe
1910 1915 1920

Asp Asn Val Asn Glu Asn Ser Ser Pro Glu Lys Thr Asp Ala Thr
1925 1930 1935

Ser Ser Thr Thr Ser Pro Pro Ser Tyr Asp Ser Val Thr Lys Pro
1940 1945 1950

Asp Lys Glu Lys Tyr Glu Gln Asp Arg Thr Glu Lys Glu Asp Lys
1955 1960 1965

Gly Lys Asp Ser Lys Glu Ser Lys Lys
1970 1975

<210> 28
<211> 1956
<212> PRT
<213> Homo sapiens

<400> 28

```
Met Glu Phe Pro Ile Gly Ser Leu Glu Thr Asn Asn Phe Arg Arg Phe
1               5                   10                  15

Thr Pro Glu Ser Leu Val Glu Ile Glu Lys Gln Ile Ala Ala Lys Gln
            20                  25                  30

Gly Thr Lys Lys Ala Arg Glu Lys His Arg Glu Gln Lys Asp Gln Glu
        35                  40                  45

Glu Lys Pro Arg Pro Gln Leu Asp Leu Lys Ala Cys Asn Gln Leu Pro
    50                  55                  60

Lys Phe Tyr Gly Glu Leu Pro Ala Glu Leu Ile Gly Glu Pro Leu Glu
65                  70                  75                  80

Asp Leu Asp Pro Phe Tyr Ser Thr His Arg Thr Phe Met Val Leu Asn
                85                  90                  95

Lys Gly Arg Thr Ile Ser Arg Phe Ser Ala Thr Arg Ala Leu Trp Leu
            100                 105                 110

Phe Ser Pro Phe Asn Leu Ile Arg Arg Thr Ala Ile Lys Val Ser Val
        115                 120                 125
```

```
His Ser Trp Phe Ser Leu Phe Ile Thr Val Thr Ile Leu Val Asn Cys
    130                 135                 140

Val Cys Met Thr Arg Thr Asp Leu Pro Glu Lys Ile Glu Tyr Val Phe
145                 150                 155                 160

Thr Val Ile Tyr Thr Phe Glu Ala Leu Ile Lys Ile Leu Ala Arg Gly
                165                 170                 175

Phe Cys Leu Asn Glu Phe Thr Tyr Leu Arg Asp Pro Trp Asn Trp Leu
            180                 185                 190

Asp Phe Ser Val Ile Thr Leu Ala Tyr Val Gly Thr Ala Ile Asp Leu
        195                 200                 205

Arg Gly Ile Ser Gly Leu Arg Thr Phe Arg Val Leu Arg Ala Leu Lys
    210                 215                 220

Thr Val Ser Val Ile Pro Gly Leu Lys Val Ile Val Gly Ala Leu Ile
225                 230                 235                 240

His Ser Val Lys Lys Leu Ala Asp Val Thr Ile Leu Thr Ile Phe Cys
                245                 250                 255

Leu Ser Val Phe Ala Leu Val Gly Leu Gln Leu Phe Lys Gly Asn Leu
            260                 265                 270

Lys Asn Lys Cys Val Lys Asn Asp Met Ala Val Asn Glu Thr Thr Asn
        275                 280                 285

Tyr Ser Ser His Arg Lys Pro Asp Ile Tyr Ile Asn Lys Arg Gly Thr
    290                 295                 300

Ser Asp Pro Leu Leu Cys Gly Asn Gly Ser Asp Ser Gly His Cys Pro
305                 310                 315                 320

Asp Gly Tyr Ile Cys Leu Lys Thr Ser Asp Asn Pro Asp Phe Asn Tyr
                325                 330                 335

Thr Ser Phe Asp Ser Phe Ala Trp Ala Phe Leu Ser Leu Phe Arg Leu
            340                 345                 350

Met Thr Gln Asp Ser Trp Glu Arg Leu Tyr Gln Gln Thr Leu Arg Thr
        355                 360                 365

Ser Gly Lys Ile Tyr Met Ile Phe Phe Val Leu Val Ile Phe Leu Gly
    370                 375                 380

Ser Phe Tyr Leu Val Asn Leu Ile Leu Ala Val Val Thr Met Ala Tyr
```

385 390 395 400

Glu Glu Gln Asn Gln Ala Thr Thr Asp Glu Ile Glu Ala Lys Glu Lys
405 410 415

Lys Phe Gln Glu Ala Leu Glu Met Leu Arg Lys Glu Gln Glu Val Leu
420 425 430

Ala Ala Leu Gly Ile Asp Thr Thr Ser Leu His Ser His Asn Gly Ser
435 440 445

Pro Leu Thr Ser Lys Asn Ala Ser Glu Arg Arg His Arg Ile Lys Pro
450 455 460

Arg Val Ser Glu Gly Ser Thr Glu Asp Asn Lys Ser Pro Arg Ser Asp
465 470 475 480

Pro Tyr Asn Gln Arg Arg Met Ser Phe Leu Gly Leu Ala Ser Gly Lys
485 490 495

Arg Arg Ala Ser His Gly Ser Val Phe His Phe Arg Ser Pro Gly Arg
500 505 510

Asp Ile Ser Leu Pro Glu Gly Val Thr Asp Asp Gly Val Phe Pro Gly
515 520 525

Asp His Glu Ser His Arg Gly Ser Leu Leu Leu Gly Gly Gly Ala Gly
530 535 540

Gln Gln Gly Pro Leu Pro Arg Ser Pro Leu Pro Gln Pro Ser Asn Pro
545 550 555 560

Asp Ser Arg His Gly Glu Asp Glu His Gln Pro Pro Pro Thr Ser Glu
565 570 575

Leu Ala Pro Gly Ala Val Asp Val Ser Ala Phe Asp Ala Gly Gln Lys
580 585 590

Lys Thr Phe Leu Ser Ala Glu Tyr Leu Asp Glu Pro Phe Arg Ala Gln
595 600 605

Arg Ala Met Ser Val Val Ser Ile Ile Thr Ser Val Leu Glu Glu Leu
610 615 620

Glu Glu Ser Glu Gln Lys Cys Pro Pro Cys Leu Thr Ser Leu Ser Gln
625 630 635 640

Lys Tyr Leu Ile Trp Asp Cys Cys Pro Met Trp Val Lys Leu Lys Thr
645 650 655

Ile Leu Phe Gly Leu Val Thr Asp Pro Phe Ala Glu Leu Thr Ile Thr
660 665 670

Leu Cys Ile Val Val Asn Thr Ile Phe Met Ala Met Glu His His Gly
675 680 685

Met Ser Pro Thr Phe Glu Ala Met Leu Gln Ile Gly Asn Ile Val Phe
690 695 700

Thr Ile Phe Phe Thr Ala Glu Met Val Phe Lys Ile Ile Ala Phe Asp
705 710 715 720

Pro Tyr Tyr Tyr Phe Gln Lys Lys Trp Asn Ile Phe Asp Cys Ile Ile
725 730 735

Val Thr Val Ser Leu Leu Glu Leu Gly Val Ala Lys Lys Gly Ser Leu
740 745 750

Ser Val Leu Arg Ser Phe Arg Leu Leu Arg Val Phe Lys Leu Ala Lys
755 760 765

Ser Trp Pro Thr Leu Asn Thr Leu Ile Lys Ile Ile Gly Asn Ser Val
770 775 780

Gly Ala Leu Gly Asn Leu Thr Ile Ile Leu Ala Ile Ile Val Phe Val
785 790 795 800

Phe Ala Leu Val Gly Lys Gln Leu Leu Gly Glu Asn Tyr Arg Asn Asn
805 810 815

Arg Lys Asn Ile Ser Ala Pro His Glu Asp Trp Pro Arg Trp His Met
820 825 830

His Asp Phe Phe His Ser Phe Leu Ile Val Phe Arg Ile Leu Cys Gly
835 840 845

Glu Trp Ile Glu Asn Met Trp Ala Cys Met Glu Val Gly Gln Lys Ser
850 855 860

Ile Cys Leu Ile Leu Phe Leu Thr Val Met Val Leu Gly Asn Leu Val
865 870 875 880

Val Leu Asn Leu Phe Ile Ala Leu Leu Leu Asn Ser Phe Ser Ala Asp
885 890 895

Asn Leu Thr Ala Pro Glu Asp Asp Gly Glu Val Asn Asn Leu Gln Val
900 905 910

Ala Leu Ala Arg Ile Gln Val Phe Gly His Arg Thr Lys Gln Ala Leu
915 920 925

Cys Ser Phe Phe Ser Arg Ser Cys Pro Phe Pro Gln Pro Lys Ala Glu
930 935 940

Pro Glu Leu Val Val Lys Leu Pro Leu Ser Ser Ser Lys Ala Glu Asn
945 950 955 960

His Ile Ala Ala Asn Thr Ala Arg Gly Ser Ser Gly Gly Leu Gln Ala
965 970 975

Pro Arg Gly Pro Arg Asp Glu His Ser Asp Phe Ile Ala Asn Pro Thr
980 985 990

Val Trp Val Ser Val Pro Ile Ala Glu Gly Glu Ser Asp Leu Asp Asp
995 1000 1005

Leu Glu Asp Asp Gly Gly Glu Asp Ala Gln Ser Phe Gln Gln Glu
1010 1015 1020

Val Ile Pro Lys Gly Gln Gln Glu Gln Leu Gln Gln Val Glu Arg
1025 1030 1035

Cys Gly Asp His Leu Thr Pro Arg Ser Pro Gly Thr Gly Thr Ser
1040 1045 1050

Ser Glu Asp Leu Ala Pro Ser Leu Gly Glu Thr Trp Lys Asp Glu
1055 1060 1065

Ser Val Pro Gln Val Pro Ala Glu Gly Val Asp Asp Thr Ser Ser
1070 1075 1080

Ser Glu Gly Ser Thr Val Asp Cys Leu Asp Pro Glu Glu Ile Leu
1085 1090 1095

Arg Lys Ile Pro Glu Leu Ala Asp Asp Leu Glu Glu Pro Asp Asp
1100 1105 1110

Cys Phe Thr Glu Gly Cys Ile Arg His Cys Pro Cys Cys Lys Leu
1115 1120 1125

Asp Thr Thr Lys Ser Pro Trp Asp Val Gly Trp Gln Val Arg Lys
1130 1135 1140

Thr Cys Tyr Arg Ile Val Glu His Ser Trp Phe Glu Ser Phe Ile
1145 1150 1155

```
Ile Phe  Met Ile Leu Leu Ser  Ser Gly Ser Leu Ala  Phe Glu Asp
    1160                 1165                 1170

Tyr Tyr  Leu Asp Gln Lys Pro  Thr Val Lys Ala Leu  Leu Glu Tyr
    1175                 1180                 1185

Thr Asp  Arg Val Phe Thr Phe  Ile Phe Val Phe Glu  Met Leu Leu
    1190                 1195                 1200

Lys Trp  Val Ala Tyr Gly Phe  Lys Lys Tyr Phe Thr  Asn Ala Trp
    1205                 1210                 1215

Cys Trp  Leu Asp Phe Leu Ile  Val Asn Ile Ser Leu  Ile Ser Leu
    1220                 1225                 1230

Thr Ala  Lys Ile Leu Glu Tyr  Ser Glu Val Ala Pro  Ile Lys Ala
    1235                 1240                 1245

Leu Arg  Thr Leu Arg Ala Leu  Arg Pro Leu Arg Ala  Leu Ser Arg
    1250                 1255                 1260

Phe Glu  Gly Met Arg Val Val  Val Asp Ala Leu Val  Gly Ala Ile
    1265                 1270                 1275

Pro Ser  Ile Met Asn Val Leu  Leu Val Cys Leu Ile  Phe Trp Leu
    1280                 1285                 1290

Ile Phe  Ser Ile Met Gly Val  Asn Leu Phe Ala Gly  Lys Phe Trp
    1295                 1300                 1305

Arg Cys  Ile Asn Tyr Thr Asp  Gly Glu Phe Ser Leu  Val Pro Leu
    1310                 1315                 1320

Ser Ile  Val Asn Asn Lys Ser  Asp Cys Lys Ile Gln  Asn Ser Thr
    1325                 1330                 1335

Gly Ser  Phe Phe Trp Val Asn  Val Lys Val Asn Phe  Asp Asn Val
    1340                 1345                 1350

Ala Met  Gly Tyr Leu Ala Leu  Leu Gln Val Ala Thr  Phe Lys Gly
    1355                 1360                 1365

Trp Met  Asp Ile Met Tyr Ala  Ala Val Asp Ser Arg  Glu Val Asn
    1370                 1375                 1380

Met Gln  Pro Lys Trp Glu Asp  Asn Val Tyr Met Tyr  Leu Tyr Phe
    1385                 1390                 1395

Val Ile  Phe Ile Ile Phe Gly  Gly Phe Phe Thr Leu  Asn Leu Phe
```

```
        1400                1405                1410

Val Gly  Val Ile Ile Asp Asn  Phe Asn Gln Gln Lys  Lys Lys Leu
    1415                1420                1425

Gly Gly  Gln Asp Ile Phe Met  Thr Glu Glu Gln Lys  Lys Tyr Tyr
    1430                1435                1440

Asn Ala  Met Lys Lys Leu Gly  Ser Lys Lys Pro Gln  Lys Pro Ile
    1445                1450                1455

Pro Arg  Pro Leu Asn Lys Phe  Gln Gly Phe Val Phe  Asp Ile Val
    1460                1465                1470

Thr Arg  Gln Ala Phe Asp Ile  Thr Ile Met Val Leu  Ile Cys Leu
    1475                1480                1485

Asn Met  Ile Thr Met Met Val  Glu Thr Asp Asp Gln  Ser Glu Glu
    1490                1495                1500

Lys Thr  Lys Ile Leu Gly Lys  Ile Asn Gln Phe Phe  Val Ala Val
    1505                1510                1515

Phe Thr  Gly Glu Cys Val Met  Lys Met Phe Ala Leu  Arg Gln Tyr
    1520                1525                1530

Tyr Phe  Thr Asn Gly Trp Asn  Val Phe Asp Phe Ile  Val Val Val
    1535                1540                1545

Leu Ser  Ile Ala Ser Leu Ile  Phe Ser Ala Ile Leu  Lys Ser Leu
    1550                1555                1560

Gln Ser  Tyr Phe Ser Pro Thr  Leu Phe Arg Val Ile  Arg Leu Ala
    1565                1570                1575

Arg Ile  Gly Arg Ile Leu Arg  Leu Ile Arg Ala Ala  Lys Gly Ile
    1580                1585                1590

Arg Thr  Leu Leu Phe Ala Leu  Met Met Ser Leu Pro  Ala Leu Phe
    1595                1600                1605

Asn Ile  Gly Leu Leu Leu Phe  Leu Val Met Phe Ile  Tyr Ser Ile
    1610                1615                1620

Phe Gly  Met Ser Ser Phe Pro  His Val Arg Trp Glu  Ala Gly Ile
    1625                1630                1635

Asp Asp  Met Phe Asn Phe Gln  Thr Phe Ala Asn Ser  Met Leu Cys
    1640                1645                1650
```

Leu Phe Gln Ile Thr Thr Ser Ala Gly Trp Asp Gly Leu Leu Ser
1655 1660 1665

Pro Ile Leu Asn Thr Gly Pro Pro Tyr Cys Asp Pro Asn Leu Pro
1670 1675 1680

Asn Ser Asn Gly Thr Arg Gly Asp Cys Gly Ser Pro Ala Val Gly
1685 1690 1695

Ile Ile Phe Phe Thr Thr Tyr Ile Ile Ile Ser Phe Leu Ile Met
1700 1705 1710

Val Asn Met Tyr Ile Ala Val Ile Leu Glu Asn Phe Asn Val Ala
1715 1720 1725

Thr Glu Glu Ser Thr Glu Pro Leu Ser Glu Asp Asp Phe Asp Met
1730 1735 1740

Phe Tyr Glu Thr Trp Glu Lys Phe Asp Pro Glu Ala Thr Gln Phe
1745 1750 1755

Ile Thr Phe Ser Ala Leu Ser Asp Phe Ala Asp Thr Leu Ser Gly
1760 1765 1770

Pro Leu Arg Ile Pro Lys Pro Asn Arg Asn Ile Leu Ile Gln Met
1775 1780 1785

Asp Leu Pro Leu Val Pro Gly Asp Lys Ile His Cys Leu Asp Ile
1790 1795 1800

Leu Phe Ala Phe Thr Lys Asn Val Leu Gly Glu Ser Gly Glu Leu
1805 1810 1815

Asp Ser Leu Lys Ala Asn Met Glu Glu Lys Phe Met Ala Thr Asn
1820 1825 1830

Leu Ser Lys Ser Ser Tyr Glu Pro Ile Ala Thr Thr Leu Arg Trp
1835 1840 1845

Lys Gln Glu Asp Ile Ser Ala Thr Val Ile Gln Lys Ala Tyr Arg
1850 1855 1860

Ser Tyr Val Leu His Arg Ser Met Ala Leu Ser Asn Thr Pro Cys
1865 1870 1875

Val Pro Arg Ala Glu Glu Glu Ala Ala Ser Leu Pro Asp Glu Gly
1880 1885 1890

```
Phe Val  Ala Phe Thr Ala Asn  Glu Asn Cys Val Leu  Pro Asp Lys
    1895                 1900                 1905

Ser Glu  Thr Ala Ser Ala Thr  Ser Phe Pro Pro Ser  Tyr Glu Ser
    1910                 1915                 1920

Val Thr  Arg Gly Leu Ser Asp  Arg Val Asn Met Arg  Thr Ser Ser
    1925                 1930                 1935

Ser Ile  Gln Asn Glu Asp Glu  Ala Thr Ser Met Glu  Leu Ile Ala
    1940                 1945                 1950

Pro Gly  Pro
    1955
```

<210> 29
<211> 1791
<212> PRT
<213> Homo sapiens

<400> 29

```
Met Asp Asp Arg Cys Tyr Pro Val Ile Phe Pro Asp Glu Arg Asn Phe
1               5                   10                  15

Arg Pro Phe Thr Ser Asp Ser Leu Ala Ala Ile Glu Lys Arg Ile Ala
            20                  25                  30

Ile Gln Lys Glu Lys Lys Lys Ser Lys Asp Gln Thr Gly Glu Val Pro
        35                  40                  45

Gln Pro Arg Pro Gln Leu Asp Leu Lys Ala Ser Arg Lys Leu Pro Lys
    50                  55                  60

Leu Tyr Gly Asp Ile Pro Arg Glu Leu Ile Gly Lys Pro Leu Glu Asp
65                  70                  75                  80

Leu Asp Pro Phe Tyr Arg Asn His Lys Thr Phe Met Val Leu Asn Arg
                85                  90                  95

Lys Arg Thr Ile Tyr Arg Phe Ser Ala Lys His Ala Leu Phe Ile Phe
            100                 105                 110

Gly Pro Phe Asn Ser Ile Arg Ser Leu Ala Ile Arg Val Ser Val His
        115                 120                 125

Ser Leu Phe Ser Met Phe Ile Ile Gly Thr Val Ile Ile Asn Cys Val
    130                 135                 140

Phe Met Ala Thr Gly Pro Ala Lys Asn Ser Asn Ser Asn Asn Thr Asp
145                 150                 155                 160
```

```
Ile Ala Glu Cys Val Phe Thr Gly Ile Tyr Ile Phe Glu Ala Leu Ile
                165                 170                 175

Lys Ile Leu Ala Arg Gly Phe Ile Leu Asp Glu Phe Ser Phe Leu Arg
            180                 185                 190

Asp Pro Trp Asn Trp Leu Asp Ser Ile Val Ile Gly Ile Ala Ile Val
        195                 200                 205

Ser Tyr Ile Pro Gly Ile Thr Ile Lys Leu Leu Pro Leu Arg Thr Phe
    210                 215                 220

Arg Val Phe Arg Ala Leu Lys Ala Ile Ser Val Val Ser Arg Leu Lys
225                 230                 235                 240

Val Ile Val Gly Ala Leu Leu Arg Ser Val Lys Lys Leu Val Asn Val
                245                 250                 255

Ile Ile Leu Thr Phe Phe Cys Leu Ser Ile Phe Ala Leu Val Gly Gln
            260                 265                 270

Gln Leu Phe Met Gly Ser Leu Asn Leu Lys Cys Ile Ser Arg Asp Cys
        275                 280                 285

Lys Asn Ile Ser Asn Pro Glu Ala Tyr Asp His Cys Phe Glu Lys Lys
    290                 295                 300

Glu Asn Ser Pro Glu Phe Lys Met Cys Gly Ile Trp Met Gly Asn Ser
305                 310                 315                 320

Ala Cys Ser Ile Gln Tyr Glu Cys Lys His Thr Lys Ile Asn Pro Asp
                325                 330                 335

Tyr Asn Tyr Thr Asn Phe Asp Asn Phe Gly Trp Ser Phe Leu Ala Met
            340                 345                 350

Phe Arg Leu Met Thr Gln Asp Ser Trp Glu Lys Leu Tyr Gln Gln Thr
        355                 360                 365

Leu Arg Thr Thr Gly Leu Tyr Ser Val Phe Phe Phe Ile Val Val Ile
    370                 375                 380

Phe Leu Gly Ser Phe Tyr Leu Ile Asn Leu Thr Leu Ala Val Val Thr
385                 390                 395                 400

Met Ala Tyr Glu Glu Gln Asn Lys Asn Val Ala Ala Glu Ile Glu Ala
                405                 410                 415
```

```
Lys Glu Lys Met Phe Gln Glu Ala Gln Gln Leu Leu Lys Glu Glu Lys
            420                 425                 430

Glu Ala Leu Val Ala Met Gly Ile Asp Arg Ser Ser Leu Thr Ser Leu
        435                 440                 445

Glu Thr Ser Tyr Phe Thr Pro Lys Lys Arg Lys Leu Phe Gly Asn Lys
    450                 455                 460

Lys Arg Lys Ser Phe Phe Leu Arg Glu Ser Gly Lys Asp Gln Pro Pro
465                 470                 475                 480

Gly Ser Asp Ser Asp Glu Asp Cys Gln Lys Lys Pro Gln Leu Leu Glu
                485                 490                 495

Gln Thr Lys Arg Leu Ser Gln Asn Leu Ser Leu Asp His Phe Asp Glu
            500                 505                 510

His Gly Asp Pro Leu Gln Arg Gln Arg Ala Leu Ser Ala Val Ser Ile
        515                 520                 525

Leu Thr Ile Thr Met Lys Glu Gln Glu Lys Ser Gln Glu Pro Cys Leu
    530                 535                 540

Pro Cys Gly Glu Asn Leu Ala Ser Lys Tyr Leu Val Trp Asn Cys Cys
545                 550                 555                 560

Pro Gln Trp Leu Cys Val Lys Lys Val Leu Arg Thr Val Met Thr Asp
                565                 570                 575

Pro Phe Thr Glu Leu Ala Ile Thr Ile Cys Ile Ile Ile Asn Thr Val
            580                 585                 590

Phe Leu Ala Met Glu His His Lys Met Glu Ala Ser Phe Glu Lys Met
        595                 600                 605

Leu Asn Ile Gly Asn Leu Val Phe Thr Ser Ile Phe Ile Ala Glu Met
    610                 615                 620

Cys Leu Lys Ile Ile Ala Leu Asp Pro Tyr His Tyr Phe Arg Arg Gly
625                 630                 635                 640

Trp Asn Ile Phe Asp Ser Ile Val Ala Leu Leu Ser Phe Ala Asp Val
                645                 650                 655

Met Asn Cys Val Leu Gln Lys Arg Ser Trp Pro Phe Leu Arg Ser Phe
            660                 665                 670
```

```
Arg Val Leu Arg Val Phe Lys Leu Ala Lys Ser Trp Pro Thr Leu Asn
        675                 680                 685

Thr Leu Ile Lys Ile Ile Gly Asn Ser Val Gly Ala Leu Gly Ser Leu
    690                 695                 700

Thr Val Val Leu Val Ile Val Ile Phe Ile Phe Ser Val Val Gly Met
705                 710                 715                 720

Gln Leu Phe Gly Arg Ser Phe Asn Ser Gln Lys Ser Pro Lys Leu Cys
                725                 730                 735

Asn Pro Thr Gly Pro Thr Val Ser Cys Leu Arg His Trp His Met Gly
            740                 745                 750

Asp Phe Trp His Ser Phe Leu Val Val Phe Arg Ile Leu Cys Gly Glu
        755                 760                 765

Trp Ile Glu Asn Met Trp Glu Cys Met Gln Glu Ala Asn Ala Ser Ser
    770                 775                 780

Ser Leu Cys Val Ile Val Phe Ile Leu Ile Thr Val Ile Gly Lys Leu
785                 790                 795                 800

Val Val Leu Asn Leu Phe Ile Ala Leu Leu Leu Asn Ser Phe Ser Asn
                805                 810                 815

Glu Glu Arg Asn Gly Asn Leu Glu Gly Glu Ala Arg Lys Thr Lys Val
            820                 825                 830

Gln Leu Ala Leu Asp Arg Phe Arg Arg Ala Phe Cys Phe Val Arg His
        835                 840                 845

Thr Leu Glu His Phe Cys His Lys Trp Cys Arg Lys Gln Asn Leu Pro
    850                 855                 860

Gln Gln Lys Glu Val Ala Gly Gly Cys Ala Ala Gln Ser Lys Asp Ile
865                 870                 875                 880

Ile Pro Leu Val Met Glu Met Lys Arg Gly Ser Glu Thr Gln Glu Glu
                885                 890                 895

Leu Gly Ile Leu Thr Ser Val Pro Lys Thr Leu Gly Val Arg His Asp
            900                 905                 910

Trp Thr Trp Leu Ala Pro Leu Ala Glu Glu Glu Asp Asp Val Glu Phe
        915                 920                 925

Ser Gly Glu Asp Asn Ala Gln Arg Ile Thr Gln Pro Glu Pro Glu Gln
```

```
    930                 935                 940

Gln Ala Tyr Glu Leu His Gln Glu Asn Lys Lys Pro Thr Ser Gln Arg
945                 950                 955                 960

Val Gln Ser Val Glu Ile Asp Met Phe Ser Glu Asp Glu Pro His Leu
                965                 970                 975

Thr Ile Gln Asp Pro Arg Lys Lys Ser Asp Val Thr Ser Ile Leu Ser
            980                 985                 990

Glu Cys Ser Thr Ile Asp Leu Gln  Asp Gly Phe Gly Trp  Leu Pro Glu
        995                 1000                 1005

Met Val  Pro Lys Lys Gln Pro  Glu Arg Cys Leu Pro  Lys Gly Phe
    1010                 1015                 1020

Gly Cys  Cys Phe Pro Cys Cys  Ser Val Asp Lys Arg  Lys Pro Pro
    1025                 1030                 1035

Trp Val  Ile Trp Trp Asn Leu  Arg Lys Thr Cys Tyr  Gln Ile Val
    1040                 1045                 1050

Lys His  Ser Trp Phe Glu Ser  Phe Ile Ile Phe Val  Ile Leu Leu
    1055                 1060                 1065

Ser Ser  Gly Ala Leu Ile Phe  Glu Asp Val His Leu  Glu Asn Gln
    1070                 1075                 1080

Pro Lys  Ile Gln Glu Leu Leu  Asn Cys Thr Asp Ile  Ile Phe Thr
    1085                 1090                 1095

His Ile  Phe Ile Leu Glu Met  Val Leu Lys Trp Val  Ala Phe Gly
    1100                 1105                 1110

Phe Gly  Lys Tyr Phe Thr Ser  Ala Trp Cys Cys Leu  Asp Phe Ile
    1115                 1120                 1125

Ile Val  Ile Val Ser Val Thr  Thr Leu Ile Asn Leu  Met Glu Leu
    1130                 1135                 1140

Lys Ser  Phe Arg Thr Leu Arg  Ala Leu Arg Pro Leu  Arg Ala Leu
    1145                 1150                 1155

Ser Gln  Phe Glu Gly Met Lys  Val Val Val Asn Ala  Leu Ile Gly
    1160                 1165                 1170

Ala Ile  Pro Ala Ile Leu Asn  Val Leu Leu Val Cys  Leu Ile Phe
    1175                 1180                 1185
```

Trp Leu Val Phe Cys Ile Leu Gly Val Tyr Phe Phe Ser Gly Lys
1190 1195 1200

Phe Gly Lys Cys Ile Asn Gly Thr Asp Ser Val Ile Asn Tyr Thr
1205 1210 1215

Ile Ile Thr Asn Lys Ser Gln Cys Glu Ser Gly Asn Phe Ser Trp
1220 1225 1230

Ile Asn Gln Lys Val Asn Phe Asp Asn Val Gly Asn Ala Tyr Leu
1235 1240 1245

Ala Leu Leu Gln Val Ala Thr Phe Lys Gly Trp Met Asp Ile Ile
1250 1255 1260

Tyr Ala Ala Val Asp Ser Thr Glu Lys Glu Gln Gln Pro Glu Phe
1265 1270 1275

Glu Ser Asn Ser Leu Gly Tyr Ile Tyr Phe Val Val Phe Ile Ile
1280 1285 1290

Phe Gly Ser Phe Phe Thr Leu Asn Leu Phe Ile Gly Val Ile Ile
1295 1300 1305

Asp Asn Phe Asn Gln Gln Gln Lys Lys Leu Gly Gly Gln Asp Ile
1310 1315 1320

Phe Met Thr Glu Glu Gln Lys Lys Tyr Tyr Asn Ala Met Lys Lys
1325 1330 1335

Leu Gly Ser Lys Lys Pro Gln Lys Pro Ile Pro Arg Pro Leu Asn
1340 1345 1350

Lys Cys Gln Gly Leu Val Phe Asp Ile Val Thr Ser Gln Ile Phe
1355 1360 1365

Asp Ile Ile Ile Ile Ser Leu Ile Ile Leu Asn Met Ile Ser Met
1370 1375 1380

Met Ala Glu Ser Tyr Asn Gln Pro Lys Ala Met Lys Ser Ile Leu
1385 1390 1395

Asp His Leu Asn Trp Val Phe Val Val Ile Phe Thr Leu Glu Cys
1400 1405 1410

Leu Ile Lys Ile Phe Ala Leu Arg Gln Tyr Tyr Phe Thr Asn Gly
1415 1420 1425

```
Trp Asn  Leu Phe Asp Cys Val  Val Val Leu Leu Ser  Ile Val Ser
    1430                 1435                 1440

Thr Met  Ile Ser Thr Leu Glu  Asn Gln Glu His Ile  Pro Phe Pro
    1445                 1450                 1455

Pro Thr  Leu Phe Arg Ile Val  Arg Leu Ala Arg Ile  Gly Arg Ile
    1460                 1465                 1470

Leu Arg  Leu Val Arg Ala Ala  Arg Gly Ile Arg Thr  Leu Leu Phe
    1475                 1480                 1485

Ala Leu  Met Met Ser Leu Pro  Ser Leu Phe Asn Ile  Gly Leu Leu
    1490                 1495                 1500

Leu Phe  Leu Ile Met Phe Ile  Tyr Ala Ile Leu Gly  Met Asn Trp
    1505                 1510                 1515

Phe Ser  Lys Val Asn Pro Glu  Ser Gly Ile Asp Asp  Ile Phe Asn
    1520                 1525                 1530

Phe Lys  Thr Phe Ala Ser Ser  Met Leu Cys Leu Phe  Gln Ile Ser
    1535                 1540                 1545

Thr Ser  Ala Gly Trp Asp Ser  Leu Leu Ser Pro Met  Leu Arg Ser
    1550                 1555                 1560

Lys Glu  Ser Cys Asn Ser Ser  Ser Glu Asn Cys His  Leu Pro Gly
    1565                 1570                 1575

Ile Ala  Thr Ser Tyr Phe Val  Ser Tyr Ile Ile Ile  Ser Phe Leu
    1580                 1585                 1590

Ile Val  Val Asn Met Tyr Ile  Ala Val Ile Leu Glu  Asn Phe Asn
    1595                 1600                 1605

Thr Ala  Thr Glu Glu Ser Glu  Asp Pro Leu Gly Glu  Asp Asp Phe
    1610                 1615                 1620

Asp Ile  Phe Tyr Glu Val Trp  Glu Lys Phe Asp Pro  Glu Ala Thr
    1625                 1630                 1635

Gln Phe  Ile Lys Tyr Ser Ala  Leu Ser Asp Phe Ala  Asp Ala Leu
    1640                 1645                 1650

Pro Glu  Pro Leu Arg Val Ala  Lys Pro Asn Lys Tyr  Gln Phe Leu
    1655                 1660                 1665
```

```
Val Met Asp Leu Pro Met Val Ser Glu Asp Arg Leu His Cys Met
    1670                1675                1680

Asp Ile Leu Phe Ala Phe Thr Ala Arg Val Leu Gly Gly Ser Asp
    1685                1690                1695

Gly Leu Asp Ser Met Lys Ala Met Met Glu Glu Lys Phe Met Glu
    1700                1705                1710

Ala Asn Pro Leu Lys Lys Leu Tyr Glu Pro Ile Val Thr Thr Thr
    1715                1720                1725

Lys Arg Lys Glu Glu Glu Arg Gly Ala Ala Ile Ile Gln Lys Ala
    1730                1735                1740

Phe Arg Lys Tyr Met Met Lys Val Thr Lys Gly Asp Gln Gly Asp
    1745                1750                1755

Gln Asn Asp Leu Glu Asn Gly Pro His Ser Pro Leu Gln Thr Leu
    1760                1765                1770

Cys Asn Gly Asp Leu Ser Ser Phe Gly Val Ala Lys Gly Lys Val
    1775                1780                1785

His Cys Asp
    1790
```

<210> 30
<211> 218
<212> PRT
<213> Homo sapiens

<400> 30

```
Met Gly Arg Leu Leu Ala Leu Val Val Gly Ala Ala Leu Val Ser Ser
1               5                   10                  15

Ala Cys Gly Gly Cys Val Glu Val Asp Ser Glu Thr Glu Ala Val Tyr
            20                  25                  30

Gly Met Thr Phe Lys Ile Leu Cys Ile Ser Cys Lys Arg Arg Ser Glu
        35                  40                  45

Thr Asn Ala Glu Thr Phe Thr Glu Trp Thr Phe Arg Gln Lys Gly Thr
    50                  55                  60

Glu Glu Phe Val Lys Ile Leu Arg Tyr Glu Asn Glu Val Leu Gln Leu
65                  70                  75                  80

Glu Glu Asp Glu Arg Phe Glu Gly Arg Val Val Trp Asn Gly Ser Arg
                85                  90                  95

Gly Thr Lys Asp Leu Gln Asp Leu Ser Ile Phe Ile Thr Asn Val Thr
            100                 105                 110

Tyr Asn His Ser Gly Asp Tyr Glu Cys His Val Tyr Arg Leu Leu Phe
        115                 120                 125

Phe Glu Asn Tyr Glu His Asn Thr Ser Val Val Lys Lys Ile His Ile
    130                 135                 140

Glu Val Val Asp Lys Ala Asn Arg Asp Met Ala Ser Ile Val Ser Glu
145                 150                 155                 160

Ile Met Met Tyr Val Leu Ile Val Val Leu Thr Ile Trp Leu Val Ala
                165                 170                 175

Glu Met Ile Tyr Cys Tyr Lys Lys Ile Ala Ala Ala Thr Glu Thr Ala
            180                 185                 190

Ala Gln Glu Asn Ala Ser Glu Tyr Leu Ala Ile Thr Ser Glu Ser Lys
        195                 200                 205

Glu Asn Cys Thr Gly Val Gln Val Ala Glu
    210                 215
```

<210> 31
<211> 215
<212> PRT
<213> Homo sapiens

<400> 31

```
Met His Arg Asp Ala Trp Leu Pro Arg Pro Ala Phe Ser Leu Thr Gly
1               5                   10                  15

Leu Ser Leu Phe Phe Ser Leu Val Pro Pro Gly Arg Ser Met Glu Val
            20                  25                  30

Thr Val Pro Ala Thr Leu Asn Val Leu Asn Gly Ser Asp Ala Arg Leu
        35                  40                  45

Pro Cys Thr Phe Asn Ser Cys Tyr Thr Val Asn His Lys Gln Phe Ser
    50                  55                  60

Leu Asn Trp Thr Tyr Gln Glu Cys Asn Asn Cys Ser Glu Glu Met Phe
65                  70                  75                  80

Leu Gln Phe Arg Met Lys Ile Ile Asn Leu Lys Leu Glu Arg Phe Gln
                85                  90                  95

Asp Arg Val Glu Phe Ser Gly Asn Pro Ser Lys Tyr Asp Val Ser Val
            100                 105                 110

Met Leu Arg Asn Val Gln Pro Glu Asp Glu Gly Ile Tyr Asn Cys Tyr
        115                 120                 125

Ile Met Asn Pro Pro Asp Arg His Arg Gly His Gly Lys Ile His Leu
    130                 135                 140

Gln Val Leu Met Glu Glu Pro Pro Glu Arg Asp Ser Thr Val Ala Val
145                 150                 155                 160

Ile Val Gly Ala Ser Val Gly Gly Phe Leu Ala Val Val Ile Leu Val
                165                 170                 175

Leu Met Val Val Lys Cys Val Arg Arg Lys Lys Glu Gln Lys Leu Ser
            180                 185                 190

Thr Asp Asp Leu Lys Thr Glu Glu Glu Gly Lys Thr Asp Gly Glu Gly
        195                 200                 205

Asn Pro Asp Asp Gly Ala Lys
    210                 215
```

<210> 32
<211> 215
<212> PRT
<213> Homo sapiens

<400> 32

```
Met Pro Ala Phe Asn Arg Leu Phe Pro Leu Ala Ser Leu Val Leu Ile
1               5                   10                  15

Tyr Trp Val Ser Val Cys Phe Pro Val Cys Val Glu Val Pro Ser Glu
            20                  25                  30

Thr Glu Ala Val Gln Gly Asn Pro Met Lys Leu Arg Cys Ile Ser Cys
        35                  40                  45

Met Lys Arg Glu Glu Val Glu Ala Thr Thr Val Val Glu Trp Phe Tyr
    50                  55                  60

Arg Pro Glu Gly Gly Lys Asp Phe Leu Ile Tyr Glu Tyr Arg Asn Gly
65                  70                  75                  80

His Gln Glu Val Glu Ser Pro Phe Gln Gly Arg Leu Gln Trp Asn Gly
                85                  90                  95

Ser Lys Asp Leu Gln Asp Val Ser Ile Thr Val Leu Asn Val Thr Leu
            100                 105                 110

Asn Asp Ser Gly Leu Tyr Thr Cys Asn Val Ser Arg Glu Phe Glu Phe


        115                 120                 125

Glu Ala His Arg Pro Phe Val Lys Thr Thr Arg Leu Ile Pro Leu Arg
    130                 135                 140

Val Thr Glu Glu Ala Gly Glu Asp Phe Thr Ser Val Val Ser Glu Ile
145                 150                 155                 160

Met Met Tyr Ile Leu Leu Val Phe Leu Thr Leu Trp Leu Leu Ile Glu
                165                 170                 175

Met Ile Tyr Cys Tyr Arg Lys Val Ser Lys Ala Glu Glu Ala Ala Gln
            180                 185                 190

Glu Asn Ala Ser Asp Tyr Leu Ala Ile Pro Ser Glu Asn Lys Glu Asn
        195                 200                 205

Ser Ala Val Pro Val Glu Glu
    210                 215
```

<210> 33
<211> 228
<212> PRT
<213> Homo sapiens

<400> 33

```
Met Pro Gly Ala Gly Asp Gly Gly Lys Ala Pro Ala Arg Trp Leu Gly
1               5                   10                  15

Thr Gly Leu Leu Gly Leu Phe Leu Leu Pro Val Thr Leu Ser Leu Glu
            20                  25                  30

Val Ser Val Gly Lys Ala Thr Asp Ile Tyr Ala Val Asn Gly Thr Glu
        35                  40                  45

Ile Leu Leu Pro Cys Thr Phe Ser Ser Cys Phe Gly Phe Glu Asp Leu
    50                  55                  60

His Phe Arg Trp Thr Tyr Asn Ser Ser Asp Ala Phe Lys Ile Leu Ile
65                  70                  75                  80

Glu Gly Thr Val Lys Asn Glu Lys Ser Asp Pro Lys Val Thr Leu Lys
                85                  90                  95

Asp Asp Asp Arg Ile Thr Leu Val Gly Ser Thr Lys Glu Lys Met Asn
            100                 105                 110

Asn Ile Ser Ile Val Leu Arg Asp Leu Glu Phe Ser Asp Thr Gly Lys
        115                 120                 125

Tyr Thr Cys His Val Lys Asn Pro Lys Glu Asn Asn Leu Gln His His
    130                 135                 140

Ala Thr Ile Phe Leu Gln Val Val Asp Arg Leu Glu Glu Val Asp Asn
145                 150                 155                 160

Thr Val Thr Leu Ile Ile Leu Ala Val Val Gly Gly Val Ile Gly Leu
                165                 170                 175

Leu Ile Leu Ile Leu Leu Ile Lys Lys Leu Ile Ile Phe Ile Leu Lys
            180                 185                 190

Lys Thr Arg Glu Lys Lys Lys Glu Cys Leu Val Ser Ser Ser Gly Asn
        195                 200                 205

Asp Asn Thr Glu Asn Gly Leu Pro Gly Ser Lys Ala Glu Glu Lys Pro
    210                 215                 220

Pro Ser Lys Val
225
```

<210> 34
<211> 34
<212> DNA
<213> Artificial Sequence

<220>
<221> source
<223> /note="Description of Artificial Sequence: Synthetic probe"

<400> 34
gcgagagcga caagcagacc ctatagaacc tcgc 34

<210> 35
<211> 6
<212> PRT
<213> Artificial Sequence

<220>
<221> source
<223> /note="Description of Artificial Sequence: Synthetic 6xHis tag"

<400> 35

```
His His His His His His
1               5
```

## Description

## Background

The voltage-gated sodium ion channel family referred to as the NaV family are large and complex molecules that are expressed in the central nervous system, including the brain, in the peripheral nervous system and in muscle, including cardiac muscle. All of the family members are important clinical targets for managing a variety of conditions including epilepsy, muscle paralysis and pain. NaV channels are cell membrane embedded proteins comprising an alpha subunit and one or more beta subunits. Genes coding for ten alpha subunits and four beta subunits have been identified (see, *e.g.,* Catterall et al., Pharmacol Rev. 55:575-578 (2003); Isom, Neuroscientist, 7:42-54 (2001)). The alpha subunit forms the ion pore and is thought to be responsible for selective sodium conduction and voltage-dependent activation and inactivation (see, *e.g.,* Liu et al., Assay Drug Dev Tech, 4(1):37-48 (2006)). Beta subunits have been shown to modify expression levels and biophysical characteristics of some alpha subunits. Liu *et al., supra.* Both the alpha and beta subunits are differentially expressed in different tissues. *Id.*

The discovery of new and improved therapeutics that specifically target NaV family members has been hampered by the lack of cell-based systems and especially cell-based systems that are amenable to high throughput formats for identifying and testing NaV modulators. Cell-based systems are preferred for drug discovery and validation because they provide a functional assay for a compound as opposed to cell-free systems, which only provide a binding assay. Moreover, cell-based systems have the advantage of simultaneously testing cytotoxicity. The present invention addresses this need..

WO 2007/109324 discloses assays for identification of NaV 1.7 channel blockers.

## Summary of the Invention

We have discovered new and useful cells and cell lines that express various forms of NaV, including functional NaV and various combinations of subunits of NaV. These cells and cell lines are useful in cell-based assays, in particular high throughput assays to study the functions of NaV and to screen for NaV modulators.

Accordingly, the invention provides a cell or cell line engineered (altered) to stably express a human NaV 1.7 comprising a NaV alpha 9 subunit, a NaV beta 1 subunit, and a NaV beta 2 subunit wherein the cell line is produced by a method as defined in claim 1, and in which cell or cell line produces a Z' factor of at least 0.6 in an assay. In some embodiments, the Z' factor can be at least 0.65, 0.70, 0.75, 0.80, or 0.85. The cells and cell lines of the invention may be grown (e.g., maintained) in culture in the absence of selective pressure, and may continue to express the NaV for at least 15 days, 30 days, 45 days, 60 days, 75 days, 100 days, 120 days, or 150 days despite the absence of selective pressure. In some embodiments, the cells or cell lines growing in the absence of selective pressure express NaV at a consistent level for at least 15 days, 30 days, 45 days, 60 days, 75 days, 100 days, 120 days, or 150 days.

. In some embodiments, the NaV comprises at least one subunit that is expressed from an introduced nucleic acid encoding it, and/or comprises at least one NaV subunit that is expressed from an endogenous gene activated by gene activation. In some embodiments, the NaV is native, e.g., containing no polypeptide tag.

The cells or cell lines of the invention may be eukaryotic cells (e.g., mammalian cells), and optionally do not express NAV endogenously (or in the case of gene activation, do not express NAV endogenously prior to gene activation). The cells may be primary or immortalized cells, and may be cells of, for example, primate (e.g., human or monkey), rodent (e.g., mouse, rat, or hamster), or insect (e.g., fruit fly) origin.

The NaV alpha subunit is
an alpha 9 subunit having the amino acid sequence set forth in SEQ ID NO:27;
a polypeptide with at least 95% sequence identity, or substantially identical, to SEQ ID NO:27, where the polypeptide may form a voltage-gated ion channel; and
a polypeptide that is an allelic variant to SEQ ID NO:27.

In further embodiments, the NaV alpha subunit is encoded by a nucleic acid sequence SEQ ID NO:13; a nucleic acid sequence that hybridizes under stringent conditions to SEQ ID NO: 13; a nucleic acid sequence with at least 95% sequence identity, or substantially identical, to SEQ ID NO: 13 and a nucleic acid sequence that is an allelic variant of SEQ ID NOS:13.

The NaV beta subunit is selected from the group consisting of:

a beta 1 subunit having the amino acid sequence set forth in SEQ ID NO:30;
a beta 2 subunit having the amino acid sequence set forth in SEQ ID NO:31;
a polypeptide with at least 95% sequence identity, or substantially identical, to any one of SEQ ID NOS:30-31, wherein the polypeptide may modulate a voltage-gated ion channel; and
a polypeptide that is an allelic variant to any one of SEQ ID NOS:30-31.

In further embodiments, the beta subunit is encoded by a nucleic acid sequence individually selected from the group consisting of: SEQ ID NOS:16-17; a nucleic acid that hybridizes under stringent conditions to any one of SEQ ID NOS:16-17; a nucleic acid with at least 95% sequence identity, or substantially identical, to any one of SEQ ID NOS:16-17; and a nucleotide that is an allelic variant of any one. As claimed the NaV 1.7 comprises a human NaV 1.7 alpha 9 subunit, a human beta 1 subunit, and a human beta 2 subunit. The human alpha 9 subunit may comprise (1) the amino acid sequence set forth in SEQ ID NO:27; or (2) an amino acid sequence encoded by a nucleic acid sequence set forth in SEQ ID NO:13. The human beta 1 subunit may comprise (1) the amino acid sequence set forth in SEQ ID NO: 30, or (2) an amino acid sequence encoded by a nucleic acid sequence set forth in SEQ ID NO:16. The human beta 2 subunit may comprise (1) the amino acid sequence set forth in SEQ ID NO:31, or (2) an amino acid sequence encoded by a nucleic acid sequence set forth in SEQ ID NO: 17. In some embodiments, the native NaV may comprise a polypeptide comprising an amino acid sequence set forth in SEQ ID NO:27; a polypeptide comprising the amino acid sequence set forth in SEQ ID NO:30; and a polypeptide comprising the amino acid sequence set forth in SEQ ID NO:31.

The invention further provides a method for identifying a human NaV 1.7 modulator as defined in claim 4, comprising the steps of contacting a cell, a cell line, or a cell (line) collection of the invention with a test compound; and detecting a change in a NaV 1.7 function in a cell
compared to a cell not contacted with the test compound, wherein a change in said function indicates that the test compound is a human NaV 1.7 modulator. The test compound may be a small molecule, a polypeptide, a peptide, or an antibody or an antigen-binding portion thereof. The test compound may be in a library of compounds. The library may be a small molecule library, a combinatorial library, a peptide library or an antibody library. In the present method, the detecting step may be selected from a membrane potential assay, an electrophysiology assay, a binding assay, and the method may be implemented in a high throughout manner.

The invention also provides a collection of cells engineered to stably express a human NaV 1.7 comprising a NaV alpha 9 subunit, a NaV beta 1 subunit and a NaV beta 2 subunit at a consistent level over time as defined in claim 7, the cell made by a method comprising the steps of: (a) providing a plurality of cells that express mRNA(s) encoding the human NaV 1.7; (b) dispersing the cells individually into individual culture vessels, thereby providing a plurality of separate cell cultures; (c) culturing the cells under a set of desired culture conditions using automated cell culture methods characterized in that the conditions are substantially identical for each of the separate cell cultures, during which culturing the number of cells per separate cell culture is normalized, and wherein the separate cultures are passaged on the same schedule; (d) assaying the separate cell cultures to measure expression of the human NaV 1.7 at least twice; and (e) identifying a separate cell culture that expresses the human NaV 1.7 at a consistent level in both assays, thereby obtaining said cell.

## Brief Description of the Figures

**FIG. 1** is a bar graph depicting relative expression of the heterologous human NaV 1.7 α, β1, and β2 subunits in stable NaV 1.7-expressing cell lines. The expression levels were assayed by quantitative RT-PCR and normalized to the expression level of a control GAPDH gene. (+) lanes indicate reactions with reverse transcriptase enzyme added and (-) lanes indicate reactions without reverse transcriptase enzyme.

**FIG. 2** shows the regulation of NaV 1.7 α subunit expression by auxiliary β subunits. Comparative RT-PCR illustrated increased detection of α subunit expression in drug-selected cells when all three NaV 1.7 subunits were co-transfected, compared to cells transfected with only the α subunit.

**FIGS. 3A-C** show electrophysiology data for a produced cell line stably expressing all three NaV 1.7 subunits, indicating the signature response for NaV 1.7. **FIG. 3A** shows sodium currents in response to 20 ms depolarization pulses from -80 mV to +50 mV. **FIG. 3B** shows the resulting current-voltage (I-V) relationship for peak sodium channel currents. **FIG. 3C** shows the inactivation graph for the sodium channel.

**FIG. 4** shows that cells stably expressing all three NaV 1.7 subunits responded to two known NaV activators, veratridine and scorpion venom, while control cells did not. The response was measured by a functional membrane potential cell-based assay.

**FIGS. 5A and 5B** show the activation of cells stably expressing NaV 1.7 in response to test compounds. **FIG. 5A** depicts the activation response of clone C44 (cells expressing all three NaV 1.7 subunits) when exposed to test compounds C18 and K21. **FIG. 5B** depicts the completely blocked response to the same test compounds of clone 60 (cells expressing only a NaV 1.7 alpha subunit). % Control was calculated relative to the response of the two clones to buffer only (*i.e.,* no test compounds added)

## Detailed Disclosure

Unless otherwise defined, all technical and scientific terms used herein have the same meaning as commonly understood by one of ordinary skill in the art to that this invention belongs. In case of conflict, the present specification, including definitions, will control. Throughout this specification and claims, the word "comprise," or variations such as "comprises" or "comprising" will be understood to imply the inclusion of a stated integer or group of integers but not the exclusion of any other integer or group of integers. The materials, methods, and examples are illustrative only and not intended to be limiting.

In order that the present invention may be more readily understood, certain terms are first defined. Additional definitions are set forth throughout the specification.

As used herein, the term "native" protein (*e.g.*, ion channel protein) refers to a protein that does not have a heterologous amino acid sequence appended or inserted to it. For example, "native NaV" used herein includes NaV proteins that do not have a tag sequence that is expressed on the polypeptide level. In some embodiments, a native NaV comprises all the subunits of a naturally occurring NaV where the subunits are intact and properly assembled.

The term "stable" or "stably expressing" is meant to distinguish the cells and cell lines of the invention from cells with transient expression as the terms "stable expression" and "transient expression" would be understood by a person of skill in the art.

The term "cell line" or "clonal cell line" refers to a population of cells that are all progeny of a single original cell. As used herein, cell lines are maintained *in vitro* in cell culture and may be frozen in aliquots to establish banks of clonal cells.

The term "stringent conditions" or "stringent hybridization conditions" describe temperature and salt conditions for hybridizing one or more nucleic acid probes to a nucleic acid sample and washing off probes that have not bound specifically to target nucleic acids in the sample. Stringent conditions are known to those skilled in the art and can be found in Current Protocols in Molecular Biology, John Wiley & Sons, N.Y. (1989), 6.3.1-6.3.6. Aqueous and nonaqueous methods are described in that reference and either can be used. An example of stringent hybridization conditions is hybridization in 6X SSC at about 45°C, followed by at least one wash in 0.2X SSC, 0.1% SDS at 60°C. A further example of stringent hybridization conditions is hybridization in 6X SSC at about 45°C, followed by at least one wash in 0.2X SSC, 0.1% SDS at 65°C. Stringent conditions include hybridization in 0.5M sodium phosphate, 7% SDS at 65°C, followed by at least one

The phrase "percent identical" or "percent identity" in connection with amino acid and/or nucleic acid sequences refers to the similarity between at least two different sequences. This percent identity can be determined by standard alignment algorithms, for example, the Basic Local Alignment Tool (BLAST) described by Altshul et al. ((1990) J. Mol. Biol., 215: 403-410); the algorithm of Needleman et al. ((1970) J. Mol. Biol., 48: 444-453); or the algorithm of Meyers et al. ((1988) Comput. Appl. Biosci., 4: 11-17). A set of parameters may be the Blosum 62 scoring matrix with a gap penalty of 12, a gap extend penalty of 4, and a frameshift gap penalty of 5. The percent identity between two amino acid or nucleotide sequences can also be determined using the algorithm of E. Meyers and W. Miller ((1989) CABIOS, 4:11-17) that has been incorporated into the ALIGN program (version 2.0), using a PAM120 weight residue table, a gap length penalty of 12 and a gap penalty of 4. The percent identity is usually calculated by comparing sequences of similar length. Protein analysis software matches similar sequences using measures of similarity assigned to various substitutions, deletions and other modifications, including conservative amino acid substitutions. For instance, GCG contains programs such as "Gap" and "Bestfit" that can be used with default parameters to determine sequence homology or sequence identity between closely related polypeptides, such as homologous polypeptides from different species of organisms or between a wild type protein and a mutein thereof. See, *e.g.,* GCG Version 6.1. Polypeptide sequences also can be compared using FASTA using default or recommended parameters, a program in GCG Version 6.1. FASTA (*e.g.*, FASTA2 and FASTA3) provides alignments and percent sequence identity of the regions of the best overlap between the query and search sequences (Pearson, Methods Enzymol. 183:63-98 (1990); Pearson, Methods Mol. Biol. 132:185-219 (2000)). The length of polypeptide sequences compared for homology will generally be at least about 16 amino acid residues, usually at least about 20 residues, more usually at least about 24 residues, typically at least about 28 residues, and preferably more than about 35 residues.

The phrase "substantially as set out," "substantially identical" or "substantially homologous" means that the relevant amino acid or nucleotide sequence will be identical to or have insubstantial differences (through conserved amino acid substitutions) in comparison to the sequences that are set out. Insubstantial differences include minor amino acid changes, such as 1 or 2 substitutions in a 50 amino acid sequence of a specified region.

A NaV "modulator" refers to a compound that alters a biological activity of a NaV, *e.g.*, ion conductance via a NaV. A NaV modulator may act upon all or upon a specific subset of NaVs or NaV subunits. Modulators include, but are not limited to, agonists (potentiators or activators) and antagonists (inhibitors or blockers). A NaV agonist refers to a compound that increases a biological activity of a NaV. A NaV antagonist refers to a compound that decreases a biological activity of a NaV.

A "functional NaV" refers to a NaV that has one or more of the biological activities of a naturally occurring or endogenously expressed NaV. Biological activities of NaV include, but are not limited to, voltage-dependent sodium conductance, and can be assessed via pharmacological responses such as inhibition by lidocaine and tetrodotoxin (TTX). Other compounds that are pharmacologically active on NaV and can thus be used to assess the functionality of an introduced NaV include sodium channel openers - compounds that hold the channel in its open state, for example, veratridine, and various scorpion and other venoms.

A "heterologous" or "introduced" NaV subunit means that the NaV subunit is encoded by a polynucleotide introduced into a host cell, or by an endogenous NaV-coding sequence whose expression is activated (*e.g.*, by gene activation technology) by externally introduced factors such as transcriptional regulatory elements. A "heterologous NaV" refers to NaV comprising one or more heterologous NaV subunits.

In a first aspect, the invention as defined in claim 1 provides cells (*e.g.,* isolated cells, clonal cells, or mixtures of clonal cells) and cell lines that express stably one or more heterologous (introduced) human NaV 1.7 subunits (*e.g.*, native NaV1.7 subunits). The cells and cell lines may constitutively express the NaV subunits. The cells and cell lines may be modulated by channel openers such as veratridine and scorpion venom, or membrane voltage changes. In related embodiments, the cells or cell lines stably express a functional heterologous NaV 1.7. The NaV 1.7 cells and cell lines of the invention have enhanced properties compared to cells and cell lines made by conventional methods. For example, the NaV 1.7 cells and cell lines have enhanced stability of expression (even when maintained in culture without selective pressure such as antibiotics) and possess high Z' values in cell-based assays. The cells and cell lines of the invention provide detectable signal-to-noise ratios, e.g., a signal-to-noise ratio greater than 1:1. The cells and cell lines of the invention provide reliable readouts when used in high throughput assays such as membrane potential assays, producing results that can match those from assays that are considered gold-standard in the field but too labor-intensive to carry out in a high-throughput manner (*e.g.*, electrophysiology assays).

In various embodiments, the cells or cell lines of the invention express human NaV 1.7 at a consistent level of expression for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, 200 days or over 200 days, where consistent expression refers to a level of expression that does not vary by more than: 1%, 2%, 3%, 4%, 5%, 6%, 7%, 8% 9% or 10% over 2 to 4 days of continuous cell culture,; 2%, 4%, 6%, 8%, 10% or 12% over 5 to 15 days of continuous cell culture; 2%, 4%, 6%, 8%, 10%, 12%, 14%, 16%, 18% or 20% over 16 to 20 days of continuous cell culture; 2%, 4%, 6%, 8%, 10%, 12%, 14%, 16%, 18%, 20%, 22%, 24% over 21 to 30 days of continuous cell culture; 2%, 4%, 6%, 8%, 10%, 12%, 14%, 16%, 18%, 20%, 22%, 24%, 26%, 28% or 30% over 30 to 40 days of continuous cell culture; 2%, 4%, 6%, 8%, 10%, 12%, 14%, 16%, 18%, 20%, 22%, 24%, 26%, 28% or 30% over 41 to 45 days of continuous cell culture; 2%, 4%, 6%, 8%, 10%, 12%, 14%, 16%, 18%, 20%, 22%, 24%, 26%, 28% or 30% over 45 to 50 days of continuous cell culture; 2%, 4%, 6%, 8%, 10%, 12%, 14%, 16%, 18%, 20%, 22%, 24%, 26%, 28%, 30% or 35% over 45 to 50 days of continuous cell culture, 2%, 4%, 6%, 8%, 10%, 12%, 14%, 16%, 18%, 20%, 22%, 24%, 26%, 28% or 30% over 50 to 55 days of continuous cell culture; 2%, 4%, 6%, 8%, 10%, 12%, 14%, 16%, 18%, 20%, 22%, 24%, 26%, 28%, 30% or 35% over 50 to 55 days of continuous cell culture; 1%, 2%, 3%, 4%, 5%, 10%, 15%, 20%, 25%, 30%, 35% or 40% over 55 to 75 days of continuous cell culture; 1%, 2%, 3%, 4%, 5%, 6%, 10%, 15%, 20%, 25%, 30%, 35%, 40% or 45% over 75 to 100 days of continuous cell culture; 1%, 2%, 3%, 4%, 5%, 6%, 10%, 15%, 20%, 25%, 30%, 35%, 40% or 45% over 101 to 125 days of continuous cell culture; 1%, 2%, 3%, 4%, 5%, 6%, 10%, 15%, 20%, 25%, 30%, 35%, 40% or 45% over 126 to 150 days of continuous cell culture; 1%, 2%, 3%, 4%, 5%, 6%, 10%, 15%, 20%, 25%, 30%, 35%, 40% or 45% over 151 to 175 days of continuous cell culture; 1%, 2%, 3%, 4%, 5%, 6%, 10%, 15%, 20%, 25%, 30%, 35%, 40% or 45% over 176 to 200 days of continuous cell culture; or 1%, 2%, 3%, 4%, 5%, 6%, 10%, 15%, 20%, 25%, 30%, 35%, 40% or 45% over more than 200 days of continuous cell culture.

As defined in claim 1, the cells are triply transfected with nucleic acids encoding, and expresses, a human NaV alpha 9/SCN9A subunit, a human NaV betal/SCN1B subunit and a human NaV beta 2/SCN2B subunit. In some embodiments, coding sequences for two or more of the introduced NaV subunits are placed on the same vector. In other embodiments, each subunit's coding sequence is placed on a different vector.

Also disclosed herein, the present cells and cell lines express an introduced alpha subunit, selected from any one of alpha 1-11, and an introduced beta subunit, selected from any one of beta 1-4, with each combination indicated by a "+" in the following table:

| | Beta 1 | Beta 2 | Beta 3 | Beta 4 |
|---|---|---|---|---|
| Alpha 1 | + | + | + | + |
| Alpha 2 | + | + | + | + |
| Alpha 3 | + | + | + | + |

(continued)

| | Beta 1 | Beta 2 | Beta 3 | Beta 4 |
|---|---|---|---|---|
| Alpha 4 | + | + | + | + |
| Alpha 5 | + | + | + | + |
| Alpha 7 | + | + | + | + |
| Alpha 8 | + | + | + | + |
| Alpha 9 | + | + | + | + |
| Alpha 10 | + | + | + | + |
| Alpha 11 | + | + | + | + |

These cells and cells lines can further express one or more introduced beta subunits independently selected from any one of beta 1-4. Also disclosed herein, the cells and cell lines of the invention express a NaV channel containing a combination of alpha and beta subunits as shown in the above table, and, the NaV channel in these cell lines further comprise one or more beta subunits selected from any one of beta 1-4.

The nucleic acid encoding the NaV subunit can be genomic DNA or cDNA. In some embodiments, the nucleic acid encoding the NaV subunit comprises one or more substitutions, insertions, or deletions that may or may not result in an amino acid substitution. NaV subunits with modifications within the scope of the invention retain at least one biological property, *e.g.*, its ability to function as, or modulate, a voltage-gated sodium channel or to respond to ion channel openers such as veratridine and scorpion and other venoms and channel blockers such as lidocaine and tetrodotoxin (TTX). Accordingly, nucleic acid sequences substantially identical (*e.g.*, at least about 85% sequence identity) or homologous (*e.g.*, at least about 85% sequence homology) to the sequences disclosed herein are also encompassed by this invention. In some embodiment, the sequence identity can be about 85%, 90%, 95%, 96%, 97%, 98%, 99%, or higher. Alternatively, substantial identity or homology exists when the nucleic acid segments will hybridize under stringent hybridization conditions (*e.g.*, highly stringent hybridization conditions) to the complement of the reference sequence.

In some embodiments, where the nucleotide mutation involves an amino acid substitution, the native amino acid may be replaced by a conservative or non-conservative substitution. In some embodiments, the sequence identity between the original and modified polypeptide sequences can be at least 85%, 90%, 95%, 96%, 97%, 98%, 99% or higher. Those of skill in the art will understand that a conservative amino acid substitution is one in which the amino acid side chains are similar in structure and/or chemical properties and the substitution should not substantially change the structural characteristics of the wild type sequence. In embodiments using a nucleic acid comprising a mutation, the mutation may be a random mutation or a site-specific mutation.

Conservative modifications will produce NaV receptors having functional and chemical characteristics similar to those of the unmodified NaV receptor. A "conservative amino acid substitution" is one in which an amino acid residue is substituted by another amino acid residue having a side chain R group) with similar chemical properties (e.g., charge or hydrophobicity). In general, a conservative amino acid substitution will not substantially change the functional properties of a protein. In cases where two or more amino acid sequences differ from each other by conservative substitutions, the percent sequence identity or degree of similarity may be adjusted upwards to correct for the conservative nature of the substitution. Means for making this adjustment are well-known to those of skill in the art. See *e.g.* Pearson, Methods Mol. Biol. 243:307-31 (1994).

Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative replacement is any change having a positive value in the PAM250 log-likelihood matrix disclosed in Gonnet et al., Science 256:1443-45 (1992). A "moderately conservative" replacement is any change having a nonnegative value in the PAM250 log-likelihood matrix.

In some embodiments, the NaV subunit-coding nucleic acid sequence further comprises an epitope tag. Such tags may encode, for example, yellow fluorescent protein (YFP), green fluorescent protein (GFP), 6x-HIS (SEQ ID NO: 35), myc, FLAG, or hemagglutinin (HA), S-tag, thioredoxin, autofluorescent proteins, GST, V5, TAP, CBP, BCCP, Maltose binding protein-tag, Nus-tag, Softag 1, Softag 3, Strep-tag, or a variant of the aforementioned. A tag may be used as a marker to determine the expression levels, intracellular localization, protein-protein interaction, regulation, and function of a NaV or a subunit thereof. A tag also may be used to facilitate protein purification and fractionation. These and other tag sequences are known to one of skill in the art and typically correspond to amino acid sequences that may be incorporated into expressed protein products and often selected based on the availability of robust antibodies or protein detection reagents that may be used to report their presence. However, tag sequences described herein are not meant to refer solely to sequences that may be used to modify, at the amino acid level, protein products encoded by the RNAs that are tagged, or to aid in the subsequent detection of any such modified protein products through use of the corresponding antibody or protein detection reagents. See, for example, discussions below in regard to using RNA tags used as "molecular beacons."

Host cells used to produce a cell line of the invention may express one or more endogenous NaV proteins or lack expression of one or more of any NaV protein. The host cell may be a primary, germ, or stem cell, including an embryonic stem cell. The host cell may also be an immortalized cell. The host cell may be derived from a primary or immortalized cell from mesoderm, ectoderm, or endoderm layers. The host cell may be endothelial, epidermal, mesenchymal, neural, renal, hepatic, hematopoietic, or immune cells. For example, the host cells may be intestinal crypt or villi cells, clara cells, colon cells, intestinal cells, goblet cells, enterochromafin cells, enteroendocrine cells. The host cells may be eukaryotic, prokaryotic, mammalian, human, primate, bovine, porcine, feline, rodent, marsupial, murine or other cells. The host cells may also be nonmammalian, such as yeast, insect, fungus, plant, lower eukaryotes and prokaryotes. Such host cells may provide backgrounds that are more divergent for testing with a greater likelihood for the absence of expression products provided by the cell that may interact with the target. In preferred embodiments, the host cell is a mammalian cell. Examples of host cells that may be used for a cell line of the invention include but are not limited to: Chinese hamster ovary (CHO) cells, established neuronal cell lines, pheochromocytomas, neuroblastomas fibroblasts, rhabdomyosarcomas, dorsal root ganglion cells, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), L-cells, HEK-293 (ATCC CRL1573), PC12 (ATCC CRL-1721), HEK293T (ATCC CRL-11268), RBL (ATCC CRL-1378), SH-SY5Y (ATCC CRL-2266), MDCK (ATCC CCL-34), SJ-RH30 (ATCC CRL-2061), HepG2 (ATCC HB-8065), ND7/23 (ECACC 92090903), CHO (ECACC 85050302), Vero (ATCC CCL 81), Caco-2 (ATCC HTB 37), K562 (ATCC CCL 243), Jurkat (ATCC TIB-152), Per.C6 (Crucell, Leiden, The Netherlands), Huvec (ATCC Human Primary PCS 100-010, Mouse CRL 2514, CRL 2515, CRL 2516), HuH-7D12 (ECACC 01042712), 293 (ATCC CRL 10852), A549 (ATCC CCL 185), IMR-90 (ATCC CCL 186), MCF-7 (ATC HTB-22), U-2 OS (ATCC HTB-96), T84 (ATCC CCL 248), or any established cell line (polarized or nonpolarized) or any cell line available from repositories such as The American Type Culture Collection (ATCC, 10801 University Blvd. Manassas, Va. 20110-2209 USA) or European Collection of Cell Cultures (ECACC, Salisbury Wiltshire SP4 0JG England). One of ordinary skill in the art will understand that different known or unknown accessory factors that may interact with or alter the function or expression of the target depending on the choice of host cell type.

In one embodiment, the host cell is an embryonic stem cell that is then used as the basis for the generation of transgenic animals. Embryonic stem cells stably expressing at least one NaV subunit, and preferably a functional heterologous NaV receptor, may be implanted into organisms directly, or their nuclei may be transferred into other recipient cells and these may then be implanted *in vivo* for studying growth and development. The embryonic stem cells also may be used to create transgenic animals.

As will be appreciated by those of skill in the art, any vector that is suitable for use with the host cell may be used to introduce a nucleic acid encoding a NaV alpha or beta subunit into the host cell. The vectors comprising the alpha and each of the beta subunits may be the same type or may be of different types. Examples of vectors that may be used to introduce the NaV subunit-encoding nucleic acids into host cells include but are not limited to plasmids, viruses, including retroviruses and lentiviruses, cosmids, artificial chromosomes and may include for example, pFN11A (BIND) Flexi®, pGL4.31, pFC14A (HaloTag® 7) CMV Flexi®, pFC14K (HaloTag® 7) CMV Flexi®, pFN24A (HaloTag® 7) CMVd3 Flexi®, pFN24K (HaloTag® 7) CMVd3 Flexi®, HaloTag™ pHT2, pACT, pAdVAntage™, pALTER®-MAX, pBIND, pCAT®3-Basic, pCAT®3-Control, pCAT®3-Enhancer, pCAT®3-Promoter, pCI,pCMVTNT™, pG51uc, pSI, pTARGET™, pTNT™, pF12A RM Flexi®, pF12K RM Flexi®, pReg neo, pYES2/GS, pAd/CMV/V5-DEST Gateway® Vector, pAd/PL-DEST™ Gateway® Vector, Gateway® pDEST™27 Vector,Gateway® pEF-DEST51 Vector, Gateway® pcDNA™-DEST47 vector, pCMV/Bsd Vector, pEF6/His A, B, & C, pcDNA™6.2-DEST, pLenti6/TR, pLP-AcGFPl-C, pLPS-AcGFP1-N, pLP-IRESneo, pLP-TRE2, pLP-RevTRE, pLP-LNCX, pLP-CMV-HA, pLP-CMV-Myc, pLP-RetroQ, pLP-CMVneo, pCMV-Script, pcDNA3.1 Hygro, pcDNA3.1neo, pcDNA3.1puro, pSV2neo, pIRES puro, and pSV2 zeo. In some embodiments, the vectors comprise expression control sequences such as constitutive or conditional promoters. One of ordinary skill in the art will be able to select such sequences. For example, suitable promoters include but are not limited to CMV, TK, SV40 and EF-1$\alpha$. In some embodiments, the promoters are inducible, temperature regulated, tissue specific, repressible, heat-shock, developmental, cell lineage specific, prokaryotic and/or eukaryotic expressible or temporal promoters or a combination or recombination of unmodified or mutagenized, randomized, shuffled sequences of any one or more of the above. Nucleic acids encoding NaV subunits are preferably constitutively expressed.

In some embodiments, the vector lacks a selectable marker or drug resistance gene. In other embodiments, the vector optionally comprises a nucleic acid encoding a selectable marker such as a protein that confers drug or antibiotic resistance. Each vector for a sequence encoding a different NaV subunit may have the same or a different drug resistance or other selectable marker. If more than one of the drug resistance markers are the same, simultaneous selection may be achieved by increasing the level of the drug. Suitable markers will be well-known to those of skill in the art and include but are not limited to genes conferring resistance to any one of the following: Neomycin/G418, Puromycin, hygromycin, Zeocin, methotrexate and blasticidin. Although drug selection (or selection using any other suitable selection marker) is not a required step, it may be used, if desired, to enrich the transfected cell population for stably transfected cells, provided that the transfected constructs are designed to confer drug resistance. If selection is accomplished using signaling probes, selection performed too soon following transfection can result in some positive cells that may only be transiently and not stably transfected. However, this can be minimized by allowing sufficient cell passage, allowing for dilution of transiently transfected cells, stably integrated cells that do not express the introduced DNA, or cells that generate RNA that may not be efficiently detected by the signaling probes.

In another aspect of the invention, cells and cell lines of the invention stably express human NaV 1.7 or a NaV 1.7 subunit. To identify stable expression, a cell line's expression of each NaV subunit is measured over a time course and the expression levels are compared. Stable cell lines will continue expressing the human NaV 1.7 subunits throughout the time course at substantially the same level (*e.g.,* no more than 40%, 30%, 20%, 15%, 10%, 5%, or 2% variation). In some aspects of the invention, the time course may be for at least one week, two weeks, three weeks, or four weeks; or at least one, two, three, four, five, six, seven, eight, or nine months, or at least any length of time in between. Isolated cells can be further characterized, such as by qRT-PCR and single end-point RT-PCR to determine the absolute and/or relative amounts of each NaV subunit being expressed, or by any other conventional method of protein expression analysis.

Cells and cell lines of the invention have the further advantageous property of providing assays with high reproducibility as evidenced by their Z' factor. See Zhang JH, Chung TD, Oldenburg KR, "A Simple Statistical Parameter for Use in Evaluation and Validation of High Throughput Screening Assays." J. Biomol. Screen. 1999;4(2):67-73. Z' values pertain to the quality of a cell or cell line because it reflects the degree to which a cell or cell line will respond consistently to modulators. Z' is a statistical calculation that takes into account the signal-to-noise range and signal variability *(i.e.,* from well to well) of the functional response to a reference compound across a multiwell plate. Z' is calculated using data obtained from multiple wells with a positive control and multiple wells with a negative control. The ratio of their combined standard deviations multiplied by three to the difference in their mean values is subtracted from one to give the Z' factor, according the equation below:

$$Z'\square\text{factor} = 1 - ((3\sigma_{\text{positive control}} + 3\sigma_{\text{negative control}}) / (\mu_{\text{positive control}} - \mu_{\text{negative control}}))$$

The theoretical maximum Z' factor is 1.0, which would indicate an ideal assay with no variability and limitless dynamic range. As used herein, a "high Z'" refers to a Z' factor of Z' of at least 0.6, at least 0.7, at least 0.75 or at least 0.8, or any decimal in between 0.6 and 1.0. A score less than 0 is undesirable because it indicates that there is overlap between positive and negative controls. In the industry, for simple cell-based assays, Z' scores up to 0.3 are considered marginal scores, Z' scores between 0.3 and 0.5 are considered acceptable, and Z' scores above 0.5 are considered excellent. Cell-free or biochemical assays may approach higher Z' scores, but Z' scores for cell-based systems tend to be lower because cell-based systems are complex.

As those of ordinary skill in the art will recognize, historically, cell-based assays using cells expressing a single chain protein do not typically achieve a Z' higher than 0.5 to 0.6. Such cells would not be reliable to use in an assay because the results are not reproducible. Cells and cell lines of the invention, on the other hand, have high Z' values and advantageously produce consistent results in assays. NaV cells and cell lines of the invention provide the basis for high throughput screening (HTS) compatible assays because they generally have Z' factors at least 0.7. In some aspects of the invention, the cells and cell lines result in Z' of at least 0.3, at least 0.4, at least 0.5, at least 0.6, at least 0.7, or at least 0.8. In other aspects of the invention, the cells and cell lines of the invention result in a Z' of at least 0.7, at least 0.75 or at least 0.8 maintained for multiple passages, e.g., between 5-20 passages, including any integer in between 5 and 20. In some aspects of the invention, the cells and cell lines result in a Z' of at least 0.7, at least 0.75 or at least 0.8 maintained for 1, 2, 3, 4 or 5 weeks or 2, 3, 4, 5, 6, 7, 8 or 9 months, including any period of time in between.

Also according to the invention, cells and cell lines that express a recombinant form of a naturally occurring NaV hetero-multimer (in contrast to cell lines expressing non-naturally occurring combinations of alpha and beta subunits or expressing just the alpha or beta subunits) can be characterized for NaV functions, e.g., sodium ion conductance. In some embodiments, the cells and cell lines of the invention display "physiologically relevant" activity of an introduced ion channel. As used herein, "physiological relevance" refers to a property of a cell or a cell line expressing an introduced ion channel whereby the introduced ion channel behaves, e.g., responds to a modulator, in substantially the same way as a naturally occurring channel of the same type, *e.g.,* a naturally occurring receptor having the same combination of alpha and beta subunits. Cells and cell lines of this invention preferably demonstrate comparable function to cells that normally express endogenous (native) NaV (*e.g.*, primary cells) in a suitable assay, such as a membrane potential assay using sodium as a NaV activator, an electrophysiology assay, and a binding or panning assay. Such comparisons are used to determine a cell or cell line's physiological relevance.

In another aspect of the invention, modulators identified using the cell lines of the invention can be used in additional assays to confirm functionality. A further advantageous property of cells and cell lines of the inventions is that modulators identified in initial screening are functional in secondary functional assays, *e.g.*, membrane potential assay or an electrophysiology assay. As those of ordinary skill in the art will recognize, compounds identified in initial screening assays typically must be modified, such as by combinatorial chemistry, medicinal chemistry or synthetic chemistry, for their derivatives or analogs to be functional in secondary functional assays. However, due to the high physiological relevance of the present cells and cell lines, compounds identified therewith may not require such "coarse" tuning.

In some embodiments, the cells and cell lines of the invention have increased sensitivity to modulators of NaV. Cells and cell lines of the invention respond to modulators with physiological range $EC_{50}$ or $IC_{50}$ values for NaV. As used herein, $EC_{50}$ refers to the concentration of a compound or substance required to induce a half-maximal activating response in the cell or cell line. As used herein, $IC_{50}$ refers to the concentration of a compound or substance required to induce a half-maximal inhibitory response in the cell or cell line. $EC_{50}$ and $IC_{50}$ values may be determined using techniques that are well-known in the art, for example, a dose-response curve that correlates the concentration of a compound or substance to the response of the NaV-expressing cell line. For example, the $IC_{50}$ for tetrodotoxin (TTX) in a cell line of the invention is about 1-100 nM, e.g., 1, 2, 5, 10, 20, 30, 40, 50, 60, 70, 80, or 90 nM.

In some embodiments, properties of the cells and cell lines of the invention, such as stability, physiological relevance, reproducibility in an assay (Z'), or physiological $EC_{50}$ or $IC_{50}$ values, are achievable under specific culture conditions. In some embodiments, the culture conditions are standardized and rigorously maintained without variation, for example, by automation. Culture conditions may include any suitable conditions under which the cells or cell lines are grown and may include those known in the art. A variety of culture conditions may result in advantageous biological properties for NAV, or its mutants or allelic variants.

In other embodiments, the cells and cell lines of the invention with desired properties, such as stability, physiological relevance, reproducibility in an assay (Z'), or physiological $EC_{50}$ or $IC_{50}$ values, can be obtained within one month or less. For example, the cells or cell lines may be obtained within 2, 3, 4, 5, or 6 days, or within 1, 2, 3 or 4 weeks, or any length of time in between.

The present cells and cell lines can be used in a collection or panel, each set of cells or each cell line expressing one form of NaV [*e.g.*, NaV comprised of various combinations *(e.g.,* dimers, trimers, etc.) of alpha and beta subunits or variants *(e.g.,* mutants, fragments, or spliced variants) of the subunits, or a mono- or multi-mer of only an alpha or beta subunit]. The collection may include, for example, cell lines expressing two or more of the aforementioned NaV receptors. In some embodiments, the collection or panel may further comprise members expressing the same NaV or expressing control proteins.

When collections or panels of cells or cell lines are produced, e.g., for drug screening, the cells or cell lines in the collection or panel may be matched such that they are the same (including substantially the same) with regard to one or more selective physiological properties. The "same physiological property" in this context means that the selected physiological property is similar enough amongst the members in the collection or panel such that the cell collection or panel can produce reliable results in drug screening assays; for example, variations in readouts in a drug screening assay will be due to, e.g., the different biological activities of test compounds on cells expressing different forms of NaV, rather than due to inherent variations in the cells. For example, the cells or cell lines may be matched to have the same growth rate, i.e., growth rates with no more than one, two, three, four, or five hour difference amongst the members of the cell collection or panel. This may be achieved by, for example, binning cells by their growth rate into five, six, seven, eight, nine, or ten groups, and creating a panel using cells from the same binned group. Methods of determining cell growth rate are well known in the art. The cells or cell lines in a panel also can be matched to have the same Z' factor (e.g., Z' factors that do not differ by more than 0.1), NaV expression level (e.g., NaV expression levels that do not differ by more than 5%, 10%, 15%, 20%, 25%, or 30%), adherence to tissue culture surfaces. Matched cells and cell lines can be grown under identical conditions, achieved by, e.g., automated parallel processing, to maintain the selected physiological property.

Matched cell panels of the invention can be used to, for example, identify modulators with defined activity (e.g., agonist or antagonist) on NaV; to profile compound activity across different forms of NaV; to identify modulators active on just one form of NaV; and to identify modulators active on just a subset of NaVs. The matched cell panels of the invention allow high throughput screening. Screenings that used to take months to accomplish can now be accomplished within weeks.

To make cells and cell lines of the invention, one can use, for example, the technology described in U.S. Patent 6,692,965 and WO/2005/079462. This technology provides real-time assessment of millions of cells such that any desired number of clones (from hundreds to thousands of clones). Using cell sorting techniques, such as flow cytometric cell sorting *(e.g.,* with a FACS machine) or magnetic cell sorting *(e.g.,* with a MACS machine), one cell per well is automatically deposited with high statistical confidence in a culture vessel (such as a 96 well culture plate). The speed and automation of the technology allows multigene recombinant cell lines to be readily isolated.

Using the technology, the RNA sequence for each NaV subunit may be detected using a signaling probe, also referred to as a molecular beacon or fluorogenic probe. In some embodiments, the vector containing the NaV subunit-coding sequence has an additional sequence coding for an RNA tag sequence. "Tag sequence" refers to a nucleic acid sequence that is an expressed RNA or portion of an RNA that is to be detected by a signaling probe. Signaling probes may detect a variety of RNA sequences, any of which may be used as tags, including those encoding peptide and protein tags described above. Signaling probes may be directed against the tag by designing the probes to include a portion that is complementary to the sequence of the tag. The tag sequence may be a 3' untranslated region of the plasmid that is cotranscribed with a NaV transcript and comprises a target sequence for signaling probe binding. The tag sequence can be in frame with the protein-coding portion of the message of the gene or out of frame with it, depending on whether one wishes to tag the protein produced. Thus, the tag sequence does not have to be translated for detection by the signaling probe. The tag sequences may comprise multiple target sequences that are the same or different, wherein one signaling probe hybridizes to each target sequence. The tag sequence may be located within the RNA encoding the gene of interest, or the tag sequence may be located within a 5'- or 3'-untranslated region. The tag sequences may be an RNA having secondary structure. The structure may be a three-arm junction structure. In some embodiments, the signaling probe detects a sequence within the NaV subunit-coding sequence.

Nucleic acids comprising a sequence encoding a NaV subunit, optionally a sequence coding for a tag sequence, and optionally a nucleic acid encoding a selectable marker may be introduced into selected host cells by well known methods. The methods include transfection, viral delivery, protein or peptide mediated insertion, coprecipitation methods, lipid based delivery reagents (lipofection), cytofection, lipopolyamine delivery, dendrimer delivery reagents, electroporation or mechanical delivery. Examples of transfection reagents are GENEPORTER, GENEPORTER2, LIPOFECTAMINE, LIPOFECTAMINE 2000, OLIGOFECTAMINE, TRANSFAST, TRANSFECTAM, GENESHUTTLE, TROJENE, GENESILENCER, X-TREMEGENE, PERFECTIN, CYTOFECTIN, SIPORT, UNIFECTOR, FUGENE 6, FUGENE HD, TFX-10, TFX-20, TFX-50, SIFECTOR, TRANSIT-LT1, TRANSIT-LT2, TRANSIT-EXPRESS, IFECT, RNAI SHUTTLE, METAFECTENE, LYOVEC, LIPOTAXI, GENEERASER, GENEJUICE, CYTOPURE, JETSI, JETPEI, MEGAFECTIN, POLYFECT, TRANSMESSANGER, RNAiFECT, SUPERFECT, EFFECTENE, TF-PEI-KIT, CLONFECTIN, AND METAFECTINE.

Following transfection of the DNA constructs into cells and subsequent drug selection (if used), or following gene activation as described above, molecular beacons (e.g., fluorogenic probes), each of which is targeted to a different tag sequence and differentially labeled, may be introduced into the cells, and a flow cytometric cell sorter is used to isolate cells positive for their signals (multiple rounds of sorting may be carried out). In one embodiment, the flow cytometric cell sorter is a FACS machine. MACS (magnetic cell sorting) or laser ablation of negative cells using laser-enabled analysis and processing can also be used. Other fluorescence plate readers, including those that are compatible with high-throughput screening can also be used. Signal-positive cells have taken up and may have integrated into their genomes at least one copy of the introduced NaV sequence(s). Cells introduced with one or more of the NaV subunits are identified. By way of example, the NaV subunit sequences may be integrated at different locations of the genome in the cell. The expression level of the introduced genes encoding the NaV subunits may vary based upon copy number or integration site. Further, cells comprising one or more of the NaV subunits may be obtained wherein one or more of the introduced genes encoding a NaV subunit is episomal or results from gene activation.

Signaling probes useful in this invention are known in the art and generally are oligonucleotides comprising a sequence complementary to a target sequence and a signal emitting system so arranged that no signal is emitted when the probe is not bound to the target sequence and a signal is emitted when the probe binds to the target sequence. By way of non-limiting illustration, the signaling probe may comprise a fluorophore and a quencher positioned in the probe so that the quencher and fluorophore are brought together in the unbound probe. Upon binding between the probe and the target sequence, the quencher and fluorophore separate, resulting in emission of signal. International publication WO/2005/079462, for example, describes a number of signaling probes that may be used in the production of the present cells and cell lines. The methods described above for introducing nucleic acids into cells may be used to introduce signaling probes.

Where tag sequences are used, the vector for each of the NaV subunit can comprise the same or a different tag sequence. Whether the tag sequences are the same or different, the signaling probes may comprise different signal emitters, such as different colored fluorophores so that expression of each subunit may be separately detected. By way of illustration, the signaling probe that specifically detects NaV alpha subunit mRNA can comprise a red fluorophore, the probe that detects the first NaV beta subunit can comprise a green fluorophore, and the probe that detects the second NaV beta subunit can comprise a blue fluorophore. Those of skill in the art will be aware of other means for differentially detecting the expression of the three subunits with a signaling probe in a triply transfected cell.

In one embodiment, the signaling probes are designed to be complementary to either a portion of the RNA encoding a NaV subunit or to portions of their 5' or 3' untranslated regions. Even if the signaling probe designed to recognize a messenger RNA of interest is able to detect spuriously endogenously expressed target sequences, the proportion of these in comparison to the proportion of the sequence of interest produced by transfected cells is such that the sorter is able to discriminate the two cell types.

The expression level of an introduced NaV subunit may vary from cell line to cell line. The expression level in a cell line also may decrease over time due to epigenetic events such as DNA methylation and gene silencing and loss of transgene copies. These variations can be attributed to a variety of factors, for example, the copy number of the transgene taken up by the cell, the site of genomic integration of the transgene, and the integrity of the transgene following genomic integration. One may use FACS to evaluate expression levels. Cells expressing an introduced NaV subunit at desired levels can be isolated by, *e.g.,* FACS. Signaling probes also may be re-applied to previously generated cells or cell lines, for example, to determine if and to what extent the cells are still positive for any one or more of the RNAs for which they were originally isolated.

Once cells expressing all three NaV subunits are isolated, they may be cultured for a length of time sufficient to identify those stably expressing all the desired subunits. In another embodiment of the invention, adherent cells can be adapted to suspension before or after cell sorting and isolating single cells. In other embodiments, isolated cells may be grown individually or pooled to give rise to populations of cells. Individual or multiple cell lines may also be grown separately or pooled. If a pool of cell lines is producing a desired activity or has a desired property, it can be further fractionated until the cell line or set of cell lines having this effect is identified. Pooling cells or cell lines may make it easier to maintain large numbers of cell lines without the requirements for maintaining each separately. Thus, a pool of cells or cell lines may be enriched for positive cells. An enriched pool may have at least 50%, at least 60%, at least 70%, at least 80%, at least 90% or 100% are positive for the desired property or activity.

In a further aspect, the invention provides a method for producing the cells and cell lines of the invention as defined in claim 8.

According to the method, the cells are cultured under a desired set of culture conditions. The conditions can be any desired conditions. Those of skill in the art will understand what parameters are comprised within a set of culture conditions. For example, culture conditions include but are not limited to: the media (Base media (DMEM, MEM, RPMI, serum-free, with serum, fully chemically defined, without animal-derived components), mono and divalent ion (sodium, potassium, calcium, magnesium) concentration, additional components added (amino acids, antibiotics, glutamine, glucose or other carbon source, HEPES, channel blockers, modulators of other targets, vitamins, trace elements, heavy metals, co-factors, growth factors, anti-apoptosis reagents), fresh or conditioned media, with HEPES, pH, depleted of certain nutrients or limiting (amino acid, carbon source)), level of confluency at which cells are allowed to attain before split/passage, feeder layers of cells, or gamma-irradiated cells, CO2, a three gas system (oxygen, nitrogen, carbon dioxide), humidity, temperature, still or on a shaker, which will be well known to those of skill in the art.

The cell culture conditions may be chosen for convenience or for a particular desired use of the cells. Advantageously, the invention provides cells and cell lines that are optimally suited for a particular desired use. That is, in embodiments of the invention in which cells are cultured under conditions for a particular desired use, cells are selected that have desired characteristics under the condition for the desired use. By way of illustration, if cells will be used in assays in plates where it is desired that the cells are adherent, cells that display adherence under the conditions of the assay may be selected. Similarly, if the cells will be used for protein production, cells may be cultured under conditions appropriate for protein production and selected for advantageous properties for this use.

In some embodiments, the method comprises the additional step of measuring the growth rates of the separate cell cultures. Growth rates may be determined using any of a variety of techniques means that will be well known to the skilled worker. Such techniques include but are not limited to measuring ATP, cell confluency, light scattering, optical density (e.g., OD 260 for DNA). Preferably growth rates are determined using means that minimize the amount of time that the cultures spend outside the selected culture conditions.

In some embodiments, cell confluency is measured and growth rates are calculated from the confluency values. In some embodiments, cells are dispersed and clumps removed prior to measuring cell confluency for improved accuracy. Means for monodispersing cells are well-known and can be achieved, for example, by addition of a dispersing reagent to a culture to be measured. Dispersing agents are well-known and readily available, and include but are not limited to enzymatic dispering agents, such as trypsin, and EDTA-based dispersing agents. Growth rates can be calculated from confluency date using commercially available software for that purpose such as HAMILTON VECTOR. Automated confluency measurement, such as using an automated microscopic plate reader is particularly useful. Plate readers that measure confluency are commercially available and include but are not limited to the CLONE SELECT IMAGER (Genetix). Typically, at least 2 measurements of cell confluency are made before calculating a growth rate. The number of confluency values used to determine growth rate can be any number that is convenient or suitable for the culture. For example, confluency can be measured multiple times over e.g., a week, 2 weeks, 3 weeks or any length of time and at any frequency desired.

When the growth rates are known, according to the method, the plurality of separate cell cultures are divided into groups by similarity of growth rates. By grouping cultures into growth rate bins, one can manipulate the cultures in the group together, thereby providing another level of standardization that reduces variation between cultures. For example, the cultures in a bin can be passaged at the same time, treated with a desired reagent at the same time, etc. Further, functional assay results are typically dependent on cell density in an assay well. A true comparison of individual clones is only accomplished by having them plated and assayed at the same density. Grouping into specific growth rate cohorts enables the plating of clones at a specific density that allows them to be functionally characterized in a high throughput format.

The range of growth rates in each group can be any convenient range. It is particularly advantageous to select a range of growth rates that permits the cells to be passaged at the same time and avoid frequent renormalization of cell numbers. Growth rate groups can include a very narrow range for a tight grouping, for example, average doubling times within an hour of each other. But according to the method, the range can be up to 2 hours, up to 3 hours, up to 4 hours, up to 5 hours or up to 10 hours of each other or even broader ranges. The need for renormalization arises when the growth rates in a bin are not the same so that the number of cells in some cultures increases faster than others. To maintain substantially identical conditions for all cultures in a bin, it is necessary to periodically remove cells to renormalize the numbers across the bin. The more disparate the growth rates, the more frequently renormalization is needed.

The cells and cell lines may be tested for and selected for any physiological property including: a change in a cellular process encoded by the genome ;a change in a cellular process regulated by the genome; a change in a pattern of chromosomal activity; a change in a pattern of chromosomal silencing; a change in a pattern of gene silencing; a change in a pattern or in the efficiency of gene activation; a change in a pattern or in the efficiency of gene expression; a change in a pattern or in the efficiency of RNA expression; a change in a pattern or in the efficiency of RNAi expression; a change in a pattern or in the efficiency of RNA processing; a change in a pattern or in the efficiency of RNA transport; a change in a pattern or in the efficiency of protein translation; a change in a pattern or in the efficiency of protein folding; a change in a pattern or in the efficiency of protein assembly; a change in a pattern or in the efficiency of protein modification; a change in a pattern or in the efficiency of protein transport; a change in a pattern or in the efficiency of transporting a membrane protein to a cell surface change in growth rate; a change in cell size; a change in cell shape; a change in cell morphology; a change in % RNA content; a change in % protein content; a change in % water content; a change in % lipid content; a change in ribosome content; a change in mitochondrial content; a change in ER mass; a change in plasma membrane surface area; a change in cell volume; a change in lipid composition of plasma membrane; a change in lipid composition of nuclear envelope; a change in protein composition of plasma membrane; a change in protein; composition of nuclear envelope; a change in number of secretory vesicles; a change in number of lysosomes; a change in number of vacuoles; a change in the capacity or potential of a cell for: protein production, protein secretion, protein folding, protein assembly, protein modification, enzymatic modification of protein, protein glycosylation, protein phosphorylation, protein dephosphorylation, metabolite biosynthesis, lipid biosynthesis, DNA synthesis, RNA synthesis, protein synthesis, nutrient absorption, cell growth, mitosis, meiosis, cell division, to dedifferentiate, to transform into a stem cell, to transform into a pluripotent cell, to transform into a omnipotent cell, to transform into a stem cell type of any organ (i.e. liver, lung, skin, muscle, pancreas, brain, testis, ovary, blood, immune system, nervous system, bone, cardiovascular system, central nervous system, gastro-intestinal tract, stomach, thyroid, tongue, gall bladder, kidney, nose, eye, nail, hair, taste bud), to transform into a differentiated any cell type (i.e. muscle, heart muscle, neuron, skin, pancreatic, blood, immune, red blood cell, white blood cell, killer T-cell, enteroendocrine cell, taste, secretory cell, kidney, epithelial cell, endothelial cell, also including any of the animal or human cell types already listed that can be used for introduction of nucleic acid sequences), to uptake DNA, to uptake small molecules, to uptake fluorogenic probes, to uptake RNA, to adhere to solid surface, to adapt to serum-free conditions, to adapt to serum-free suspension conditions, to adapt to scaled-up cell culture, for use for large scale cell culture, for use in drug discovery, for use in high throughput screening, for use in a functional cell based assay, for use in membrane potential assays, for use in reporter cell based assays, for use in ELISA studies, for use in *in vitro* assays, for use *in vivo* applications, for use in secondary testing, for use in compound testing, for use in a binding assay, for use in panning assay, for use in an antibody panning assay, for use in imaging assays, for use in microscopic imaging assays, for use in multiwell plates, for adaptation to automated cell culture, for adaptation to miniaturized automated cell culture, for adaptation to large-scale automated cell culture, for adaptation to cell culture in multiwell plates (6, 12, 24, 48, 96, 384, 1536 or higher density), for use in cell chips, for use on slides, for use on glass slides, for microarray on slides or glass slides, for use in biologics production, and for use in the production of reagents for research.

Tests that may be used to characterize cells and cell lines of the invention and/or matched panels of the invention include but are not limited to: amino acid analysis, DNA sequencing, protein sequencing, NMR, a test for protein transport, a test for nucelocytoplasmic transport, a test for subcellular localization of proteins, a test for subcellular localization of nucleic acids, microscopic analysis, submicroscopic analysis, fluorescence microscopy, electron microscopy, confocal microscopy, laser ablation technology, cell counting and Dialysis. The skilled worker would understand how to use any of the above-listed tests.

According to the method, cells may be cultured in any cell culture format so long as the cells or cell lines are dispersed in individual cultures prior to the step of measuring growth rates. For example, for convenience, cells may be initially pooled for culture under the desired conditions and then individual cells separated one cell per well or vessel. Cells may be cultured in multi-well tissue culture plates with any convenient number of wells. Such plates are readily commercially available and will be well knows to a person of skill in the art. In some cases, cells may preferably be cultured in vials or in any other convenient format, the various formats will be known to the skilled worker and are readily commercially available.

In embodiments comprising the step of measuring growth rate, prior to measuring growth rates, the cells are cultured for a sufficient length of time for them to acclimate to the culture conditions. As will be appreciated by the skilled worker, the length of time will vary depending on a number of factors such as the cell type, the chosen conditions, the culture format and may be any amount of time from one day to a few days, a week or more.

Preferably, each individual culture in the plurality of separate cell cultures is maintained under substantially identical conditions a discussed below, including a standardized maintenance schedule. Another advantageous feature of the method is that large numbers of individual cultures can be maintained simultaneously, so that a cell with a desired set of traits may be identified even if extremely rare. For those and other reasons, according to the invention, the plurality of separate cell cultures are cultured using automated cell culture methods so that the conditions are substantially identical for each well. Automated cell culture prevents the unavoidable variability inherent to manual cell culture.

Any automated cell culture system may be used in the method of the invention. A number of automated cell culture systems are commercially available and will be well-known to the skilled worker. In some embodiments, the automated system is a robotic system. Preferably, the system includes independently moving channels, a multichannel head (for instance a 96-tip head) and a gripper or cherry-picking arm and a HEPA filtration device to maintain sterility during the procedure. The number of channels in the pipettor should be suitable for the format of the culture. Convenient pipettors have, e.g., 96 or 384 channels. Such systems are known and are commercially available. For example, a MICROLAB STAR™ instrument (Hamilton) may be used in the method of the invention. The automated system should be able to perform a variety of desired cell culture tasks. Such tasks will be known by a person of skill in the art. They include but are not limited to: removing media, replacing media, adding reagents, cell washing, removing wash solution, adding a dispersing agent, removing cells from a culture vessel, adding cells to a culture vessel an the like.

The production of a cell or cell line of the invention may include any number of separate cell cultures. However, the advantages provided by the method increase as the number of cells increases. There is no theoretical upper limit to the number of cells or separate cell cultures that can be utilized in the method. According to the invention, the number of separate cell cultures can be two or more but more advantageously is at least 3, 4, 5, 6, 7, 8, 9, 10 or more separate cell cultures, for example, at least 12, at least 15, at least 20, at least 24, at least 25, at least 30, at least 35, at least 40, at least 45, at least 48, at least 50, at least 75, at least 96, at least 100, at least 200, at least 300, at least 384, at least 400, at least 500, at least 1000, at least 10,000, at least 100,000, at least 500,000 or more.

The ease to isolate and re-isolate from a mixed cell population those cells with desired properties (e.g., expressing desired RNAs at appropriate levels) makes it possible to maintain cell lines under no or minimal drug selection pressure. Selection pressure is applied in cell culture to select cells with desired sequences or traits, and is usually achieved by linking the expression of a polypeptide of interest with the expression of a selection marker that imparts to the cells resistance to a corresponding selective agent or pressure. Antibiotic selection includes, without limitation, the use of antibiotics (e.g., puromycin, neomycin, G418, hygromycin, bleomycin). Non-antibiotic selection includes, without limitation, the use of nutrient deprivation, exposure to selective temperatures, exposure to mutagenic conditions and expression of fluorescent markers where the selection marker may be e.g., glutamine synthetase, dihydrofolate reductase (DHFR), oabain, thymidine kinase (TK), hypoxanthine guanine phosphororibosyltransferase (HGPRT) or a fluorescent protein such as GFP. In the instant aspects of the invention, none of such selection steps are applied to the cells in culture. In some preferred embodiments, cells and cell lines of the invention are maintained in culture without any selective pressure. In further embodiments, cells and cell lines are maintained without any antibiotics. As used herein, cell maintenance refers to culturing cells after they have been selected for their NaV expression through, *e.g.,* cell sorting. Maintenance does not refer to the optional step of growing cells in a selective drug *(e.g.,* an antibiotic) prior to cell sorting where drug resistance marker(s) introduced into the cells allow enrichment of stable transfectants in a mixed population.

Drug-free cell maintenance provides a number of advantages. For examples, drug-resistant cells do not always express the co-transfected transgene of interest at adequate levels, because the selection relies on survival of the cells that have taken up the drug resistant gene, with or without the transgene. Further, selective drugs are often mutagenic or otherwise interferes the physiology of the cells, leading to skewed results in cell-based assays. For example, selective drugs may decrease susceptibility to apoptosis (Robinson et al., Biochemistry, 36(37):1169-11178 (1997)), increase DNA repair and drug metabolism (Deffie et al., Cancer Res. 48(13):3595-3602 (1988)), increase cellular pH (Thiebaut et al., J Histochem Cytochem. 38(5):685-690 (1990); Roepe et al., Biochemistry. 32(41):1042-11056 (1993); Simon et al., Proc Natl Acad Sci USA. 91(3): 1128-1132 (1994)), decrease lysosomal and endosomal pH (Schindler et al., Biochemistry. 35(9):2811-2817 (1996); Altan et al., J Exp Med. 187(10):1583-1598 (1998)), decrease plasma membrane potential (Roepe et al., Biochemistry. 32(41):11042-11056 (1993)), increase plasma membrane conductance to chloride (Gill et al., Cell. 71(1):23-32 (1992)) and ATP (Abraham et al., Proc Natl Acad Sci USA. 90(1):312-316 (1993)), and increase rates of vesicle transport (Altan et al., Proc Natl Acad Sci USA. 96(8):4432-4437 (1999)). GFP, a commonly used non-antibiotic selective marker, may cause cell death in certain cell lines (Hanazono et al., Hum Gene Ther. 8(11): 1313-1319 (1997)). Thus, the cells and cell lines of this invention allow screening assays that are free from any artifact caused by selective drugs or markers. In some preferred embodiments, cells are not cultured with selective drugs such as antibiotics before or after cell sorting so that cells with desired properties are isolated by sorting even without beginning with an enriched cell population.

Also disclosed herein are methods of using the cells and cell lines of the invention. The cells and cell lines of the invention may be used in any application for which a functional NaV subunit(s) or complete NaV ion channel is needed. The cells and cell lines may be used, for example, in an *in vitro* cell-based assay or an *in vivo* assay where the cells are implanted in an animal *(e.g.,* a non-human mammal) to, *e.g.,* screen for NaV modulators; produce protein for crystallography and binding studies; and investigate compound selectivity and dosing, receptor/compound binding kinetic and stability, and effects of receptor expression on cellular physiology (*e.g*., electrophysiology, protein trafficking, protein folding, and protein regulation). The present cells and cell lines also can be used in knock down studies to study the roles of specific NaV subunits.

Cell lines expressing various combinations of alpha and beta subunits (*e.g*., naturally occurring heterotrimers or nonnaturally occurring heterotrimers) can be used separately or together as a collection to identify NaV modulators, including those specific for a particular NaV, a particular subunit of a NaV, or a particular combination of NaV subunits, and to obtain information about the activities of individual subunits. The invention also provides methods for using modulators specific for particular modified forms; such information may be useful in determining whether NaV has naturally occurring modified forms. Using the present cell and cell lines can help determine whether different forms of NaV are implicated in different NaV pathologies and allow selection of disease- or tissue-specific NaV modulators for highly targeted treatment of NaV-related pathologies.

As used herein, a "modulator" includes any substance or compound that has modulating activity with respect to at least one NaV subunit. The modulator can be a NaV agonist (potentiator or activator) or antagonist (inhibitor or blocker), including partial agonists or antagonists, selective agonists or antagonists and inverse agonists, and can be an allosteric modulator. A substance or compound is a modulator even if its modulating activity changes under different conditions or concentrations. In some aspects of the invention, the modulator alters the selectivity of an ion channel. For example, a modulator may affect what ions are able to pass through an ion channel.

To identify a NaV modulator, one can expose a cell line of the invention to a test compound under conditions in which the NaV would be expected to be functional and detect a statistically significant change (*e.g.,* $p < 0.05$) in NaV activity compared to a suitable control, *e.g.,* cells from the cell line that are not contacted with the test compound. Alternatively, or in addition positive and/or negative controls using known agonists or antagonists, cells expressing different combinations of NaV subunits may be used. In some embodiments, the NaV activity to be detected and/or measured is membrane depolarization, change in membrane potential, or fluorescence resulting from such membrane changes.

In some embodiments, one or more cell lines of the invention are exposed to a plurality of test compounds, for example, a library of test compounds. A library of test compounds can be screened using the cell lines of the invention to identify one or more modulators. The test compounds can be chemical moieties including small molecules, polypeptides, peptides, peptide mimetics, antibodies or antigen-binding portions thereof. In the case of antibodies, they may be non-human antibodies, chimeric antibodies, humanized antibodies, or fully human antibodies. The antibodies may be intact antibodies comprising a full complement of heavy and light chains or antigen-binding portions, including antibody fragments (such as Fab and Fab, Fab', $F(ab')_2$, Fd, Fv, dAb), single subunit antibodies (scFv), single domain antibodies, all or an antigen-binding portion of a heavy or light chain.

Also disclosed herein, in some embodiments, prior to exposure to a test compound, the cells may be modified by pretreatment with, for example, enzymes, including mammalian or other animal enzymes, plant enzymes, bacterial enzymes, protein modifying enzymes and lipid modifying enzymes, and enzymes in the oral cavity, gastrointestinal tract, stomach or saliva. Such enzymes can include, for example, kinases, proteases, phosphatases, glycosidases, oxidoreductases, transferases, hydrolases, lyases, isomerases, ligases. For example, in some embodiments, cells are pretreated with at least one proteolytic enzyme such as trypsin or furin. Alternatively, the cells may be exposed to the test compound first followed by treatment to identify compounds that alter the modification of the NaV by the treatment.

Also disclosed herein, large compound collections are tested for NaV-modulating activity in a cell-based, functional, high-throughput screen (HTS), e.g., using 96, 384, 1536, or higher density well format. Hits from the HTS screen may be subsequently tested in additional assays to confirm function, e.g., determination of their chemical structures, testing of structurally related compounds to optimize activity and specificity, and further testing in animal models. Also disclosed herein, the therapeutic potential of modulators is tested in animal models to assess their usefulness in the treatment of human diseases and conditions, including epilepsy, periodic paralysis, cardiac diseases, CNS diseases, ataxia, and pain (chronic or acute), loss of ability to feel pain. By way of example, a human NaV 1.7 expressing cell line of the invention can be used to identify a NaV 1.7 antagonist for use as an analgesic to reduce or eliminate pain.

These and other embodiments of the invention may be further illustrated in the following non-limiting Examples.

## EXAMPLES

### Example 1 Generating a Stable NaV 1.7 Heterotrimer-Expressing Cell Line

#### *Generating Expression Constructs*

Plasmid expression vectors that allowed streamlined cloning were generated based on pCMV-SCRIPT (Stratagene) and contained various necessary components for transcription and translation of a gene of interest, including: CMV and SV40 eukaryotic promoters; SV40 and HSV-TK polyadenylation sequences; multiple cloning sites; Kozak sequences; and Neomycin/Kanamycin resistance cassettes (or Ampicillin, Hygromycin, Puromycin, Zeocin resistance cassettes).

#### *Generation of Cell Lines*

293T cells were cotransfected with three separate plasmids, one encoding a human NaV 1.7 a subunit (SEQ ID NO: 13), one encoding a human NaV 1.7 β1 subunit (SEQ ID NO: 16) and one encoding a human NaV 1.7 β2 subunit (SEQ ID NO: 17), using standard techniques. (Examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE® 6, DOTAP/DOPE, Metafectine or FECTURIN™.)

Although drug selection is optional to produce the cells or cell lines of this invention, we included one drug resistance marker per plasmid. The sequences were under the control of the CMV promoter. An untranslated sequence encoding a Target Sequence for detection by a signaling probe was also present along with the sequence encoding the drug resistance marker. The Target Sequences utilized were Target Sequence 1 (SEQ ID NO: 1), Target Sequence 2 (SEQ ID NO: 2) and Target Sequence 3 (SEQ ID NO: 3). In this example, the NaV 1.7 α subunit gene-containing vector comprised Target Sequence 1 (SEQ ID NO: 1); the NaV 1.7 β1 subunit gene-containing vector comprised Target Sequence 2 (SEQ ID NO: 2); and the NaV 1.7 β2 subunit gene-containing vector comprised Target Sequence 3 (SEQ ID NO: 3).

Transfected cells were grown for 2 days in DMEM-FBS media, followed by 10 days in antibiotic-containing DMEM-FBS media. During the antibiotic containing period, antibiotics were added to the media as follows: puromycin (0.1 μg/ml), hygromycin (100 μg/ml), and zeocin (200 μg/ml).

Following enrichment on antibiotic, cells were passaged 6-18 times in the absence of antibiotic selection to allow time for expression that was not stable over the selected period of time to subside.

Cells were harvested and transfected with signaling probes (SEQ ID NOS: 4, 5, 34) using standard techniques. (Examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE® 6, DOTAP/DOPE, Metafectine or FECTURIN™.)

Signaling Probe 1(SEQ ID NO: 4) bound Target Sequence 1 (SEQ ID NO: 1); Signaling Probe 2 (SEQ ID NO: 5) bound Target Sequence 2 (SEQ ID NO: 2); and Signaling Probe 3 (SEQ ID NO: 34) bound Target Sequence 3 (SEQ ID NO: 3). The cells were then dissociated and collected for analysis and sorted using a fluorescence activated cell sorter.

### Target Sequences detected by signaling probes

The following tag sequences were used for the NaV 1.7 subunit transgenes.

**Target Sequence 1**
5'-GTTCTTAAGGCACAGGAACTGGGAC-3' (SEQ ID NO: 1) (NaV 1.7 α subunit)
**Target Sequence 2**
5'-GAAGTTAACCCTGTCGTTCTGCGAC-3' (SEQ ID NO: 2) (NaV 1.7 β1 subunit)
**Target Sequence 3**
5'-GTTCTATAGGGTCTGCTTGTCGCTC-3' (SEQ ID NO: 3) (NaV 1.7 β2 subunit)

**Signaling probes**

Supplied as 100 μM stocks.

**Signaling probe 1** - This probe binds target sequence 1.
**5' - Cy5** GCCAGTCCCAGTTCCTGTGCCTTAAGAACCTCGC **BHQ3 quench -3'** (SEQ ID NO: 4)
**Signaling probe 2** - This probe binds target sequence 2.
**5'- Cy5.5** CGAGTCGCAGAACGACAGGGTTAACTTCCTCGC **BHQ3 quench -3'** (SEQ ID NO: 5)
**Signaling probe 3** - This probe binds target sequence 3.
**5'- Fam** CGAGAGCGACAAGCAGACCCTATAGAACCTCGC **BHQ1 quench -3'** (SEQ ID NO: 34)

BHQ3 in Signaling probes 1 and 2 can be replaced by BHQ2 or gold particle. BHQ1 in Signaling probe 3 can be replaced by BHQ2, gold particle, or DABCYL.

In addition, a similar probe using a Quasar® Dye (BioSearch) with spectral properties similar to Cy5 was used in certain experiments. In some experiments, 5-MedC and 2-amino dA mixmer probes were used rather than DNA probes.

Standard analytical methods were used to gate cells fluorescing above background and to isolate cells falling within the defined gate directly into 96-well plates. Flow cytometric cell sorting was operated such that a single cell was deposited per well. After selection, the cells were expanded in media lacking drug. The following gating hierarchy was used:

coincidence gate → singlets gate → live gate → Sort gate in plot FAM vs. Cy5: 0.1 - 1.0% of live cells.

The above steps were repeated to obtain a greater number of cells. At least four independent rounds of the above steps were completed, and for each of these rounds, at least two internal cycles of cell passsaging and isolation were performed.

The plates were transferred to a Microlabstar automated liquid handler (Hamilton Robotics). Cells were incubated for 5-7 days in a 1:1 mix of fresh complete growth medium (DMEM/10% FBS) and 2-3 day conditioned growth medium, supplemented with 100 units/ml penicillin and 0.1mg/ml streptomycin. Then the cells were dispersed by trypsinization to minimize clumps and transferred to new 96-well plates. After the clones were dispersed, plates were imaged to determine confluency of wells (Genetix). Each plate was focused for reliable image acquisition across the plate. Reported confluencies of greater than 70% were not relied upon. Confluency measurements were obtained at days every 3 times over 9 days (*i.e,* between days 1 and 10 post-dispersal) and used to calculate growth rates.

Cells were binned (independently grouped and plated as a cohort) according to growth rate between 10-11 days following the dispersal step in step 7. Bins were independently collected and plated on individual 96 well plates for downstream handling; some growth bins resulted in more than one 96-well plate. Bins were calculated by considering the spread of growth rates and bracketing a high percentage of the total number of populations of cells. Depending on the sort iteration described in Step 5, between 5 and 9 growth bins were used with a partition of 1- 4 days. Therefore, each bin corresponded to a growth rate or population doubling time between 8 and 14.4 hours depending on the iteration.

Cells can have doubling times from less 1 day to more than 2 weeks. In order to process the most diverse clones that at the same time can be reasonably binned according to growth rate, it is preferable to use 3-9 bins with a 0.25 to 0.7 day doubling time per bin. One skilled in the art will appreciate that the tightness of the bins and number of bins can be adjusted for the particular situation and that the tightness and number of bins can be further adjusted if cells are synchronized for their cell cycle.

The plates were incubated under standard and fixed conditions (humidified 37°C, 5%CO2) in antibiotics-free DMEM-10%FBS media. The plates of cells were split to produce 4 sets of target plates. These 4 sets of plates comprised all plates with all growth bins to ensure there were 4 replicates of the initial set. Up to 3 target plate sets were committed for cryopreservation (described in step 10), and the remaining set was scaled and further replica plated for passage and functional assay experiments. Distinct and independent tissue culture reagents, incubators, personnel, and carbon dioxide sources were used for downstream replica plates. Quality control steps were taken to ensure the proper production and quality of all tissue culture reagents: each component added to each bottle of media prepared for use was added by one designated person in one designated hood with only that reagent in the hood while a second designated person monitored to avoid mistakes. Conditions for liquid handling were set to eliminate cross contamination across wells. Fresh tips were used for all steps, or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

Three sets of plates were frozen at -70 to -80°C. Plates in each set were first allowed to attain confluencies of 70 to 80%. Medium was aspirated and 90%FBS and 5%-10% DMSO was added. The plates were sealed with Parafilm, individually surrounded by 1 to 5cm of foam, and then placed into a -80°C freezer.

The remaining set of plates was maintained as described in step 9. All cell splitting was performed using automated liquid handling steps, including media removal, cell washing, trypsin addition and incubation, quenching and cell dispersal steps. For some assay plating steps, cells were dissociated with cell dissociation buffer (e.g., CDB, Invitrogen or CellStripper, CellGro) rather than trypsin.

The consistency and standardization of cell and culture conditions for all populations of cells was controlled. Differences across plates due to slight differences in growth rates were controlled by periodic normalization of cell numbers across plates every 2 to 8 passages. Populations of cells that were outliers were detected and eliminated.

The cells were maintained for 3 to 8 weeks to allow for their *in vitro* evolution under these conditions. During this time, we observed size, morphology, fragility, response to trypsinization or dissociation, roundness/average circularity post-dissociation, percentage viability, tendency towards microconfluency, or other aspects of cell maintenance such as adherence to culture plate surfaces.

Populations of cells were tested using functional criteria. Membrane potential assay kits (Molecular Devices/MDS) were used according to manufacturer's instructions. Cells were tested at multiple different densities in 96- or 384-well plates and responses were analyzed. A variety of post-plating time points were used, *e.g.,* 12-48 hours post plating. Different densities of plating were also tested for assay response differences.

The functional responses from experiments performed at low and higher passage numbers were compared to identify cells with the most consistent responses over defined periods of time, ranging from 3 to 9 weeks. Other characteristics of the cells that changed over time were also noted.

Populations of cells meeting functional and other criteria were further evaluated to determine those most amenable to production of viable, stable and functional cell lines. Selected populations of cells were expanded in larger tissue culture vessels and the characterization steps described above were continued or repeated under these conditions. At this point, additional standardization steps, such as different plating cell densities; time of passage; culture dish size/format and coating); fluidics optimization; cell dissociation optimization (*e.g.*, type, volume used, and length of time); and washing steps, were introduced for consistent and reliable passages. Temperature differences were also used for standardization *(i.e.,* 30°C vs 37°C).

In addition, viability of cells at each passage was determined. Manual intervention was increased and cells were more closely observed and monitored. This information was used to help identify and select final cell lines that retained the desired properties. Final cell lines and back-up cell lines were selected that showed consistent growth, appropriate adherence, and functional response.

The low passage frozen plates described above corresponding to the final cell line and back-up cell lines were thawed at 37°C, washed two times with DMEM-10% FBS and incubated in humidified 37°C /5% CO2 conditions. The cells were then expanded for a period of 2-3 weeks. Cell banks for each final and back-up cell line consisting of 15-20 vials were established.

The following step can also be conducted to confirm that the cell lines are viable, stable, and functional. At least one vial from the cell bank is thawed and expanded in culture. The resulting cells are tested to determine if they meet the same characteristics for which they were originally selected.

## <u>Example 2 Characterizing Relative Expression of Heterologous NaV 1.7 Subunits in Stable NaV 1.7-Expressing Cell Lines</u>

Quantitative RT-PCR (qRT-PCR) was used to determine the relative expression of the heterologous human NaV 1.7 $\alpha$, $\beta$1, and $\beta$2 subunits in the produced stable NaV 1.7-expressing cell lines. Total RNA was purified from 1-3x$10^6$ mammalian cells using an RNA extraction kit (RNeasy Mini Kit, Qiagen). DNase treatment was done according to rigorous DNase treatment protocol (TURBO DNA-free Kit, Ambion). First strand cDNA synthesis was performed using a reverse transcriptase kit (SuperScript III, Invitrogen) in 20 $\mu$L reaction volume with 1 $\mu$g DNA-free total RNA and 250 ng Random Primers (Invitrogen). Samples without reverse transcriptase and sample without RNA were used as negative controls for this reaction. Synthesis was done in a thermal cycler (Mastercycler, Eppendorf) at the following conditions: 5 min at 25°C, 60 min at 50°C; reaction termination was conducted for 15 min at 70°C.

For analysis of gene expression, primers and probes for qRT-PCR (MGB TaqMan probes, Applied Biosystems) were designed to specifically anneal to the target sequences (SEQ ID NOS: 1 - 3). For sample normalization, control (glyceraldehyde 3-phosphate dehydrogenase (GAPDH)) Pre-Developed Assay reagents (TaqMaN, Applied Biosystems) were used. Reactions, including negative controls and positive controls (plasmid DNA), were set up in triplicates with 40 ng of cDNA in 50 $\mu$L reaction volume. The relative amounts of each of the three NaV 1.7 subunits being expressed were determined. As shown in **FIG. 1**, all three subunits were successfully expressed in the produced stable NaV 1.7-expressing cell line.

**Example 3 Characterizing Stable NaV 1.7-Expressing Cell Lines for Native NaV Function using Electrophysiological Assay**

Automated patch-clamp system was used to record sodium currents from the produced stable HEK293T cell lines expressing NaV 1.7 α, β1, and β2 subunits. The following illustrated protocol can also be used for QPatch, Sophion or Patchliner, Nanion systems. The extracellular Ringer's solution contained 140 mM NaCl, 4.7 mM KCl, 2.6 mM $MgCl_2$, 11 mM glucose and 5 mM HEPES, pH 7.4 at room temperature. The intracellular Ringer's solution contained 120 mM CsF, 20 mM Cs-EGTA, 1 mM $CaCl_2$, 1 mM $MgCl_2$, and 10 mM HEPES, pH 7.2. Experiments were conducted at room temperature.

Cells stably expressing NaV 1.7 α, β1, and β2 subunits were grown under standard culturing protocols as described in **Example 1**. Cells were harvested and kept in suspension with continuous stirring for up to 4 hours with no significant change in quality or ability to patch. Electrophysiological experiment (whole-cell) was performed using the standard patch plate. The patch-clamp hole (micro-etched in the chip) is approximately 1 μm in diameter and has a resistance of ~2 MΩ. The membrane potential was clamped to a holding potential of -100 mV.

Current-voltage relation and inactivation characteristics of voltage-gated human NaV 1.7 sodium channel stably expressed in HEK293T cells are shown on **FIGS. 3A-C. FIG. 3A** shows sodium currents in response to 20 ms depolarization pulses from - 80 mV to +50 mV. The holding potential was -100 mV. **FIG.3B** shows the resulting current-voltage (I-V) relationship for peak sodium channel currents. The activation threshold was - 35 mV (midpoint of activation, Va = - 24.9 mV +/- 3.7 mV), and the maximal current amplitude was obtained at -10 mV. **FIG. 3C** shows the inactivation graph for the sodium channel. The membrane potential was held at a holding potential of - 100 mV, subsequently shifted to conditioning potentials ranging from -110 mV to +10 mV for 1000 ms, and finally the current was measured upon a step to 0 mV. The resulting current amplitude indicates the fraction of sodium channels in the inactivated state. At potentials more negative than - 85 mV the channels were predominantly in the closed state, whereas at potentials above -50 mV they were predominantly in the inactivated state. The curve represents the Boltzmann fit from which the $V_{1/2}$ for steady-state inactivation was estimated to be - 74 mV. The current-voltage profile for the produced stable NaV 1.7-expressing cell lines is consistent with previously reported current-voltage profile (Va= - 28.0 mV ±1.1 mV; $V_{1/2}$ = -71.3 mV ± 0.8 mV) (Sheets et al., J Physiol. 581(Pt 3):1019-1031. (2007)).

**Example 4 Characterizing Stable NaV 1.7-Expressing Cell Lines for Native NaV Function using Membrane Potential Assay**

The produced stable cells expressing NaV 1.7 a, β1, and β2 subunits were maintained under standard cell culture conditions in Dulbecco's Modified Eagles medium supplemented with 10% fetal bovine serum, glutamine and HEPES. On the day before assay, the cells were harvested from stock plates using cell dissociation buffer, e.g., CDB (GIBCO) or cell-stripper (Mediatech), and plated at 10, 000 - 25,000 cells per well in 384 well plates in growth media. The assay plates were maintained in a 37°C cell culture incubator under 5%CO2 for 22-24 hours. The media were then removed from the assay plates and blue fluorescence membrane potential dye (Molecular Devices Inc.) diluted in load buffer (137 mM NaCl, 5 mM KCl,1.25 mM $CaCl_2$, 25 mM HEPES, 10 mM glucose) was added. The cells were incubated with blue membrane potential dye for 1 hour at 37°C. The assay plates were then loaded onto the high-throughput fluorescent plate reader (Hamamastu FDSS). The fluorescent plate reader measures cell fluorescence in images taken of the cell plate once per second and displays the data as relative florescence units.

**FIG. 4** demonstrates the assay response of stable NaV 1.7-expressing cells and control cells *(i.e.,* HEK293T parental cells) to addition of buffer and channel activators *(i.e.,* veratridine and scorpion venom (SV)). In a first addition step (Addition 1 in **FIG. 4**), only buffer was added, with no test compounds added. If desired, test compounds can be added in this step. In a second addition step, veratridine and scorpion venom, which are sodium channels activators, were diluted in assay buffer to the desired concentration (*i.e.,* 25 μM veratridine and 5 - 25 μg/ml scorpion venom) and added into 384 well polypropylene microtiter plates. Once bound, veratridine and scorpion venom proteins modulate the activity of voltage-gated sodium channels through a combination of mechanisms, including an alteration of the activation and inactivation kinetics. The resulted activation of sodium channels in stable NaV 1.7-expressing cells changes cells membrane potential and the fluorescent signal increases. The above-described functional assay can also be used to characterize the relative potencies of test compounds at NaV 1.7 ion channels.

**Example 5 Characterizing Regulation of NaV 1.7 Alpha Subunit by Beta Subunits**

***Regulation of Alpha Subunit Gene Expression by Beta Subunits***

Pools of HEK293T cells were engineered to express various ratios of α and β subunits by manipulating the molar ratios of independent plasmid DNAs or α and control plasmids (*e.g.*, α:β1:β2 = 1:1:1). After drug selection the subunits expression in six different cell pools were evaluated with qRT-PCR as described in **Example 2**. Comparative qRT-PCR indicated that α subunit expression in drug-selected cells detection was increased when all three human NaV 1.7 subunits (*i.e.,* α, β1, and β2) were co-transfected (**FIG**. **2**, left panel) in compared to only α subunit and control plasmid transfected (**FIG**. **2**, right panel). The presence of the β subunit transcripts affects α subunit gene expression, demonstrating the importance of co-expressing all three NaV 1.7 subunits for a physiologically relevant functional assay.

***Regulation of Pharmacological Properties by Beta Subunits***

A membrane potential cell-based assay was used to measure the response to test compounds of the cells stably co-expressing all three NaV 1.7 subunits (*i.e.,* α, β1, and β2) and control cells stably expressing only a NaV 1.7 α subunit. Two compounds (**FIG. 5**) *(i.e.,* C18 and K21) were tested in the membrane potential assay performed substantially according to the protocol in **Example 4**. Specifically for this example, the test compounds were added in the first addition step.

As shown in **FIG. 5**, C18 and K21 potentiated the response of clone C44 (expressing NaV 1.7 α, β1, and β2 subunits, **FIG. 5A**) and blocked the response of clone C60 (expressing NaV 1.7 α subunit only, **FIG. 5B**). The assay response of the two test compounds was normalized to the response of buffer alone for each of the two clones.

**LISTING OF SEQUENCES**

Target sequence 1
5'-GTTCTTAAGGCACAGGAACTGGGAC-3' (SEQ ID NO: 1)

Target sequence 2
5'-GAAGTTAACCCTGTCGTTCTGCGAC-3' (SEQ ID NO: 2)

Target sequence 3
5'-GTTCTATAGGGTCTGCTTGTCGCTC-3' (SEQ ID NO: 3)

Signaling probe 1 (binds target 1)
**5**' - **Cy5** GCCAGTCCCAGTTCCTGTGCCTTAAGAACCTCGC **BHQ3 quench** -**3**' (SEQ ID NO: 4)

Signaling probe 2 - (binds target 2)
**5'- Cy5.5** GCGAGTCGCAGAACGACAGGGTTAACTTCCTCGC **BHQ3 quench** - **3'** (SEQ ID NO: 5)

Homo sapiens (H.s.) SCN1A

atggagcaaacagtgcttgtaccaccaggacctgacagcttcaacttcttcaccagagaatctcttgcggctattgaaagac
gcattgcagaagaaaaggcaaagaatcccaaaccagacaaaaaagatgacgacgaaaatggcccaaagccaaatagtg
acttggaagctggaaagaaccttccatttatttatggagacattcctccagagatggtgtcagagcccctggaggacctgga
cccctactatatcaataagaaaacttttatagtattgaataaagggaaggccatcttccggttcagtgccacctctgccctgtac
attttaactcccttcaatcctcttaggaaaatagctattaagattttggtacattcattattcagcatgctaattatgtgcactattttg
acaaactgtgtgtttatgacaatgagtaaccctcctgattggacaaagaatgtagaatacaccttcacaggaatatatacttttg
aatcacttataaaaattattgcaaggggattctgtttagaagattttactttccttcgggatccatggaactggctcgatttcactg
tcattacatttgcgtacgtcacagagtttgtggacctgggcaatgtctcggcattgagaacattcagagttctccgagcattga
agacgatttcagtcattccaggcctgaaaaccattgtgggagccctgatccagtctgtgaagaagctctcagatgtaatgatc
ctgactgtgttctgtctgagcgtatttgctctaattgggctgcagctgttcatgggcaacctgaggaataaatgtatacaatggc
ctcccaccaatgcttccttggaggaacatagtatagaaaagaatataactgtgaattataatggtacacttataaatgaaactgt
ctttgagtttgactggaagtcatatattcaagattcaagatatcattatttcctggagggtttttagatgcactactatgtggaaat
agctctgatgcaggccaatgtccagagggatatatgtgtgtgaaagctggtagaaatcccaattatggctacacaagctttga
taccttcagttgggctttttgtccttgtttcgactaatgactcaggacttctgggaaaatctttatcaactgacattacgtgctgct
gggaaaacgtacatgatatttttgtattggtcattttcttgggctcattctacctaataaatttgatcctggctgtggtggccatgg
cctacgaggaacagaatcaggccaccttggaagaagcagaacagaaagaggccgaatttcagcagatgattgaacagct
taaaaagcaacaggaggcagctcagcaggcagcaacggcaactgcctcagaacattccagagagcccagtgcagcag
gcaggctctcagacagctcatctgaagcctctaagttgagttccaagagtgctaaggaaagaagaaatcggaggaagaaa

agaaaacagaaagagcagtctggtggggaagagaaagatgaggatgaattccaaaaatctgaatctgaggacagcatca
ggaggaaaggttttcgcttctccattgaagggaaccgattgacatatgaaaagaggtactcctccccacaccagtctttgttg
agcatccgtggctccctattttcaccaaggcgaaatagcagaacaagccttttcagctttagagggcgagcaaaggatgtg
ggatctgagaacgacttcgcagatgatgagcacagcacctttgaggataacgagagccgtagagattccttgtttgtgcccc
gacgacacggagagagacgcaacagcaacctgagtcagaccagtaggtcatcccggatgctggcagtgtttccagcgaa
tgggaagatgcacagcactgtggattgcaatggtgtggtttccttggttggtggaccttcagttcctacatcgcctgttggaca
gcttctgccagagggaacaaccactgaaactgaaatgagaaagagaaggtcaagttctttccacgtttccatggactttctag
aagatccttcccaaaggcaacgagcaatgagtatagccagcattctaacaaatacagtagaagaacttgaagaatccaggc
agaaatgcccaccctgttggtataaattttccaacatattcttaatctgggactgttctccatattggttaaaagtgaaacatgttg
tcaacctggttgtgatggacccatttgttgacctggccatcaccatctgtattgtcttaaatactcttttcatggccatggagcact
atccaatgacggaccatttcaataatgtgcttacagtaggaaacttggttttcactgggatctttacagcagaaatgtttctgaa
aattattgccatggatccttactattatttccaagaaggctggaatatctttgacggttttattgtgacgcttagcctggtagaactt
ggactcgccaatgtggaaggattatctgttctccgttcatttcgattgctgcgagttttcaagttggcaaaatcttggccaacgtt
aaatatgctaataaagatcatcggcaattccgtgggggctctgggaaatttaaccctcgtcttggccatcatcgtcttcatttttg
ccgtggtcggcatgcagctctttggtaaaagctacaaagattgtgtctgcaagatcgccagtgattgtcaactcccacgctgg
cacatgaatgacttcttccactccttcctgattgtgttccgcgtgctgtgtggggagtggatagagaccatgtgggactgtatg
gaggttgctggtcaagccatgtgccttactgtcttcatgatggtcatggtgattggaaacctagtggtcctgaatctctttctgg
ccttgcttctgagctcatttagtgcagacaaccttgcagccactgatgatgataatgaaatgaataatctccaaattgctgtgga
taggatgcacaaaggagtagcttatgtgaaaagaaaaatatatgaatttattcaacagtccttcattaggaaacaaaagattta
gatgaaattaaaccacttgatgatctaaacaacaagaaagacagttgtatgtccaatcatacagcagaaattgggaaagatct
tgactatcttaaagatgtaaatggaactacaagtggtataggaactggcagcagtgttgaaaaatacattattgatgaaagtga
ttacatgtcattcataaacaaccccagtcttactgtgactgtaccaattgctgtaggagaatctgactttgaaaatttaaacacg
gaagactttagtagtgaatcggatctggaagaaagcaaagagaaactgaatgaaagcagtagctcatcagaaggtagcac
tgtggacatcggcgcacctgtagaagaacagcccgtagtggaacctgaagaaactcttgaaccagaagcttgtttcactga
aggctgtgtacaaagattcaagtgttgtcaaatcaatgtggaagaaggcagaggaaaacaatggtggaacctgagaagga
cgtgtttccgaatagttgaacataactggtttgagaccttcattgttttcatgattctccttagtagtggtgctctggcatttgaaga
tatatatattgatcagcgaaagacgattaagacgatgttggaatatgctgacaaggttttcacttacattttcattctggaaatgct
tctaaaatgggtggcatatggctatcaaacatatttcaccaatgcctggtgttggctggacttcttaattgttgatgtttcattggt
cagtttaacagcaaatgccttgggttactcagaacttggagccatcaaatctctcaggacactaagagctctgagacctctaa
gagccttatctcgatttgaagggatgagggtggttgtgaatgcccttttaggagcaattccatccatcatgaatgtgcttctggt
ttgtcttatattctggctaattttcagcatcatgggcgtaaatttgtttgctggcaaattctaccactgtattaacaccacaactggt
gacaggtttgacatcgaagacgtgaataatcatactgattgcctaaaactaatagaaagaaatgagactgctcgatggaaaa
atgtgaaagtaaactttgataatgtaggatttgggtatctctctttgcttcaagttgccacattcaaaggatggatggatataatgt
atgcagcagttgattccagaaatgtggaactccagcctaagtatgaagaaagtctgtacatgtatctttactttgttattttcatca
tctttgggtccttcttcaccttgaacctgtttattggtgtcatcatagataatttcaaccagcagaaaaagaagtttggaggtcaa
gacatctttatgacagaagaacagaagaaatactataatgcaatgaaaaaattaggatcgaaaaaaccgcaaaagcctata
cctcgaccaggaaacaaatttcaaggaatggtctttgacttcgtaaccagacaagtttttgacataagcatcatgattctcatct
gtcttaacatggtcacaatgatggtggaaacagatgaccagagtgaatatgtgactaccattttgtcacgcatcaatctggtgt
tcattgtgctatttactggagagtgtgtactgaaactcatctctctacgccattattattttaccattggatggaatatttttgattttg
tggttgtcattctctccattgtaggtatgtttcttgccgagctgatagaaaagtatttcgtgtcccctaccctgttccgagtgatcc
gtcttgctaggattggccgaatcctacgtctgatcaaaggagcaaaggggatccgcacgctgctctttgctttgatgatgtcc
cttcctgcgttgtttaacatcggcctcctactcttcctagtcatgttcatctacgccatctttgggatgtccaactttgcctatgtta
agagggaagttgggatcgatgacatgttcaactttgagacctttggcaacagcatgatctgcctattccaaattacaacctctg
ctggctgggatggattgctagcacccattctcaacagtaagccacccgactgtgaccctaataaagttaaccctggaagctc
agttaagggagactgtgggaacccatctgttggaattttctttttgtcagttacatcatcatatccttcctggttgtggtgaacat
gtacatcgcggtcatcctggagaacttcagtgttgctactgaagaaagtgcagagcctctgagtgaggatgactttgagatgt
tctatgaggtttgggagaagtttgatcccgatgcaactcagttcatggaatttgaaaaattatctcagtttgcagctgcgcttga
accgcctctcaatctgccacaaccaaacaaactccagctcattgccatggatttgcccatggtgagtggtgaccggatccac
tgtcttgatatcttatttgcttttacaaagcgggttctaggagagagtggagagatggatgctctacgaatacagatggaagag
cgattcatggcttccaatccttccaaggtctcctatcagccaatcactactactttaaaacgaaaacaagaggaagtatctgct

gtcattattcagcgtgcttacagacgccaccttttaaagcgaactgtaaaacaagcttcctttacgtacaataaaaacaaaatc
aaaggtggggctaatcttcttataaaagaagacatgataattgacagaataaatgaaaactctattacagaaaaaactgatct
gaccatgtccactgcagcttgtccaccttcctatgaccgggtgacaaagccaattgtggaaaaacatgagcaagaaggcaa
agatgaaaaagccaaagggaaataa (SEQ ID NO: 6)

H.s. SCN2A

atggcacagtcagtgctggtaccgccaggacctgacagcttccgcttctttaccagggaatcccttgctgctattgaacaac
gcattgcagaagagaaagctaagagacccaaacaggaacgcaaggatgaggatgatgaaaatggcccaaagccaaac
agtgacttggaagcaggaaaatctcttccatttatttatggagacattcctccagagatggtgtcagtgcccctggaggatctg
gacccctactatatcaataagaaaacgtttatagtattgaataaagggaaagcaatctctcgattcagtgccacccctgccctt
tacattttaactcccttcaaccctattagaaaattagctattaagattttggtacattctttattcaatatgctcattatgtgcacgatt
cttaccaactgtgtatttatgaccatgagtaaccctccagactggacaaagaatgtggagtatacctttacaggaatttatacttt
tgaatcacttattaaaatacttgcaaggggcttttgtttagaagatttcacatttttacgggatccatggaattggttggatttcaca
gtcattacttttgcatatgtgacagagtttgtggacctgggcaatgtctcagcgttgagaacattcagagttctccgagcattga
aaacaatttcagtcattccaggcctgaagaccattgtgggggccctgatccagtcagtgaagaagctttctgatgtcatgatct
tgactgtgttctgtctaagcgtgtttgcgctaataggattgcagttgttcatgggcaacctacgaaataaatgtttgcaatggcct
ccagataattcttcctttgaaataaatatcacttccttctttaacaattcattggatgggaatggtactactttcaataggacagtg
agcatatttaactgggatgaatatattgaggataaaagtcacttttatttttttagaggggcaaaatgatgctctgctttgtggcaa
cagctcagatgcaggccagtgtcctgaaggatacatctgtgtgaaggctggtagaaaccccaactatggctacacgagctt
tgacacctttagttgggcctttttgtccttatttcgtctcatgactcaagacttctgggaaaacctttatcaactgacactacgtgct
gctgggaaaacgtacatgatatttttgtgctggtcattttcttgggctcattctatctaataaatttgatcttggctgtggtggcca
tggcctatgaggaacagaatcaggccacattggaagaggctgaacagaaggaagctgaatttcagcagatgctcgaaca
gttgaaaaagcaacaagaagaagctcaggcggcagctgcagccgcatctgctgaatcaagagacttcagtggtgctggtg
ggataggagtttttcagagagttcttcagtagcatctaagttgagctccaaaagtgaaaaagagctgaaaaacagaagaaa
gaaaaagaaacagaaagaacagtctggagaagaagagaaaaatgacagagtccgaaaatcggaatctgaagacagcat
aagaagaaaaggtttccgtttttccttggaaggaagtaggctgacatatgaaaagagattttcttctccacaccagtccttactg
agcatccgtggctccctttctctccaagacgcaacagtagggcgagccttttcagcttcagaggtcgagcaaaggacattg
gctctgagaatgactttgctgatgatgagcacagcacctttgaggacaatgacagccgaagagactctctgttcgtgccgca
cagacatggagaacggcgccacagcaatgtcagccaggccagccgtgcctccagggtgctccccatcctgcccatgaat
gggaagatgcatagcgctgtggactgcaatggtgtggtctccctggtcgggggcccttctaccctcacatctgctgggcag
ctcctaccagagggcacaactactgaaacagaaataagaaagagacggtccagttcttatcatgtttccatggatttattgga
agatcctacatcaaggcaaagagcaatgagtatagccagtattttgaccaacaccatggaagaacttgaagaatccagaca
gaaatgcccaccatgctggtataaatttgctaatatgtgtttgatttgggactgttgtaaaccatggttaaaggtgaaacacctt
gtcaacctggttgtaatggacccatttgttgacctggccatcaccatctgcattgtcttaaatacactcttcatggctatggagc
actatcccatgacggagcagttcagcagtgtactgtctgttggaaacctggtcttcacagggatcttcacagcagaaatgtttc
tcaagataattgccatggatccatattattactttcaagaaggctggaatatttttgatggttttattgtgagccttagtttaatgga
acttggtttggcaaatgtggaaggattgtcagttctccgatcattccggctgctccgagttttcaagttggcaaaatcttggcca
actctaaatatgctaattaagatcattggcaattctgtgggggctctaggaaacctcaccttggtattggccatcatcgtcttcat
ttttgctgtggtcggcatgcagctctttggtaagagctacaaagaatgtgtctgcaagatttccaatgattgtgaactcccacgc
tggcacatgcatgactttttccactccttcctgatcgtgttccgcgtgctgtgtggagagtggatagagaccatgtgggactgt
atggaggtcgctggccaaaccatgtgccttactgtcttcatgatggtcatggtgattggaaatctagtggttctgaacctcttctt
ggccttgcttttgagttccttcagttctgacaatcttgctgccactgatgatgataacgaaatgaataatctccagattgctgtgg
gaaggatgcagaaaggaatcgattttgttaaaagaaaaatacgtgaatttattcagaaagcctttgttaggaagcagaaagct
ttagatgaaattaaaccgcttgaagatctaaataataaaaagacagctgtatttccaaccataccaccatagaaataggcaa
agacctcaattatctcaaagacggaaatggaactactagtggcataggcagcagtgtagaaaaatatgtcgtggatgaaagt
gattacatgtcatttataaacaaccctagcctcactgtgacagtaccaattgctgttggagaatctgactttgaaaatttaaatac
tgaagaattcagcagcgagtcagatatggaggaaagcaaagagaagctaaatgcaactagttcatctgaaggcagcacg
gttgatattggagctcccgccgagggagaacagcctgaggttgaacctgaggaatcccttgaacctgaagcctgttttacag

aagactgtgtacggaagttcaagtgttgtcagataagcatagaagaaggcaaagggaaactctggtggaatttgaggaaaa
catgctataagatagtggagcacaattggttcgaaaccttcattgtcttcatgattctgctgagcagtggggctctggcctttga
agatatatacattgagcagcgaaaaaccattaagaccatgttagaatatgctgacaaggttttcacttacatattcattctggaa
atgctgctaaagtgggttgcatatggttttcaagtgtattttaccaatgcctggtgctggctagacttcctgattgttgatgtctca
ctggttagcttaactgcaaatgccttgggttactcagaacttggtgccatcaaatccctcagaacactaagagctctgaggcc
actgagagctttgtcccggtttgaaggaatgagggttgttgtaaatgctcttttaggagccattccatctatcatgaatgtacttc
tggtttgtctgatcttttggctaatattcagtatcatgggagtgaatctctttgctggcaagttttaccattgtattaattacaccact
ggagagatgtttgatgtaagcgtggtcaacaactacagtgagtgcaaagctctcattgagagcaatcaaactgccaggtgg
aaaaatgtgaaagtaaactttgataacgtaggacttggatatctgtctctacttcaagtagccacgtttaagggatggatggat
attatgtatgcagctgttgattcacgaaatgtagaattacaacccaagtatgaagacaacctgtacatgtatctttattttgtcatc
tttattatttttggttcattctttaccttgaatcttttcattggtgtcatcatagataacttcaaccaacagaaaaagaagtttggaggt
caagacatttttatgacagaagaacagaagaaatactacaatgcaatgaaaaaactgggttcaaagaaaccacaaaaaccc
atacctcgacctgctaacaaattccaaggaatggtctttgattttgtaaccaaacaagtctttgatatcagcatcatgatcctcat
ctgccttaacatggtcaccatgatggtggaaaccgatgaccagagtcaagaaatgacaaacattctgtactggattaatctgg
tgtttattgttctgttcactggagaatgtgtgctgaaactgatctctcttcgttactactatttcactattggatggaatatttttgattt
tgtggtggtcattctctccattgtaggaatgtttctggctgaactgatagaaaagtattttgtgtcccctaccctgttccgagtgat
ccgtcttgccaggattggccgaatcctacgtctgatcaaaggagcaaaggggatccgcacgctgctctttgctttgatgatgt
cccttcctgcgttgtttaacatcggcctccttcttttcctggtcatgttcatctacgccatctttgggatgtccaattttgcctatgtt
aagagggaagttgggatcgatgacatgttcaactttgagacctttggcaacagcatgatctgcctgttccaaattacaacctct
gctggctgggatggattgctagcacctattcttaatagtggacctccagactgtgaccctgacaaagatcaccctggaagct
cagttaaaggagactgtgggaacccatctgttgggattttctttttgtcagttacatcatcatatccttcctggttgtggtgaaca
tgtacatcgcggtcatcctggagaacttcagtgttgctactgaagaaagtgcagagcctctgagtgaggatgactttgagatg
ttctatgaggtttgggagaagtttgatcccgatgcgacccagtttatagagtttgccaaactttctgattttgcagatgccctgga
tcctcctcttctcatagcaaaacccaacaaagtccagctcattgccatggatctgcccatggtgagtggtgaccggatccact
gtcttgacatcttatttgcttttacaaagcgtgttttgggtgagagtggagagatggatgcccttcgaatacagatggaagagc
gattcatggcatcaaacccctccaaagtctcttatgagcccattacgaccacgttgaaacgcaaacaagaggaggtgtctgc
tattattatccagagggcttacagacgctacctcttgaagcaaaaagttaaaaaggtatcaagtatatacaagaaagacaaag
gcaaagaatgtgatggaacacccatcaaagaagatactctcattgataaactgaatgagaattcaactccagagaaaaccg
atatgacgccttccaccacgtctccaccctcgtatgatagtgtgaccaaaccagaaaaagaaaaatttgaaaaagacaaatc
agaaaaggaagacaaagggaaagatatcagggaaagtaaaaagtaa (SEQ ID NO: 7)

H.s. SCN3A

atggcacaggcactgttggtacccccaggacctgaaagcttccgcctttttactagagaatctcttgctgctatcgaaaaacg
tgctgcagaagagaaagccaagaagcccaaaaaggaacaagataatgatgatgagaacaaaccaaagccaaatagtga
cttggaagctggaaagaaccttccatttatttatggagacattcctccagagatggtgtcagagcccctggaggacctggatc
cctactatatcaataagaaaacttttatagtaatgaataaaggaaaggcaattttccgattcagtgccacctctgccttgtatattt
taactccactaaaccctgttaggaaaattgctatcaagattttggtacattctttattcagcatgcttatcatgtgcactattttgac
caactgtgtatttatgaccttgagcaaccctcctgactggacaaagaatgtagagtacacattcactggaatctatacctttga
gtcacttataaaaatcttggcaagagggttttgcttagaagattttacgtttcttcgtgatccatggaactggctggatttcagtgt
cattgtgatggcatatgtgacagagtttgtggacctgggcaatgtctcagcgttgagaacattcagagttctccgagcactga
aaacaatttcagtcattccaggtttaaagaccattgtgggggccctgatccagtcggtaaagaagctttctgatgtgatgatcc
tgactgtgttctgtctgagcgtgtttgctctcattgggctgcagctgttcatgggcaatctgaggaataaatgtttgcagtggcc
cccaagcgattctgcttttgaaaccaacaccacttcctactttaatggcacaatggattcaaatgggacatttgttaatgtaaca
atgagcacatttaactggaaggattacattggagatgacagtcacttttatgttttggatgggcaaaaagaccctttactctgtg
gaaatggctcagatgcaggccagtgtccagaaggatacatctgtgtgaaggctggtcgaaaccccaactatggctacaca
agctttgacacctttagctgggctttcctgtctctatttcgactcatgactcaagactactgggaaaatctttaccagttgacatta
cgtgctgctgggaaaacatacatgatatttttgtcctggtcattttcttgggctcattttatttggtgaatttgatcctggctgtggt
ggccatggcctatgaggagcagaatcaggccaccttggaagaagcagaacaaaaagaggccgaatttcagcagatgctc

gaacagcttaaaaagcaacaggaagaagctcaggcagttgcggcagcatcagctgcttcaagagatttcagtggaatagg
tgggttaggagagctgttggaaagttcttcagaagcatcaaagttgagttccaaaagtgctaaagaatggaggaaccgaag
gaagaaaagaagacagagagagcaccttgaaggaaacaacaaaggagagagagacagctttcccaaatccgaatctga
agacagcgtcaaaagaagcagcttccttttctccatggatggaaacagactgaccagtgacaaaaaattctgctcccctcat
cagtctctcttgagtatccgtggctccctgttttccccaagacgcaatagcaaaacaagcattttcagtttcagaggtcgggca
aaggatgttggatctgaaaatgactttgctgatgatgaacacagcacatttgaagacagcgaaagcaggagagactcactg
tttgtgccgcacagacatggagagcgacgcaacagtaacgttagtcaggccagtatgtcatccaggatggtgccagggctt
ccagcaaatgggaagatgcacagcactgtggattgcaatggtgtggtttccttggtgggtggaccttcagctctaacgtcac
ctactggacaacttcccccagagggcaccaccacagaaacggaagtcagaaagagaaggttaagctcttaccagatttca
atggagatgctggaggattcctctggaaggcaaagagccgtgagcatagccagcattctgaccaacacaatggaagaact
tgaagaatctagacagaaatgtccgccatgctggtatagatttgccaatgtgttcttgatctgggactgctgtgatgcatggtta
aaagtaaaacatcttgtgaatttaattgttatggatccatttgttgatcttgccatcactatttgcattgtcttaaataccctctttatg
gccatggagcactaccccatgactgagcaattcagtagtgtgttgactgtaggaaacctggtctttactgggattttcacagc
agaaatggttctcaagatcattgccatggatccttattactatttccaagaaggctggaatatctttgatggaattattgtcagcct
cagtttaatggagcttggtctgtcaaatgtggagggattgtctgtactgcgatcattcagactgcttagagttttcaagttggca
aaatcctggcccacactaaatatgctaattaagatcattggcaattctgtgggggctctaggaaacctcaccttggtgttggcc
atcatcgtcttcatttttgctgtggtcggcatgcagctctttggtaagagctacaaagaatgtgtctgcaagatcaatgatgact
gtacgctcccacggtggcacatgaacgacttcttccactccttcctgattgtgttccgcgtgctgtgtggagagtggatagag
accatgtgggactgtatggaggtcgctggccaaaccatgtgccttattgttttcatgttggtcatggtcattggaaaccttgtgg
ttctgaacctctttctggccttattgttgagttcatttagctcagacaaccttgctgctactgatgatgacaatgaaatgaataatct
gcagattgcagtaggaagaatgcaaaagggaattgattatgtgaaaaataagatgcgggagtgtttccaaaaagccttttta
gaaagccaaaagttatagaaatccatgaaggcaataagatagacagctgcatgtccaataatactggaattgaaataagca
aagagcttaattatcttagagatgggaatggaaccaccagtggtgtaggtactggaagcagtgttgaaaaatacgtaatcga
tgaaaatgattatatgtcattcataaacaaccccagcctcaccgtcacagtgccaattgctgttggagagtctgactttgaaaa
cttaaatactgaagagttcagcagtgagtcagaactagaagaaagcaaagagaaattaaatgcaaccagctcatctgaagg
aagcacagttgatgttgttctaccccgagaaggtgaacaagctgaaactgaacccgaagaagaccttaaaccggaagcttg
ttttactgaaggatgtattaaaaagtttccattctgtcaagtaagtacagaagaaggcaaagggaagatctggtggaatcttcg
aaaaacctgctacagtattgttgagcacaactggtttgagactttcattgtgttcatgatccttctcagtagtggtgcattggcctt
tgaagatatatacattgaacagcgaaagactatcaaaaccatgctagaatatgctgacaaagtctttacctatatattcattctg
gaaatgcttctcaaatgggttgcttatggatttcaaacatatttcactaatgcctggtgctggctagatttcttgatcgttgatgttt
ctttggttagcctggtagccaatgctcttggctactcagaactcggtgccatcaaatcattacggacattaagagctttaagac
ctctaagagccttatcccggtttgaaggcatgagggtggttgtgaatgctcttgttggagcaattccctctatcatgaatgtgct
gttggtctgtctcatcttctggttgatctttagcatcatgggtgtgaatttgtttgctggcaagttctaccactgtgttaacatgaca
acgggtaacatgtttgacattagtgatgttaacaatttgagtgactgtcaggctcttggcaagcaagctcggtggaaaaacgt
gaaagtaaactttgataatgttggcgctggctatcttgcactgcttcaagtggccacatttaaaggctggatggatattatgtat
gcagctgttgattcacgagatgttaaacttcagcctgtatatgaagaaaatctgtacatgtatttatactttgtcatctttatcatctt
tgggtcattcttcactctgaatctattcattggtgtcatcatagataacttcaaccagcagaaaaagaagtttggaggtcaagac
atctttatgacagaggaacagaaaaaatattacaatgcaatgaagaaacttggatccaagaaacctcagaaacccatacctc
gcccagcaaacaaattccaaggaatggtctttgattttgtaaccagacaagtctttgatatcagcatcatgatcctcatctgcct
caacatggtcaccatgatggtggaaacggatgaccagggcaaatacatgaccctagttttgtcccggatcaacctagtgttc
attgttctgttcactggagaatttgtgctgaagctcgtctccctcagacactactacttcactataggctggaacatctttgacttt
gtggtggtgattctctccattgtaggtatgtttctggctgagatgatagaaaagtattttgtgtcccctaccttgttccgagtgatc
cgtcttgccaggattggccgaatcctacgtctgatcaaaggagcaaaggggatccgcacgctgctctttgctttgatgatgtc
ccttcctgcgttgtttaacatcggcctcctgctcttcctggtcatgtttatctatgccatctttgggatgtccaactttgcctatgtta
aaaaggaagctggaattgatgacatgttcaactttgagacctttggcaacagcatgatctgcttgttccaaattacaacctctg
ctggctgggatggattgctagcacctattcttaatagtgcaccacccgactgtgaccctgacacaattcaccctggcagctca
gttaagggagactgtgggaacccatctgttgggattttctttttgtcagttacatcatcatatccttcctggttgtggtgaacatg
tacatcgcggtcatcctggagaacttcagtgttgctactgaagaaagtgcagagcccctgagtgaggatgactttgagatgtt
ctatgaggtttgggaaaagtttgatcccgatgcgacccagtttatagagttctctaaactctctgattttgcagctgccctggat
cctcctcttctcatagcaaaacccaacaaagtccagcttattgccatggatctgcccatggtcagtggtgaccggatccactg

tcttgatattttatttgcctttacaaagcgtgttttgggtgagagtggagagatggatgcccttcgaatacagatggaagacag
gtttatggcatcaaacccctccaaagtctcttatgagcctattacaaccactttgaaacgtaaacaagaggaggtgtctgccg
ctatcattcagcgtaatttcagatgttatcttttaaagcaaaggttaaaaaatatatcaagtaactataacaaagaggcaattaaa
gggaggattgacttacctataaaacaagacatgattattgacaaactaaatgggaactccactccagaaaaaacagatggg
agttcctctaccacctctcctccttcctatgatagtgtaacaaaaccagacaaggaaaagtttgagaaagacaaaccagaaa
aagaaagcaaaggaaaagaggtcagagaaaatcaaaagtaa (SEQ ID NO: 8)

H.s. SCN4A

atggccagaccatctctgtgcaccctggtgcctctgggccctgagtgcttgcgccccttcacccgggagtcactggcagcc
atagaacagcgggcggtggaggaggaggcccggctgcagcggaataagcagatggagattgaggagcccgaacgga
agccacgaagtgacttggaggctggcaagaacctacccatgatctacggagacccccccgccggaggtcatcggcatccc
cctggaggacctggatccctactacagcaataagaagaccttcatcgtactcaacaagggcaaggccatcttccgcttctcc
gccacacctgctctctacctgctgagccccttcagcgtagtcaggcgcggggccatcaaggtgctcatccatgcgctgttca
gcatgttcatcatgatcaccatcttgaccaactgcgtattcatgaccatgagtgacccgcctccctggtccaagaatgtggag
tacaccttcacagggatctacacctttgagtccctcatcaagatactggcccgaggcttctgtgtcgacgacttcacattcctc
cgggacccctggaactggctggacttcagtgtcatcatgatggcgtacctgacagagtttgtggacttgggcaacatctcag
ccctgaggaccttccgggtgctgcgggccctcaaaaccatcacggtcatcccagggctgaagacgatcgtgggggccct
gatccagtcggtgaaaaagctgtcggatgtgatgatcctcactgtcttctgcctgagcgtctttgcgctggtaggactgcagc
tcttcatgggaaacctgaggcagaagtgtgtgcgctggcccccgccgttcaacgacaccaacaccacgtggtacagcaat
gacacgtggtacggcaatgacacatggtatggcaatgagatgtggtacggcaatgactcatggtatgccaacgacacgtg
gaacagccatgcaagctgggccaccaacgatacctttgattgggacgcctacatcagtgatgaagggaacttctacttcctg
gagggctccaacgatgccctgctctgtgggaacagcagtgatgctgggcactgccctgagggttatgagtgcatcaagac
cgggcggaaccccaactatggctacaccagctatgacaccttcagctgggccttcttggctctcttccgcctcatgacacag
gactattgggagaacctcttccagctgacccttcgagcagctggcaagacctacatgatcttcttcgtggtcatcatcttcctg
ggctctttctacctcatcaatctgatcctggccgtggtggccatggcatatgccgagcagaatgaggccaccctggccgag
gataaggagaaagaggaggagtttcagcagatgcttgagaagttcaaaaagcaccaggaggagctggagaaggccaag
gccgcccaagctctggaaggtggggaggcagatggggacccagcccatggcaaagactgcaatggcagcctggacac
atcgcaaggggagaagggagccccgaggcagagcagcagcggagacagcggcatctccgacgccatggaagaactg
gaagaggcccaccaaaagtgcccaccatggtggtacaagtgcgcccacaaagtgctcatatggaactgctgcgccccgt
ggctgaagttcaagaacatcatccacctgatcgtcatggacccgttcgtggacctgggcatcaccatctgcatcgtgctcaa
cacccctcttcatggccatggaacattaccccatgacggagcactttgacaacgtgctcactgtgggcaacctggtcttcaca
ggcatcttcacagcagagatggttctgaagctgattgccatggacccctacgagtatttccagcagggttggaatatcttcga
cagcatcatcgtcaccctcagcctggtagagctaggcctggccaacgtacagggactgtctgtgctacgctccttccgtctg
ctgcgggtcttcaagctggccaagtcgtggccaacgctgaacatgctcatcaagatcattggcaattcagtgggggcgctg
ggtaacctgacgctggtgctggctatcatcgtgttcatcttcgccgtggtgggcatgcagctgtttggcaagagctacaagg
agtgcgtgtgcaagattgccttggactgcaacctgccgcgctggcacatgcatgatttcttccactccttcctcatcgtcttcc
gcatcctgtgcggggagtggatcgagaccatgtgggactgcatggaggtggccggccaagccatgtgcctcaccgtcttc
ctcatggtcatggtcatcggcaatcttgtggtcctgaacctgttcctggctctgctgctgagctccttcagcgccgacagtctg
gcagcctcggatgaggatggcgagatgaacaacctgcagattgccatcgggcgcatcaagttgggcatcggctttgccaa
ggccttcctcctggggctgctgcatggcaagatcctgagccccaaggacatcatgctcagcctcggggaggctgacggg
gccggggaggctggagaggcgggggagactgcccccgaggatgagaagaaggagccgcccgaggaggacctgaa
gaaggacaatcacatcctgaaccacatgggcctggctgacggcccccccatccagcctcgagctggaccaccttaacttcat
caacaacccctacctgaccatacaggtgcccatcgcctccgaggagtccgacctggagatgcccaccgaggaggaaac
cgacactttctcagagcctgaggatagcaagaagccgccgcagcctctctatgatgggaactcgtccgtctgcagcacag
ctgactacaagcccccgaggaggaccctgaggagcaggcagaggagaaccccgagggggagcagcctgaggagtg
cttcactgaggcctgcgtgcagcgctggccctgcctctacgtggacatctcccagggccgtgggaagaagtggtggactc
tgcgcagggcctgcttcaagattgtcgagcacaactggttcgagaccttcattgtcttcatgatcctgctcagcagtggggct
ctggccttcgaggacatctacattgagcagcggcgagtcattcgcaccatcctagaatatgccgacaaggtcttcacctaca

tcttcatcatggagatgctgctcaaatgggtggcctacggctttaaggtgtacttcaccaacgcctggtgctggctcgacttcc
tcatcgtggatgtctccatcatcagcttggtggccaactggctgggctactcggagctgggacccatcaaatccctgcggac
actgcgggccctgcgtcccctgagggcactgtcccgattcgagggcatgagggtggtggtgaacgccctcctaggcgcc
atcccctccatcatgaatgtgctgcttgtctgcctcatcttctggctgatcttcagcatcatgggtgtcaacctgtttgccggcaa
gttctactactgcatcaacaccaccacctctgagaggttcgacatctccgaggtcaacaacaagtctgagtgcgagagcctc
atgcacacaggccaggtccgctggctcaatgtcaaggtcaactacgacaacgtgggtctgggctacctctccctcctgcag
gtggccaccttcaagggttggatggacatcatgtatgcagccgtggactcccgggagaaggaggagcagccgcagtacg
aggtgaacctctacatgtacctctactttgtcatcttcatcatctttggctccttcttcaccctcaacctcttcattggcgtcatcatt
gacaacttcaaccagcagaagaagaagttagggggaaagacatctttatgacggaggaacagaagaaatactataacg
ccatgaagaagcttggctccaagaagcctcagaagccaattccccggccccagaacaagatccagggcatggtgtatga
cctcgtgacgaagcaggccttcgacatcaccatcatgatcctcatctgcctcaacatggtcaccatgatggtggagacagac
aaccagagccagctcaaggtggacatcctgtacaacatcaacatgatcttcatcatcatcttcacaggggagtgcgtgctca
agatgctcgccctgcgccagtactacttcaccgttggctggaacatctttgacttcgtggtcgtcatcctgtccattgtgggcct
tgccctctctgacctgatccagaagtacttcgtgtcacccacgctgttccgtgtgatccgcctggcgcggattgggcgtgtcc
tgcggctgatccgcggggccaagggcatccggacgctgctgttcgccctcatgatgtcgctgcctgccctcttcaacatcg
gcctcctcctcttcctggtcatgttcatctactccatcttcggcatgtccaactttgcctacgtcaagaaggagtcgggcatcga
tgatatgttcaacttcgagaccttcggcaacagcatcatctgcctgttcgagatcaccacgtcggccggctgggacgggctc
ctcaaccccatcctcaacagcgggcccccagactgtgaccccaacctggagaacccgggcaccagtgtcaagggtgact
gcggcaacccctccatcggcatctgcttcttctgcagctatatcatcatctccttcctcatcgtggtcaacatgtacatcgccat
catcctggagaacttcaatgtggccacagaggagagcagcgagccccttggtgaagatgactttgagatgttctacgagac
atgggagaagttcgaccccgacgccacccagttcatcgcctacagccgcctctcagacttcgtggacaccctgcaggaac
cgctgaggattgccaagcccaacaagatcaagctcatcacactggacttgcccatggtgccaggggacaagatccactgc
ctggacatcctctttgccctgaccaaagaggtcctgggtgactctggggaaatggacgccctcaagcagaccatggagga
gaagttcatggcagccaacccctccaaggtgtcctacgagcccatcaccaccaccctcaagaggaagcacgaggaggtg
tgcgccatcaagatccagagggcctaccgccggcacctgctacagcgctccatgaagcaggcatcctacatgtaccgcca
cagccacgacggcagcggggatgacgcccctgagaaggaggggctgcttgccaacaccatgagcaagatgtatggcc
acgagaatgggaacagcagctcgccaagcccggaggagaagggcgaggcaggggacgccggacccactatggggc
tgatgcccatcagcccctcagacactgcctggcctcccgcccctcccccagggcagactgtgcgcccaggtgtcaagga
gtctcttgtctag (SEQ ID NO: 9)

H.s. SCN5A

atggcaaacttcctattacctcggggcaccagcagcttccgcaggttcacacgggagtccctggcagccatcgagaagcg
catggcagagaagcaagcccgcggctcaaccaccttgcaggagagccgagaggggctgcccgaggaggaggctccc
cggccccagctggacctgcaggcctccaaaaagctgccagatctctatggcaatccaccccaagagctcatcggagagc
ccctggaggacctggaccccttctatagcacccaaaagactttcatcgtactgaataaaggcaagaccatcttccggttcagt
gccaccaacgccttgtatgtcctcagtcccttccaccccatccggagagcggctgtgaagattctggttcactcgctcttcaa
catgctcatcatgtgcaccatcctcaccaactgcgtgttcatggcccagcacgaccctccaccctggaccaagtatgtcgag
tacaccttcaccgccatttacacctttgagtctctggtcaagattctggctcgaggcttctgcctgcacgcgttcactttccttcg
ggacccatggaactggctggactttagtgtgattatcatggcatacacaactgaatttgtggacctgggcaatgtctcagcctt
acgcaccttccgagtcctccgggccctgaaaactatatcagtcatttcagggctgaagaccatcgtgggggccctgatcca
gtctgtgaagaagctggctgatgtgatggtcctcacagtcttctgcctcagcgtctttgccctcatcggcctgcagctcttcat
gggcaacctaaggcacaagtgcgtgcgcaacttcacagcgctcaacggcaccaacggctccgtggaggccgacggctt
ggtctgggaatccctggacctttacctcagtgatccagaaaattacctgctcaagaacggcacctctgatgtgttactgtgtg
ggaacagctctgacgctgggacatgtccggagggctaccggtgcctaaaggcaggcgagaaccccgaccacggctaca
ccagcttcgattcctttgcctgggcctttcttgcactcttccgcctgatgacgcaggactgctgggagcgcctctatcagcag
accctcaggtccgcagggaagatctacatgatcttcttcatgcttgtcatcttcctggggtccttctacctggtgaacctgatcc
tggccgtggtcgcaatggcctatgaggagcaaaaccaagccaccatcgctgagaccgaggagaaggaaaagcgcttcc
aggaggccatggaaatgctcaagaaagaacacgaggccctcaccatcaggggtgtggataccgtgtcccgtagctccttg

gagatgtcccctttggccccagtaaacagccatgagagaagaagcaagaggagaaaacggatgtcttcaggaactgagg
agtgtggggaggacaggctccccaagtctgactcagaagatggtcccagagcaatgaatcatctcagcctcacccgtggc
ctcagcaggacttctatgaagccacgttccagccgcgggagcattttcacctttcgcaggcgagacctgggttctgaagca
gattttgcagatgatgaaaacagcacagcgggggagagcgagagccaccacacatcactgctggtgccctggcccctgc
gccggaccagtgcccagggacagcccagtcccggaacctcggctcctggccacgccctccatggcaaaaagaacagc
actgtggactgcaatggggtggtctcattactgggggcaggcgacccagaggccacatccccaggaagccacctcctcc
gccctgtgatgctagagcacccgccagacacgaccacgccatcggaggagccaggcgggccccagatgctgacctccc
aggctccgtgtgtagatggcttcgaggagccaggagcacggcagcgggccctcagcgcagtcagcgtcctcaccagcg
cactggaagagttagaggagtctcgccacaagtgtccaccatgctggaaccgtctcgcccagcgctacctgatctgggagt
gctgcccgctgtggatgtccatcaagcagggagtgaagttggtggtcatggacccgtttactgacctcaccatcactatgtg
catcgtactcaacacactcttcatggcgctggagcactacaacatgacaagtgaattcgaggagatgctgcaggtcggaaa
cctggtcttcacagggattttcacagcagagatgaccttcaagatcattgccctcgacccctactactacttccaacagggct
ggaacatcttcgacagcatcatcgtcatccttagcctcatggagctgggcctgtcccgcatgagcaacttgtcggtgctgcg
ctccttccgcctgctgcgggtcttcaagctggccaaatcatggcccaccctgaacacactcatcaagatcatcgggaactca
gtgggggcactggggaacctgacactggtgctagccatcatcgtgttcatctttgctgtggtgggcatgcagctctttggcaa
gaactactcggagctgagggacagcgactcaggcctgctgcctcgctggcacatgatggacttctttcatgccttcctcatc
atcttccgcatcctctgtggagagtggatcgagaccatgtgggactgcatggaggtgtcggggcagtcattatgcctgctgg
tcttcttgcttgttatggtcattggcaaccttgtggtcctgaatctcttcctggccttgctgctcagctccttcagtgcagacaacc
tcacagcccctgatgaggacagagagatgaacaacctccagctggccctggcccgcatccagaggggcctgcgctttgt
caagcggaccacctgggatttctgctgtggtctcctgcggcagcggcctcagaagcccgcagcccttgccgcccagggc
cagctgcccagctgcattgccacccccctactccccgccacccccagagacggagaaggtgcctcccacccgcaaggaa
acacggtttgaggaaggcgagcaaccaggccagggcacccccggggatccagagcccgtgtgtgtgcccatcgctgtg
gccgagtcagacacagatgaccaagaagaagatgaggagaacagcctgggcacggaggaggagtccagcaagcagc
aggaatcccagcctgtgtccggtggcccagaggcccctccggattccaggacctggagccaggtgtcagcgactgcctc
ctctgaggccgaggccagtgcatctcaggccgactggcggcagcagtggaaagcggaaccccaggccccagggtgcg
gtgagaccccagaggacagttgctccgagggcagcacagcagacatgaccaacaccgctgagctcctggagcagatcc
ctgacctcggccaggatgtcaaggacccagaggactgcttcactgaaggctgtgtccggcgctgtccctgctgtgcggtg
gacaccacacaggccccagggaaggtctggtggcggttgcgcaagacctgctaccacatcgtggagcacagctggttcg
agacattcatcatcttcatgatcctactcagcagtggagcgctggccttcgaggacatctacctagaggagcggaagaccat
caaggttctgcttgagtatgccgacaagatgttcacatatgtcttcgtgctggagatgctgctcaagtgggtggcctacggctt
caagaagtacttcaccaatgcctggtgctggctcgacttcctcatcgtagacgtctctctggtcagcctggtggccaacaccc
tgggctttgccgagatgggccccatcaagtcactgcggacgctgcgtgcactccgtcctctgagagctctgtcacgatttga
gggcatgagggtggtggtcaatgccctggtgggcgccatcccgtccatcatgaacgtcctcctcgtctgcctcatcttctgg
ctcatcttcagcatcatgggcgtgaacctctttgcggggaagtttgggaggtgcatcaaccagacagagggagacttgcctt
tgaactacaccatcgtgaacaacaagagccagtgtgagtccttgaacttgaccggagaattgtactggaccaaggtgaaag
tcaactttgacaacgtgggggccgggtacctggcccttctgcaggtggcaacatttaaaggctggatggacattatgtatgc
agctgtggactccaggggggtatgaagagcagcctcagtgggaatacaacctctacatgtacatctattttgtcattttcatcat
ctttgggtctttcttcaccctgaacctctttattggtgtcatcattgacaacttcaaccaacagaagaaaaagttagggggccag
gacatcttcatgacagaggagcagaagaagtactacaatgccatgaagaagctgggctccaagaagccccagaagccca
tcccacggcccctgaacaagtaccagggcttcatattcgacattgtgaccaagcaggcctttgacgtcaccatcatgtttctg
atctgcttgaatatggtgaccatgatggtggagacagatgaccaaagtcctgagaaaatcaacatcttggccaagatcaacc
tgctctttgtggccatcttcacaggcgagtgtattgtcaagctggctgccctgcgccactactacttcaccaacagctggaata
tcttcgacttcgtggttgtcatcctctccatcgtgggcactgtgctctcggacatcatccagaagtacttcttctccccgacgct
cttccgagtcatccgcctggcccgaataggccgcatcctcagactgatccgaggggccaaggggatccgcacgctgctct
ttgccctcatgatgtccctgcctgccctcttcaacatcgggctgctgctcttcctcgtcatgttcatctactccatctttggcatgg
ccaacttcgcttatgtcaagtgggaggctggcatcgacgacatgttcaacttccagaccttcgccaacagcatgctgtgcctc
ttccagatcaccacgtcggccggctgggatggcctcctcagccccatcctcaacactgggccgccctactgcgaccccac
tctgcccaacagcaatggctctcgggggggactgcgggagcccagccgtgggcatcctcttcttcaccacctacatcatcat
ctccttcctcatcgtggtcaacatgtacattgccatcatcctggagaacttcagcgtggccacggaggagagcaccgagcc
cctgagtgaggacgacttcgatatgttctatgagatctgggagaaatttgacccagaggccactcagtttattgagtattcggt

cctgtctgactttgccgatgccctgtctgagccactccgtatcgccaagcccaaccagataagcctcatcaacatggacctg
cccatggtgagtggggaccgcatccattgcatggacattctctttgccttcaccaaaagggtcctgggggagtctggggag
atggacgccctgaagatccagatggaggagaagttcatggcagccaacccatccaagatctcctacgagcccatcaccac
cacactccggcgcaagcacgaagaggtgtcggccatggttatccagagagccttccgcaggcacctgctgcaacgctctt
tgaagcatgcctccttcctcttccgtcagcaggcgggcagcggcctctccgaagaggatgcccctgagcgagagggcctc
atcgcctacgtgatgagtgagaacttctcccgaccccttggcccaccctccagctcctccatctcctccacttccttcccaccc
tcctatgacagtgtcactagagccaccagcgataacctccaggtgcgggggtctgactacagccacagtgaagatctcgc
cgacttcccccccttctccggacagggaccgtgagtccatcgtgtga (SEQ ID NO: 10)

H.s. SCN7A

atgttggcttcaccagaacctaagggccttgttcccttcactaaagagtcttttgaacttataaaacagcatattgctaaaacac
ataatgaagaccatgaagaagaagacttaaagccaactcctgatttggaagttggcaaaaagcttccatttatttatggaaac
ctttctcaaggaatggtgtcagagcccttggaagatgtggacccatattactacaagaaaaaaaatactttcatagtattaaata
aaaatagaacaatcttcagattcaatgcggcttccatcttgtgtacattgtctcctttcaattgtattagaagaacaactatcaag
gttttggtacatccctttttccaactgtttattctaattagtgtcctgattgattgcgtattcatgtccctgactaatttgccaaaatgg
agaccagtattagagaatactttgcttggaatttacacatttgaaatacttgtaaaactctttgcaagaggtgtctgggcaggat
cattttccttcctcggtgatccatggaactggctcgatttcagcgtaactgtgtttgaggttattataagatactcacctctggact
tcattccaacgcttcaaactgcaagaactttgagaattttaaaaattattcctttaaatcaaggtctgaaatcccttgtaggggtc
ctgatccactgcttgaagcagcttattggtgtcattatcctaactctgtttttctgagcatattttctctaattgggatggggctctt
catgggcaacttgaaacataaatgttttcgatggccccaagagaatgaaaatgaaaccctgcacaacagaactggaaaccc
atattatattcgagaaacagaaaacttttattatttggaaggagaaagatatgctctcctttgtggcaacaggacagatgctggt
cagtgtcctgaaggatatgtgtgtgtaaaagctggcataaatcctgatcaaggcttcacaaattttgacagttttggctgggcc
ttatttgccctatttcggttaatggctcaggattaccctgaagtactttatcaccagatactttatgcttctgggaaggtctacatg
atattttttgtggtggtaagtttttgttttcctttatatggcaagtttgttcttaggcatacttgccatggcctatgaagaagaaaag
cagagagttggtgaaatatctaagaagattgaaccaaaatttcaacagactggaaaagaacttcaagaaggaaatgaaaca
gatgaggccaagaccatacaaatagaaatgaagaaaaggtcaccaatttccacagacacatcattggatgtgttggaagat
gctactctcagacataaggaagaacttgaaaaatccaagaagatatgcccattatactggtataagtttgctaaaactttcttga
tctggaattgttctccctgttggttaaaattgaaagagtttgtccataggattataatggcaccatttactgatcttttccttatcata
tgcataattttaaacgtatgttttctgaccttggagcattatccaatgagtaaacaaactaacactcttctcaacattggaaacct
ggttttcattggaattttcacagcagaaatgatttttaaaataattgcaatgcatccatatgggtatttccaagtaggttggaacat
ttttgatagcatgatagtgttccatggtttaatagaactttgtctagcaaatgttgcaggaatggctcttcttcgattattcaggatg
ttaagaattttcaagttgggaaagtattggccaacattccagattttgatgtggtctcttagtaactcatgggtggccctgaaag
acttggtcctgttgttgttcacattcatcttcttttctgctgcattcggcatgaagctgtttggtaagaattatgaagaatttgtctgc
cacatagacaaagactgtcaactcccacgctggcacatgcatgactttttccactccttcctgaatgtgttccgaattctctgtg
gagagtgggtagagaccttgtgggactgtatggaggttgcaggccaatcctggtgtattcctttttacctgatggtcattttaat
tggaaatttactggtactttacctgtttctggcattggtgagctcatttagttcatgcaaggatgtaacagctgaagagaataatg
aagcaaaaaatctccagcttgcagtggcaagaattaaaaaggaataaactatgtgcttcttaaaatactatgcaaaacacaa
aatgtcccaaaggacacaatggaccatgtaaatgaggtatatgttaaagaagatatttctgaccataccctttctgaattgagc
aacacccaagattttctcaaagataaggaaaaaagcagtggcacagagaaaaacgctactgaaaatgagagccaatcact
tatccccagtcctagtgtctcagaaactgtaccaattgcttcaggagaatctgatatagaaaatctggataataaggagattca
gagtaagtctggtgatggaggcagcaaagagaaaataaagcaatctagctcatctgaatgcagtactgttgatattgctatct
ctgaagaagaagaaatgttctatggaggtgaaagatcaaagcatctgaaaaatggttgcagacgcggatcttcacttggtca
aatcagtggagcatccaagaaaggaaaaatctggcagaacatcaggaaaacctgctgcaagattgtagagaacaattggtt
taagtgttttattgggcttgttactctgctcagcactggcactctggcttttgaagatatatatatggatcagagaaagacaatta
aaattttattagaatatgctgacatgatctttacttatatcttcattctggaaatgcttctaaaatggatggcatatggttttaaggcc
tatttctctaatggctggtacaggctggacttcgtggttgttattgtgttttgtcttagcttaataggcaaaactcgggaagaacta
aaacctcttatttccatgaaattccttcggcccctcagagttctatctcaatttgaaagaatgaaggtggttgtgagagctttgat
caaaacaaccttacccactttgaatgtgtttcttgtctgcctgatgatctggctgatttttagtatcatgggagtagacttatttgct

ggcagattctatgaatgcattgacccaacaagtggagaaaggtttccttcatctgaagtcatgaataagagtcggtgtgaaag
ccttctgtttaacgaatccatgctatgggaaaatgcaaaaatgaactttgataatgttggaaatggtttcctttctctgcttcaagt
agcaacatttaatggatggatcactattatgaattcagcaattgattctgttgctgttaatatacagcctcattttgaagtcaacat
ctacatgtattgttactttatcaactttattatatttggagtatttctccctctgagtatgctgattactgttattattgataatttcaaca
agcataaaataaagctgggaggctcaaatatctttataacggttaaacagagaaaacagtaccgcaggctgaagaagctaa
tgtatgaggattctcaaagaccagtacctcgcccattaaacaagctccaaggattcatctttgatgtggtaacaagccaagctt
ttaatgtcattgttatggttcttatatgtttccaagcaatagccatgatgatagacactgatgttcagagtctacaaatgtccattg
ctctctactggattaactcaatttttgttatgctatatactatggaatgtatactgaagctcatcgctttccgttgtttttatttcaccat
tgcgtggaacatttttgattttatggtggttattttctccatcacaggactatgtctgcctatgacagtaggatcctaccttgtgcct
ccttcacttgtgcaactgatacttctctcacggatcattcacatgctgcgtcttggaaaaggaccaaaggtgtttcataatctgat
gcttcctttgatgctgtccctcccagcattattgaacatcattcttctcatcttcctggtcatgttcatctatgccgtatttggaatgt
ataattttgcctatgttaaaaaagaagctggaattaatgatgtgtctaattttgaaacctttggcaacagtatgctctgtcttttca
agttgcaatatttgctggttgggatgggatgcttgatgcaattttcaacagtaaatggtctgactgtgatcctgataaaattaacc
ctgggactcaagttagaggagattgtgggaacccctctgttgggatttttattttgtcagttatatcctcatatcatggctgatca
ttgtaaatatgtacattgttgttgtcatggagtttttaaatattgcttctaagaagaaaaacaagaccttgagtgaagatgattttag
gaaattctttcaggtatggaaaaggtttgatcctgataggacccagtacatagactctagcaagctttcagattttgcagctgct
cttgatcctcctcttttcatggcaaaaccaaacaagggccagctcattgctttggacctccccatggctgttggggacagaatt
cattgcctcgatatcttacttgcttttacaaagagagttatgggtcaagatgtgaggatggagaaagttgtttcagaaatagaat
cagggtttttgttagccaacccttttaagatcacatgtgagccaattacgactactttgaaacgaaaacaagaggcagtttcag
caaccatcattcaacgtgcttataaaaattaccgcttgaggcgaaatgacaaaaatacatcagatattcatatgatagatggtg
acagagatgttcatgctactaaagaaggtgcctattttgacaaagctaaggaaaagtcacctattcaaagccagatctaa
(SEQ ID NO: 11)

H.s. SCN8A

atggcagcgcggctgcttgcaccaccaggccctgatagtttcaagcctttcacccctgagtcactggcaaacattgagagg
cgcattgctgagagcaagctcaagaaaccaccaaaggccgatggcagtcatcgggaggacgatgaggacagcaagcc
caagccaaacagcgacctggaagcagggaagagtttgcctttcatctacggggacatcccccaaggcctggttgcagttc
ccctggaggactttgacccatactatttgacgcagaaaacctttgtagtattaaacagagggaaaactctcttcagatttagtg
ccacgcctgccttgtacattttaagtccttttaacctgataagaagaatagctattaaaattttgatacattcagtatttagcatgat
cattatgtgcactattttgaccaactgtgtattcatgacttttagtaaccctcctgactggtcgaagaatgtggagtacacgttca
cagggatttatacatttgaatcactagtgaaaatcattgcaagaggtttctgcatagatggctttacctttttacgggacccatgg
aactggttagatttcagtgtcatcatgatggcgtatataacagagtttgtaaacctaggcaatgtttcagctctacgcactttcag
ggtactgagggctttgaaaactatttcggtaatcccaggcctgaagacaattgtgggtgccctgattcagtctgtgaagaaac
tgtcagatgtgatgatcctgacagtgttctgcctgagtgtttttgccttgatcggactgcagctgttcatggggaaccttcgaaa
caagtgtgttgtgtggcccataaacttcaacgagagctatcttgaaaatggcaccaaaggctttgattgggaagagtatatca
acaataaaacaaatttctacacagttcctggcatgctggaacctttactctgtgggaacagttctgatgctgggcaatgccca
gagggataccagtgtatgaaagcaggaaggaaccccaactatggttacacaagttttgacacttttagctgggccttcttggc
attatttcgccttatgacccaggactattgggaaaacttgtatcaattgactttacgagcagccgggaaaacatacatgatcttc
ttcgtcttggtcatctttgtgggttctttctatctggtgaacttgatcttggctgtggtggccatggcttatgaagaacagaatcag
gcaacactggaggaggcagaacaaaaagaggctgaatttaaagcaatgttggagcaacttaagaagcaacaggaagag
gcacaggctgctgcgatggccacttcagcaggaactgtctcagaagatgccatagaggaagaaggtgaagaaggaggg
ggctcccctcggagctcttctgaaatctctaaactcagctcaaagagtgcaaaggaaagacgtaacaggagaaagaagag
gaagcaaaaggaactctctgaaggagaggagaaaggggatcccgagaaggtgtttaagtcagagtcagaagatggcat
gagaaggaaggcctttcggctgccagacaacagaatagggaggaaattttccatcatgaatcagtcactgctcagcatccc
aggctcgcccttcctctcccgccacaacagcaagagcagcatcttcagtttcaggggacctgggcggttccgagacccgg
gctccgagaatgagttcgcggatgacgagcacagcacggtggaggagagcgagggccgccgggactccctcttcatcc
ccatccgggcccgcgagcgccggagcagctacagcggctacagcggctacagccagggcagccgctcctcgcgcatc
ttccccagcctgcggcgcagcgtgaagcgcaacagcacggtggactgcaacggcgtggtgtccctcatcggcggcccc

ggctcccacatcggcgggcgtctcctgccagaggctacaactgaggtggaaattaagaagaaaggccctggatctcttta
gtttccatggaccaattagcctcctacgggcggaaggacagaatcaacagtataatgagtgttgttacaaatacactagtaga
agaactggaagagtctcagagaaagtgcccgccatgctggtataaatttgccaacactttcctcatctgggagtgccacccc
tactggataaaactgaaagagattgtgaacttgatagttatggacccttttgtggatttagccatcaccatctgcatcgtcctga
atacactgtttatggcaatggagcaccatcctatgacaccacaatttgaacatgtcttggctgtaggaaatctggttttcactgg
aattttcacagcggaaatgttcctgaagctcatagccatggatccctactattatttccaagaaggttggaacatttttgacgga
tttattgtctccctcagtttaatggaactgagtctagcagacgtggaggggctttcagtgctgcgatctttccgattgctccgag
tcttcaaattggccaaatcctggcccaccctgaacatgctaatcaagattattggaaattcagtgggtgccctgggcaacctg
acactggtgctggccattattgtcttcatctttgccgtggtggggatgcaactctttggaaaaagctacaaagagtgtgtctgc
aagatcaaccaggactgtgaactccctcgctggcatatgcatgactttttccattccttcctcattgtctttcgagtgttgtgcgg
ggagtggattgagaccatgtgggactgcatggaagtggcaggccaggccatgtgcctcattgtctttatgatggtcatggtg
attggcaacttggtggtgctgaacctgtttctggccttgctcctgagctccttcagtgcagacaacctggctgccacagatga
cgatggggaaatgaacaacctccagatctcagtgatccgtatcaagaagggtgtggcctggaccaaactaaaggtgcacg
ccttcatgcaggcccactttaagcagcgtgaggctgatgaggtgaagcctctggatgagttgtatgaaaagaaggccaact
gtatcgccaatcacaccggtgcagacatccaccggaatggtgacttccagaagaatggcaatggcacaaccagcggcatt
ggcagcagcgtggagaagtacatcattgatgaggaccacatgtccttcatcaacaaccccaacttgactgtacgggtaccc
attgctgtgggcgagtctgactttgagaacctcaacacagaggatgttagcagcgagtcggatcctgaaggcagcaaagat
aaactagatgacaccagctcctctgaaggaagcaccattgatatcaaaccagaagtagaagaggtccctgtggaacagcc
tgaggaatacttggatccagatgcctgcttcacagaaggttgtgtccagcggttcaagtgctgccaggtcaacatcgaggaa
gggctaggcaagtcttggtggatcctgcggaaaacctgcttcctcatcgtggagcacaactggtttgagaccttcatcatctt
catgattctgctgagcagtggcgccctggccttcgaggacatctacattgagcagagaaagaccatccgcaccatcctgga
atatgctgacaaagtcttcacctatatcttcatcctggagatgttgctcaagtggacagcctatggcttcgtcaagttcttcacca
atgcctggtgttggctggacttcctcattgtggctgtctctttagtcagccttatagctaatgccctgggctactcggaactagg
tgccataaagtcccttaggaccctaagagctttgagacccttaagagccttatcacgatttgaagggatgagggtggtggtg
aatgccttggtgggcgccatcccctccatcatgaatgtgctgctggtgtgtctcatcttctggctgattttcagcatcatgggag
ttaacttgtttgcgggaaagtaccactactgctttaatgagacttctgaaatccgatttgaaattgaagatgtcaacaataaaact
gaatgtgaaaagcttatggaggggaacaatacagagatcagatggaagaacgtgaagatcaactttgacaatgttggggc
aggatacctggcccttcttcaagtagcaaccttcaaaggctggatggacatcatgtatgcagctgtagattcccggaagcct
gatgagcagcctaagtatgaggacaatatctacatgtacatctattttgtcatcttcatcatcttcggctccttcttcaccctgaac
ctgttcattggtgtcatcattgataacttcaatcaacaaaagaaaaagttcggaggtcaggacatcttcatgaccgaagaaca
gaagaagtactacaatgccatgaaaaagctgggctcaaagaagccacagaaacctattccccgccccttgaacaaaatcc
aaggaatcgtctttgattttgtcactcagcaagcctttgacattgttatcatgatgctcatctgccttaacatggtgacaatgatg
gtggagacagacactcaaagcaagcagatggagaacatcctctactggattaacctggtgtttgttatcttcttcacctgtgag
tgtgtgctcaaaatgtttgcgttgaggcactactacttcaccattggctggaacatcttcgacttcgtggtagtcatcctctccat
tgtgggaatgttcctggcagatataattgagaaatactttgtttccccaaccctattccgagtcatccgattggcccgtattggg
cgcatcttgcgtctgatcaaaggcgccaaagggattcgtaccctgctctttgccttaatgatgtccttgcctgccctgttcaaca
tcggccttctgctcttcctggtcatgttcatcttctccatttttgggatgtccaattttgcatatgtgaagcacgaggctggtatcg
atgacatgttcaactttgagacatttggcaacagcatgatctgcctgtttcaaatcacaacctcagctggttgggatggcctgc
tgctgcccatcctaaaccgcccccctgactgcagcctagataaggaacacccagggagtggctttaagggagattgtggg
aacccctcagtgggcatcttcttctttgtaagctacatcatcatctctttcctaattgtcgtgaacatgtacattgccatcatcctg
gagaacttcagtgtagccacagaggaaagtgcagaccctctgagtgaggatgactttgagaccttctatgagatctgggag
aagttcgaccccgatgccacccagttcattgagtactgtaagctggcagactttgcagatgccttggagcatcctctccgagt
gcccaagcccaataccattgagctcatcgctatggatctgccaatggtgagcggggatcgcatccactgcttggacatcctt
tttgccttcaccaagcgggtcctgggagatagcggggagttggacatcctgcggcagcagatggaagagcggttcgtggc
atccaatccttccaaagtgtcttacgagccaatcacaaccacactgcgtcgcaagcaggaggaggtatctgcagtggtcct
gcagcgtgcctaccggggacatttggcaaggcggggcttcatctgcaaaaagacaacttctaataagctggagaatggag
gcacacaccgggagaaaaagagagcaccccatctacagcctccctcccgtcctatgacagtgtaactaaacctgaaaag
gagaaacagcagcgggcagaggaaggaagaagggaaagagccaaaagacaaaaagaggtcagagaatccaagtgtt
ag (SEQ ID NO: 12)

H.s. SCN9A

atggcaatgttgcctcccccaggacctcagagctttgtccatttcacaaaacagtctcttgccctcattgaacaacgcattgct
gaaagaaaatcaaaggaacccaaagaagaaaagaaagatgatgatgaagaagccccaaagccaagcagtgacttggaa
gctggcaaacaactgcccttcatctatggggacattcctcccggcatggtgtcagagcccctggaggacttggacccctact
atgcagacaaaaagactttcatagtattgaacaaagggaaaacaatcttccgtttcaatgccacacctgctttatatatgctttct
cctttcagtcctctaagaagaatatctattaagattttagtacactccttattcagcatgctcatcatgtgcactattctgacaaact
gcatatttatgaccatgaataacccgccggactggaccaaaaatgtcgagtacacttttactggaatatatacttttgaatcact
tgtaaaaatccttgcaagaggcttctgtgtaggagaattcacttttcttcgtgacccgtggaactggctggattttgtcgtcattg
tttttgcgtatttaacagaatttgtaaacctaggcaatgtttcagctcttcgaactttcagagtattgagagctttgaaaactatttct
gtaatcccaggcctgaagacaattgtaggggctttgatccagtcagtgaagaagctttctgatgtcatgatcctgactgtgttc
tgtctgagtgtgtttgcactaattggactacagctgttcatgggaaacctgaagcataaatgttttcgaaattcacttgaaaataa
tgaaacattagaaagcataatgaataccctagagagtgaagaagactttagaaaatatttttattacttggaaggatccaaaga
tgctctcctttgtggtttcagcacagattcaggtcagtgtccagaggggtacacctgtgtgaaaattggcagaaaccctgatta
tggctacacgagctttgacactttcagctgggccttcttagccttgtttaggctaatgacccaagattactgggaaaacctttac
caacagacgctgcgtgctgctggcaaaacctacatgatcttctttgtcgtagtgattttcctgggctccttttatctaataaacttg
atcctggctgtggttgccatggcatatgaagaacagaaccaggcaaacattgaagaagctaaacagaaagaattagaattt
caacagatgttagaccgtcttaaaaaagagcaagaagaagctgaggcaattgcagcggcagcggctgaatatacaagtat
taggagaagcagaattatgggcctctcagagagttcttctgaaacatccaaactgagctctaaaagtgctaaagaaagaag
aaacagaagaaagaaaaagaatcaaaagaagctctccagtggagaggaaaagggagatgctgagaaattgtcgaaatca
gaatcagaggacagcatcagaagaaaaagtttccaccttggtgtcgaagggcataggcgagcacatgaaaagaggttgtc
taccccccaatcagtcaccactcagcattcgtggctccttgttttctgcaaggcgaagcagcagaacaagtctttttagtttcaaa
ggcagaggaagagatataggatctgagactgaatttgccgatgatgagcacagcatttttggagacaatgagagcagaag
gggctcactgtttgtgccccacagaccccaggagcgacgcagcagtaacatcagccaagccagtaggtccccaccaatg
ctgccggtgaacgggaaaatgcacagtgctgtggactgcaacggtgtggtctccctggttgatggacgctcagccctcatg
ctccccaatggacagcttctgccagagggcacgaccaatcaaatacacaagaaaaggcgttgtagttcctatctcctttcag
aggatatgctgaatgatcccaacctcagacagagagcaatgagtagagcaagcatattaacaaacactgtggaagaacttg
aagagtccagacaaaaatgtccaccttggtggtacagatttgcacacaaattcttgatctggaattgctctccatattggataa
aattcaaaaagtgtatctattttattgtaatggatccttttgtagatcttgcaattaccatttgcatagttttaaacacattatttatggc
tatggaacaccacccaatgactgaggaattcaaaaatgtacttgctataggaaatttggtctttactggaatctttgcagctgaa
atggtattaaaactgattgccatggatccatatgagtatttccaagtaggctggaatatttttgacagccttattgtgactttaagtt
tagtggagctctttctagcagatgtggaaggattgtcagttctgcgatcattcagactgctccgagtcttcaagttggcaaaat
cctggccaacattgaacatgctgattaagatcattggtaactcagtaggggctctaggtaacctcaccttagtgttggccatca
tcgtcttcatttttgctgtggtcggcatgcagctctttggtaagagctacaaagaatgtgtctgcaagatcaatgatgactgtac
gctcccacggtggcacatgaacgacttcttccactccttcctgattgtgttccgcgtgctgtgtggagagtggatagagacca
tgtgggactgtatggaggtcgctggtcaagctatgtgccttattgtttacatgatggtcatggtcattggaaacctggtggtcct
aaacctatttctggccttattattgagctcatttagttcagacaatcttacagcaattgaagaagaccctgatgcaaacaacctc
cagattgcagtgactagaattaaaaagggaataaattatgtgaaacaaaccttacgtgaatttattctaaaagcattttccaaaa
agccaaagatttccagggagataagacaagcagaagatctgaatactaagaaggaaaactatatttctaaccatacacttgc
tgaaatgagcaaaggtcacaatttcctcaaggaaaaagataaaatcagtggttttggaagcagcgtggacaaacacttgatg
gaagacagtgatggtcaatcatttattcacaatcccagcctcacagtgacagtgccaattgcacctggggaatccgatttgga
aaatatgaatgctgaggaacttagcagtgattcggatagtgaatacagcaaagtgagattaaaccggtcaagctcctcagag
tgcagcacagttgataaccctttgcctggagaaggagaagaagcagaggctgaacctatgaattccgatgagccagaggc
ctgtttcacagatggttgtgtacggaggttctcatgctgccaagttaacatagagtcagggaaaggaaaaatctggtggaac
atcaggaaaacctgctacaagattgttgaacacagttggtttgaaagcttcattgtcctcatgatcctgctcagcagtggtgcc
ctggcttttgaagatatttatattgaaaggaaaaagaccattaagattatcctggagtatgcagacaagatcttcacttacatctt
cattctggaaatgcttctaaaatggatagcatatggttataaaacatatttcaccaatgcctggtgttggctggatttcctaattgt
tgatgtttctttggttactttagtggcaaacactcttggctactcagatcttggccccattaaatcccttcggacactgagagcttt
aagacctctaagagccttatctagatttgaaggaatgagggtcgttgtgaatgcactcataggagcaattccttccatcatgaa

tgtgctacttgtgtgtcttatattctggctgatattcagcatcatgggagtaaatttgtttgctggcaagttctatgagtgtattaac
accacagatgggtcacggtttcctgcaagtcaagttccaaatcgttccgaatgttttgcccttatgaatgttagtcaaaatgtgc
gatggaaaaacctgaaagtgaactttgataatgtcggacttggttacctatctctgcttcaagttgcaacttttaagggatggac
gattattatgtatgcagcagtggattctgttaatgtagacaagcagcccaaatatgaatatagcctctacatgtatatttattttgt
cgtctttatcatctttgggtcattcttcactttgaacttgttcattggtgtcatcatagataatttcaaccaacagaaaaagaagctt
ggaggtcaagacatctttatgacagaagaacagaagaaatactataatgcaatgaaaaagctggggtccaagaagccaca
aaagccaattcctcgaccagggaacaaaatccaaggatgtatatttgacctagtgacaaatcaagcctttgatattagtatcat
ggttcttatctgtctcaacatggtaaccatgatggtagaaaaggagggtcaaagtcaacatatgactgaagttttatattggata
aatgtggtttttataatccttttcactggagaatgtgtgctaaaactgatctccctcagacactactacttcactgtaggatggaat
atttttgattttgtggttgtgattatctccattgtaggtatgtttctagctgatttgattgaaacgtattttgtgtcccctaccctgttcc
gagtgatccgtcttgccaggattggccgaatcctacgtctagtcaaaggagcaaaggggatccgcacgctgctctttgcttt
gatgatgtcccttcctgcgttgtttaacatcggcctcctgctcttcctggtcatgttcatctacgccatctttggaatgtccaacttt
gcctatgttaaaaaggaagatggaattaatgacatgttcaattttgagacctttggcaacagtatgatttgcctgttccaaattac
aacctctgctggctgggatggattgctagcacctattcttaacagtaagccacccgactgtgacccaaaaaaagttcatcctg
gaagttcagttgaaggagactgtggtaacccatctgttggaatattctactttgttagttatatcatcatatccttcctggttgtggt
gaacatgtacattgcagtcatactggagaattttagtgttgccactgaagaaagtactgaacctctgagtgaggatgactttga
gatgttctatgaggtttgggagaagtttgatcccgatgcgacccagtttatagagttctctaaactctctgattttgcagctgccc
tggatcctcctcttctcatagcaaaacccaacaaagtccagctcattgccatggatctgcccatggttagtggtgaccggatc
cattgtcttgacatcttatttgcttttacaaagcgtgttttgggtgagagtggggagatggattctcttcgttcacagatggaaga
aaggttcatgtctgcaaatccttccaaagtgtcctatgaacccatcacaaccacactaaaacggaaacaagaggatgtgtct
gctactgtcattcagcgtgcttatagacgttaccgcttaaggcaaaatgtcaaaaatatatcaagtatatacataaaagatgga
gacagagatgatgatttactcaataaaaaagatatggcttttgataatgttaatgagaactcaagtccagaaaaaacagatgc
cacttcatccaccacctctccaccttcatatgatagtgtaacaaagccagacaaagagaaatatgaacaagacagaacaga
aaaggaagacaaagggaaagacagcaaggaaagcaaaaaatag (SEQ ID NO: 13)

H.s. SCN10A

atggaattccccattggatccctcgaaactaacaacttccgtcgctttactccggagtcactggtggagatagagaagcaaat
tgctgccaagcagggaacaaagaaagccagagagaagcatagggagcagaaggaccaagaagagaagcctcggccc
cagctggacttgaaagcctgcaaccagctgcccaagttctatggtgagctcccagcagaactgatcggggagcccctgga
ggatctagatccgttctacagcacacaccggacatttatggtgctgaacaaagggaggaccatttcccggtttagtgccact
cgggccctgtggctattcagtcctttcaacctgatcagaagaacggccatcaaagtgtctgtccactcgtggttcagtttattta
ttacggtcactattttggttaattgtgtgtgcatgacccgaactgaccttccagagaaaattgaatatgtcttcactgtcatttaca
cctttgaagccttgataaagatactggcaagaggattttgtctaaatgagttcacgtacctgagagatccttggaactggctgg
attttagcgtcattaccctggcatatgttggcacagcaatagatctccgtgggatctcaggcctgcggacattcagagttctta
gagcattaaaaacagtttctgtgatcccaggcctgaaggtcattgtgggggccctgattcactcagtgaagaaactggctga
tgtgaccatcctcaccatcttctgcctaagtgtttttgccttggtggggctgcaactcttcaagggcaacctcaaaaataaatgt
gtcaagaatgacatggctgtcaatgagacaaccaactactcatctcacagaaaaccagatatctacataaataagcgaggc
acttctgaccccttactgtgtggcaatggatctgactcaggccactgccctgatggttatatctgccttaaaacttctgacaacc
cggattttaactacaccagctttgattcctttgcttgggctttcctctcactgttccgcctcatgacacaggattcctgggaacgc
ctctaccagcagaccctgaggacttctgggaaaatctatatgatctttttgtgctcgtaatcttcctgggatctttctacctggtc
aacttgatcttggctgtagtcaccatggcgtatgaggagcagaaccaggcaaccactgatgaaattgaagcaaaggagaa
gaagttccaggaggccctcgagatgctccggaaggagcaggaggtgctagcagcactagggattgacacaacctctctc
cactcccacaatggatcacctttaacctccaaaaatgccagtgagagaaggcatagaataaagccaagagtgtcagaggg
ctccacagaagacaacaaatcaccccgctctgatccttacaaccagcgcaggatgtcttttctaggcctcgcctctggaaaa
cgccgggctagtcatggcagtgtgttccatttccggtcccctggccgagatatctcactccctgagggagtcacagatgatg
gagtctttcctggagaccacgaaagccatcggggctctctgctgctgggtgggggtgctggccagcaaggccccctccct
agaagccctcttcctcaacccagcaaccctgactccaggcatggagaagatgaacaccaaccgccgcccactagtgagct
tgcccctggagctgtcgatgtctcggcattcgatgcaggacaaaagaagactttcttgtcagcagaatacttagatgaacctt

ccgggcccaaagggcaatgagtgttgtcagtatcataacctccgtccttgaggaactcgaggagtctgaacagaagtgccc
accctgcttgaccagcttgtctcagaagtatctgatctgggattgctgccccatgtgggtgaagctcaagacaattctctttgg
gcttgtgacggatccctttgcagagctcaccatcaccttgtgcatcgtggtgaacaccatcttcatggccatggagcaccatg
gcatgagccctaccttcgaagccatgctccagataggcaacatcgtctttaccatatttttactgctgaaatggtcttcaaaat
cattgccttcgacccatactattatttccagaagaagtggaatatctttgactgcatcatcgtcactgtgagtctgctagagctg
ggcgtggccaagaagggaagcctgtctgtgctgcggagcttccgcttgctgcgcgtattcaagctggccaaatcctggcc
caccttaaacacactcatcaagatcatcggaaactcagtgggggcactggggaacctcaccatcatcctggccatcattgtc
tttgtctttgctctggttggcaagcagctcctaggggaaaactaccgtaacaaccgaaaaaatatctccgcgccccatgaag
actggccccgctggcacatgcacgacttcttccactctttcctcattgtcttccgtatcctctgtggagagtggattgagaacat
gtgggcctgcatggaagttggccaaaaatccatatgcctcatccttttcttgacggtgatggtgctagggaacctggtggtgc
ttaacctgttcatcgccctgctattgaactctttcagtgctgacaacctcacagccccggaggacgatggggaggtgaacaa
cctgcaggtggccctggcacggatccaggtctttggccatcgtaccaaacaggctctttgcagcttcttcagcaggtcctgc
ccattcccccagcccaaggcagagcctgagctggtggtgaaactcccactctccagctccaaggctgagaaccacattgc
tgccaacactgccaggggggagctctggagggctccaagctcccagaggccccagggatgagcacagtgacttcatcgct
aatccgactgtgtgggtctctgtgcccattgctgagggtgaatctgatcttgatgacttggaggatgatggtggggaagatgc
tcagagcttccagcaggaagtgatccccaaaggacagcaggagcagctgcagcaagtcgagaggtgtggggaccacct
gacacccaggagcccaggcactggaacatcttctgaggacctggctccatccctgggtgagacgtggaaagatgagtctg
ttcctcaggtccctgctgagggagtggacgacacaagctcctctgagggcagcacggtggactgcctagatcctgaggaa
atcctgaggaagatccctgagctggcagatgacctggaagaaccagatgactgcttcacagaaggatgcattcgccactgt
ccctgctgcaaactggataccaccaagagtccatgggatgtgggctggcaggtgcgcaagacttgctaccgtatcgtgga
gcacagctggtttgagagcttcatcatcttcatgatcctgctcagcagtggatctctggcctttgaagactattacctggacca
gaagcccacggtgaaagctttgctggagtacactgacagggtcttcacctttatctttgtgttcgagatgctgcttaagtgggt
ggcctatggcttcaaaaagtacttcaccaatgcctggtgctggctggacttcctcattgtgaatatctcactgataagtctcaca
gcgaagattctggaatattctgaagtggctcccatcaaagcccttcgaacccttcgcgctctgcggccactgcgggctctttc
tcgatttgaaggcatgcgggtggtggtggatgccctggtgggcgccatcccatccatcatgaatgtcctcctcgtctgcctca
tcttctggctcatcttcagcatcatgggtgtgaacctcttcgcagggaagttttggaggtgcatcaactataccgatggagagt
tttcccttgtacctttgtcgattgtgaataacaagtctgactgcaagattcaaaactccactggcagcttcttctgggtcaatgtg
aaagtcaactttgataatgttgcaatgggttaccttgcacttctgcaggtggcaacctttaaaggctggatggacattatgtatg
cagctgttgattcccgggaggtcaacatgcaacccaagtgggaggacaacgtgtacatgtatttgtactttgtcatcttcatca
tttttggaggcttcttcacactgaatctctttgttggggtcataattgacaacttcaatcaacagaaaaaaaagttagggggcca
ggacatcttcatgacagaggagcagaagaaatactacaatgccatgaagaagttgggctccaagaagccccagaagccc
atcccacggcccctgaacaagttccagggttttgtctttgacatcgtgaccagacaagcttttgacatcaccatcatggtcctc
atctgcctcaacatgatcaccatgatggtggagactgatgaccaaagtgaagaaaagacgaaaattctgggcaaaatcaac
cagttctttgtggccgtcttcacaggcgaatgtgtcatgaagatgttcgctttgaggcagtactacttcacaaatggctggaat
gtgtttgacttcattgtggtggttctctccattgcgagcctgatttttctgcaattcttaagtcacttcaaagttacttctccccaac
gctcttcagagtcatccgcctggcccgaattggccgcatcctcagactgatccgagcggccaaggggatccgcacactgc
tctttgccctcatgatgtccctgcctgccctcttcaacatcgggctgttgctattccttgtcatgttcatctactctatcttcggtatg
tccagctttccccatgtgaggtgggaggctggcatcgacgacatgttcaacttccagaccttcgccaacagcatgctgtgcc
tcttccagattaccacgtcggccggctgggatggcctcctcagccccatcctcaacacagggcccccctactgtgacccca
atctgcccaacagcaatggcaccagaggggactgtgggagcccagccgtaggcatcatcttcttcaccacctacatcatca
tctccttcctcatcatggtcaacatgtacattgcagtgattctggagaacttcaatgtggccacggaggagagcactgagccc
ctgagtgaggacgactttgacatgttctatgagacctgggagaagtttgacccagaggccactcagtttattaccttttctgctc
tctcggactttgcagacactctctctggtcccctgagaatcccaaaacccaatcgaaatatactgatccagatggacctgcctt
tggtccctggagataagatccactgcttggacatcctttttgctttcaccaagaatgtcctaggagaatccggggagttggatt
ctctgaaggcaaatatggaggagaagtttatggcaactaatctttcaaaatcatcctatgaaccaatagcaaccactctccgat
ggaagcaagaagacatttcagccactgtcattcaaaaggcctatcggagctatgtgctgcaccgctccatggcactctctaa
caccccatgtgtgcccagagctgaggaggaggctgcatcactcccagatgaaggttttgttgcattcacagcaaatgaaaat
tgtgtactcccagacaaatctgaaactgcttctgccacatcattcccaccgtcctatgagagtgtcactagaggccttagtgat
agagtcaacatgaggacatctagctcaatacaaaatgaagatgaagccaccagtatggagctgattgcccctgggccctag
(SEQ ID NO: 14)

H.s. SCN11A

atggatgacagatgctacccagtaatctttccagatgagcggaatttccgccccttcacttccgactctctggctgcaattgag
aagcggattgccatccaaaaggagaaaaagaagtctaaagaccagacaggagaagtaccccagcctcggcctcagcttg
acctaaaggcctccaggaagttgcccaagctctatggcgacattcctcgtgagctcataggaaagcctctggaagacttgg
acccattctaccgaaatcataagacatttatggtgttaaacagaaagaggacaatctaccgcttcagtgccaagcatgccttg
ttcatttttgggcctttcaattcaatcagaagtttagccattagagtctcagtccattcattgttcagcatgttcattatcggcaccg
ttatcatcaactgcgtgttcatggctacagggcctgctaaaaacagcaacagtaacaatactgacattgcagagtgtgtcttca
ctgggatttatatttttgaagctttgattaaaatattggcaagaggtttcattctggatgagttttctttccttcgagatccatggaac
tggctggactccattgtcattggaatagcgattgtgtcatatattccaggaatcaccatcaaactattgcccctgcgtaccttcc
gtgtgttcagagctttgaaagcaatttcagtagtttcacgtctgaaggtcatcgtgggggccttgctacgctctgtgaagaagc
tggtcaacgtgattatcctcaccttcttttgcctcagcatctttgccctggtaggtcagcagctcttcatgggaagtctgaacctg
aaatgcatctcgagggactgtaaaaatatcagtaacccggaagcttatgaccattgctttgaaaagaaagaaaattcacctga
attcaaaatgtgtggcatctggatgggtaacagtgcctgttccatacaatatgaatgtaagcacaccaaaattaatcctgacta
taattatacgaattttgacaactttggctggtcttttcttgccatgttccggctgatgacccaagattcctgggagaagctttatca
acagaccctgcgtactactgggctctactcagtcttcttcttcattgtggtcattttcctgggctccttctacctgattaacttaacc
ctggctgttgttaccatggcatatgaggagcagaacaagaatgtagctgcagagatagaggccaaggaaaagatgtttcag
gaagcccagcagctgttaaaggaggaaaaggaggctctggttgccatgggaattgacagaagttcacttacttcccttgaa
acatcatattttaccccaaaaaagagaaagctctttggtaataagaaaaggaagtccttctttttgagagagtctgggaaagac
cagcctcctgggtcagattctgatgaagattgccaaaaaaagccacagctcctagagcaaaccaaacgactgtcccagaat
ctatcactggaccactttgatgagcatggagatcctctccaaaggcagagagcactgagtgctgtcagcatcctcaccatca
ccatgaaggaacaagaaaaatcacaagagccttgtctcccttgtggagaaaacctggcatccaagtacctcgtgtggaact
gttgcccccagtggctgtgcgttaagaaggtcctgagaactgtgatgactgacccgtttactgagctggccatcaccatctg
catcatcatcaacactgtcttcttggccatggagcatcacaagatggaggccagttttgagaagatgttgaatatagggaattt
ggttttcactagcatttttatagcagaaatgtgcctaaaaatcattgcgctcgatccctaccactactttcgccgaggctggaac
atttttgacagcattgttgctcttctgagttttgcagatgtaatgaactgtgtacttcaaaagagaagctggccattcttgcgttcc
ttcagagtgctcagggtcttcaagttagccaaatcctggccaactttgaacacactaattaagataatcggcaactctgtcgga
gcccttggaagcctgactgtggtcctggtcattgtgatctttattttctcagtagttggcatgcagctttttggccgtagcttcaat
tcccaaaagagtccaaaactctgtaacccgacaggcccgacagtctcatgtttacggcactggcacatgggggatttctgg
cactccttcctagtggtattccgcatcctctgcggggaatggatcgaaaatatgtgggaatgtatgcaagaagcgaatgcatc
atcatcattgtgtgttattgtcttcatattgatcacggtgataggaaaacttgtggtgctcaacctcttcattgccttactgctcaatt
cctttagcaatgaggaaagaaatggaaacttagaaggagaggccaggaaaactaaagtccagttagcactggatcgattc
cgccgggctttttgttttgtgagacacactcttgagcatttctgtcacaagtggtgcaggaagcaaaacttaccacagcaaaa
agaggtggcaggaggctgtgctgcacaaagcaaagacatcattcccctggtcatggagatgaaaaggggctcagagacc
caggaggagcttggtatactaacctctgtaccaaagaccctgggcgtcaggcatgattggacttggttggcaccacttgcg
gaggaggaagatgacgttgaattttctggtgaagataatgcacagcgcatcacacaacctgagcctgaacaacaggcctat
gagctccatcaggagaacaagaagcccacgagccagagagttcaaagtgtggaaattgacatgttctctgaagatgagcc
tcatctgaccatacaggatccccgaaagaagtctgatgttaccagtatactatcagaatgtagcaccattgatcttcaggatgg
ctttggatggttacctgagatggttcccaaaaagcaaccagagagatgtttgcccaaaggctttggttgctgctttccatgctg
tagcgtggacaagagaaagcctccctgggtcatttggtggaacctgcggaaaacctgctaccaaatagtgaaacacagct
ggtttgagagctttattatctttgtgattctgctgagcagtggggcactgatatttgaagatgttcaccttgagaaccaacccaa
aatccaagaattactaaattgtactgacattatttttacacatatttttatcctggagatggtactaaaatgggtagccttcggattt
ggaaagtatttcaccagtgcctggtgctgccttgatttcatcattgtgattgtctctgtgaccaccctcattaacttaatggaattg
aagtccttccggactctacgagcactgaggcctcttcgtgcgctgtcccagtttgaaggaatgaaggtggtggtcaatgctct
cataggtgccatacctgccattctgaatgttttgcttgtctgcctcattttctggctcgtattttgtattctgggagtatacttcttttct
ggaaaatttgggaaatgcattaatggaacagactcagttataaattataccatcattacaaataaaagtcaatgtgaaagtggc
aatttctcttggatcaaccagaaagtcaactttgacaatgtgggaaatgcttacctcgctctgctgcaagtggcaacatttaag
ggctggatggatattatatatgcagctgttgattccacagagaaagaacaacagccagagtttgagagcaattcactcggtta

catttacttcgtagtctttatcatctttggctcattcttcactctgaatctcttcattggcgttatcattgacaacttcaaccaacagc
agaaaaagttaggtggccaagacatttttatgacagaagaacagaagaaatactataatgcaatgaaaaaattaggatccaa
aaaacctcaaaaacccattccacggcctctgaacaaatgtcaaggtctcgtgttcgacatagtcacaagccagatctttgaca
tcatcatcataagtctcattatcctaaacatgattagcatgatggctgaatcatacaaccaacccaaagccatgaaatccatcct
tgaccatctcaactgggtctttgtggtcatctttacgttagaatgtctcatcaaaatctttgctttgaggcaatactacttcaccaat
ggctggaatttatttgactgtgtggtcgtgcttctttccattgttagtacaatgatttctaccttggaaaatcaggagcacattcctt
tccctccgacgctcttcagaattgtccgcttggctcggattggccgaatcctgaggcttgtccgggctgcacgaggaatcag
gactctcctctttgctctgatgatgtcgcttccttctctgttcaacattggtcttctactctttctgattatgtttatctatgccattctg
ggtatgaactggttttccaaagtgaatccagagtctggaatcgatgacatattcaacttcaagacttttgccagcagcatgctct
gtctcttccagataagcacatcagcaggttgggattccctgctcagccccatgctgcgatcaaaagaatcatgtaactcttcct
cagaaaactgccacctccctggcatagccacatcctactttgtcagttacattatcatctcctttctcattgttgtcaacatgtaca
ttgctgtgattttagagaacttcaatacagccactgaagaaagtgaggaccctttgggtgaagatgactttgacatattttatga
agtgtgggaaaagtttgacccagaagcaacacaatttatcaaatattctgccctttctgactttgctgatgccttgcctgagcct
ttgcgtgtcgcaaagccaaataaatatcaatttctagtaatggacttgcccatggtgagtgaagatcgcctccactgcatggat
attcttttcgccttcaccgctagggtactcggtggctctgatggcctagatagtatgaaagcaatgatggaagagaagttcatg
gaagccaatcctctcaagaagttgtatgaacccatagtcaccaccaccaagagaaaggaagaggaaagaggtgctgctat
tattcaaaaggcctttcgaaagtacatgatgaaggtgaccaagggtgaccaaggtgaccaaaatgacttggaaaacgggc
ctcattcaccactccagactctttgcaatggagacttgtctagctttggggtggccaagggcaaggtccactgtgactga
(SEQ ID NO: 15)

H.s. SCN1B

Atggggaggctgctggccttagtggtcggcgcggcactggtgtcctcagcctgcgggggctgcgtggaggtggactcg
gagaccgaggccgtgtatgggatgaccttcaaaattctttgcatctcctgcaagcgccgcagcgagaccaacgctgagac
cttcaccgagtggaccttccgccagaagggcactgaggagtttgtcaagatcctgcgctatgagaatgaggtgttgcagct
ggaggaggatgagcgcttcgagggccgcgtggtgtggaatggcagccggggcaccaaagacctgcaggatctgtctat
cttcatcaccaatgtcacctacaaccactcgggcgactacgagtgccacgtctaccgcctgctcttcttcgaaaactacgag
cacaacaccagcgtcgtcaagaagatccacattgaggtagtggacaaagccaacagagacatggcatccatcgtgtctga
gatcatgatgtatgtgctcattgtggtgttgaccatatggctcgtggcagagatgatttactgctacaagaagatcgctgccgc
cacggagactgctgcacaggagaatgcctcggaatacctggccatcacctctgaaagcaaagagaactgcacgggcgtc
caggtggccgaatag (SEQ ID NO: 16)

H.s. SCN2B

Atgcacagagatgcctggctacctcgccctgccttcagcctcacggggctcagtctctttttctctttggtgccaccaggacg
gagcatggaggtcacagtacctgccaccctcaacgtcctcaatggctctgacgcccgcctgccctgcaccttcaactcctg
ctacacagtgaaccacaaacagttctccctgaactggacttaccaggagtgcaacaactgctctgaggagatgttcctccag
ttccgcatgaagatcattaacctgaagctggagcggtttcaagaccgcgtggagttctcagggaaccccagcaagtacgat
gtgtcggtgatgctgagaaacgtgcagccggaggatgaggggatttacaactgctacatcatgaaccccccctgaccgcca
ccgtggccatggcaagatccatctgcaggtcctcatggaagagccccctgagcgggactccacggtggccgtgattgtgg
gtgcctccgtcgggggcttcctggctgtggtcatcttggtgctgatggtggtcaagtgtgtgaggagaaaaaaagagcaga
agctgagcacagatgacctgaagaccgaggaggagggcaagacggacggtgaaggcaacccggatgatggcgccaa
gtag (SEQ ID NO: 17)

H.s. SCN3B

Atgcctgccttcaatagattgtttcccctggcttctctcgtgcttatctactgggtcagtgtctgcttccctgtgtgtgtggaagt
gccctcggagacggaggccgtgcagggcaaccccatgaagctgcgctgcatctcctgcatgaagagagaggaggtgga
ggccaccacggtggtggaatggttctacaggcccgagggcggtaaagatttccttatttacgagtatcggaatggccacca
ggaggtggagagcccctttcaggggcgcctgcagtggaatggcagcaaggacctgcaggacgtgtccatcactgtgctc
aacgtcactctgaacgactctggcctctacacctgcaatgtgtcccgggagtttgagtttgaggcgcatcggccctttgtgaa
gacgacgcggctgatcccccctaagagtcaccgaggaggctggagaggacttcacctctgtggtctcagaaatcatgatgt
acatccttctggtcttcctcaccttgtggctgctcatcgagatgatatattgctacagaaaggtctcaaaagccgaagaggca
gcccaagaaaacgcgtctgactaccttgccatcccatctgagaacaaggagaactctgcggtaccagtggaggaatag
(SEQ ID NO: 18)

H.s. SCN4B

Atgcccggggctggggacggaggcaaagccccggcgagatggctgggcactgggcttttgggcctcttcctgctcccc
gtaaccctgtcgctggaggtgtctgtgggaaaggccaccgacatctacgctgtcaatggcacggagatcctgctgccctgc
accttctccagctgctttggcttcgaggacctccacttccggtggacctacaacagcagtgacgcattcaagattctcataga
ggggactgtgaagaatgagaagtctgaccccaaggtgacgttgaaagacgatgaccgcatcactctggtaggctctacta
aggagaagatgaacaacatttccattgtgctgagggacctggagttcagcgacacgggcaaatacacctgccatgtgaag
aaccccaaggagaataatctccagcaccacgccaccatcttcctccaagtcgttgatagactggaagaagtggacaacac
agtgacactcatcatcctggctgtcgtgggcggggtcatcgggctcctcatcctcatcctgctgatcaagaaactcatcatctt
catcctgaagaagactcgggagaagaagaaggagtgtctcgtgagctcctcggggaatgacaacacggagaacggcttg
cctggctccaaggcagaggagaaaccaccttcaaaagtgtga (SEQ ID NO: 19)

H.s. SCN1A

meqtvlvppgpdsfnfftreslaaierriaeekaknpkpdkkdddengpkpnsdleagknlpfiygdippemvsepl
edldpyyinkktfivlnkgkaifrfsatsalyiltpfnplrkiaikilvhslfsmlimctiltncvfmtmsnppdwtknvey
tftgiytfeslikiiargfcledftflrdpwnwldftvitfayvtefvdlgnvsalrtfrvlralktisvipglktivgaliqsvkkl
sdvmiltvfclsvfaliglqlfmgnlrnkciqwpptnasleehsieknitvnyngtlinetvfefdwksyiqdsryhyfle
gfldallcgnssdagqcpegymcvkagrnpnygytsfdtfswaflslfrlmtqdfwenlyqltlraagktymiffvlvifl
gsfylinlilavvamayeeqnqatleeaeqkeaefqqmieqlkkqqeaaqqaatatasehsrepsaagrlsdssseaskl
ssksakerrnrrkkrkqkeqsggeekdedefqksesedsirrkgfrfsiegnrltyekryssphqsllsirgslfsprrnsrts
lfsfrgrakdvgsendfaddehstfednesrrdslfvprrhgerrnsnlsqtsrssrmlavfpangkmhstvdcngvvsl
vggpsvptspvgqllpegtttetemrkrrsssfhvsmdfledpsqrqramsiasiltntveeleesrqkcppcwykfsni
fliwdcspywlkvkhvvnlvvmdpfvdlaiticivlntlfmamehypmtdhfnnvltvgnlvftgiftaemflkiiam
dpyyyfqegwnifdgfivtlslvelglanveglsvlrsfrllrvfklakswptlnmlikiignsvgalgnltlvlaiivfifav
vgmqlfgksykdcvckiasdcqlprwhmndffhsflivfrvlcgewietmwdcmevagqamcltvfmmvmvig
nlvvlnlflalllssfsadnlaatdddnemnnlqiavdrmhkgvayvkrkiyefiqqsfirkqkildeikplddlnnkkds
cmsnhtaeigkdldylkdvngttsgigtgssvekyiidesdymsfinnpsltvtvpiavgesdfenlntedfssesdlees
keklnesssssegstvdigapveeqpvvepeetlepeacftegcvqrfkccqinveegrgkqwwnlrrtcfrivehnwf
etfivfmillssgalafediyidqrktiktmleyadkvftyifilemllkwvaygyqtyftnawcwldflivdvslvsltan
algyselgaikslrtlralrplralsrfegmrvvvnallgaipsimnvllvclifwlifsimgvnlfagkfyhcintttgdrfdi
edvnnhtdclkliernetarwknvkvnfdnvgfgylsllqvatfkgwmdimyaavdsrnvelqpkyeeslymylyf
vifiifgsfftlnlfigviidnfnqqkkkfggqdifmteeqkkyynamkklgskkpqkpiprpgnkfqgmvfdfvtrq
vfdisimiliclnmvtmmvetddqseyvttilsrinlvfivlftgecvlklislrhyyftigwnifdfvvvilsivgmflaeli
ekyfvsptlfrvirlarigrilrlikgakgirtllfalmmslpalfniglllflvmfiyaifgmsnfayvkrevgiddmfnfetf
gnsmiclfqittsagwdgllapilnskppdcdpnkvnpgssvkgdcgnpsvgifffvsyiiisflvvvnmyiavilenfs
vateesaeplseddfemfyevwekfdpdatqfmefeklsqfaaaleppInlpqpnklqliamdlpmvsgdrihcldil

faftkrvlgesgemdalriqmeerfmasnpskvsyqpitttlkrkqeevsaviiqrayrrhllkrtvkqasftynknkikg
ganllikedmiidrinensitektdltmstaacppsydrvtkpivekheqegkdekakgk (SEQ ID NO: 20)

H.s. SCN2A

maqsvlvppgpdsfrfftreslaaieqriaeekakrpkqerkdeddengpkpnsdleagkslpfiygdippemvsvpl
edldpyyinkktfivlnkgkaisrfsatpalyiltpfnpirklaikilvhslfnmlimctiltncvfmtmsnppdwtknve
ytftgiytfeslikilargfcledftflrdpwnwldftvitfayvtefvdlgnvsalrtfrvlralktisvipglktivgaliqsvk
klsdvmiltvfclsvfaliglqlfmgnlrnkclqwppdnssfeinitsffnnsldgngttfnrtvsifnwdeyiedkshfyfl
egqndallcgnssdagqcpegyicvkagrnpnygytsfdtfswaflslfrlmtqdfwenlyqltlraagktymiffvlvi
flgsfylinlilavvamayeeqnqatleeaeqkeaefqqmleqlkkqqeeaqaaaaaasaesrdfsgaggigvfsesss
vasklssksekelknrrkkkkqkeqsgeeekndrvrksesedsirrkgfrfslegsrltyekrfssphqsllsirgslfsprr
nsraslfsfrgrakdigsendfaddehstfedndsrrdslfvphrhgerrhsnvsqasrasrvlpilpmngkmhsavdcn
gvvslvggpstltsagqllpegttteteirkrrsssyhvsmdlledptsrqramsiasiltntmeeleesrqkcppcwykfa
nmcliwdcckpwlkvkhlvnlvvmdpfvdlaiticivlntlfmamehypmteqfssvlsvgnlvftgiftaemflkii
amdpyyyfqegwnifdgfivslslmelglanveglsvlrsfrllrvfklakswptlnmlikiignsvgalgnltlvlaiivfi
favvgmqlfgksykecvckisndcelprwhmhdffhsflivfrvlcgewietmwdcmevagqtmcltvfmmvm
vignlvvlnlflalllssfssdnlaatdddnemnnlqiavgrmqkgidfvkrkirefiqkafvrkqkaldeikpledlnnk
kdscisnhttieigkdlnylkdgngttsgigssvekyvvdesdymsfinnpsltvtvpiavgesdfenlnteefssesdm
eeskeklnatsssegstvdigapaegeqpevepeeslepeacftedcvrkfkccqisieegkgklwwnlrktcykiveh
nwfetfivfmillssgalafediyieqrktiktmleyadkvftyifilemllkwvaygfqvyftnawcwldflivdvslvs
ltanalgyselgaikslrtlralrplralsrfegmrvvvnallgaipsimnvllvclifwlifsimgvnlfagkfyhcinyttg
emfdvsvvnnyseckaliesnqtarwknvkvnfdnvglgylsllqvatfkgwmdimyaavdsrnvelqpkyednl
ymylyfvifiifgsfftlnlfigviidnfnqqkkkfggqdifmteeqkkyynamkklgskkpqkpiprpankfqgmvf
dfvtkqvfdisimiliclnmvtmmvetddqsqemtnilywinlvfivlftgecvlklislryyyftigwnifdfvvvilsi
vgmflaeliekyfvsptlfrvirlarigrilrlikgakgirtllfalmmslpalfniglllflvmfiyaifgmsnfayvkrevgi
ddmfnfetfgnsmiclfqittsagwdgllapilnsgppdcdpdkdhpgssvkgdcgnpsvgifffvsyiiisflvvvn
myiavilenfsvateesaeplseddfemfyevwekfdpdatqfiefaklsdfadaldppllliakpnkvqliamdlpmv
sgdrihcldilfaftkrvlgesgemdalriqmeerfmasnpskvsyepitttlkrkqeevsaiiiqrayrryllkqkvkkvs
siykkdkgkecdgtpikedtlidklnenstpektdmtpsttsppsydsvtkpekekfekdksekedkgkdireskk
(SEQ ID NO: 21)

H.s. SCN3A

maqallvppgpesfrlftreslaaiekraaeekakkpkkeqdnddenkpkpnsdleagknlpfiygdippemvseple
dldpyyinkktfivmnkgkaifrfsatsalyiltplnpvrkiaikilvhslfsmlimctiltncvfmtlsnppdwtknveyt
ftgiytfeslikilargfcledftflrdpwnwldfsvivmayvtefvdlgnvsalrtfrvlralktisvipglktivgaliqsvk
klsdvmiltvfclsvfaliglqlfmgnlrnkclqwppsdsafetnttsyfngtmdsngtfvnvtmstfnwkdyigddsh
fyvldgqkdpllcgngsdagqcpegyicvkagrnpnygytsfdtfswaflslfrlmtqdywenlyqltlraagktymif
fvlviflgsfylvnlilavvamayeeqnqatleeaeqkeaefqqmleqlkkqqeeaqavaaasaasrdfsgigglgelle
ssseasklssksakewrnrrkkrrqrehlegnnkgerdsfpksesedsvkrssflfsmdgnrltsdkkfcsphqsllsirg
slfsprrnsktsifsfrgrakdvgsendfaddehstfedsesrrdslfvphrhgerrnsnvsqasmssrmvpglpangk
mhstvdcngvvslvggpsaltsptgqlppegtttetevrkrrlssyqismemledssgrqravsiasiltntmeeleesrq
kcppcwyrfanvfliwdccdawlkvkhlvnlivmdpfvdlaiticivlntlfmamehypmteqfssvltvgnlvftgi
ftaemvlkiiamdpyyyfqegwnifdgiivslslmelglsnveglsvlrsfrllrvfklakswptlnmlikiignsvgalg
nltlvlaiivfifavvgmqlfgksykecvckinddctlprwhmndffhsflivfrvlcgewietmwdcmevagqtmcl
ivfmlvmvignlvvlnlflalllssfssdnlaatdddnemnnlqiavgrmqkgidyvknkmrecfqkaffrkpkviei
hegnkidscmsnntgieiskelnylrdgngttsgvgtgssvekyvidendymsfinnpsltvtvpiavgesdfenlnte

efsseseleeskeklnatsssegstvdvvlpregeqaetepeedlkpeacftegcikkfpfcqvsteegkgkiwwnlrkt
cysivehnwfetfivfmillssgalafediyieqrktiktmleyadkvftyifilemllkwvaygfqtyftnawcwldfli
vdvslvslvanalgyselgaikslrtlralrplralsrfegmrvvvnalvgaipsimnvllvclifwlifsimgvnlfagkfy
hcvnmttgnmfdisdvnnlsdcqalgkqarwknvkvnfdnvgagylallqvatfkgwmdimyaavdsrdvklqp
vyeenlymylyfvifiifgsfftlnlfigviidnfnqqkkkfggqdifmteeqkkyynamkklgskkpqkpiprpankf
qgmvfdfvtrqvfdisimiliclnmvtmmvetddqgkymtlvlsrinlvfivlftgefvlklvslrhyyftigwnifdfv
vvilsivgmflaemiekyfvsptlfrvirlarigrilrlikgakgirtllfalmmslpalfniglllflvmfiyaifgmsnfayv
kkeagiddmfnfetfgnsmiclfqittsagwdgllapilnsappdcdpdtihpgssvkgdcgnpsvgifffvsyiiisflv
vvnmyiavilenfsvateesaeplseddfemfyevwekfdpdatqfiefsklsdfaaaldppllliakpnkvqliamdlp
mvsgdrihcldilfaftkrvlgesgemdalriqmedrfmasnpskvsyepitttlkrkqeevsaaiiqrnfrcyllkqrlk
nissnynkeaikgridlpikqdmiidklngnstpektdgsssttsppsydsvtkpdkekfekdkpekeskgkevrenq
k (SEQ ID NO: 22)

H.s. SCN4A

marpslctlvplgpeclrpftreslaaieqraveeearlqrnkqmeieeperkprsdleagknlpmiygdpppevigipl
edldpyysnkktfivlnkgkaifrfsatpalyllspfsvvrrgaikvlihalfsmfimitiltncvfmtmsdpppwsknve
ytftgiytfeslikilargfcvddftflrdpwnwldfsvimmayltefvdlgnisalrtfrvlralktitvipglktivgaliqsv
kklsdvmiltvfclsvfalvglqlfmgnlrqkcvrwpppfndtnttwysndtwygndtwygnemwygndswyan
dtwnshaswatndtfdwdayisdegnfyflegsndallcgnssdaghcpegyeciktgrnpnygytsydtfswaflal
frlmtqdywenlfqltlraagktymiffvviiflgsfylinlilavvamayaeqneatlaedkekeeefqqmlekfkkhq
eelekakaaqaleggeadgdpahgkdcngsldtsqgekgaprqsssgdsgisdameeleeahqkcppwwykcah
kvliwnccapwlkfkniihlivmdpfvdlgiticivlntlfmamehypmtehfdnvltvgnlvftgiftaemvlkliam
dpyeyfqqgwnifdsiivtlslvelglanvqglsvlrsfrllrvfklakswptlnmlikiignsvgalgnltlvlaiivfifav
vgmqlfgksykecvckialdcnlprwhmhdffhsflivfrilcgewietmwdcmevagqamcltvflmvmvignl
vvlnlflalllssfsadslaasdedgemnnlqiaigriklgigfakafllgllhgkilspkdimlslgeadgageageageta
pedekkeppeedlkkdnhilnhmgladgppssleldhlnfinnpyltiqvpiaseesdlempteeetdtfsepedskkp
pqplydgnssvcstadykppeedpeeqaeenpegeqpeecfteacvqrwpclyvdisqgrgkkwwtlrracfkive
hnwfetfivfmillssgalafediyieqrrvirtileyadkvftyifimemllkwvaygfkvyftnawcwldflivdvsiis
lvanwlgyselgpikslrtlralrplralsrfegmrvvvnallgaipsimnvllvclifwlifsimgvnlfagkfyycinttts
erfdisevnnkseceslmhtgqvrwlnvkvnydnvglgylsllqvatfkgwmdimyaavdsrekeeqpqyevnly
mylyfvifiifgsfftlnlfigviidnfnqqkkklggkdifmteeqkkyynamkklgskkpqkpiprpqnkiqgmvy
dlvtkqafditimiliclnmvtmmvetdnqsqlkvdilyninmifiiiftgecvlkmlalrqyyftvgwnifdfvvvilsi
vglalsdliqkyfvsptlfrvirlarigrvlrlirgakgirtllfalmmslpalfniglllflvmfiysifgmsnfayvkkesgid
dmfnfetfgnsiiclfeittsagwdgllnpilnsgppdcdpnlenpgtsvkgdcgnpsigicffcsyiiisflivvnmyiai
ilenfnvateesseplgeddfemfyetwekfdpdatqfiaysrlsdfvdtlqeplriakpnkiklitldlpmvpgdkihcl
dilfaltkevlgdsgemdalkqtmeekfmaanpskvsyepitttlkrkheevcaikiqrayrrhllqrsmkqasymyrh
shdgsgddapekegllantmskmyghengnssspspeekgeagdagptmglmpispsdtawppapppgqtvrp
gvkeslv (SEQ ID NO: 23)

H.s. SCN5A

manfllprgtssfrrftreslaaiekrmaekqargsttlqesreglpeeeaprpqldlqaskklpdlygnppqeligepledl
dpfystqktfivlnkgktifrfsatnalyvlspfhpirraavkilvhslfnmlimctiltncvfmaqhdpppwtkyveytft
aiytfeslvkilargfclhaftflrdpwnwldfsviimayttefvdlgnvsalrtfrvlralktisvisglktivgaliqsvkkla
dvmvltvfclsvfaliglqlfmgnlrhkcvrnftalngtngsveadglvwesldlylsdpenyllkngtsdvllcgnssda
gtcpegyrclkagenpdhgytsfdsfawaflalfrlmtqdcwerlyqqtlrsagkiymiffmlviflgsfylvnlilavva
mayeeqnqatiaeteekekrfqeamemlkkehealtirgvdtvsrsslemsplapvnsherrskrrkrmssgteecge

drlpksdsedgpramnhlsltrglsrtsmkprssrgsiftfrrrdlgseadfaddenstageseshhtsllvpwplrrtsaqg
qpspgtsapghalhgkknstvdcngvvsllgagdpeatspgshllrpvmlehppdtttpseepggpqmltsqapcvd
gfeepgarqralsavsvltsaleeleesrhkcppcwnrlaqryliweccplwmsikqgvklvvmdpftdltitmcivlnt
lfmalehynmtsefeemlqvgnlvftgiftaemtfkiialdpyyyfqqgwnifdsiivilslmelglsrmsnlsvlrsfrll
rvfklakswptlntlikiignsvgalgnltlvlaiivfifavvgmqlfgknyselrdsdsgllprwhmmdffhafliifrilc
gewietmwdcmevsgqslcllvfllvmvignlvvlnlflalllssfsadnltapdedremnnlqlalariqrglrfvkrtt
wdfccgllrqrpqkpaalaaqgqlpsciatpyspppppetekvpptrketrfeegeqpgqgtpgdpepvcvpiavaesd
tddqeedeenslgteeesskqqesqpvsggpeappdsrtwsqvsatasseaeasasqadwrqqwkaepqapgcget
pedscsegstadmtntaelleqipdlgqdvkdpedcftegcvrrcpccavdttqapgkvwwrlrktcyhivehswfet
fiifmillssgalafediyleerktikvlleyadkmftyvfvlemllkwvaygfkkyftnawcwldflivdvslvslvantl
gfaemgpikslrtlralrplralsrfegmrvvvnalvgaipsimnvllvclifwlifsimgvnlfagkfgrcinqtegdlpl
nytivnnksqceslnltgelywtkvkvnfdnvgagylallqvatfkgwmdimyaavdsrgyeeqpqweynlymyi
yfvifiifgsfftlnlfigviidnfnqqkkklggqdifmteeqkkyynamkklgskkpqkpiprplnkyqgfifdivtkq
afdvtimflicnlmvtmmvetddqspekinilakinllfvaiftgecivklaalrhyyftnswnifdfvvvilsivgtvlsd
iiqkyffsptlfrvirlarigrilrlirgakgirtllfalmmslpalfnigllflvmfiysifgmanfayvkweagiddmfnfq
tfansmlclfqittsagwdgllspilntgppycdptlpnsngsrgdcgspavgilffttyiiisflivvnmyiaiilenfsvat
eesteplseddfdmfyeiwekfdpeatqfieysvlsdfadalseplriakpnqislinmdlpmvsgdrihcmdilfaftk
rvlgesgemdalkiqmeekfmaanpskisyepitttlrrkheevsamviqrafrrhllqrslkhasflfrqqagsglseed
aperegliayvmsenfsrplgppssssisstsfppsydsvtratsdnlqvrgsdyshsedladfppspdrdresiv
(SEQ ID NO: 24)

H.s. SCN7A (occasionally referred to as SCN6A)

mlaspepkglvpftkesfelikqhiakthnedheeedlkptpdlevgkklpfiygnlsqgmvsepledvdpyyykkk
ntfivlnknrtifrfnaasilctlspfncirrttikvlvhpffqlfilisvlidcvfmsltnlpkwrpvlentllgiytfeilvklfar
gvwagsfsflgdpwnwldfsvtvfeviirysplldfiptlqtartlrilkiiplnqglkslvgvlihclkqligviiltlfflsifsli
gmglfmgnlkhkcfrwpqenenetlhnrtgnpyyiretenfyylegeryallcgnrtdagqcpegyvcvkaginpdq
gftnfdsfgwalfalfrlmaqdypevlyhqilyasgkvymiffvvvsflfsfymaslflgilamayeeekqrvgeiskki
epkfqqtgkelqegnetdeaktiqiemkkrspistdtsldvledatlrhkeelekskkicplywykfaktfliwncspcw
lklkefvhriimapftdlfliiciilnvcfltlehypmskqtntllnignlvfigiftaemifkiiamhpygyfqvgwnifds
mivfhglielclanvagmallrlfrmlrifklgkywptfqilmwslsnswvalkdlvlllftfiffsaafgmklfgknyeef
vchidkdcqlprwhmhdffhsflnvfrilcgewvetlwdcmevagqswcipfylmvilignllvlylflalvssfssck
dvtaeenneaknlqlavarikkginyvllkilcktqnvpkdtmdhvnevyvkedisdhtlselsntqdflkdkekssgt
eknatenesqslipspsvsetvpiasgesdienldnkeiqsksgdggskekikqssssecstvdiaiseeeemfyggers
khlkngcrrgsslgqisgaskkgkiwqnirktcckivennwfkcfiglvtllstgtlafediymdqrktikilleyadmift
yifilemllkwmaygfkayfsngwyrldfvvvivfclsligktreelkplismkflrplrvlsqfermkvvvralikttlpt
lnvflvclmiwlifsimgvdlfagrfyecidptsgerfpssevmnksrcesllfnesmlwenakmnfdnvgngflsllq
vatfngwitimnsaidsvavniqphfevniymycyfinfiifgvflplsmlitviidnfnkhkiklggsnifitvkqrkqy
rrlkklmyedsqrpvprplnklqgfifdvvtsqafnvivmvlicfqaiammidtdvqslqmsialywinsifvmlytm
ecilkliafrcfyftiawnifdfmvvifsitglclpmtvgsylvppslvqlillsriihmlrlgkgpkvfhnlmlplmlslpal
lniilliflvmfiyavfgmynfayvkkeagindvsnfetfgnsmlclfqvaifagwdgmldaifnskwsdcdpdkinp
gtqvrgdcgnpsvgifyfvsyiliswliivnmyivvvmeflniaskkknktlseddfrkffqvwkrfdpdrtqyidsskl
sdfaaaldpplfmakpnkgqlialdlpmavgdrihcldillaftkrvmgqdvrmekvvseiesgfllanpfkitcepittt
lkrkqeavsatiiqrayknyrlrrndkntsdihmidgdrdvhatkegayfdkakekspiqsqi (SEQ ID NO: 25)

H.s. SCN8A

maarllappgpdsfkpftpeslanierriaesklkkppkadgshreddedskpkpnsdleagkslpfiygdipqglvav
pledfdpyyltqktfvvlnrgktlfrfsatpalyilspfnlirriaikilihsvfsmiimctiltncvfmtfsnppdwsknvey
tftgiytfeslvkiiargfcidgftflrdpwnwldfsvimmayitefvnlgnvsalrtfrvlralktisvipglktivgaliqsv
kklsdvmiltvfclsvfaliglqlfmgnlrnkcvvwpinfnesylengtkgfdweeyinnktnfytvpgmlepllcgns
sdagqcpegyqcmkagrnpnygytsfdtfswaflalfrlmtqdywenlyqltlraagktymiffvlvifvgsfylvnlil
avvamayeeqnqatleeaeqkeaefkamleqlkkqqeeaqaaamatsagtvsedaieeegeegggsprssseiskls
sksakerrnrrkkrkqkelsegeekgdpekvfksesedgmrrkafrlpdnrigrkfsimnqsllsipgspflsrhnskssi
fsfrgpgrfrdpgsenefaddehstveesegrrdslfipirarerrssysgysgysqgsrssrifpslrrsvkrnstvdcngv
vsliggpgshiggrllpeatteveikkkgpgsllvsmdqlasygrkdrinsimsvvtntlveleesqrkcppcwykfan
tfliwechpywiklkeivnlivmdpfvdlaiticivlntlfmamehhpmtpqfehvlavgnlvftgiftaemflkliam
dpyyyfqegwnifdgfivslslmelsladveglsvlrsfrllrvfklakswptlnmlikiignsvgalgnltlvlaiivfifav
vgmqlfgksykecvckinqdcelprwhmhdffhsflivfrvlcgewietmwdcmevagqamclivfmmvmvi
gnlvvlnlflalllssfsadnlaatdddgemnnlqisvirikkgvawtklkvhafmqahfkqreadevkpldelyekka
ncianhtgadihrngdfqkngngttsgigssvekyiidedhmsfinnpnltvrvpiavgesdfenlntedvssesdpeg
skdklddtsssegstidikpeveevpveqpeeyldpdacftegcvqrfkccqvnieeglgkswwilrktcflivehnwf
etfiifmillssgalafediyieqrktirtileyadkvftyifilemllkwtaygfvkfftnawcwldflivavslvslianalg
yselgaikslrtlralrplralsrfegmrvvvnalvgaipsimnvllvclifwlifsimgvnlfagkyhycfnetseirfeied
vnnkteceklmegnnteirwknvkinfdnvgagylallqvatfkgwmdimyaavdsrkpdeqpkyedniymyiy
fvifiifgsfftlnlfigviidnfnqqkkkfggqdifmteeqkkyynamkklgskkpqkpiprplnkiqgivfdfvtqqa
fdivimmliclnmvtmmvetdtqskqmenilywinlvfvifftcecvlkmfalrhyyftigwnifdfvvvilsivgmf
ladiiekyfvsptlfrvirlarigrilrlikgakgirtllfalmmslpalfnigllflvmfifsifgmsnfayvkheagiddmf
nfetfgnsmiclfqittsagwdglllpilnrppdcsldkehpgsgfkgdcgnpsvgifffvsyiiisflivvnmyiaiilenf
svateesadplseddfetfyeiwekfdpdatqfieyckladfadalehplrvpkpntieliamdlpmvsgdrihcldilfa
ftkrvlgdsgeldilrqqmeerfvasnpskvsyepitttlrrkqeevsavvlqrayrghlarrgfickkttsnklenggthre
kkestpstaslpsydsvtkpekekqqraeegrrerakrqkevreskc (SEQ ID NO: 26)

H.s. SCN9A

mamlpppgpqsfvhftkqslalieqriaerkskepkeekkdddeeapkpssdleagkqlpfiygdippgmvsepled
ldpyyadkktfivlnkgktifrfnatpalymlspfsplrrisikilvhslfsmlimctiltncifmtmnnppdwtknveytf
tgiytfeslvkilargfcvgeftflrdpwnwldfvvivfayltefvnlgnvsalrtfrvlralktisvipglktivgaliqsvkkl
sdvmiltvfclsvfaliglqlfmgnlkhkcfrnslennetlesimntleseedfrkyfyylegskdallcgfstdsgqcpeg
ytcvkigrnpdygytsfdtfswaflalfrlmtqdywenlyqqtlraagktymiffvvviflgsfylinlilavvamayeeq
nqanieeakqkelefqqmldrlkkeqeeaeaiaaaaaeytsirrsrimglsesssetsklssksakerrnrrkkknqkkls
sgeekgdaeklsksesedsirrksfhlgveghrrahekrlstpnqsplsirgslfsarrssrtslfsfkgrgrdigsetefadde
hsifgdnesrrgslfvphrpqerrssnisqasrsppmlpvngkmhsavdcngvvslvdgrsalmlpngqllpegttnqi
hkkrrcssyllsedmlndpnlrqramsrasiltntveeleesrqkcppwwyrfahkfliwncspywikfkkciyfivm
dpfvdlaiticivlntlfmamehhpmteefknvlaignlvftgifaaemvlkliamdpyeyfqvgwnifdslivtlslve
lfladveglsvlrsfrllrvfklakswptlnmlikiignsvgalgnltlvlaiivfifavvgmqlfgksykecvckinddctlp
rwhmndffhsflivfrvlcgewietmwdcmevagqamclivymmvmvignlvvlnlflalllssfssdnltaieedp
dannlqiavtrikkginyvkqtlrefilkafskkpkisreirqaedlntkkenyisnhtlaemskghnflkekdkisgfgss
vdkhlmedsdgqsfihnpsltvtvpiapgesdlenmnaeelssdsdseyskvrlnrssssecstvdnplpgegeeaeae
pmnsdepeacftdgcvrrfsccqvniesgkgkiwwnirktcykivehswfesfivlmillssgalafediyierkktikii
leyadkiftyifilemllkwiaygyktyftnawcwldflivdvslvtlvantlgysdlgpikslrtlralrplralsrfegmrv
vvnaligaipsimnvllvclifwlifsimgvnlfagkfyecinttdgsrfpasqvpnrsecfalmnvsqnvrwknlkvnf
dnvglgylsllqvatfkgwtiimyaavdsvnvdkqpkyeyslymyiyfvvfiifgsfftlnlfigviidnfnqqkkklgg
qdifmteeqkkyynamkklgskkpqkpiprpgnkiqgcifdlvtnqafdisimvliclnmvtmmvekegqsqhm
tevlywinvvfiilftgecvlklislrhyyftvgwnifdfvvviisivgmfladlietyfvsptlfrvirlarigrilrlvkgakg
irtllfalmmslpalfnigllflvmfiyaifgmsnfayvkkedgindmfnfetfgnsmiclfqittsagwdgllapilnsk

ppdcdpkkvhpgssvegdcgnpsvgifyfvsyiiisflvvvnmyiavilenfsvateesteplseddfemfyevwekf dpdatqfiefsklsdfaaaldpplliakpnkvqliamdlpmvsgdrihcldilfaftkrvlgesgemdslrsqmeerfms anpskvsyepitttlkrkqedvsatviqrayrryrlrqnvknissiyikdgdrdddllnkkdmafdnvnensspektdat ssttsppsydsvtkpdkekyeqdrtekedkgkdskeskk (SEQ ID NO: 27)

H.s. SCN10A

mefpigsletnnfrrftpeslveiekqiaakqgtkkarekhreqkdqeekprpqldlkacnqlpkfygelpaeligeple dldpfysthrtfmvlnkgrtisrfsatralwlfspfnlirrtaikvsvhswfslfitvtilvncvcmtrtdlpekieyvftviytf ealikilargfclneftylrdpwnwldfsvitlayvgtaidlrgisglrtfrvlralktvsvipglkvivgalihsvkkladvtilt ifclsvfalvglqlfkgnlknkcvkndmavnettnysshrkpdiyinkrgtsdpllcgngsdsghcpdgyiclktsdnp dfnytsfdsfawaflslfrlmtqdswerlyqqtlrtsgkiymiffvlviflgsfylvnlilavvtmayeeqnqattdeieake kkfqealemlrkeqevlaalgidttslhshngspltsknaserrhrikprvsegstednksprsdpynqrrmsflglasgk rrashgsvfhfrspgrdislpegvtddgvfpgdheshrgslllgggagqqgplprsplpqpsnpdsrhgedehqpppts elapgavdvsafdagqkktflsaeyldepfraqramsvvsiitsvleeleeseqkcppcltslsqkyliwdccpmwvkl ktilfglvtdpfaeltitlcivvntifmamehhgmsptfeamlqignivftifftaemvfkiiafdpyyyfqkkwnifdcii vtvsllelgvakkgslsvlrsfrllrvfklakswptlntlikiignsvgalgnltiilaiivfvfalvgkqllgenyrnnrknisa phedwprwhmhdffhsflivfrilcgewienmwacmevgqksiclilfltvmvlgnlvvlnlfialllnsfsadnltap eddgevnnlqvalariqvfghrtkqalcsffsrscpfpqpkaepelvvklplssskaenhiaantargssgglqaprgprd ehsdfianptvwvsvpiaegesdlddleddggedaqsfqqevipkgqqeqlqqvercgdhltprspgtgtssedlapsl getwkdesvpqvpaegvddtsssegstvdcldpeeilrkipeladdleepddcftegcirhcpcckldttkspwdvgw qvrktcyrivehswfesfiifmillssgslafedyyldqkptvkalleytdrvftfifvfemllkwvaygfkkyftnawcw ldflivnislisltakileysevapikalrtlralrplralsrfegmrvvvdalvgaipsimnvllvclifwlifsimgvnlfag kfwrcinytdgefslvplsivnnksdckiqnstgsffwvnvkvnfdnvamgylallqvatfkgwmdimyaavdsre vnmqpkwednvymylyfvifiifggfftlnlfvgviidnfnqqkkklggqdifmteeqkkyynamkklgskkpqk piprplnkfqgfvfdivtrqafditimvliclnmitmmvetddqseektkilgkinqffvavftgecvmkmfalrqyyft ngwnvfdfivvvlsiaslifsailkslqsyfsptlfrvirlarigrilrliraakgirtllfalmmslpalfniglllflvmfiysifg mssfphvrweagiddmfnfqtfansmlclfqittsagwdgllspilntgppycdpnlpnsngtrgdcgspavgiifftty iiisflimvnmyiavilenfnvateesteplseddfdmfyetwekfdpeatqfitfsalsdfadtlsgplripkpnrniliq mdlplvpgdkihcldilfaftknvlgesgeldslkanmeekfmatnlskssyepiattlrwkqedisatviqkayrsyvl hrsmalsntpcvpraeeeaaslpdegfvaftanencvlpdksetasatsfppsyesvtrglsdrvnmrtsssiqnedeats meliapgp (SEQ ID NO: 28)

H.s. SCN11A

mddrcypvifpdernfrpftsdslaaiekriaiqkekkkskdqtgevpqprpqldlkasrklpklygdipreligkpledl dpfyrnhktfmvlnrkrtiyrfsakhalfifgpfnsirslairvsvhslfsmfiigtviincvfmatgpaknsnsnntdiaec vftgiyifealikilargfildefsflrdpwnwldsivigiaivsyipgitikllplrtfrvfralkaisvvsrlkvivgallrsvkk lvnviiltffclsifalvgqqlfmgslnlkcisrdcknisnpeaydhcfekkenspefkmcgiwmgnsacsiqyeckht kinpdynytnfdnfgwsflamfrlmtqdsweklyqqtlrttglysvfffivviflgsfylinltlavvtmayeeqnknvaa eieakekmfqeaqqllkeekealvamgidrssltsletsyftpkkrklfgnkkrksfflresgkdqppgsdsdedcqkk pqlleqtkrlsqnlsldhfdehgdplqrqralsavsiltitmkeqeksqepclpcgenlaskylvwnccpqwlcvkkvlr tvmtdpftelaiticiiintvflamehhkmeasfekmlnignlvftsifiaemclkiialdpyhyfrrgwnifdsivallsfa dvmncvlqkrswpflrsfrvlrvfklakswptlntlikiignsvgalgsltvvlvivififsvvgmqlfgrsfnsqkspklc nptgptvsclrhwhmgdfwhsflvvfrilcgewienmwecmqeanassslcvivfilitvigklvvlnlfialllnsfsn eerngnlegearktkvqlaldrfrrafcfvrhtlehfchkwcrkqnlpqqkevaggcaaqskdiiplvmemkrgsetqe elgiltsvpktlgvrhdwtwlaplaeeeddvefsgednaqritqpepeqqayelhqenkkptsqrvqsveidmfsede phltiqdprkksdvtsilsecstidlqdgfgwlpemvpkkqperclpkgfgccfpccsvdkrkppwviwwnlrktcy qivkhswfesfiifvillssgalifedvhlenqpkiqellnctdiifthifilemvlkwvafgfgkyftsawccldfiivivsv ttlinlmelksfrtlralrplralsqfegmkvvvnaligaipailnvllvclifwlvfcilgvyffsgkfgkcingtdsvinytii tnksqcesgnfswinqkvnfdnvgnaylallqvatfkgwmdiiyaavdstekeqqpefesnslgyiyfvvfiifgsfftl nlfigviidnfnqqqkklggqdifmteeqkkyynamkklgskkpqkpiprplnkcqglvfdivtsqifdiiiisliilnm ismmaesynqpkamksildhlnwvfvviftleclikifalrqyyftngwnlfdcvvvllsivstmistlenqehipfpptl frivrlarigrilrlvraargirtllfalmmslpslfnigllflimfiyailgmnwfskvnpesgiddifnfktfassmlclfqi stsagwdsllspmlrskescnsssenchlpgiatsyfvsyiiisflivvnmyiavilenfntateesedplgeddfdifyev wekfdpeatqfikysalsdfadalpeplrvakpnkyqflvmdlpmvsedrlhcmdilfaftarvlggsdgldsmkam meekfmeanplkklyepivtttkrkeeergaaiiqkafrkymmkvtkgdqgdqndlengphsplqtlcngdlssfgv akgkvhcd (SEQ ID NO: 29)

H.s. SCN1B

Mgrllalvvgaalvssacggcvevdseteavygmtfkilcisckrrsetnaetftewtfrqkgteefvkilryenevlqlee derfegrvvwngsrgtkdlqdlsifitnvtynhsgdyechvyrllffenyehntsvvkkihievvdkanrdmasivsei mmyvlivvltiwlvaemiycykkiaaatetaaqenaseylaitseskenctgvqvae (SEQ ID NO: 30)

H.s. SCN2B

Mhrdawlprpafsltglslffslvppgrsmevtvpatlnvlngsdarlpctfnscytvnhkqfslnwtyqecnncseem flqfrmkiinlklerfqdrvefsgnpskydvsvmlrnvqpedegiyncyimnppdrhrghgkihlqvlmeepperds tvavivgasvggflavvilvlmvvkcvrrkkeqklstddlkteeegktdgegnpddgak (SEQ ID NO: 31)

H.s. SCN3B

Mpafnrlfplaslvliywvvsvcfpvcvevpseteavqgnpmklrciscmkreeveattvvewfyrpeggkdfliyey rnghqevespfqgrlqwngskdlqdvsitvlnvtlndsglytcnvsrefefeahrpfvkttrliplrvteeagedftsvvse immyillvfltlwlliemiycyrkvskaeeaaqenasdylaipsenkensavpvee (SEQ ID NO: 32)

H.s. SCN4B

Mpgagdggkaparwlgtgllglfllpvtlslevsvgkatdiyavngteillpctfsscfgfedlhfrwtynssdafkiliegt vkneksdpkvtlkdddritlvgstkekmnnisivlrdlefsdtgkytchvknpkennlqhhatiflqvvdrleevdntvtl iilavvggvigllilillikkliifilkktrekkkeclvsssgndntenglpgskaeekppskv (SEQ ID NO: 33)

Signaling probe 3 - (binds target 3)
**5'- Fam** GCGAGAGCGACAAGCAGACCCTATAGAACCTCGC **BHQ1 quench** - 3' (SEQ ID NO: 34)

**Claims**

1. A cell line engineered to stably express a human NaV 1.7 comprising a NaV alpha 9 subunit, a NaV beta 1 subunit, and a NaV beta 2 subunit, wherein the cell line is produced by a method comprising the steps of:

a) providing a plurality of cells that express mRNA(s) encoding the human NaV 1.7;
b) dispersing the cells individually into individual culture vessels, thereby providing a plurality of separate cell cultures;
c) culturing the cells under a set of desired culture conditions using an automated cell culture method **characterized in that** the conditions are substantially identical for each of the separate cell cultures, during which culturing the number of cells per separate cell culture is normalized, and wherein the separate cultures are passaged on the same schedule;
d) assaying the separate cell cultures to measure expression of the human NaV 1.7 at least twice and to measure a Z' factor in the membrane potential assay; and
e) identifying a separate cell culture that expresses the human NaV 1.7 at a consistent level in both assays and produces a Z' factor of at least 0.6 in the membrane potential assay, thereby obtaining said collection of cells;

wherein the membrane potential assay comprises:

i) plating cells from the cell line at 10,000 - 25,000 cells per well in a 384-well plate in growth media;
ii) maintaining the cells in the 384-well plate in a 37°C cell culture incubator under 5% $CO_2$ for 22-24 hours;
iii) removing the growth media from the 384-well plate;
iv) incubating the cells in the 384-well plate with a blue florescence membrane potential dye diluted in a load buffer comprising 137 mM NaCl, 5 mM KCl,1.25 mM $CaCl_2$, 25 mM HEPES, 10 mM glucose for 1 hour at 37°C;
v) adding to the incubated cells a) 25 μM of veratridine and 5-25 μg/ml of scorpion venom for positive control wells; or b) a control buffer for negative control wells;
vi) loading the 384-well plate onto a high-throughput fluorescent plate reader; and
vii) measuring cell fluorescence signals of the positive control wells and the negative control wells,

wherein the Z' factor is calculated using the measured cell fluorescence signals from step vii) and using the equation:

$$Z' \text{ factor} = 1 - ((3\sigma_{\text{positive control}} + 3\sigma_{\text{negative control}}) / (\mu_{\text{positive control}} - \mu_{\text{negative control}})),$$

wherein $\sigma_{positive}$ control represents a standard deviation from the positive controls of the assay; $\sigma_{negative}$ control represents a standard deviation from the negative controls of the assay; $\mu_{positive}$ control represents the mean value of the positive controls; and $\mu_{negative}$ control represents the mean value of the negative controls.

**2.** The cell line of claim 1, wherein the cell line is capable of producing a Z' factor of at least 0.7 maintainable for multiple passages, or wherein the cell line is grown in the absence of selective pressure.

**3.** The cell line of claim 1 or 2, wherein the NaV does not comprise any polypeptide tag.

**4.** A method for identifying a human NaV 1.7 modulator, comprising
contacting a cell in the cell line of any one of claims 1-3 with a test compound; and
detecting a change in a human NaV 1.7 function in the cell when compared to a cell not contacted with the test compound, wherein a change in said function indicates that the test compound is a human NaV 1.7 modulator.

**5.** The method of claim 4, wherein the test compound is a small molecule, a polypeptide, a peptide, or an antibody or an antigen-binding portion thereof.

**6.** The method of claim 4 or 5, wherein the steps are conducted in a high throughout manner.

**7.** A collection of cells engineered to stably express a human NaV 1.7 comprising a NaV alpha 9 subunit, a NaV beta 1 subunit and a NaV beta 2 subunit at a consistent level over time, wherein the collection of cells is capable of producing a Z' factor of at least 0.6 in a membrane potential assay, wherein the membrane potential assay comprises:

i) plating cells from the cell line at 10,000 - 25,000 cells per well in a 384-well plate in growth media;
ii) maintaining the cells in the 384-well plate in a 37°C cell culture incubator under 5% $CO_2$ for 22-24 hours;
iii) removing the growth media from the 384-well plate;
iv) incubating the cells in the 384-well plate with a blue florescence membrane potential dye diluted in a load buffer comprising 137 mM NaCl, 5 mM KCl,1.25 mM $CaCl_2$, 25 mM HEPES, 10 mM glucose for 1 hour at 37°C;
v) adding to the incubated cells a) 25 μM of veratridine and 5-25 μg/ml of scorpion venom for positive control wells; or b) a control buffer for negative control wells;
vi) loading the 384-well plate onto a high-throughput fluorescent plate reader; and
vii) measuring cell fluorescence signals of the positive control wells and the negative control wells,

wherein the Z' factor is calculated using the measured cell fluorescence signals from step vii) and using the equation:

$$Z' \text{ factor} = 1 - ((3\sigma_{\text{positive control}} + 3\sigma_{\text{negative control}}) / (\mu_{\text{positive control}} - \mu_{\text{negative control}})),$$

wherein $\sigma_{positive}$ control represents a standard deviation from the positive controls of the assay; $\sigma_{negative}$ control represents a standard deviation from the negative controls of the assay; $\mu_{positive}$ control represents the mean value of the positive controls; and $\mu_{negative}$ control represents the mean value of the negative controls,
and wherein the collection of cells is made by a method comprising the steps of

a) providing a plurality of cells that express mRNA(s) encoding the human NaV 1.7;
b) dispersing the cells individually into individual culture vessels, thereby providing a plurality of separate cell cultures;
c) culturing the cells under a set of desired culture conditions using an automated cell culture method **characterized in that** the conditions are substantially identical for each of the separate cell cultures, during which culturing the number of cells per separate cell culture is normalized, and wherein the separate cultures are passaged on the same schedule;
d) assaying the separate cell cultures to measure expression of the human NaV 1.7 at least twice and to measure the Z' factor in the membrane potential assay; and
e) identifying a separate cell culture that expresses the human NaV 1.7 at a consistent level in both assays and produces a Z' factor is at least 0.6 in the membrane potential assay, thereby obtaining said collection of cells.

**8.** A method for producing a collection of cells engineered to stably express a human NaV 1.7 comprising a NaV alpha 9 subunit, a NaV beta 1 subunit and a NaV beta 2 subunit at a consistent level over time, wherein the collection of cells is capable of producing a Z' factor of at least 0.6 in a membrane potential assay, wherein the membrane potential assay comprises:

i) plating cells from the cell line at 10,000 - 25,000 cells per well in a 384-well plate in growth media;
ii) maintaining the cells in the 384-well plate in a 37°C cell culture incubator under 5% $CO_2$ for 22-24 hours;
iii) removing the growth media from the 384-well plate;
iv) incubating the cells in the 384-well plate with a blue florescence membrane potential dye diluted in a load buffer comprising 137 mM NaCl, 5 mM KCl,1.25 mM $CaCl_2$, 25 mM HEPES, 10 mM glucose for 1 hour at 37°C;
v) adding to the incubated cells a) 25 $\mu$M of veratridine and 5-25 $\mu$g/ml of scorpion venom for positive control wells; or b) a control buffer for negative control wells;
vi) loading the 384-well plate onto a high-throughput fluorescent plate reader; and
vii) measuring cell fluorescence signals of the positive control wells and the negative control wells,

wherein the Z' factor is calculated using the measured cell fluorescence signals from step vii) and using the equation:

$$Z' \text{ factor} = 1 - ((3\sigma_{\text{positive control}} + 3\sigma_{\text{negative control}}) / (\mu_{\text{positive control}} - \mu_{\text{negative control}})),$$

wherein $\sigma_{positive}$ control represents a standard deviation from the positive controls of the assay; $\sigma_{negative}$ control represents a standard deviation from the negative controls of the assay; $\mu_{positive}$ control represents the mean value of the positive controls; and $\mu_{negative}$ control represents the mean value of the negative controls,
wherein the method comprises the steps of:

a) providing a plurality of cells that express mRNA(s) encoding the human NaV 1.7;
b) dispersing the cells individually into individual culture vessels, thereby providing a plurality of separate cell cultures;
c) culturing the cells under a set of desired culture conditions using an automated cell culture method **characterized in that** the conditions are substantially identical for each of the separate cell cultures, during which culturing the number of cells per separate cell culture is normalized, and wherein the separate cultures are passaged on the same schedule;
d) assaying the separate cell cultures to measure expression of the human NaV 1.7 at least twice and to measure a Z' factor in the membrane potential assay; and
e) identifying a separate cell culture that expresses the human NaV 1.7 at a consistent level in both assays and produces a Z' factor of at least 0.6 in the membrane potential assay, thereby obtaining said collection of cells.

## Patentansprüche

**1.** Zelllinie, die zur stabilen Expression eines menschlichen NaV 1.7 konstruiert ist, der eine NaV-alpha-9-Untereinheit, eine NaV-beta-1-Untereinheit und eine NaV-beta-2-Untereinheit umfasst, wobei die Zelllinie durch ein Verfahren hergestellt wird, das die folgenden Schritte umfasst:

a) Bereitstellen einer Vielzahl von Zellen, die mRNA(s) exprimieren, die den menschlichen NaV 1.7 kodiert/kodieren;
b) einzelnes Dispergieren der Zellen in einzelnen Kulturgefäßen, wodurch eine Vielzahl von getrennten Zellkulturen bereitgestellt wird;
c) Kultivieren der Zellen unter einer Reihe von gewünschten Kulturbedingungen unter Verwendung eines automatisierten Zellkulturverfahrens, **dadurch gekennzeichnet, dass** die Bedingungen im Wesentlichen für jede der getrennten Zellkulturen identisch sind, während welchem Kultivieren die Anzahl der Zellen pro getrennter Zellkultur normalisiert wird, und wobei die getrennten Kulturen nach dem gleichen Zeitplan passagiert werden;
d) Untersuchen der getrennten Zellkulturen, um die Expression des menschlichen NaV 1.7 mindestens zweimal zu messen und um einen Z'-Faktor in dem Membranpotential-Assay zu messen; und
e) Identifizieren einer getrennten Zellkultur, die den menschlichen NaV 1.7 bei konstantem Niveau in beiden Assays exprimiert und einen Z'-Faktor von mindestens 0,6 in dem Membranpotential-Assay erzeugt, wodurch die Sammlung von Zellen erhalten wird;

wobei der Membranpotential-Assay Folgendes umfasst:

i) Ausplattieren von Zellen aus der Zelllinie bei 10 000-25 000 Zellen pro Well in einer 384-Well-Platte in Nährmedium;
ii) Erhalten der Zellen in der 384-Well-Platte in einem 37 °C-Zellkulturinkubator unter 5 % $CO_2$ für 22-24 Stunden;
iii) Entfernen des Nährmediums aus der 384-Well-Platte;
iv) Inkubieren der Zellen in der 384-Well-Platte mit einem blauen Fluoreszenz-Membranpotential-Farbstoff, der in einem Ladungspuffer verdünnt ist, der 137 mM NaCl, 5 mM KCl, 1,25 mM $CaCl_2$, 25 mM HEPES, 10 mM Glucose umfasst, für 1 Stunde bei 37 °C;
v) Zugeben zu den inkubierten Zellen von a) 25 μM Veratridin und 5-25 μg/ml Skorpiongift für positive Kontroll-Wells; oder b) einem Kontrollpuffer für negative Kontroll-Wells;
vi) Laden der 384-Well-Platte in einen Hochdurchsatz-Fluoreszenzplattenleser; und
vii) Messen von Zellfluoreszenzsignalen der positiven Kontroll-Wells und der negativen Kontroll-Wells,

wobei der Z'-Faktor unter Verwendung der gemessenen Zellfluoreszenzsignale von Schritt vii) und unter Verwendung der folgenden Gleichung berechnet wird:

$$Z'\text{-Faktor} = 1 - ((3\sigma_{\text{positive Kontrolle}} + 3\sigma_{\text{negative Kontrolle}})/(\mu_{\text{positive Kontrolle}} - \mu_{\text{negative Kontrolle}})),$$

wobei $\sigma_{\text{positive Kontrolle}}$ eine Standardabweichung von den positiven Kontrollen des Assays darstellt; $\sigma_{\text{negative Kontrolle}}$ eine Standardabweichung von den negativen Kontrollen des Assays darstellt; $\mu_{\text{positive Kontrolle}}$ den Mittelwert der positiven Kontrollen darstellt; und $\mu_{\text{negative Kontrolle}}$ den Mittelwert der negativen Kontrollen darstellt.

**2.** Zelllinie nach Anspruch 1, wobei die Zelllinie einen Z'-Faktor von mindestens 0,7 erzeugen kann, der für mehrere Passagen erhaltbar ist, oder wobei die Zelllinie in Abwesenheit von selektivem Druck kultiviert wird.

**3.** Zelllinie nach Anspruch 1 oder 2, wobei der NaV kein Polypeptid-Tag umfasst.

**4.** Verfahren zur Identifizierung eines Modulators von menschlichem NaV 1.7, das Folgendes umfasst:

Inkontaktbringen einer Zelle in der Zelllinie nach einem der Ansprüche 1-3 mit einer Testverbindung; und
Nachweisen einer Veränderung der Funktion von menschlichem NaV 1.7 in der Zelle im Vergleich zu einer Zelle, die nicht mit der Testverbindung in Kontakt gebracht wurde, wobei eine Veränderung der Funktion anzeigt, dass die Testverbindung ein Modulator von menschlichem NaV 1.7 ist.

**5.** Verfahren nach Anspruch 4, wobei die Testverbindung ein kleines Molekül, ein Polypeptid, ein Peptid oder ein Antikörper oder ein antigenbindender Teil davon ist.

**6.** Verfahren nach Anspruch 4 oder 5, wobei die Schritte im Hochdurchsatz durchgeführt werden.

**7.** Sammlung von Zellen, die zur stabilen Expression eines menschlichen NaV 1.7, der eine NaV-alpha-9-Untereinheit, eine NaV-beta-1-Untereinheit und eine NaV-beta-2-Untereinheit umfasst, bei konstantem Niveau im Zeitverlauf konstruiert sind, wobei die Sammlung von Zellen einen Z'-Faktor von mindestens 0,6 in einem Membranpotential-Assay erzeugen kann, wobei der Membranpotential-Assay Folgendes umfasst:

i) Ausplattieren von Zellen aus der Zelllinie bei 10 000-25 000 Zellen pro Well in einer 384-Well-Platte in Nährmedium;
ii) Erhalten der Zellen in der 384-Well-Platte in einem 37 °C-Zellkulturinkubator unter 5 % $CO_2$ für 22-24 Stunden;
iii) Entfernen des Nährmediums aus der 384-Well-Platte;
iv) Inkubieren der Zellen in der 384-Well-Platte mit einem blauen Fluoreszenz-Membranpotential-Farbstoff, der in einem Ladungspuffer verdünnt ist, der 137 mM NaCl, 5 mM KCl, 1,25 mM $CaCl_2$, 25 mM HEPES, 10 mM Glucose umfasst, für 1 Stunde bei 37 °C;
v) Zugeben zu den inkubierten Zellen von a) 25 μM Veratridin und 5-25 μg/ml Skorpiongift für positive Kontroll-Wells; oder b) einem Kontrollpuffer für negative Kontroll-Wells;
vi) Laden der 384-Well-Platte in einen Hochdurchsatz-Fluoreszenzplattenleser; und

vii) Messen von Zellfluoreszenzsignalen der positiven Kontroll-Wells und der negativen Kontroll-Wells,

wobei der Z'-Faktor unter Verwendung der gemessenen Zellfluoreszenzsignale von Schritt vii) und unter Verwendung der folgenden Gleichung berechnet wird:

$$Z\text{'-Faktor} = 1 - ((3\sigma_{\text{positive Kontrolle}} + 3\sigma_{\text{negative Kontrolle}})/(\mu_{\text{positive Kontrolle}} - \mu_{\text{negative Kontrolle}})),$$

wobei $\sigma_{\text{positive Kontrolle}}$ eine Standardabweichung von den positiven Kontrollen des Assays darstellt; $\sigma_{\text{negat}^{\text{i}}\text{ve Kontrolle}}$ eine Standardabweichung von den negativen Kontrollen des Assays darstellt; $\mu_{\text{positive Kontrolle}}$ den Mittelwert der positiven Kontrollen darstellt; und $\sigma_{\text{negative Kontrolle}}$ den Mittelwert der negativen Kontrollen darstellt,
und wobei die Sammlung von Zellen durch ein Verfahren hergestellt wird, das die folgenden Schritte umfasst:

a) Bereitstellen einer Vielzahl von Zellen, die mRNA(s) exprimieren, die den menschlichen NaV 1.7 kodiert/kodieren;
b) einzelnes Dispergieren der Zellen in einzelnen Kulturgefäßen, wodurch eine Vielzahl von getrennten Zellkulturen bereitgestellt wird;
c) Kultivieren der Zellen unter einer Reihe von gewünschten Kulturbedingungen unter Verwendung eines automatisierten Zellkulturverfahrens, **dadurch gekennzeichnet, dass** die Bedingungen im Wesentlichen für jede der getrennten Zellkulturen identisch sind, während welchem Kultivieren die Anzahl der Zellen pro getrennter Zellkultur normalisiert wird, und wobei die getrennten Kulturen nach dem gleichen Zeitplan passagiert werden;
d) Untersuchen der getrennten Zellkulturen, um die Expression des menschlichen NaV 1.7 mindestens zweimal zu messen und um den Z'-Faktor in dem Membranpotential-Assay zu messen; und
e) Identifizieren einer getrennten Zellkultur, die den menschlichen NaV 1.7 bei konstantem Niveau in beiden Assays exprimiert und einen Z'-Faktor von mindestens 0,6 in dem Membranpotential-Assay erzeugt, wodurch die Sammlung von Zellen erhalten wird.

**8.** Verfahren zur Herstellung einer Sammlung von Zellen, die zur stabilen Expression eines menschlichen NaV 1.7, der eine NaV-alpha-9-Untereinheit, eine NaV-beta-1-Untereinheit und eine NaV-beta-2-Untereinheit umfasst, bei konstantem Niveau im Zeitverlauf konstruiert sind, wobei die Sammlung von Zellen einen Z'-Faktor von mindestens 0,6 in einem Membranpotential-Assay erzeugen kann, wobei der Membranpotential-Assay Folgendes umfasst:

i) Ausplattieren von Zellen aus der Zelllinie bei 10 000-25 000 Zellen pro Well in einer 384-Well-Platte in Nährmedium;
ii) Erhalten der Zellen in der 384-Well-Platte in einem 37 °C-Zellkulturinkubator unter 5 % $CO_2$ für 22-24 Stunden;
iii) Entfernen des Nährmediums aus der 384-Well-Platte;
iv) Inkubieren der Zellen in der 384-Well-Platte mit einem blauen Fluoreszenz-Membranpotential-Farbstoff, der in einem Ladungspuffer verdünnt ist, der 137 mM NaCl, 5 mM KCl, 1,25 mM $CaCl_2$, 25 mM HEPES, 10 mM Glucose umfasst, für 1 Stunde bei 37 °C;
v) Zugeben zu den inkubierten Zellen von a) 25 μM Veratridin und 5-25 μg/ml Skorpiongift für positive Kontroll-Wells; oder b) einem Kontrollpuffer für negative Kontroll-Wells;
vi) Laden der 384-Well-Platte in einen Hochdurchsatz-Fluoreszenzplattenleser; und
vii) Messen von Zellfluoreszenzsignalen der positiven Kontroll-Wells und der negativen Kontroll-Wells,

wobei der Z'-Faktor unter Verwendung der gemessenen Zellfluoreszenzsignale von Schritt vii) und unter Verwendung der folgenden Gleichung berechnet wird:

$$Z\text{'-Faktor} = 1 - ((3\sigma_{\text{positive Kontrolle}} + 3\sigma_{\text{negative Kontrolle}})/(\mu_{\text{positive Kontrolle}} - \mu_{\text{negative Kontrolle}})),$$

wobei $\sigma_{\text{positive Kontrolle}}$ eine Standardabweichung von den positiven Kontrollen des Assays darstellt; $\sigma_{\text{negative Kontrolle}}$ eine Standardabweichung von den negativen Kontrollen des Assays darstellt; $\mu_{\text{positive Kontrolle}}$ den Mittelwert der positiven Kontrollen darstellt; und $\mu_{\text{negative Kontrolle}}$ den Mittelwert der negativen Kontrollen darstellt,
wobei das Verfahren die folgenden Schritte umfasst:

a) Bereitstellen einer Vielzahl von Zellen, die mRNA(s) exprimieren, die den menschlichen NaV 1.7 kodiert/kodieren;

b) einzelnes Dispergieren der Zellen in einzelnen Kulturgefäßen, wodurch eine Vielzahl von getrennten Zellkulturen bereitgestellt wird;
c) Kultivieren der Zellen unter einer Reihe von gewünschten Kulturbedingungen unter Verwendung eines automatisierten Zellkulturverfahrens, **dadurch gekennzeichnet, dass** die Bedingungen im Wesentlichen für jede der getrennten Zellkulturen identisch sind, während welchem Kultivieren die Anzahl der Zellen pro getrennter Zellkultur normalisiert wird, und wobei die getrennten Kulturen nach dem gleichen Zeitplan passagiert werden;
d) Untersuchen der getrennten Zellkulturen, um die Expression des menschlichen NaV 1.7 mindestens zweimal zu messen und um einen Z'-Faktor in dem Membranpotential-Assay zu messen; und
e) Identifizieren einer getrennten Zellkultur, die den menschlichen NaV 1.7 bei konstantem Niveau in beiden Assays exprimiert und einen Z'-Faktor von mindestens 0,6 in dem Membranpotential-Assay erzeugt, wodurch die Sammlung von Zellen erhalten wird.

## Revendications

**1.** Lignée cellulaire modifiée afin d'exprimer de manière stable un NaV 1.7 humain comprenant une sous-unité NaV alpha 9, une sous-unité NaV bêta 1 et une sous-unité NaV bêta 2, où la lignée cellulaire est produite par une méthode comprenant les étapes consistant à :

a) fournir une pluralité de cellules exprimant un ou plusieurs ARNm codant pour le NaV 1.7 humain ;
b) disperser les cellules individuellement dans des récipients de culture individuels, fournissant ainsi une pluralité de cultures cellulaires séparées ;
c) cultiver les cellules dans un set de conditions de culture désirées à l'aide d'une méthode de culture cellulaire automatisée, **caractérisé en ce que** les conditions sont sensiblement identiques pour chacune des cultures cellulaires séparées, culture pendant laquelle le nombre de cellules par culture cellulaire séparée est normalisé, et où les cultures séparées sont réensemencées suivant le même programme ;
d) doser les cultures cellulaires séparées afin de mesurer l'expression du NaV 1.7 humain au moins deux fois et mesurer un facteur Z' dans le dosage de potentiel membranaire ; et
e) identifier une culture cellulaire séparée qui exprime le NaV 1.7 humain à un niveau constant dans les deux dosages et produit un facteur Z' d'au moins 0,6 dans le dosage de potentiel membranaire, conduisant ainsi à ladite collection de cellules ;

où le dosage de potentiel membranaire comprend :

i) l'ensemencement de cellules à partir de la lignée cellulaire à 10 000-25 000 cellules par puits dans une plaque de 384 puits dans du milieu de culture ;
ii) le maintien des cellules dans la plaque de 384 puits dans une étuve de culture cellulaire à 37°C avec 5% de $CO_2$ pendant 22-24 heures ;
iii) l'élimination du milieu de culture à partir de la plaque de 384 puits ;
iv) l'incubation des cellules dans la plaque de 384 puits avec un colorant de potentiel membranaire à fluorescence bleue dilué dans un tampon de chargement comprenant 137 mM de NaCl, 5 mM de KCl, 1,25 mM de $CaCl_2$, 25 mM de HEPES, 10 mM de glucose pendant 1 heure à 37°C ;
v) l'addition aux cellules incubées a) de 25 μM de vératridine et 5-25 μg/ml de venin de scorpion pour les puits témoins positifs ; ou b) d'un tampon témoin pour les puits témoins négatifs ;
vi) le chargement de la plaque de 384 puits dans un lecteur de plaques à fluorescence à débit élevé ; et
vii) la mesure des signaux de fluorescence cellulaire des puits témoins positifs et des puits témoins négatifs,

où le facteur Z' est calculé en utilisant les signaux de fluorescence cellulaire mesurés à partir de l'étape vii) et en utilisant l'équation :

$$\text{facteur } Z' = 1 - ((3\sigma_{\text{témoin positif}} + 3\sigma_{\text{témoin négatif}})/(\mu_{\text{témoin positif}} - \mu_{\text{témoin négatif}})),$$

où $\sigma_{\text{témoin}}$ positif représente un écart-type à partir des témoins positifs du dosage ; $\sigma_{\text{témoin négatif}}$ représente un écart-type à partir des témoins négatifs du dosage ; $\mu_{\text{témoin positif}}$ représente la valeur moyenne des témoins positifs ; et $\mu_{\text{témoin négatif}}$ représente la valeur moyenne des témoins négatifs.

**2.** Lignée cellulaire selon la revendication 1, où la lignée cellulaire est capable de produire un facteur Z' d'au moins 0,7 pouvant être maintenu pour des réensemencements multiples, ou où la lignée cellulaire est cultivée en l'absence de pression sélective.

**3.** Lignée cellulaire selon la revendication 1 ou 2, où le NaV ne comprend pas d'étiquette polypeptidique.

**4.** Méthode d'identification d'un modulateur du NaV 1.7 humain, comprenant
la mise en contact d'une cellule dans la lignée cellulaire selon l'une quelconque des revendications 1-3 avec un composé à tester ; et
la détection d'un changement dans une fonction du NaV 1.7 humain dans la cellule par rapport à une cellule n'ayant pas été mise en contact avec le composé à tester, où un changement de ladite fonction indique que le composé à tester est un modulateur du NaV 1.7 humain.

**5.** Méthode selon la revendication 4, où le composé à tester est une petite molécule, un polypeptide, un peptide, ou un anticorps ou une fraction de celui-ci de liaison à l'antigène.

**6.** Méthode selon la revendication 4 ou 5, où les étapes sont réalisées d'une manière ayant un débit élevé.

**7.** Collection de cellules modifiées afin d'exprimer de manière stable un NaV 1.7 humain comprenant une sous-unité NaV alpha 9, une sous-unité NaV bêta 1 et une sous-unité NaV bêta 2, à un niveau constant au cours du temps, où la collection de cellules est capable de produire un facteur Z' d'au moins 0,6 dans un dosage de potentiel membranaire, où le dosage de potentiel membranaire comprend :

i) l'ensemencement de cellules à partir de la lignée cellulaire à 10 000-25 000 cellules par puits dans une plaque de 384 puits dans du milieu de culture ;
ii) le maintien des cellules dans la plaque de 384 puits dans une étuve de culture cellulaire à 37°C avec 5% de $CO_2$ pendant 22-24 heures ;
iii) l'élimination du milieu de culture à partir de la plaque de 384 puits ;
iv) l'incubation des cellules dans la plaque de 384 puits avec un colorant de potentiel membranaire à fluorescence bleue dilué dans un tampon de chargement comprenant 137 mM de NaCl, 5 mM de KCl, 1,25 mM de $CaCl_2$, 25 mM de HEPES, 10 mM de glucose pendant 1 heure à 37°C ;
v) l'addition aux cellules incubées a) de 25 μM de vératridine et 5-25 μg/ml de venin de scorpion pour les puits témoins positifs ; ou b) d'un tampon témoin pour les puits témoins négatifs ;
vi) le chargement de la plaque de 384 puits dans un lecteur de plaques à fluorescence à débit élevé ; et
vii) la mesure des signaux de fluorescence cellulaire des puits témoins positifs et des puits témoins négatifs,

où le facteur Z' est calculé en utilisant les signaux de fluorescence cellulaire mesurés à partir de l'étape vii) et en utilisant l'équation :

$$\text{facteur Z'} = 1 - ((3\sigma_{\text{témoin positif}} + 3\sigma_{\text{témoin négatif}})/(\mu_{\text{témoin positif}} - \mu_{\text{témoin négatif}})),$$

où $\sigma_{\text{témoin}}$ positif représente un écart-type à partir des témoins positifs du dosage ; $\sigma_{\text{témoin négatif}}$ représente un écart-type à partir des témoins négatifs du dosage ; $\mu_{\text{témoin positif}}$ représente la valeur moyenne des témoins positifs ; et $\mu_{\text{témoin négatif}}$ représente la valeur moyenne des témoins négatifs,
et où la collection de cellules est préparée par une méthode comprenant les étapes consistant à :

a) fournir une pluralité de cellules exprimant un ou plusieurs ARNm codant pour le NaV 1.7 humain ;
b) disperser les cellules individuellement dans des récipients de culture individuels, fournissant ainsi une pluralité de cultures cellulaires séparées ;
c) cultiver les cellules dans un set de conditions de culture désirées à l'aide d'une méthode de culture cellulaire automatisée, **caractérisé en ce que** les conditions sont sensiblement identiques pour chacune des cultures cellulaires séparées, culture pendant laquelle le nombre de cellules par culture cellulaire séparée est normalisé, et où les cultures séparées sont réensemencées suivant le même programme ;
d) doser les cultures cellulaires séparées afin de mesurer l'expression du NaV 1.7 humain au moins deux fois et mesurer le facteur Z' dans le dosage de potentiel membranaire ; et
e) identifier une culture cellulaire séparée qui exprime le NaV 1.7 humain à un niveau constant dans les deux dosages et produit un facteur Z' qui est d'au moins 0,6 dans le dosage de potentiel membranaire, conduisant ainsi à ladite collection de cellules.

**8.** Méthode de production d'une collection de cellules modifiées afin d'exprimer de manière stable un NaV 1.7 humain comprenant une sous-unité NaV alpha 9, une sous-unité NaV bêta 1 et une sous-unité NaV bêta 2, à un niveau constant au cours du temps, où la collection de cellules est capable de produire un facteur Z' d'au moins 0,6 dans un dosage de potentiel membranaire, où le dosage de potentiel membranaire comprend :

i) l'ensemencement de cellules à partir de la lignée cellulaire à 10 000-25 000 cellules par puits dans une plaque de 384 puits dans du milieu de culture ;
ii) le maintien des cellules dans la plaque de 384 puits dans une étuve de culture cellulaire à 37°C avec 5% de $CO_2$ pendant 22-24 heures ;
iii) l'élimination du milieu de culture à partir de la plaque de 384 puits ;
iv) l'incubation des cellules dans la plaque de 384 puits avec un colorant de potentiel membranaire à fluorescence bleue dilué dans un tampon de chargement comprenant 137 mM de NaCl, 5 mM de KCl, 1,25 mM de $CaCl_2$, 25 mM de HEPES, 10 mM de glucose pendant 1 heure à 37°C ;
v) l'addition aux cellules incubées a) de 25 μM de vératridine et 5-25 μg/ml de venin de scorpion pour les puits témoins positifs ; ou b) d'un tampon témoin pour les puits témoins négatifs ;
vi) le chargement de la plaque de 384 puits dans un lecteur de plaques à fluorescence à débit élevé ; et
vii) la mesure des signaux de fluorescence cellulaire des puits témoins positifs et des puits témoins négatifs,

où le facteur Z' est calculé en utilisant les signaux de fluorescence cellulaire mesurés à partir de l'étape vii) et en utilisant l'équation :

$$\text{facteur Z'} = 1 - ((3\sigma_{\text{témoin positif}} + 3\sigma_{\text{témoin négatif}})/(\mu_{\text{témoin positif}} - \mu_{\text{témoin négatif}})),$$

où $\sigma_{\text{témoin}}$ positif représente un écart-type à partir des témoins positifs du dosage ; $\sigma_{\text{témoin négatif}}$ représente un écart-type à partir des témoins négatifs du dosage ; $\mu_{\text{témoin positif}}$ représente la valeur moyenne des témoins positifs ; et $\mu_{\text{témoin négatif}}$ représente la valeur moyenne des témoins négatifs,
et où la méthode comprend les étapes consistant à :

a) fournir une pluralité de cellules exprimant un ou plusieurs ARNm codant pour le NaV 1.7 humain ;
b) disperser les cellules individuellement dans des récipients de culture individuels, fournissant ainsi une pluralité de cultures cellulaires séparées ;
c) cultiver les cellules dans un set de conditions de culture désirées à l'aide d'une méthode de culture cellulaire automatisée, **caractérisé en ce que** les conditions sont sensiblement identiques pour chacune des cultures cellulaires séparées, culture pendant laquelle le nombre de cellules par culture cellulaire séparée est normalisé, et où les cultures séparées sont réensemencées suivant le même programme ;
d) doser les cultures cellulaires séparées afin de mesurer l'expression du NaV 1.7 humain au moins deux fois et mesurer un facteur Z' dans le dosage de potentiel membranaire ; et
e) identifier une culture cellulaire séparée qui exprime le NaV 1.7 humain à un niveau constant dans les deux dosages et produit un facteur Z' d'au moins 0,6 dans le dosage de potentiel membranaire, conduisant ainsi à ladite collection de cellules.

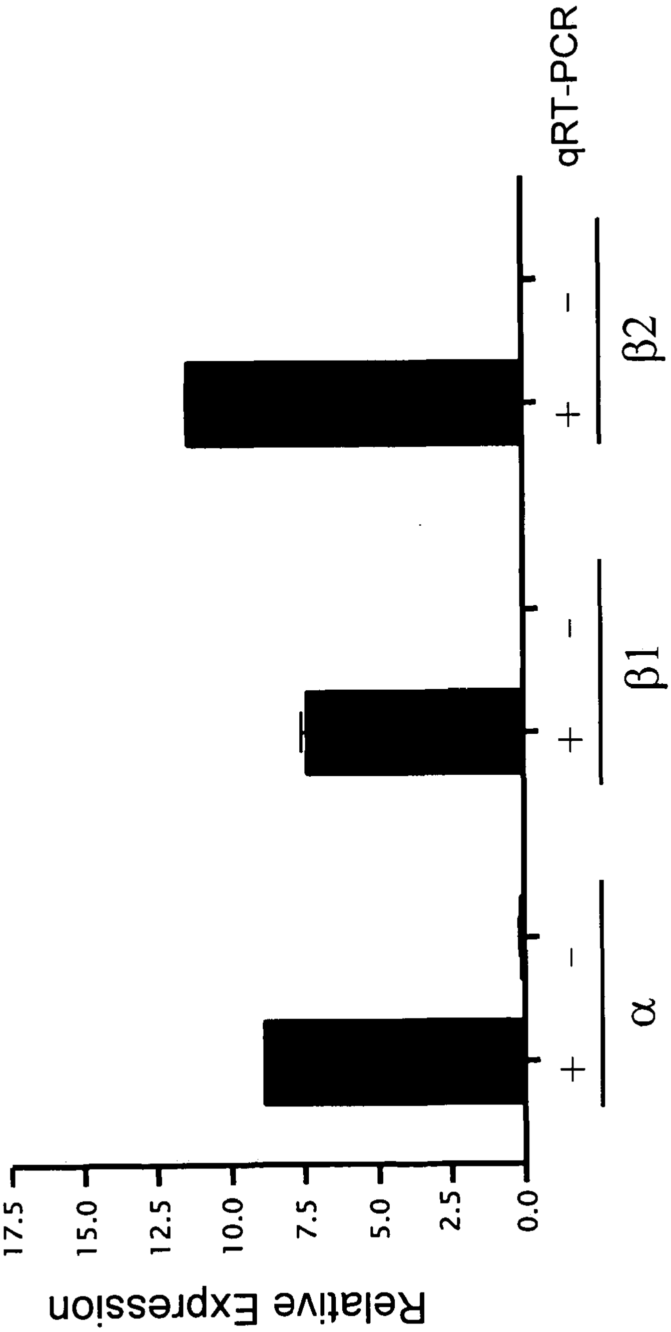


FIG. 1

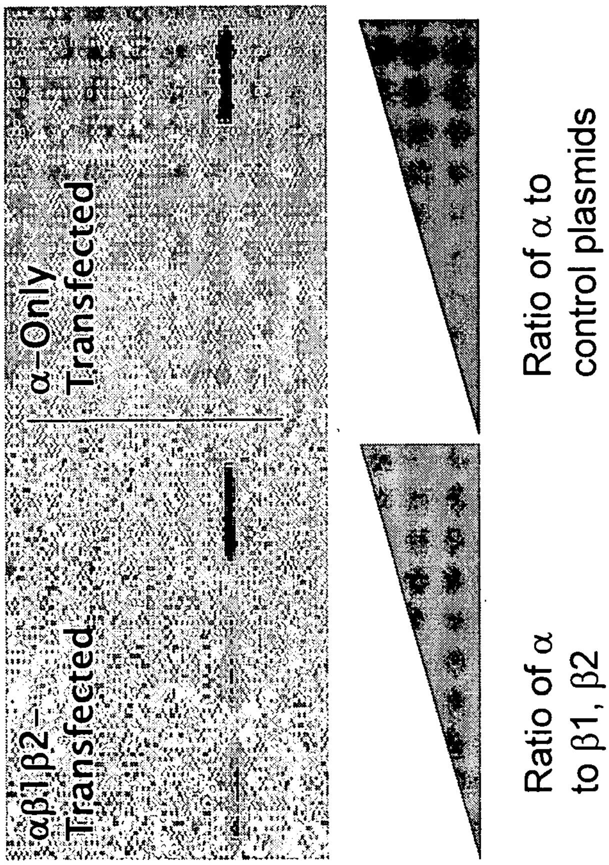
αβ1β2–Transfected
α–Only Transfected
Ratio of α to β1, β2
Ratio of α to control plasmids

FIG. 2

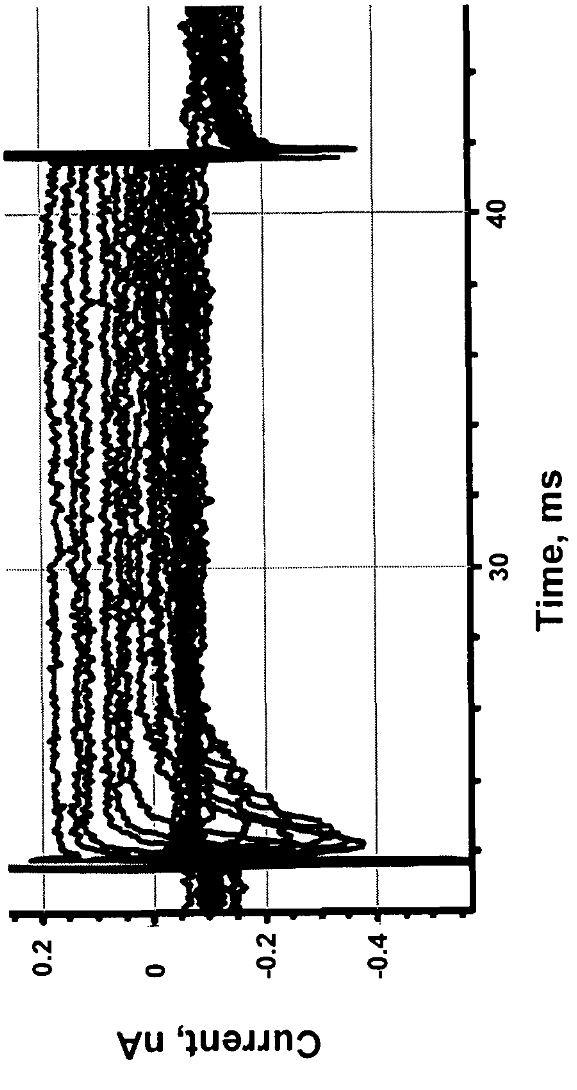


FIG. 3A

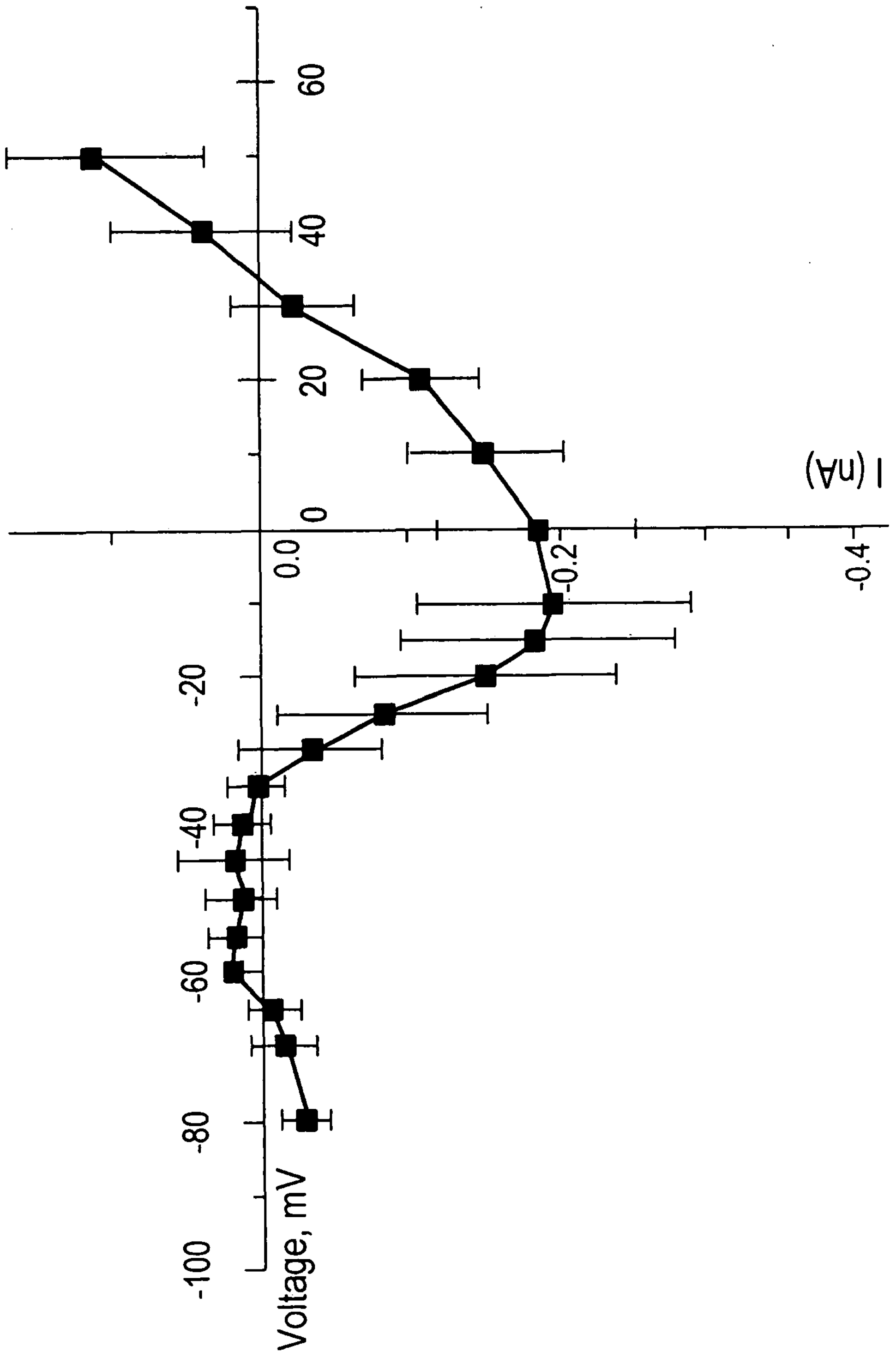


FIG. 3B

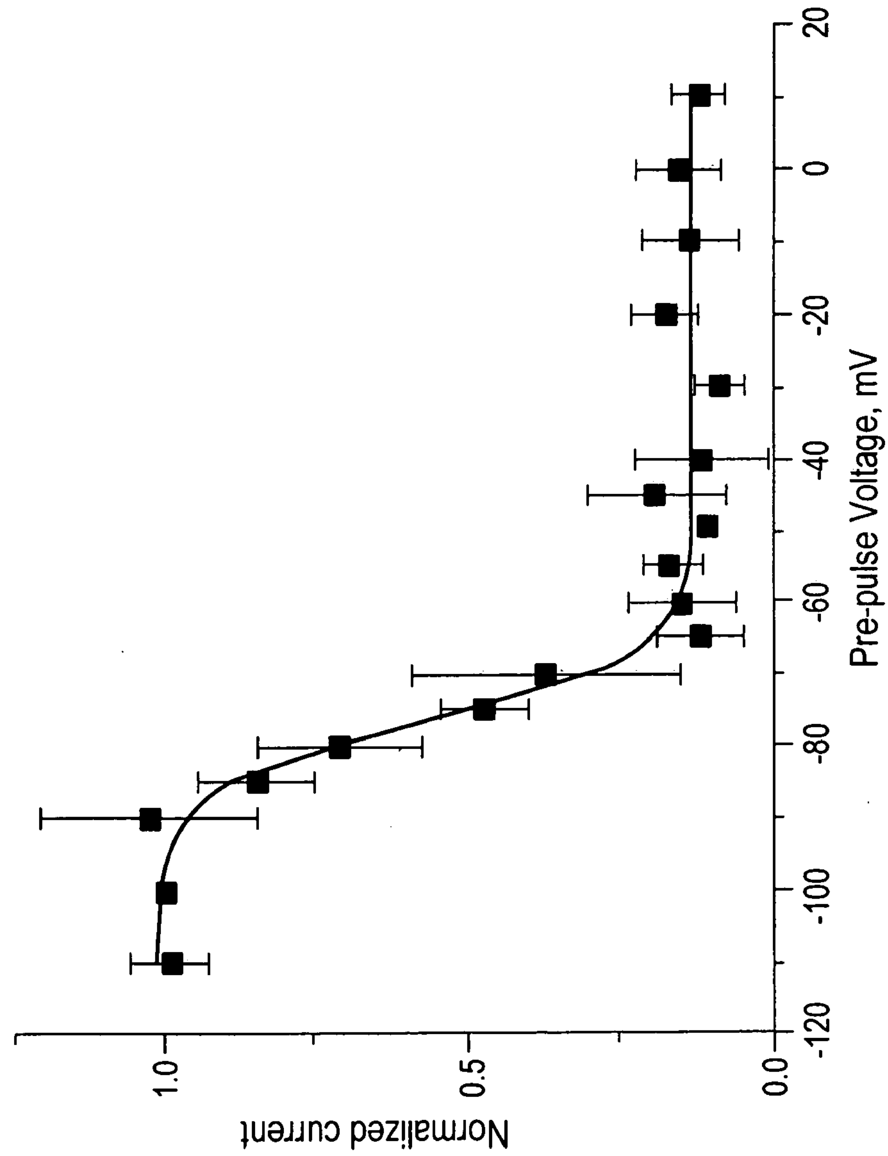
Normalized current
1.0
0.5
0.0
-120
-100
-80
-60
-40
-20
0
20
Pre-pulse Voltage, mV

FIG. 3C

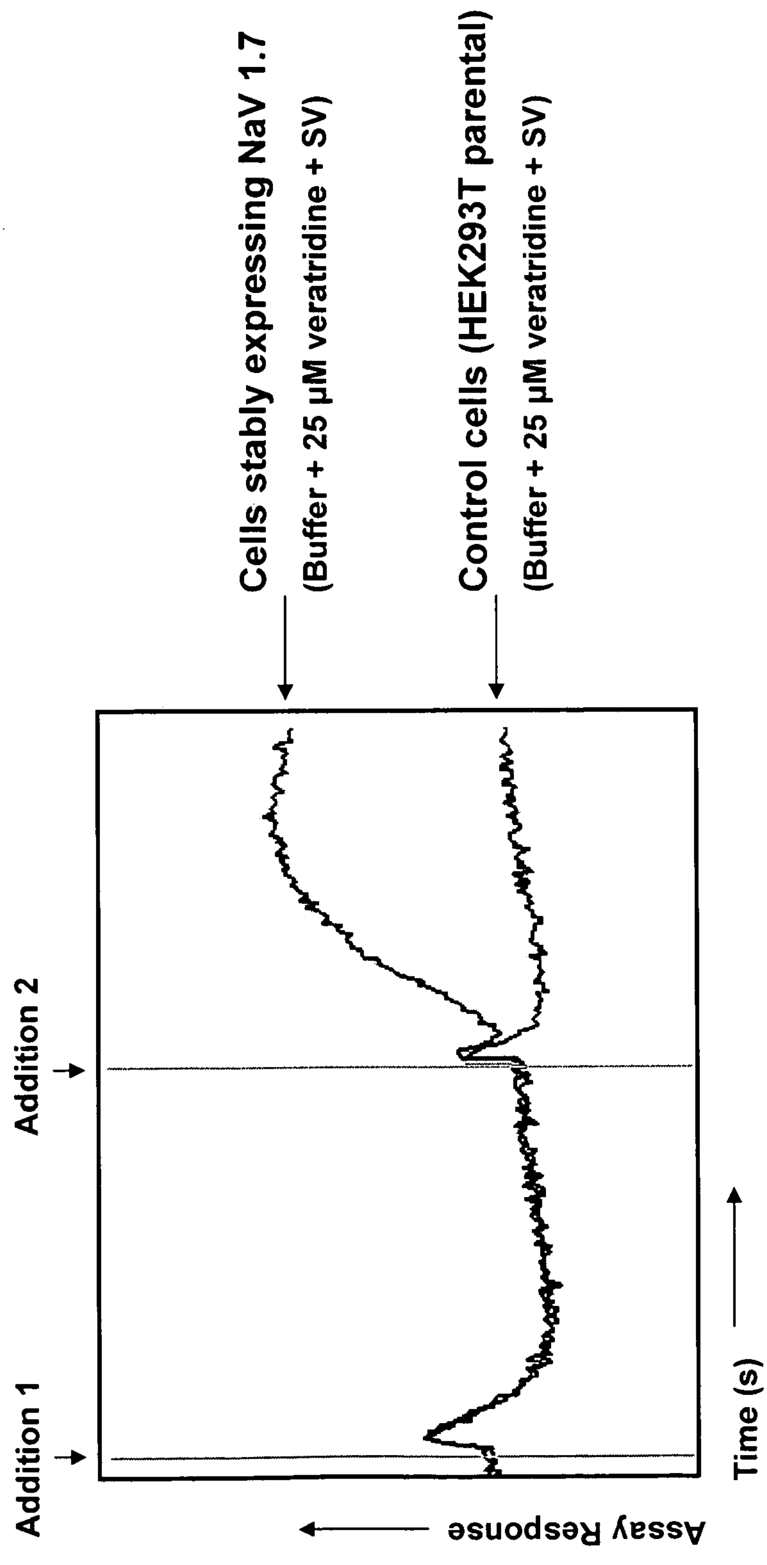

Cells stably expressing NaV 1.7
(Buffer + 25 µM veratridine + SV)
Control cells (HEK293T parental)
(Buffer + 25 µM veratridine + SV)
Addition 1
Addition 2
Assay Response
Time (s)


FIG. 4

FIG. 5A

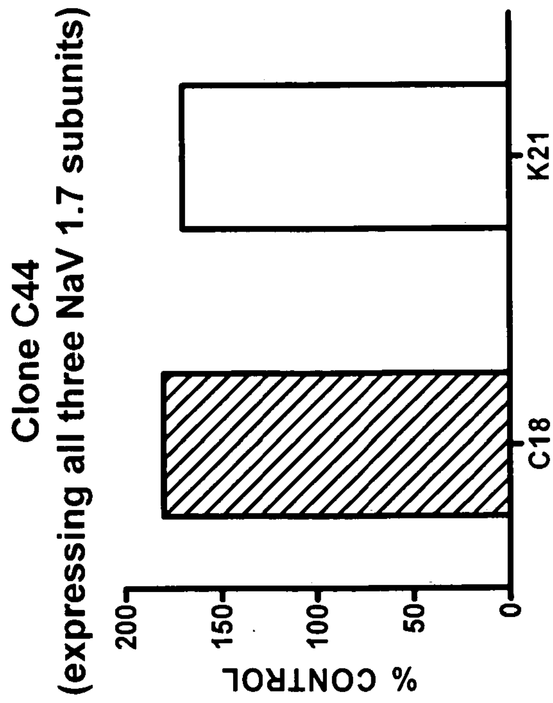
Clone C44
(expressing all three NaV 1.7 subunits)
% CONTROL
200
150
100
50
0
C18
K21

FIG. 5B

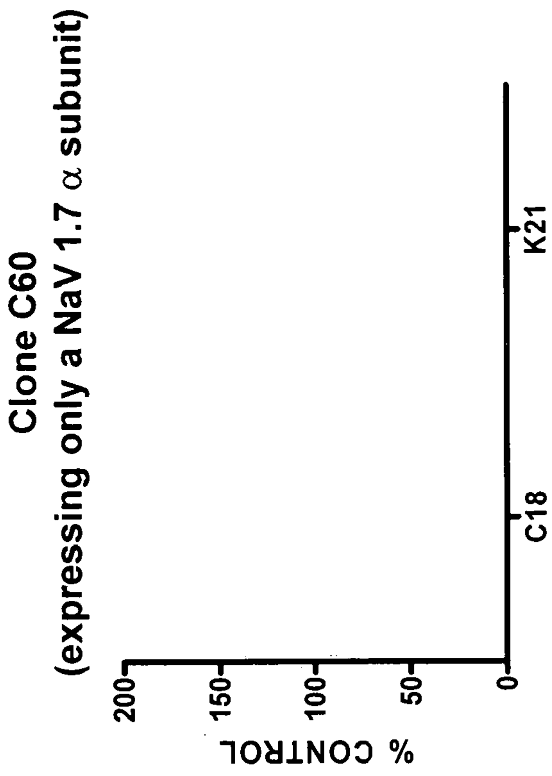
Clone C60
(expressing only a NaV 1.7 α subunit)
% CONTROL
200
150
100
50
0
C18
K21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007109324 A
- US 6692965 B
- WO 2005079462 A
- US 2009032902 W


### Non-patent literature cited in the description


- **CATTERALL et al.** *Pharmacol Rev.,* 2003, vol. 55, 575-578
- **ISOM.** *Neuroscientist,* 2001, vol. 7, 42-54
- **LIU et al.** *Assay Drug Dev Tech,* 2006, vol. 4 (1), 37-48
- Current Protocols in Molecular Biology. John Wiley & Sons, 1989, 6.3.1-6.3.6
- **ALTSHUL et al.** *J. Mol. Biol.,* 1990, vol. 215, 403-410
- **NEEDLEMAN et al.** *J. Mol. Biol.,* 1970, vol. 48, 444-453
- **MEYERS et al.** *Comput. Appl. Biosci.,* 1988, vol. 4, 11-17
- **E. MEYERS ; W. MILLER.** *CABIOS,* 1989, vol. 4, 11-17
- **PEARSON.** *Methods Enzymol.,* 1990, vol. 183, 63-98
- **PEARSON.** *Methods Mol. Biol.,* 2000, vol. 132, 185-219
- **PEARSON.** *Methods Mol. Biol.,* 1994, vol. 243, 307-31
- **GONNET et al.** *Science,* 1992, vol. 256, 1443-45
- **ZHANG JH ; CHUNG TD ; OLDENBURG KR.** A Simple Statistical Parameter for Use in Evaluation and Validation of High Throughput Screening Assays. *J. Biomol. Screen.,* 1999, vol. 4 (2), 67-73
- **ROBINSON et al.** *Biochemistry,* 1997, vol. 36 (37), 1169-11178
- **DEFFIE et al.** *Cancer Res.,* 1988, vol. 48 (13), 3595-3602
- **THIEBAUT et al.** *J Histochem Cytochem.,* 1990, vol. 38 (5), 685-690
- **ROEPE et al.** *Biochemistry,* 1993, vol. 32 (41), 1042-11056
- **SIMON et al.** *Proc Natl Acad Sci USA.,* 1994, vol. 91 (3), 1128-1132
- **SCHINDLER et al.** *Biochemistry,* 1996, vol. 35 (9), 2811-2817
- **ALTAN et al.** *J Exp Med.,* 1998, vol. 187 (10), 1583-1598
- **ROEPE et al.** *Biochemistry,* 1993, vol. 32 (41), 11042-11056
- **GILL et al.** *Cell,* 1992, vol. 71 (1), 23-32
- **ABRAHAM et al.** *Proc Natl Acad Sci USA.,* 1993, vol. 90 (1), 312-316
- **ALTAN et al.** *Proc Natl Acad Sci USA.,* 1999, vol. 96 (8), 4432-4437
- **HANAZONO et al.** *Hum Gene Ther.,* 1997, vol. 8 (11), 1313-1319
- **SHEETS et al.** *J Physiol.,* 2007, vol. 581, 1019-1031